# EUROPEAN PATENT SPECIFICATION

(11) **EP 1 517 921 B1**
(45) Date of publication and mention of the grant of the patent: **07.06.2006**
(21) Application number: 03738294.2
(22) Date of filing: 30.06.2003
(51) Int. Cl.: C07K 16/24

(54) **DUAL SPECIFIC LIGANDS WITH INCREASED SERUM HALF-LIFE**
DUAL-SPECIFISCHE LIGANDEN MIT ERHÖHTER HALBWERTSZEIT
LIGANDS A SPECIFICITE DOUBLE ET AVEC UNE DEMI-VIE AUGMENTEE

(30) Priority: 28.06.2002 WO PCT/GB02/03014; 27.12.2002 GB 0230202
(43) Date of publication of application: 30.03.2005
(60) Divisional application: 05076402.6
(73) Proprietor: Domantis Limited, Cambridge Cambridgeshire, CB4 OWG (GB)
(72) Inventor: WINTER, Greg, Cambridge CB2 2QH (GB); TOMLINSON, Ian, Cambridge CB4 0WG (GB); IGNATOVICH, Olga, Cambridge CB4 0WG (GB); HOLT, Lucy, Cambridge CB4 0WG (GB); DE ANGELIS, Elena, Cambridge CB4 0WG (GB); JONES, Phillip, Cambridge CB4 0WG (GB)
(74) Representative: Soames, Candida Jane
(86) International application number: PCT/GB2003/002804
(87) International publication number: WO 2004/003019

(56) References cited:
- EP-A1- 0 368 684
- WO-A1-00/29004
- WO-A1-91/02078
- WO-A1-97/30084
- WO-A1-98/40469
- WO-A2-02/072141
- WO-A2-03/002609
- WO-A2-03/035694
- US-A- 5 644 034
- ELS CONRATH K ET AL: "Camel single-domain antibodies as modular building units in bispecific and bivalent antibody constructs" JOURNAL OF BIOLOGICAL CHEMISTRY, AMERICAN SOCIETY OF BIOLOGICAL CHEMISTS, BALTIMORE, MD, US, vol. 276, no. 10, 9 March 2001 (2001-03-09), pages 7346-7350, XP002248402 ISSN: 0021-9258 cited in the application
- REITER Y ET AL: "An antibody single-domain phage display library of a native heavy chain variable region: isolation of functional single-domain VH molecules with a unique interface" JOURNAL OF MOLECULAR BIOLOGY, LONDON, GB, vol. 290, no. 3, 1 January 1999 (1999-01-01), pages 685-698, XP004461990 ISSN: 0022-2836
- SMITH BRYAN J ET AL: "Prolonged in vivo residence times of antibody fragments associated with albumin" BIOCONJUGATE CHEMISTRY, vol. 12, no. 5, September 2001 (2001-09), pages 750-756, XP002270731 ISSN: 1043-1802
- VAN DEN BEUCKEN T ET AL: "Building novel binding ligands to B7.1 and B7.2 based on human antibody single variable light chain domains" JOURNAL OF MOLECULAR BIOLOGY, LONDON, GB, vol. 310, no. 3, 13 July 2001 (2001-07-13), pages 591-601, XP004464206 ISSN: 0022-2836
- MUYLDERMANS S ET AL: "UNIQUE SINGLE-DOMAIN ANTIGEN BINDING FRAGMENTS DERIVES FROM NATURALLY OCCURRING CAMEL HEAVY-CHAIN ANTIBODIES" JOURNAL OF MOLECULAR RECOGNITION, HEYDEN & SON LTD., LONDON, GB, vol. 12, no. 2, March 1999 (1999-03), pages 131-140, XP009012180 ISSN: 0952-3499
- RIECHMANN L ET AL: "Single domain antibodies: Comparison of camel VH and camelised human VH domains" BIOSIS, XP004187632
- HOLLIGER P ET AL: "RETARGETING SERUM IMMUNOGLOBULIN WITH BISPECIFIC DIABODIES" NATURE BIOTECHNOLOGY, NATURE PUBLISHING, US, vol. 15, July 1997 (1997-07), pages 632-636, XP002921893 ISSN: 1087-0156
- TANHA J ET AL: "Optimal design features of camelized human single-domain antibody libraries." THE JOURNAL OF BIOLOGICAL CHEMISTRY. UNITED STATES 6 JUL 2001, vol. 276, no. 27, 6 July 2001 (2001-07-06), pages 24774-24780, XP002283749 ISSN: 0021-9258

## Description

The present invention relates to dual specific ligands comprising a first immunoglobulin single variable domain binding to a first antigen or epitope, and a second immunoglobulin single variable domain binding to a second antigen or epitope wherein binding to at least one of the first and second antigens or epitopes acts to increase the half-life of the ligand *in vivo.* Open and closed conformation ligands comprising more than one binding specificity are described.

### Introduction

The antigen binding domain of an antibody comprises two separate regions: a heavy chain variable domain (V_{H}) and a light chain variable domain (V_{L}: which can be either V_{κ} or V_{λ}). The antigen binding site itself is formed by six polypeptide loops: three from V_{H} domain (H1, H2 and H3) and three from V_{L} domain (L1, L2 and L3). A diverse primary repertoire of V genes that encode the V_{H} and V_{L} domains is produced by the combinatorial rearrangement of gene segments. The V_{H} gene is produced by the recombination of three gene segments, V_{H}, D and J_{H}. In humans, there are approximately 51 functional V_{H} segments (Cook and Tomlinson (1995) *Immunol Today,* **16**: 237), 25 functional D segments (Corbett *et al*. (1997) *J. Mol. Biol*., **268**: 69) and 6 functional J_{H} segments (Ravetch *et al*. (1981) *Cell,* **27**: 583), depending on the haplotype. The V_{H} segment encodes the region of the polypeptide chain which forms the first and second antigen binding loops of the V_{H} domain (H1 and H2), whilst the V_{H}, D and J_{H} segments combine to form the third antigen binding loop of the V_{H} domain (H3). The V_{L} gene is produced by the recombination of only two gene segments, V_{L} and J_{L}. In humans, there are approximately 40 functional V_{κ} segments (Schäble and Zachau (1993) *Biol. Chem. Hoppe-Seyler,* **374**: 1001), 31 functional V_{λ} segments (Williams *et al*. (1996) *J. Mol. Biol*., **264**: 220; Kawasaki *et al*. (1997) *Genome Res.,* **7**: 250), 5 functional J_{κ} segments (Hieter *et al*. (1982) *J. Biol. Chem*., **257**: 1516) and 4 functional J_{λ} segments (Vasicek and Leder (1990) *J. Exp. Med*., **172**: 609), depending on the haplotype. The V_{L} segment encodes the region of the polypeptide chain which forms the first and second antigen binding loops of the V_{L} domain (L1 and L2), whilst the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) *J. Mol. Biol.,* **196:** 901; Chothia *et al*. (1989) *Nature,* **342:** 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences (Chothia *et al.* (1992) *J. Mol. Biol*., **227:** 799; Tomlinson *et al*. (1995) *EMBO J*., **14**: 4628; Williams *et al.* (1996) *J. Mol. Biol*., **264:** 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin *et al*. (1996) *J. Mol. Biol*., **263**: 800; Shirai *et al*. (1996) *FEBS Letters*, **399**: 1.

Bispecific antibodies comprising complementary pairs of V_{H} and V_{L} regions are known in the art. These bispecific antibodies must comprise two pairs of V_{H} and V_{L}s, each V_{H/}V_{L} pair binding to a single antigen or epitope. Methods described involve hybrid hybridomas (Milstein & Cuello AC, Nature 305:537-40), minibodies (Hu *et al*., (1996) Cancer Res 56:3055-3061;), diabodies (Holliger *et al.,* (1993) Proc. Natl. Acad. Sci. USA 90, 6444-6448; WO 94/13804), chelating recombinant antibodies (CRAbs; (Neri *et al.,* (1995) J. Mol. Biol. 246, 367-373), biscFv (e.g. Atwell *et al*., (1996) Mol. Immunol. 33, 1301-1312), "knobs in holes" stabilised antibodies (Carter *et al.,* (1997) Protein Sci. 6, 781-788). In each case each antibody species comprises two antigen-binding sites, each fashioned by a complementary pair of V_{H} and V_{L} domains. Each antibody is thereby able to bind to two different antigens or epitopes at the same time, with the binding to EACH antigen or epitope mediated by a V_{H} and its complementary V_{L} domain. Each of these techniques presents its particular disadvantages; for instance in the case of hybrid hybridomas, inactive V_{H}/V_{L} pairs can greatly reduce the fraction of bispecific IgG. Furthermore, most bispecific approaches rely on the association of the different V_{H}/V_{L} pairs or the association of V_{H} and V_{L} chains to recreate the two different V_{H}/V_{L} binding sites. It is therefore impossible to control the ratio of binding sites to each antigen or epitope in the assembled molecule and thus many of the assembled molecules will bind to one antigen or epitope but not the other. In some cases it has been possible to engineer the heavy or light chains at the sub-unit interfaces (Carter *et al*., 1997) in order to improve the number of molecules which have binding sites to both antigens or epitopes but this never results in all molecules having binding to both antigens or epitopes.

There is some evidence that two different antibody binding specificities might be incorporated into the same binding site, but these generally represent two or more specificities that correspond to structurally related antigens or epitopes or to antibodies that are broadly cross-reactive.. For example, cross-reactive antibodies have been described, usually where the two antigens are related in sequence and structure, such as hen egg white lysozyme and turkey lysozyme (McCafferty et al., WO 92/01047) or to free hapten and to hapten conjugated to carrier (Griffiths AD et al. *EMBO J* 1994 13:14 3245-60). In a further example, WO 02/02773 (Abbott Laboratories) describes antibody molecules with "dual specificity". The antibody molecules referred to are antibodies raised or selected against multiple antigens, such that their specificity spans more than a single antigen. Each complementary V_{H}/V_{L} pair in the antibodies of WO 02/02773 specifies a single binding specificity for two or more structurally related antigens; the V_{H} and V_{L} domains in such complementary pairs do not each possess a separate specificity. The antibodies thus have a broad single specificity which encompasses two antigens, which are structurally related. Furthermore natural autoantibodies have been described that are polyreactive (Casali & Notkins, Ann. Rev. Immunol. 7, 515-531), reacting with at least two (usually more) different antigens or epitopes that are not structurally related. It has also been shown that selections of random peptide repertoires using phage display technology on a monoclonal antibody will identify a range of peptide sequences that fit the antigen binding site. Some of the sequences are highly related, fitting a consensus sequence, whereas others are very different and have been termed mimotopes (Lane & Stephen, Current Opinion in Immunology, 1993, 5, 268-271). It is therefore clear that a natural four-chain antibody, comprising associated and complementary V_{H} and V_{L} domains, has the potential to bind to many different antigens from a large universe of known antigens. It is less clear how to create a binding site to two given antigens in the same antibody, particularly those which are not necessarily structurally related.

Protein engineering methods have been suggested that may have a bearing on this. For example it has also been proposed that a catalytic antibody could be created with a binding activity to a metal ion through one variable domain, and to a hapten (substrate) through contacts with the metal ion and a complementary variable domain (Barbas et al., 1993 Proc. Natl. Acad. Sci USA 90, 6385-6389). However in this case, the binding and catalysis of the substrate (first antigen) is proposed to require the binding of the metal ion (second antigen). Thus the binding to the V_{H}/V_{L} pairing relates to a single but multicomponent antigen.

Methods have been described for the creation of bispecific antibodies from camel antibody heavy chain single domains in which binding contacts for one antigen are created in one variable domain, and for a second antigen in a second variable domain. However the variable domains were not complementary. Thus a first heavy chain variable domain is selected against a first antigen, and a second heavy chain variable domain against a second antigen, and then both domains are linked together on the same chain to give a bispecific antibody fragment (Conrath et al., J. Biol. Chem. 276, 7346-7350). However the camel heavy chain single domains are unusual in that they are derived from natural camel antibodies which have no light chains, and indeed the heavy chain single domains are unable to associate with camel light chains to form complementary V_{H} and V_{L} pairs.

Single heavy chain variable domains have also been described, derived from natural antibodies which are normally associated with light chains (from monoclonal antibodies or from repertoires of domains; see EP-A-0368684). These heavy chain variable domains have been shown to interact specifically with one or more related antigens but have not been combined with other heavy or light chain variable domains to create a ligand with a specificity for two or more different antigens. Furthermore, these single domains have been shown to have a very short in *vivo* half-life. Therefore such domains are of limited therapeutic value.

It has been suggested to make bispecific antibody fragments by linking heavy chain variable domains of different specificity together (as described above). The disadvantage with this approach is that isolated antibody variable domains may have a hydrophobic interface that normally makes interactions with the light chain and is exposed to solvent and may be "sticky" allowing the single domain to bind to hydrophobic surfaces. Furthermore, in the absence of a partner light chain the combination of two or more different heavy chain variable domains and their association, possibly via their hydrophobic interfaces, may prevent them from binding to one in not both of the ligands they are able to bind in isolation. Moreover, in this case the heavy chain variable domains would not be associated with complementary light chain variable domains and thus may be less stable and readily unfold (Worn & Pluckthun, 1998 Biochemistry 37, 13120-7).

WO 031002609 which was published on 9^{th} Januray 2003 describes multispecific ligands.

### Summary of the invention

The inventors have described, in their copending international patent application WO 03/002609, dual specific immunoglobulin ligands which comprise immunoglobulin single variable domains which each have different specificities. The domains may act in competition with each other or independently to bind antigens or epitopes on target molecules.

In a first configuration, the present invention provides a further improvement in dual specific ligands as developed by the present inventors, in which one specificity of the ligand is directed towards a protein or polypeptide present *in vivo* in an organism which can act to increase the half-life of the ligand by binding to it.

Accordingly, in a first aspect, there is provided a dual-specific ligand comprising a first immunoglobulin single variable domain having a binding specificity to a first antigen or epitope and a second complementary immunoglobulin single variable domain having a binding activity to a second antigen or epitope, wherein one or both of said antigens or epitopes acts to increase the half-life of the ligand *in vivo* and wherein said first and second domains are not a heavy chain variable domain and higher chain variable domain which share the same specificity provided that said dual specific ligand does not consist of an anti-HSA V_{H} domain and an anti-β galactosidase V_{κ} domain. Preferably, that neither of the first or second variable domains binds to human serum albumin (HSA).

Antigens or epitopes which increase the half-life of a ligand as described herein are advantageously present on proteins or polypeptides found in an organism *in vivo.* Examples include extracellular matrix proteins, blood proteins, and proteins present in various tissues in the organism. The proteins act to reduce the rate of ligand clearance from the blood, for example by acting as bulking agents, or by anchoring the ligand to a desired site of action. Examples of antigens/epitopes which increase half-life *in vivo* are given in Annex 1 below.

Increased half-life is useful in *in vivo* applications of immunoglobulins, especially antibodies and most especially antibody fragments of small size. Such fragments (Fvs, disulphide bonded Fvs, Fabs, scFvs, dAbs) suffer from rapid clearance from the body; thus, whilst they are able to reach most parts of the body rapidly, and are quick to produce and easier to handle, their *in vivo* applications have been limited by their only brief persistence *in vivo*. The invention solves this problem by providing increased half-life of the ligands *in vivo* and consequently longer persistence times in the body of the functional activity of the ligand.

Methods for pharmacokinetic analysis and determination of ligand half-life will be familiar to those skilled in the art. Details may be found in *Kenneth, A et al:* Chemical Stability of Pharmaceuticals: A Handbook for Pharmacists and in *Peters et al,* Pharmacokinetc analysis: A Practical Approach (1996). Reference is also made to "Pharmacokinetics", M Gibaldi & D Perron, published by Marcel Dekker, 2^{nd} Rev. ex edition (1982), which describes pharmacokinetic parameters such as t alpha and t beta half lives and area under the curve (AUC).

Half lives (t½ alpha and t½ beta) and AUC can be determined from a curve of serum concentration of ligand against time. The WinNonlin analysis package (available from Pharsight Corp., Mountain View, CA94040, USA) can be used, for example, to model the curve. In a first phase (the alpha phase) the ligand is undergoing mainly distribution in the patient, with some elimination. A second phase (beta phase) is the terminal phase when the ligand has been distributed and the serum concentration is decreasing as the ligand is cleared from the patient. The t alpha half life is the half life of the first phase and the t beta half life is the half life of the second phase. Thus, advantageously, the present invention provides a ligand or a composition comprising a ligand according to the invention having a tα half-life in the range of 15 minutes or more. In one embodiment, the lower end of the range is 30 minutes, 45 minutes, 1 hour, 2 hours, 3 hours, 4 hours, 5 hours, 6 hours, 7 hours, 10 hours, 11 hours or 12 hours. In addition, or alternatively,a ligand or composition according to the invention will have a tα half life in the range of up to and including 12 hours. In one embodiment, the upper end of the range is 11, 10, 9, 8, 7, 6 or 5 hours. An example of a suitable range is 1 to 6 hours, 2 to 5 hours or 3 to 4 hours.

Advantageously, the present invention provides a ligand or a composition comprising a ligand according to the invention having a tβ half-life in the range of 2.5 hours or more. In one embodiment, the lower end of the range is 3 hours, 4 hours, 5 hours, 6 hours, 7 hours, 10 hours, 11 hours, or 12 hours. In addition, or alternatively, a ligand or composition according to the invention has a tβ half-life in the range of up to and including 21 days. In one embodiment, the upper end of the range is 12 hours, 24 hours, 2 days, 3 days, 5 days, 10 days, 15 days or 20 days. Advantageously a ligand or composition according to the invention will have a tβ half life in the range 12 to 60 hours. In a further embodiment, it will be in the range 12 to 48 hours. In a further embodiment still, it will be in the range 12 to 26 hours.

In addition, or alternatively to the above criteria, the present invention provides a ligand or a composition comprising a ligand according to the invention having an AUC value (area under the curve) in the range of 1 mg.min/ml or more. In one embodiment, the lower end of the range is 5, 10, 15, 20, 30, 100, 200 or 300mg.min/ml. In addition, or alternatively, a ligand or composition according to the invention has an AUC in the range of up to 600 mg.min/ml. In one embodiment, the upper end of the range is 500, 400, 300, 200, 150, 100, 75 or 50 mg.min/ml. Advantageously a ligand according to the invention will have a AUC in the range selected from the group consisting of the following: 15 to 150mg.min/ml, 15 to 100 mg.min/ml, 15 to 75 mg.min/ml, and 15 to 50mg.min/ml.

In a first embodiment, the dual specific ligand comprises two complementary variable domains, i.e. two variable domains that, in their natural environment, are capable of operating together as a cognate pair or group even if in the context of the present invention they bind separately to their cognate epitopes. For example, the complementary variable domains may be immunoglobulin heavy chain and light chain variable domains (V_{H} and V_{L}). V_{H} and V_{L} domains are advantageously provided by scFv or Fab antibody fragments. Variable domains may be linked together to form multivalent ligands by, for example: provision of a hinge region at the C-terminus of each V domain and disulphide bonding between cysteines in the hinge regions; or provision of dAbs each with a cysteine at the C-terminus of the domain, the cysteines being disulphide bonded together; or production of V-CH & V-CL to produce a Fab format; or use of peptide linkers (for example Gly₄Ser linkers discussed hereinbelow) to produce dimers, trimers and further multimers.

The inventors have found that the use of complementary variable domains allows the two domain surfaces to pack together and be sequestered from the solvent. Furthermore the complementary domains are able to stabilise each other. In addition, it allows the creation of dual-specific IgG antibodies without the disadvantages of hybrid hybridomas as used in the prior art, or the need to engineer heavy or light chains at the sub-unit interfaces. The dual-specific ligands of the first aspect of the present invention have at least one V_{H}/V_{L} pair. A bispecific IgG according to this invention will therefore comprise two such pairs, one pair on each arm of the Y-shaped molecule. Unlike conventional bispecific antibodies or diabodies, therefore, where the ratio of chains used is determinative in the success of the preparation thereof and leads to practical difficulties, the dual specific ligands of the invention are free from issues of chain balance. Chain imbalance in conventional bi-specific antibodies results from the association of two different V_{L} chains with two different V_{H} chains, where V_{L} chain 1 together with V_{H} chain 1 is able to bind to antigen or epitope I and V_{L} chain 2 together with V_{H} chain 2 is able to bind to antigen or epitope 2 and the two correct pairings are in some way linked to one another. Thus, only when V_{L} chain 1 is paired with V_{H} chain 1 and V_{L} chain 2 is paired with V_{H} chain 2 in a single molecule is bi-specificity created. Such bi-specific molecules can be created in two different ways. Firstly, they can be created by association of two existing V_{H}/V_{L} pairings that each bind to a different antigen or epitope (for example, in a bi-specific IgG). In this case the V_{H}/V_{L} pairings must come all together in a 1:1 ratio in order to create a population of molecules all of which are bi-specific. This never occurs (even when complementary CH domain is enhanced by "knobs into holes" engineering) leading to a mixture of bi-specific molecules and molecules that are only able to bind to one antigen or epitope but not the other. The second way of creating a bi-specific antibody is by the simultaneous association of two different V_{H} chain with two different V_{L} chains (for example in a bi-specific diabody). In this case, although there tends to be a preference for V_{L} chain 1 to pair with V_{H} chain 1 and V_{L} chain 2 to pair with V_{H} chain 2 (which can be enhanced by "knobs into holes" engineering of the V_{L} and V_{H} domains), this paring is never achieved in all molecules, leading to a mixed formulation whereby incorrect pairings occur that are unable to bind to either antigen or epitope.

Bi-specific antibodies constructed according to the dual-specific ligand approach according to the first aspect of the present invention overcome all of these problems because the binding to antigen or epitope 1 resides within the V_{H} or V_{L} domain and the binding to antigen or epitope 2 resides with the complementary V_{L} or V_{H} domain, respectively. Since V_{H} and V_{L} domains pair on a 1:1 basis all V_{H}/V_{L} pairings will be bi-specific and thus all formats constructed using these V_{H}/V_{L} pairings (Fv, scfvs, Fabs, minibodies, IgGs etc) will have 100% bi-specific activity.

In the context of the present invention, first and second "epitopes" are understood to be epitopes which are not the same and are not bound by a single monospecific ligand. In the first configuration of the invention, they are advantageously on different antigens, one of which acts to increase the half-life of the ligand *in vivo*. Likewise, the first and second antigens are advantageously not the same.

The dual specific ligands of the invention do not include ligands as described in WO 02/02773. Thus, the ligands of the present invention do not comprise complementary V_{H}/V_{L} pairs which bind any one or more antigens or epitopes co-operatively. Instead, the ligands according to the first aspect of the invention comprise a V_{H}/V_{L} complementary pair, wherein the V domains have different specificities.

Moreover, the ligands according to the first aspect of the invention comprise V_{H}/V_{L} complementary pairs having different specificities for non-structurally related epitopes or antigens. Structurally related epitopes or antigens are epitopes or antigens which possess sufficient structural similarity to be bound by a conventional V_{H}/V_{L} complementary pair which acts in a co-operative manner to bind an antigen or epitope; in the case of structurally related epitopes, the epitopes are sufficiently similar in structure that they "fit" into the same binding pocket formed at the antigen binding site of the V_{H}/V_{L} dimer.

In a second aspect, the present invention provides a ligand comprising a first immunoglobulin variable domain having a first antigen or epitope binding specificity and a second immunoglobulin variable domain having a second antigen or epitope binding specificity wherein one or both of said first and second variable domains bind to an antigen which increases the half-life óf the ligand *in vivo,* and either
(i) the first and the second immunoglobulin variable domains are heavy chain variable domains; or
(ii) the first and the second immunoglobulin variable domains are light chain variable domains.
provided that when the first and second immunoglobulin variable domains are Camelid VHH domains, the VHH domain which is specific for an antigen which increases the half-life of the ligand in vivo does not bind hen egg white lysozyme (HEL), porcine, pancreatic alpha-amylase; NmC-A, hcg, BSA-linked RR6 azo dye or *S. mutans* HG982 cells.

In one embodiment, binding to one variable domain modulates the binding of the ligand to the second variable domain.

In this embodiment, the variable domains may be, for example, pairs of V_{H} domains or pairs of V_{L} domains. Binding of antigen at the first site may modulate, such as enhance or inhibit, binding of an antigen at the second site. For example, binding at the first site at least partially inhibits binding of an antigen at a second site. In such an embodiment, the ligand may for example be maintained in the body of a subject organism *in vivo* through binding to a protein which increases the half-life of the ligand until such a time as it becomes bound to the second target antigen and dissociates from the half-life increasing protein.

Modulation of binding in the above context is achieved as a consequence of the structural proximity of the antigen binding sites relative to one another. Such structural proximity can be achieved by the nature of the structural components linking the two or more antigen binding sites, eg by the provision of a ligand with a relatively rigid structure that holds the antigen binding sites in close proximity. Advantageously, the two or more antigen binding sites are in physically close proximity to one another such that one site modulates the binding of antigen at another site by a process which involves steric hindrance and/or conformational changes within the immunoglobulin molecule.

The first and the second antigen binding domains may be associated either covalently or non-covalently. In the case that the domains are covalently associated, then the association may be mediated for example by disulphide bonds or by a polypeptide linker such as (Gly₄Ser)ₙ, where n = from 1 to 8, eg, 2, 3, 4, 5 or 7.

Ligands according to the invention may be combined into non-immunoglobulin multi-ligand structures to form multivalent complexes, which bind target molecules with the same antigen, thereby providing superior avidity, while at least one variable domain binds an antigen to increase the half life of the multimer. For example natural bacterial receptors such as SpA have been used as scaffolds for the grafting of CDRs to generate ligands which bind specifically to one or more epitopes. Details of this procedure are described in US 5,831,012. Other suitable scaffolds include those based on fibronectin and affibodies. Details of suitable procedures are described in WO 98/58965. Other suitable scaffolds include lipocallin and CTLA4, as described in van den Beuken *et al*., J. MoL Biol. (2001) 310, 591-601, and scaffolds such as those described in WO0069907 (Medical Research Council), which are based for example on the ring structure of bacterial GroEL or other chaperone polypeptides.

Protein scaffolds may be combined; for example, CDRs may be grafted on to a CTLA4 scaffold and used together with immunoglobulin V_{H} or V_{L} domains to form a ligand. Likewise, fibronectin, lipocallin and other scaffolds may be combined.

In the case that the variable domains are selected from V-gene repertoires selected for instance using phage display technology as herein described, then these variable domains can comprise a universal framework region, such that is they may be recognised by a specific generic ligand as herein defined. The use of universal frameworks, generic ligands and the like is described in WO99/20749. In the present invention, reference to phage display includes the use of both phage and/or phagemids.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary pair.

In a preferred embodiment of the invention the 'dual-specific ligand' is a single chain Fv fragment. In an alternative embodiment of the invention, the 'dual-specific ligand' consists of a Fab region of an antibody. The term "Fab region" includes a Fab-like region where two VH or two VL domains are used.

The variable regions may be derived from antibodies directed against target antigens or epitopes. Alternatively they may be derived from a repertoire of single antibody domains such as those expressed on the surface of filamentous bacteriophage. Selection may be performed as described below.

In a third aspect, the invention provides a method for producing a ligand comprising a first immunoglobulin single variable domain having a first binding specificity and a second single immunoglobulin single variable domain having a second (different) binding specificity, one or both of the binding specificities being specific for an antigen which increases the half-life of the ligand *in vivo,* the method comprising the steps of:
(a) selecting a first variable domain by its ability to bind to a first epitope,
(b) selecting a second variable region by its ability to bind to a second epitope,
(c) combining the variable domains; and
(d) selecting the ligand by its ability to bind to said first epitope and to said second epitope; wherein said variable domains are a heavy and a light chain variable domain; the heavy chain variable domain is not a V_{H} domain specific for HSA.

The ligand can bind to the first and second epitopes either simultaneously or, where there is competition between the binding domains for epitope binding, the binding of one domain may preclude the binding of another domain to its cognate epitope. In one embodiment, therefore, step (d) above requires simultaneous binding to both first and second (and possibly further) epitopes; in another embodiment, the binding to the first and second epitoes is not simultaneous.

The epitopes are preferably on separate antigens.

Ligands advantageously comprise V_{H}/V_{L} combinations, or V_{H}/V_{H} or V_{L}/V_{L} combinations of immunoglobulin variable domains, as described above. The ligands may moreover comprise camelid V_{HH} domains, provided that the V_{HH} domain which is specific for an antigen which increases the half-life of the ligand *in vivo* does not bind Hen egg white lysozyme (HEL), porcine pancreatic alpha-amylase or NmC-A; hcg, BSA-linked RR6 azo dye or *S. mutans* HG982 cells, as described in Conrath *et al*., (2001) JBC 276:7346-7350 and WO99/23221, neither of which describe the use of a specificity for an antigen which increases half-life to increase the half life of the ligand *in vivo*.

In one embodiment, said first variable domain is selected for binding to said first epitope in absence of a complementary variable domain. In a further embodiment, said first variable domain is selected for binding to said first epitope/antigen in the presence of a third variable domain in which said third variable domain is different from said second variable domain and is complementary to the first domain. Similarly, the second domain may be selected in the absence or presence of a complementary variable domain.

The antigens or epitopes targeted by the ligands of the invention, in addition to the half-life enhancing protein, may be any antigen or epitope but advantageously is an antigen or epitope that is targeted with therapeutic benefit. The invention provides ligands, including open conformation, closed conformation and isolated dAb monomer ligands, specific for any such target, particularly those targets further identified herein. Such targets may be, or be part of, polypeptides, proteins or nucleic acids, which may be naturally occurring or synthetic. In this respect, the ligand of the invention may bind the epiotpe or antigen and act as an antagonist or agonist (eg, EPO receptor agonist). One skilled in the art will appreciate that the choice is large and varied. They may be for instance human or animal proteins, cytokines, cytokine receptors, enzymes co-factors for enzymes or DNA binding proteins. Suitable cytokines and growth factors include but are not limited to: ApoE, Apo-SAA, BDNF, Cardiotrophin-1, EGF, EGF receptor, ENA-78, Eotaxin, Eotaxin-2, Exodus-2, EpoR, FGF-acidic, FGF-basic, fibroblast growth factor-10, FLT3 ligand, Fractalkine (CX3C), GDNF, G-CSF, GM-CSF, GF-β1, insulin, IFN-γ, IGF-I, IGF-II, IL-1α, IL-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-γ, HCC1, 1-309, HER 1, HER 2, HER 3 and HER 4. Cytokine receptors include receptors for the foregoing cytokines. It will be appreciated that this list is by no means exhaustive.

In one embodiment of the invention, the variable domains are derived from a respective antibody directed against the antigen or epitope. In a preferred embodiment the variable domains are derived from a repertoire of single variable antibody domains.

In a further aspect, the present invention provides one or more nucleic acid molecules encoding at least a dual-specific ligand as herein defined. The dual specific ligand may be encoded on a single nucleic acid molecule; alternatively, each domain may be encoded by a separate nucleic acid molecule. Where the ligand is encoded by a single nucleic acid molecule, the domains may be expressed as a fusion polypeptide, in the manner of a scFv molecule, or may be separately expressed and subsequently linked together, for example using chemical linking agents. Ligands expressed from separate nucleic acids will be linked together by appropriate means.

The nucleic acid may further encode a signal sequence for export of the polypeptides from a host cell upon expression and may be fused with a surface component of a filamentous bacteriophage particle (or other component of a selection display system) upon expression.

In a further aspect the present invention provides a vector comprising nucleic acid encoding a dual specific ligand according to the present invention.

In a yet further aspect, the present invention provides a host cell transfected with a vector encoding a dual specific ligand according to the present invention.

Expression from such a vector may be configured to produce, for example on the surface of a bacteriophage particle, variable domains for selection. This allows selection of displayed variable regions and thus selection of 'dual-specific ligands' using the method of the present invention.

Dual-Specific ligands according to the present invention preferably comprise combinations of heavy and light chain domains. For example, the dual specific ligand may comprise a V_{H} domain and a V_{L} domain, which may be linked together in the form of an scFv. In addition, the ligands may comprise one or more C_{H} or C_{L} domains. For example, the ligands may comprise a C_{H}1 domain, C_{H}2 or C_{H}3 domain, and/or a C_{L} domain, Cµ1, Cµ2, Cµ3 or Cµ4 domains, or any combination thereof. A hinge region domain may also be included. Such combinations of domains may, for example, mimic natural antibodies, such as IgG or IgM, or fragments thereof, such as Fv, scFv, Fab or F(ab')₂ molecules. Other structures, such as a single arm of an IgG molecule comprising V_{H}, V_{L}, C_{H}1 and C_{L} domains, are envisaged.

In a preferred embodiment of the invention, the variable regions are selected from single domain V gene repertoires. Generally the repertoire of single antibody domains is displayed on the surface of filamentous bacteriophage. In a preferred embodiment each single antibody domain is selected by binding of a phage repertoire to antigen.

In a preferred embodiment of the invention each single variable domain may be selected for binding to its target antigen or epitope in the absence of a complementary variable region. In an alternative embodiment, the single variable domains may be selected for binding to its target antigen or epitope in the presence of a complementary variable region. Thus the first single variable domain may be selected in the presence of a third complementary variable domain, and the second variable domain may be selected in the presence of a fourth complementary variable domain. The complementary third or fourth variable domain may be the natural cognate variable domain having the same specificity as the single domain being tested, or a non-cognate complementary domain - such as a "dummy" variable domain.

Preferably, the dual specific ligand of the invention comprises only two variable domains although several such ligands may be incorporated together into the same protein, for example two such ligands can be incorporated into an IgG or a multimeric immunoglobulin, such as IgM. Alternatively, in another embodiment a plurality of dual specific ligands are combined to form a multimer. For example, two different dual specific ligands are combined to create a tetra-specific molecule.

It will be appreciated by one skilled in the art that the light and heavy variable regions of a dual-specific ligand produced according to the method of the present invention may be on the same polypeptide chain, or alternatively, on different polypeptide chains. In the case that the variable regions are on different polypeptide chains, then they may be linked via a linker, generally a flexible linker (such as a polypeptide chain), a chemical linking group, or any other method known in the art.

In a further aspect, the present invention provides a composition comprising a dual-specific ligand, obtainable by a method of the present invention, and a pharmaceutically acceptable carrier, diluent or excipient.

Moreover, the present invention provides a a 'dual-specific ligand' or a composition thereof according to the present invention, for use in therapy.

The inventors have realised that under certain structural conditions, non-complementary variable domains (for example two light chain variable domains or two heavy chain variable domains) may be present in a ligand such that binding of a first epitope to a first variable domain inhibits the binding of a second epitope to a second variable domain, even though such non-complementary domains do not operate together as a cognate pair.

Advantageously, the ligand comprises two or more pairs of variable domains; that is, it comprises at least four variable domains. Advantageously, the four variable domains comprise frameworks of human origin.

In a preferred embodiment, the human frameworks are identical to those of human germline sequences.

The present inventors consider that such antibodies will be of particular use in ligand binding assays for therapeutic and other uses.

According to the present invention the term 'multi-specific ligand' refers to a ligand which possesses more than one epitope binding specificity as herein defined.

As herein defined the term 'closed conformation' (multi-specific ligand) means that the epitope binding domains of the ligand are attached to or associated with each other, optionally by means of a protein skeleton, such that epitope binding by one epitope binding domain competes with epitope binding by another epitope binding domain. That is, cognate epitopes may be bound by each epitope binding domain individually but not simultaneosuly. The closed conformation of the ligand can be achieved using methods herein described.

"Open conformation" means that the epitope binding domains of the ligand are attached to or associated with each other, optionally by means of a protein skeleton, such that epitope binding by one epitope binding domain does not compete with epitope binding by another epitope binding domain.

As referred to herein, the term 'competes' means that the binding of a first epitope to its cognate epitope binding domain is inhibited when a second epitope is bound to its cognate epitope binding domain. For example, binding may be inhibited sterically, for example by physical blocking of a binding domain or by alteration of the structure or environment of a binding domain such that its affinity or avidity for an epitope is reduced.

In the context of the present invention, first and second "epitopes" are understood to be epitopes which are not the same and are not bound by a single monospecific ligand. They may be on different antigens or on the same antigen, but separated by a sufficient distance that they do not form a single entity that could be bound by a single mono-specific V_{H}/V_{L} binding pair of a conventional antibody. Experimentally, if both of the individual variable domains in single chain antibody form (domain antibodies or dAbs) are separately competed by a monospecific V_{H}/V_{L} ligand against two epitopes then those two epitopes are not sufficiently far apart to be considered separate epitopes according to the present invention.

The closed conformation multispecific ligands of the invention do not include ligands as described in WO 02/02773. Thus, the ligands of the present invention do not comprise complementary V_{H}/V_{L} pairs which bind any one or more antigens or epitopes co-operatively. Instead, the ligands according to the invention preferably comprise non-complementary V_{H}-V_{H} or V_{L}-V_{L} pairs. Advantageously, each V_{H} or V_{L} domain in each V_{H}-V_{H} or V_{L}-V_{L} pair has a different epitope binding specificity, and the epitope binding sites are so arranged that the binding of an epitope at one site competes with the binding of an epitope at another site.

According to the present invention, advantageously, each epitope binding domain comprises an immunoglobulin variable domain. More advantageously, each immunoglobulin variable domain will be either a variable light chain domain (V_{L}) or a variable heavy chain domain V_{H}. In the second configuration of the present invention, the immunoglobulin domains when present on a ligand according to the present invention are non-complementary, that is they do not associate to form a V_{H}/V_{L} antigen binding site. Thus, multi-specific ligands as defined in the second configuration of the invention comprise immunoglobulin domains of the same sub-type, that is either variable light chain domains (V_{L}) or variable heavy chain domains (V_{H}). Moreover, where the ligand according to the invention is in the closed conformation, the immunoglobulin domains maybe of the camelid V_{HH} type.

In an alternative embodiment, the ligand(s) according to the invention do not comprise a camelid V_{HH} domain. More particularly, the ligand(s) of the invention do not comprise one or more amino acid residues that are specific to camelid V_{HH} domains as compared to human V_{H} domains.

Advantageously, the single variable domains are derived from antibodies selected for binding activity against different antigens or epitopes. For example, the variable domains may be isolated at least in part by human immunisation. Alternative methods are known in the art, including isolation from human antibody libraries and synthesis of artificial antibody genes.

The variable domains advantageously bind superantigens, such as protein A or protein L. Binding to superantigens is a property of correctly folded antibody variable domains, and allows such domains to be isolated from, for example, libraries of recombinant or mutant domains.

Epitope binding domains according to the present invention comprise a protein scaffold and epitope interaction sites (which are advantageously on the surface of the protein scaffold).

Epitope binding domains may also be based on protein scaffolds or skeletons other than immunoglobulin domains. For example natural bacterial receptors such as SpA have been used as scaffolds for the grafting of CDRs to generate ligands which bind specifically to one or more epitopes. Details of this procedure are described in US 5,831,012. Other suitable scaffolds include those based on fibronectin and affibodies. Details of suitable procedures are described in WO 98/58965. Other suitable scaffolds include lipocallin and CTLA4, as described in van den Beuken *et al*., J. Mol. Biol. (2001) 310, 591-601, and scaffolds such as those described in WO0069907 (Medical Research Council), which are based for example on the ring structure of bacterial GroEL or other chaperone polypeptides.

Protein scaffolds may be combined; for example, CDRs may be grafted on to a CTLA4 scaffold and used together with immunoglobulin V_{H} or V_{L} domains to form a multivalent ligand. Likewise, fibronectin, lipocallin and other scaffolds may be combined.

It will be appreciated by one skilled in the art that the epitope binding domains of a closed conformation multispecific ligand produced according to the method of the present invention may be on the same polypeptide chain, or alternatively, on different polypeptide chains. In the case that the variable regions are on different polypeptide chains, then they may be linked via a linker, advantageously a flexible linker (such as a polypeptide chain), a chemical linking group, or any other method known in the art.

The first and the second epitope binding domains may be associated either covalently or non-covalently. In the case that the domains are covalently associated, then the association may be mediated for example by disulphide bonds.

The epitopes may be, or be part of, polypeptides, proteins or nucleic acids, which may be naturally occurring or synthetic. In this respect, the ligand of the invention may bind an epiotpe or antigen and act as an antagonist or agonist (eg, EPO receptor agonist). The epitope binding domains of the ligand in one embodiment have the same epitope specificity, and may for example simultaneously bind their epitope when multiple copies of the epitope are present on the same antigen. In another embodiment, these epitopes are provided on different antigens such that the ligand can bind the epitopes and bridge the antigens. One skilled in the art will appreciate that the choice of epitopes and antigens is large and varied. They may be for instance human or animal proteins, cytokines, cytokine receptors, enzymes co-factors for enzymes or DNA binding proteins. Suitable cytokines and growth factors include but are not limited to: ApoE, Apo-SAA, BDNF, Cardiotrophin-1, EGF, EGF receptor, ENA-78, Eotaxin, Eotaxin-2, Exodus-2, EpoR, FGF-acidic, FGF-basic, fibroblast growth factor-10, FLT3 ligand, Fractalkine (CX3C), GDNF, G-CSF, GM-CSF, GF-β1, insulin, IFN-γ, IGF-I, IGF-II, IL-1α, IL-1β, IL-2, IL-3, IL-4, IL-5, IL-6, IL-7, IL-8 (72 a,a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-γ, HCCl, 1-309, HER 1, HER 2, HER 3, HER 4, TACE recognition site, TNF BP-I and TNF BP-II, as well as any target disclosed in Annex 2 or Annex 3 hereto, whether in combination as set forth in the Annexes, in a different combination or individually. Cytokine receptors include receptors for the foregoing cytokines, e.g. IL-1 R1; IL-6R; IL-10R; IL-18R, as well as receptors for cytokines set forth in Annex 2 or Annex 3 and also receptors disclosed in Annex 2 and 3. It will be appreciated that this list is by no means exhaustive. Where the multispecific ligand binds to two epitopes (on the same or different antigens), the antigen(s) may be selected from this list.

Advantageously, dual specific ligands may be used to target cytokines and other molecules which cooperate synergistically in therapeutic situations in the body of an organism. The invention therefore provides a method for synergising the activity of two or more cytokines, comprising administering a dual specific ligand capable of binding to said two or more cytokines. In this aspect of the invention, the dual specific ligand may be any dual specific ligand, including a ligand composed of complementary and/or non-complementary domains, a ligand in an open conformation, and a ligand in a closed conformation. For example, this aspect of the invention relates to combinations of V_{H} domains and V_{L} domains, V_{H} domains only and V_{L} domains only.

Synergy in a therapeutic context may be achieved in a number of ways. For example, target combinations may be therapeutically active only if both targets are targeted by the ligand, whereas targeting one target alone is not therapeutically effective. In another embodiment, one target alone may provide some low or minimal therapeutic effect, but together with a second target the combination provides a synergistic increase in therapeutic effect.

Preferably, the cytokines bound by the dual specific ligands of this aspect of the invention are selected from the list shown in Annex 2.

Moreover, dual specific ligands may be used in oncology applications, where one specificity targets CD89, which is expressed by cytotoxic cells, and the other is tumour specific. Examples of tumour antigens which may be targetted are given in Annex 3.

In one embodiment of the second configuration of the invention, the variable domains are derived from an antibody directed against the first and/or second antigen or epitope. In a preferred embodiment the variable domains are derived from a repertoire of single variable antibody domains. In one example, the repertoire is a repertoire that is not created in an animal or a synthetic repertoire. In another example, the single variable domains are not isolated (at least in part) by animal immunisation. Thus, the single domains can be isolated from a naïve library.

In a further aspect, the present invention provides a closed conformation multi-specific ligand comprising a first epitope binding domain having a first epitope binding specificity and a non-complementary second epitope binding domain having a second epitope binding specificity wherein the first and second binding specificities are capable of competing for epitope binding such that the closed conformation multi-specific ligand cannot bind both epitopes simultaneously.

Open conformation ligands comprise non-complementary binding domains, wherein the domains are specific for a different epitope on the same target. Such ligands bind to targets with increased avidity. Similarly, the invention provides multivalent ligands comprising non-complementary binding domains specific for the same epitope and directed to targets which comprise multiple copies of said epitope, such as IL-5, PDGF-AA, PDGF-BB, TGF beta, TGF beta2, TGF beta3 and TNFα, for eample human TNF Receptor 1 and human TNFα.

Ligands can be configured to bind individual epitopes with low affinity, such that binding to individual epitopes is not therapeutically significant; but the increased avidity resulting from binding to two epitopes provides a theapeutic benefit. In a particular example, epitopes may be targetted which are present individually on normal cell types, but present together only on abnormal or diseased cells, such as tumour cells. In such a situaton, only the abnormal or diseased cells are effectively targetted by the blspecific Ligands according to the invention.

Ligand specific for multiple copies of the same epitope, or adjacent epitopes, on the same target (known as chelating dAbs) may also be trimeric or polymeric (tertrameric or more) ligands comprising three, four or more non-complementary binding domains. For example, ligands may be constructed comprising three or four V_{H} domains or V_{L} domains.

Moreover, ligands are provided which bind to multisubunit targets, wherein each binding domain is specific for a subunit of said target. The ligand may be dimeric, trimeric or polymeric.

Preferably, the multi-specific ligands according to the above aspects of the invention are obtainable by the method of the first aspect of the invention.

According to the above aspect of the second configuration of the invention, advantageously the first epitope binding domain and the second epitope binding domains are non-complementary immunoglobulin variable domains, as herein defined. That is either V_{H}-V_{H} or V_{L}-V_{L} variable domains.

Chelating dAbs in particular may be prepared according to a preferred aspect of the invention, namely the use of anchor dAbs, in which a library of dimeric, trimeric or multimeric dAbs is constructed using a vector which comprises a constant dAb upstream or downstream of a linker sequence, with a repertoire of second, third and further dAbs being inserted on the other side of the linker. For example, the anchor or guiding dAb may be TAR1-5 (Vκ), TAR1-27(Vκ), TAR2h-5(VH) or TAR2h-6(Vκ).

In alternative methodologies, the use of linkers may be avoided, for example by the use of non-covalent bonding or natural affinity between binding domains such as V_{H} and V_{κ}.

According to the present invention, the term 'immunoglobulin skeleton' refers to a protein which comprises at least one immunoglobulin fold and which acts as a nucleus for one or more epitope binding domains, as defined herein.

Preferred immunoglobulin skeletons as herein defined includes any one or more of those selected from the following: an immunoglobulin molecule comprising at least (i) the CL (kappa or lambda subclass) domain of an antibody; or (ii) the CH1 domain of an antibody heavy chain; an immunoglobulin molecule comprising the CH1 and CH2 domains of an antibody heavy chain; an immunoglobulin molecule comprising the CH1, CH2 and CH3 domains of an antibody heavy chain; or any of the subset (ii) in conjunction with the CL (kappa or lambda subclass) domain of an antibody. A hinge region domain may also be included. Such combinations of domains may, for example, mimic natural antibodies, such as IgG or IgM, or fragments thereof, such as Fv, scFv, Fab or F(ab')₂ molecules. Those skilled in the art will be aware that this list is not intended to be exhaustive.

Linking of the skeleton to the epitope binding domains, as herein defined may be achieved at the polypeptide level, that is after expression of the nucleic acid encoding the skeleton and/or the epitope binding domains. Alternatively, the linking step may be performed at the nucleic acid level. Methods of linking a protein skeleton according to the present invention, to the one or more epitope binding domains include the use of protein chemistry and/or molecular biology techniques which will be familiar to those skilled in the art and are described herein.

Advantageously, the closed conformation multispecific ligand comprises a first domain capable of binding a target molecule, and a second domain capable of binding a molecule or group which extends the half-life of the ligand. For example, the molecule or group may be a bulky agent, such as HSA or a cell matrix protein. As used herein, the phrase "molecule or group which extends the half-life of a ligand" refers to a molecule or chemical group which, when bound by a dual-specific ligand as described herein increases the in vivo half-life of such dual specific ligand when administered to an animal, relative to a ligand that does not bind that molecule or group. Examples of molecules or groups that extend the half-life of a ligand are described hereinbelow. In a preferred embodiment, the closed conformation multispecific ligand may be capable of binding the target molecule only on displacement of the half-life enhancing molecule or group. Thus, for example, a closed conformation multispecific ligand is maintained in circulation in the bloodstream of a subject by a bulky molecule such as HSA. When a target molecule is encountered, competition between the binding domains of the closed conformation multispecific ligand results in displacement of the HSA and binding of the target

Ligands according to any aspect of the present invention, as well as dAb monomers useful in constructing such ligands, may advantageously dissociate from their cognate target(s) with a K_{d} of 300nM to 5pM (ie, 3 x 10⁻⁷ to 5 x 10⁻¹²M), preferably 50nM to20pM, or 5nM to 200pM or 1nM to 100pM, 1 x 10⁻⁷ M or less, 1 x 10⁻⁸ M or less, 1 x 10⁻⁹ M or less, 1 x 10⁻¹⁰ M or less, 1 x 10⁻¹¹ M or less; and/or a K_{off} rate constant of 5 x 10⁻¹ to 1 x 10⁻⁷ S⁻¹, preferably 1 x 10⁻² to 1 x 10⁻⁶ S⁻¹, or 5 x 10⁻³ to 1 x 10⁻⁵ S⁻¹, or 5 x 10⁻¹ S⁻¹ or less, or 1 x 10⁻² S⁻¹ or less, or 1 x 10⁻³ S⁻¹ or less, or 1 x 10⁻⁴ S⁻¹ or less, or 1 x 10⁻⁵ S⁻¹ or less, or 1 x 10⁻⁶ S⁻¹ or less as determined by surface plasmon resonance. The K_{d} rate constand is defined as K_{off}/Kₒₙ.

A ligand can comprise an anti-TNFα dAb monomer (or dual specific ligand comprising such a dAb), homodimer, heterodimer or homotrimer ligand, wherein each dAb binds TNFα. The ligand binds to TNFα with a K_{d} of 300nM to 5pM (ie, 3 x 10⁻⁷ to 5 x 10⁻¹²M), preferably 50nM to 20pM, more preferably 5nM to 200pM and most preferably 1nM to 100pM; expressed in an alternative manner, the K_{d} is 1 x 10⁻⁷ M or less, preferably 1 x 10⁻⁸ M or less, more preferably 1 x 10⁻⁹ M or less, advantageously 1 x 10⁻¹⁰ M or less and most preferably 1 x 10⁻¹¹ M or less; and/or a K_{off} rate constant of 5 x 10⁻¹ to 1 x 10⁻⁷ S⁻¹, preferably 1 x 10⁻² to 1 x 10⁻⁶ S⁻¹, more preferably 5 x 10⁻³ to 1 x 10⁻⁵ S⁻¹, for example 5 x 10⁻¹ S⁻¹ or less, preferably 1 x 10⁻² S⁻¹ or less, more preferably 1 x 10⁻³ S⁻¹ or less, advantageously 1 x 10⁻⁴ S⁻¹ or less, further advantageously 1 x 10⁻⁵ S⁻¹ or less, and most preferably 1 x 10⁻⁶ S⁻¹ or less, as determined by surface plasmon resonance.

Preferably, the ligand neutralises TNFα in a standard L929 assay with an ND50 of 500nM to 50pM, preferably or 140nM to 50pM, advantageously 10nM to 100pM, more preferably 1nM to 100pM; for example 50nM or less, preferably 5nM or less, advantageously 500pM or less, more preferably 200pM or less and most preferably 100pM or less.

Preferably, the ligand inhibits binding of TNF alpha to TNF alpha Receptor I (p55 receptor) with an IC50 of 500nM to 50pM, preferably 100nM to 50pM, more preferably 10nM to 100pM, advantageously 1nM to 100pM; for example 50nM or less, preferably 5nM or less, more preferably 500pM or less, advantageously 200pM or less, and most preferably 100pM or less. Preferably, the TNFα is Human TNFα.

A ligand can comprise an anti-TNF Receptor I dAb monomer, or dual specific ligand comprising such a dAb, that binds to TNF Receptor I with a K_{d} of 300nM to 5pM (ie, 3 x 10⁻⁷ to 5 x 10⁻¹²M), preferably 50nM to20pM, more preferably 5nM to 200pM and most preferably 1nM to 100pM, for example 1 x 10⁻⁷ M or less, preferably 1 x 10⁻⁸ M or less, more preferably 1 x 10⁻⁹ M or less, advantageously 1 x 10⁻¹⁰ M or less and most preferably 1 x 10⁻¹¹ M or less; and/or a K_{off} rate constant of 5 x 10⁻¹ to 1 x 10⁻⁷ S⁻¹, preferably 1 x 10⁻² to 1 x 10⁻⁶ S⁻¹, more preferably 5 x 10⁻³ to 1 x 10⁻⁵ S⁻¹, for example 5 x 10⁻¹ S⁻¹ or less, preferably 1 x 10⁻² S⁻¹ or less, advantageously 1 x 10⁻³ S⁻¹ or less, more preferably 1 x 10⁻⁴ S⁻¹ or less, still more preferably 1 x 10⁻⁵ S⁻¹ or less, and most preferably 1 x 10⁻⁶ S⁻¹ or less as determined by surface plasmon resonance.

Preferably, the dAb monomeror ligand neutralises TNFα in a standard assay (eg, the L929 or HeLa assays described herein) with an ND50 of 500nM to 50pM, preferably 100nM to 50pM, more preferably 10nM to 100pM, advantageously 1nM to 10OpM; for example 50nM or less, preferably 5nM or less, more preferably 500pM or less, advantageously 200pM or less, and most preferably 100pM or less.

Preferably, the dAb monomer or ligand inhibits binding of TNF alpha to TNF alpha. Receptor I (p55 receptor) with an IC50 of 500nM to 50pM, preferably 100nM to 50pM, more preferably 10nM to 100pM, advantageously 1nM to 100pM; for example 50nM or less, preferably 5nM or less, more preferably 500pM or less, advantageously 200pM or less, and most preferably 100pM or less. Preferably, the TNF Receptor I target is Human TNFα.

A ligand an comprise a dAb monomer(or dual specific ligand comprising such a dAb) that binds to serum albumm (SA) with a K_{d} of 1nM to 500µM (ie, x 10⁻⁹ to 5 x 10⁻⁴), preferably 100nM to 10µM. Preferably, for a dual specific ligand comprising a first anti-SA dAb and a second dAb to another target, the affinity (eg K_{d} and/or K_{off} as measured by surface plasmon resonance, eg using BiaCore) of the second dAb for its target is frona 1 to 100000 times (preferably 100 to 100000, more preferably 1000 to 100000, or 10000 to 100000 times) the affinity of the first dAb for SA. For example, the first dAb binds SA with an affinity of approximately 10µM, while the second dAb binds its target with an affinity of 100pM. Preferably, the serum albumin is human serum albumin (HSA).

In one embodiment, the first dAb (or a dAb monomer) binds SA (eg, HSA) with a K_{d} of approximately 50, preferably 70, and more preferably 100,150 or 200 nM.

The invention moreover provides dimers, trimers and polymers of the aforementioned dAb monomers, in accordance with the foregoing aspect of the present invention.

Ligands according to the invention, including dAb monomers, dimers and trimers, can be linked to an antibody Fc region, comprising one or both of C_{H}2 and C_{H}3 domains, and optionally a hinge region. For example, vectors encoding ligands linked as a single nucleotide sequence to an Fc region may be used to prepare such polypeptides.

In a further aspect of the invention the present invention provides a vector comprising nucleic acid according to the present invention.

In a yet further aspect, the present invention provides a host cell transfected with a vector according to the present invention.

Expression from such a vector may be configured to produce, for example on the surface of a bacteriophage particle, epitope binding domains for selection. This allows selection of displayed domains and thus selection of 'multispecific ligands' using the method of the present invention.

In a further aspect still of the invention, the present invention provides a composition comprising a ligand, obtainable by a method of the present invention, and aphannaceutically acceptable carrier, diluent or excipient.

Moreover, the present invention provides a method for the treatment of disease using a 'closed confonnation multispecific ligand' or a composition according to the present invention.

In a preferred embodiment of the invention the disease is cancer or an inflammatory disease, eg rheumatoid arthritis, asthma or Crohn's disease.

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%. 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

Calculations of "homology" or "sequence identity" or "similarity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences.

Advantageously, the BLAST algorithm (version 2.0) is employed for sequence alignment, with parameters set to default values. The BLAST algorithm is described in detail at the world wide web site ("www") of the National Center for Biotechnology Information (".ncbi") of the National Institutes of Health ("nih") of the U.S. government (".gov"), in the "/Blast/" directory, in the "blast_help.html" file. The search parameters are defined as follows, and are advantageously set to the defined default parameters.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, 1990, Proc. Natl. Acad. Sci. USA 87(6):2264-8 (see the "blast_help.html" file, as described above) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul et al. (1994).

The five BLAST programs available at the National Center for Biotechnology Information web site perform the following tasks:
"blastp" compares an amino acid query sequence against a protein sequence database;
"blastn" compares a nucleotide query sequence against a nucleotide sequence database;
"blastx" compares the six-frame conceptual translation products of a nucleotide query sequence (both strands) against a protein sequence database;
"tblastn" compares a protein query sequence against a nucleotide sequence database dynamically translated in all six reading frames (both strands).
"tblastx" compares the six-frame translations of a nucleotide query sequence against the six-frame translations of a nucleotide sequence database.
BLAST uses the following search parameters:
HISTOGRAM Display a histogram of scores for each search; default is yes. (See parameter H in the BLAST Manual).
DESCRIPTIONS Restricts the number of short descriptions of matching sequences reported to the number specified; default limit is 100 descriptions. (See parameter V in the manual page). See also EXPECT and CUTOFF.
ALIGNMENTS Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).
EXPECT The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).
CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.
MATRIX Specify an alternate scoring matrix for BLASTP, BLASTX, TBLASTN and TBLASTX. The default matrix is BLOSUM62 (Henikoff & Henikoff, 1992, Proc. Natl. Aacad. Sci. USA 89(22):10915-9). The valid alternative choices include: PAM40, PAM120, PAM250 and IDENTITY. No alternate scoring matrices are available for BLASTN; specifying the MATRIX directive in BLASTN requests returns an error response.
STRAND Restrict a TBLASTN search to just the top or bottom strand of the database sequences; or restrict a BLASTN, BLASTX or TBLASTX search to just reading frames on the top or bottom strand of the query sequence.
FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States, 1993, Computers and Chemistry 17:191-201, or, for BLASTN, by the DUST program of Tatusov and Lipman (see the world wide web site of the NCBI). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "N" repeated 13 times) and the letter "X" in protein sequences (e.g., "X" repeated 9 times).

Filtering is only applied to the query sequence (or its translation products), not to database sequences. Default filtering is DUST for BLASTN, SEG for other programs. It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi Causes NCBI gi identifiers to be shown in the output, in addition to the accession and/or locus name.

Most preferably, sequence comparisons are conducted using the simple BLAST search algorithm provided at the NCBI world wide web site described above, in the "/BLAST" directory.

### Brief Description of the Figures

- Figure 1: shows the diversification of V_{H}/HSA at positions H50, H52, H52a, H53, H55, H56, H58, H95, H96, H97, H98 (DVT or NNK encoded respectively) which are in the antigen binding site of V_{H} HSA. The sequence of V_{K} is diversified at positions L50, L53.
- Figure 2: shows
Library 1: Germline V_{K}/DVT V_{H},
Library 2: Germline V_{K}/NNK V_{H},
Library 3: Germline V_{H}/DVT V_{K}
Library 4: Germline V_{H}/NNK V_{K}
In phage display/ScFv format. These libraries were pre-selected for binding to generic ligands protein A and protein L so that the majority of the clones and selected libraries are functional. Libraries were selected on HSA (first round) and β-gal (second round) or HSA β-gal selection or on β-gal (first round) and HSA (second round) β-gal HSA selection. Soluble scFv from these clones of PCR are amplified in the sequence. One clone encoding a dual specific antibody K8 was chosen for further work.

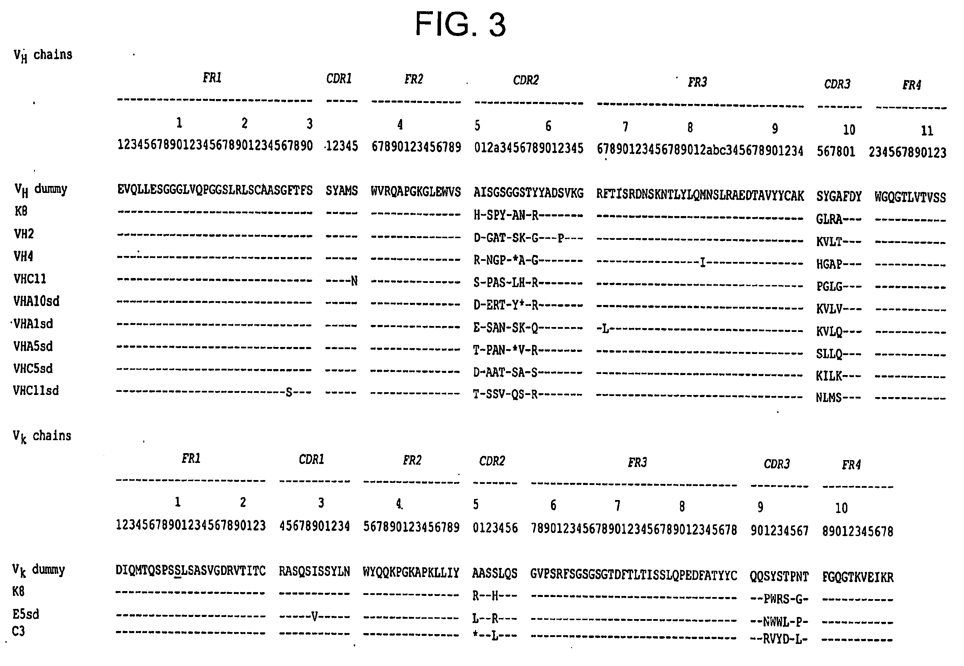

- Figure 3: shows an alignment of V_{H} chains and V_{κ} chains.

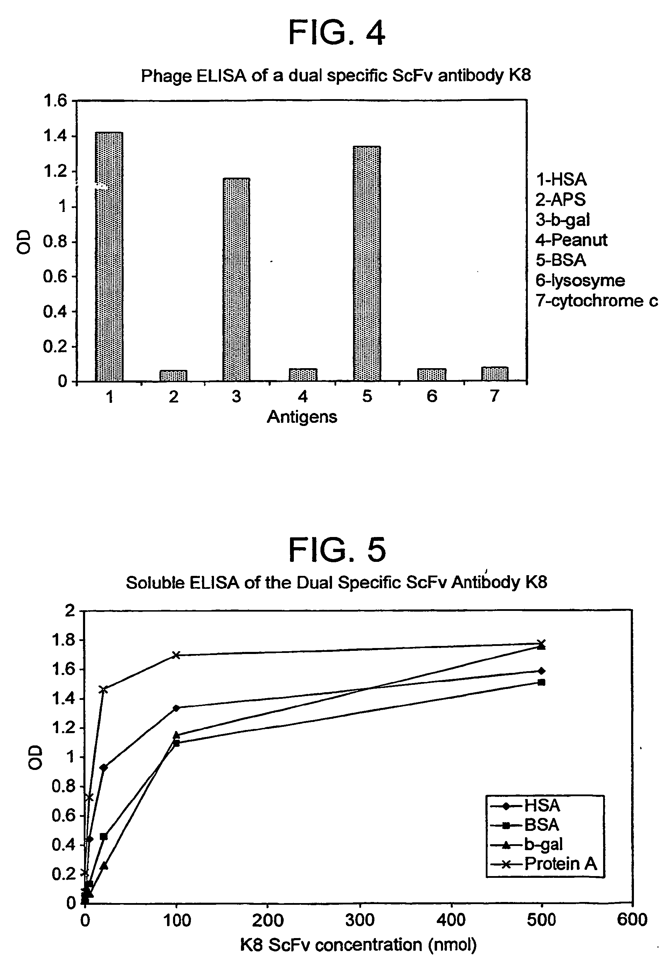

- Figure 4: shows the characterisation of the binding properties of the K8 antibody, the binding properties of the K8 antibody characterised by monoclonal phage ELISA, the dual specific K8 antibody was found to bind HSA and β-gal and displayed on the surface of the phage with absorbant signals greater than 1.0. No cross reactivity with other proteins was detected.
- Figure 5: shows soluble scFv ELISA performed using known concentrations of the K8 antibody fragment. A 96-well plate was coated with 100µg of HSA, BSA and β-gal at 10µg/ml and 100µg/ml of Protein A at 1µg/ml concentration. 50µg of the serial dilutions of the K8 scFv was applied and the bound antibody fragments were detected with Protein L-HRP. ELISA results confirm the dual specific nature of the K8 antibody.

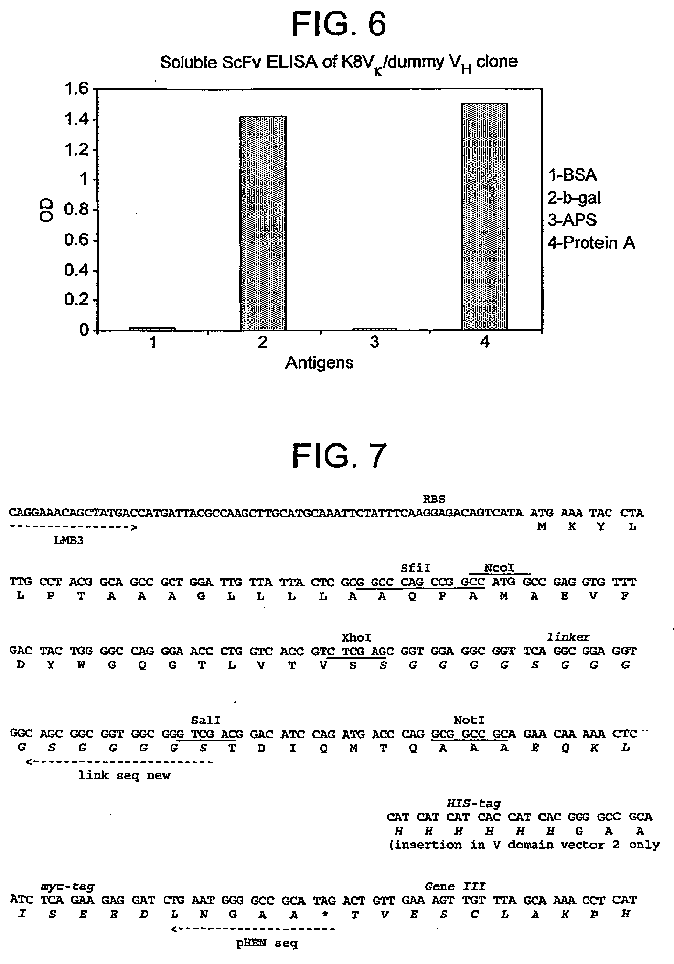

- Figure 6: shows the binding characteristics of the clone K8V_{K}/dummy V_{H} analysed using soluble scFv ELISA. Production of the soluble scFv fragments was induced by IPTG as described by Harrison *et al*, Methods Enzymol. 1996;267:83-109 and the supernatant containing scFv assayed directly. Soluble scFv ELISA is performed as described in example 1 and the bound scFvs were detected with Protein L-HRP. The ELISA results revealed that this clone was still able to bind β-gal, whereas binding BSA was abolished.
- Figure 7: shows the sequence of variable domain vectors 1 and 2.

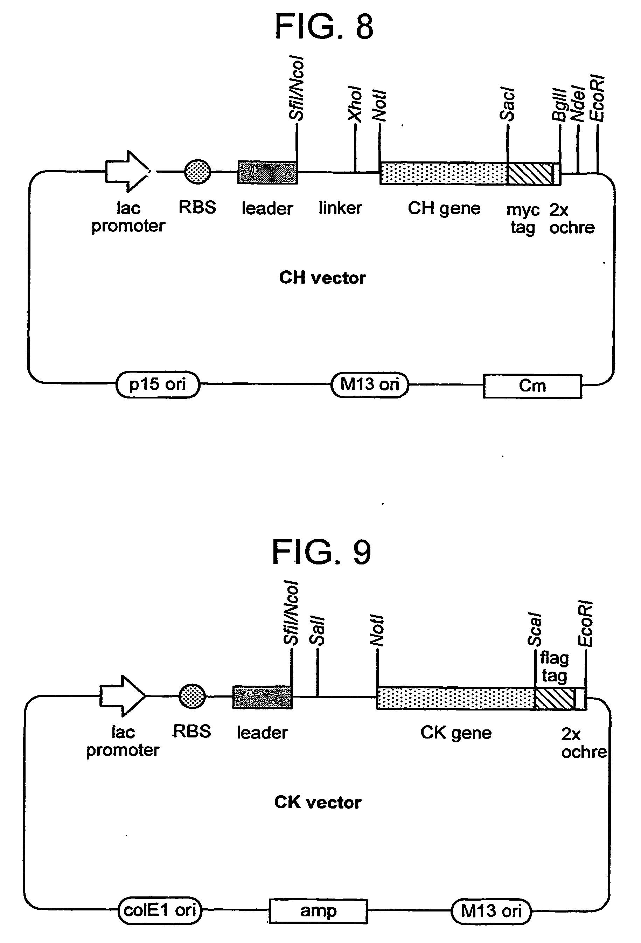

- Figure 8: is a map of the C_{H} vector used to construct a V_{H}1/V_{H}2 multipsecific ligand.
- Figure 9: is a map of the V_{κ} vector used to construct a V_{κ}1/V_{κ}2 multispecific ligand.

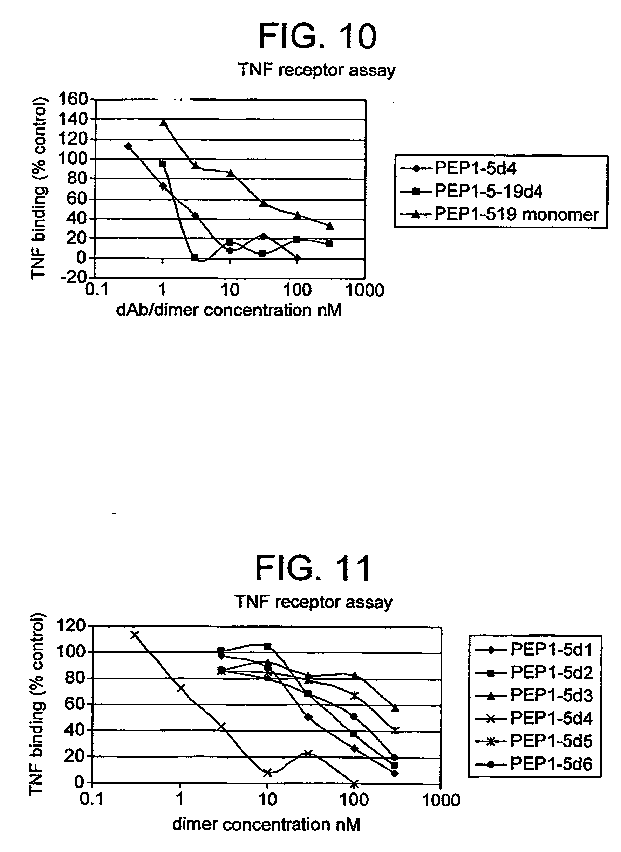

- Figure 10: TNF receptor assay comparing TAR1-5 dimer 4, TAR1-5-19 dimer 4 and TAR1-5-19 monomer.
- Figure 11: TNF receptor assay comparing TAR1-5 dimers 1-6.All dimers have been FPLC purified and the results for the optimal dimeric species are shown.

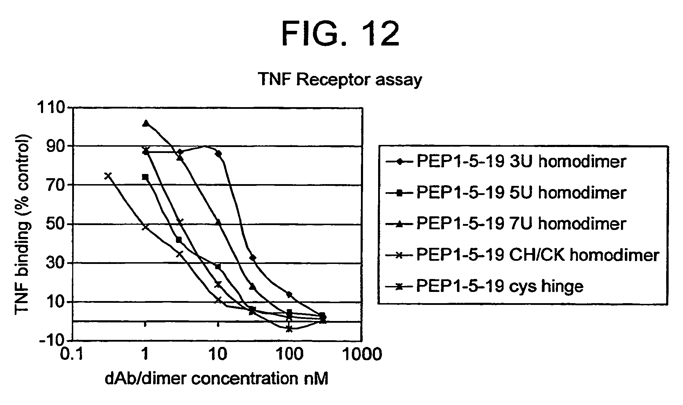

- Figure 12: TNF receptor assay of TAR1-5 19 homodimers in different formats: dAblinker-dAb format with 3U, 5U or 7U linker, Fab format and cysteine hinge linker format.

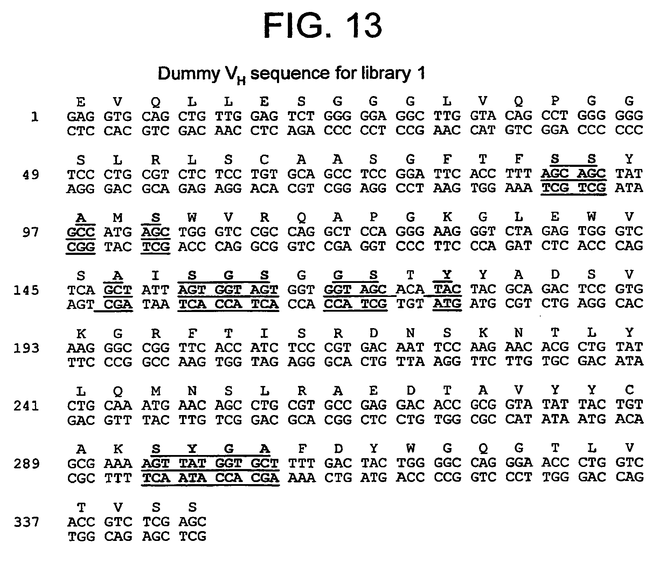

- Figure 13: Dummy VH sequence for library 1. The sequence of the VH framework based on germline sequence DP47 - JH4b. Positions where NNK randomisation (N=A or T or C or G nucleotides; K = G or T nucleotides) has been incorporated into library 1 are indicated in bold underlined text.

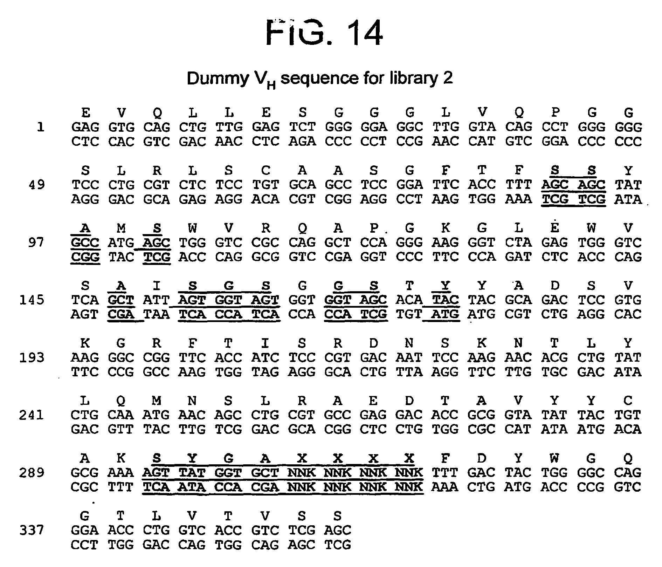

- Figure 14: Dummy VH sequence for library 2. The sequence of the VH framework based on germline sequence DP47 - JH4b. Positions where NNK randomisation (N=A or T or C or G nucleotides; K = G or T nucleotides) has been incorporated into library 2 are indicated in bold underlined text.

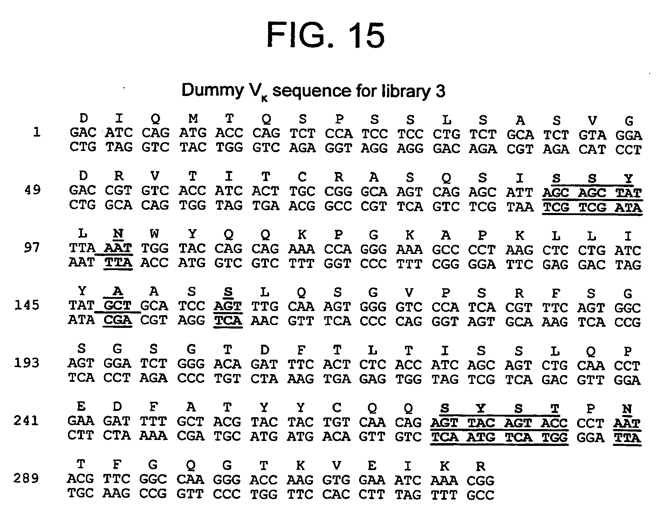

- Figure 15: Dummy Vκ sequence for library 3. The sequence of the Vκ framework based on germline sequence DP_{K}9 - J _{K}1. Positions where NNK randomisation (N=A or T or C or G nucleotides; K = G or T nucleotides) has been incorporated into library 3 are indicated in bold underlined text.

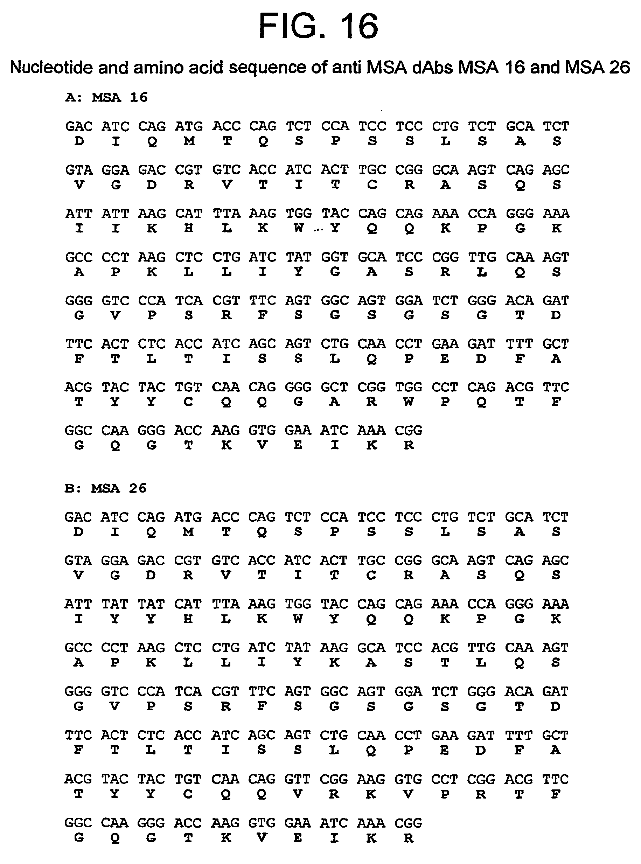

- Figure 16: Nucleotide and amino acid sequence of anti MSA dAbs MSA 16 and MSA 26.

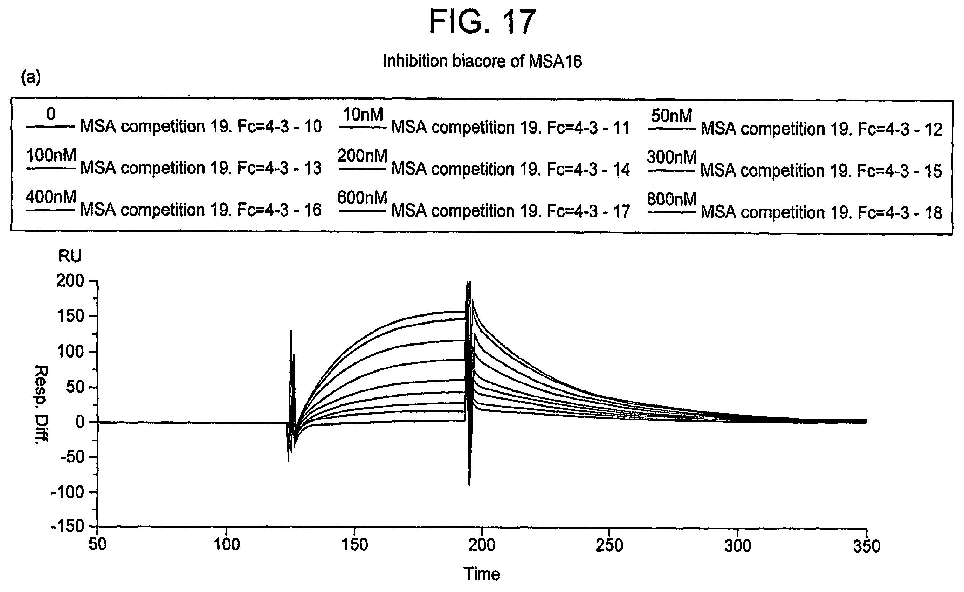

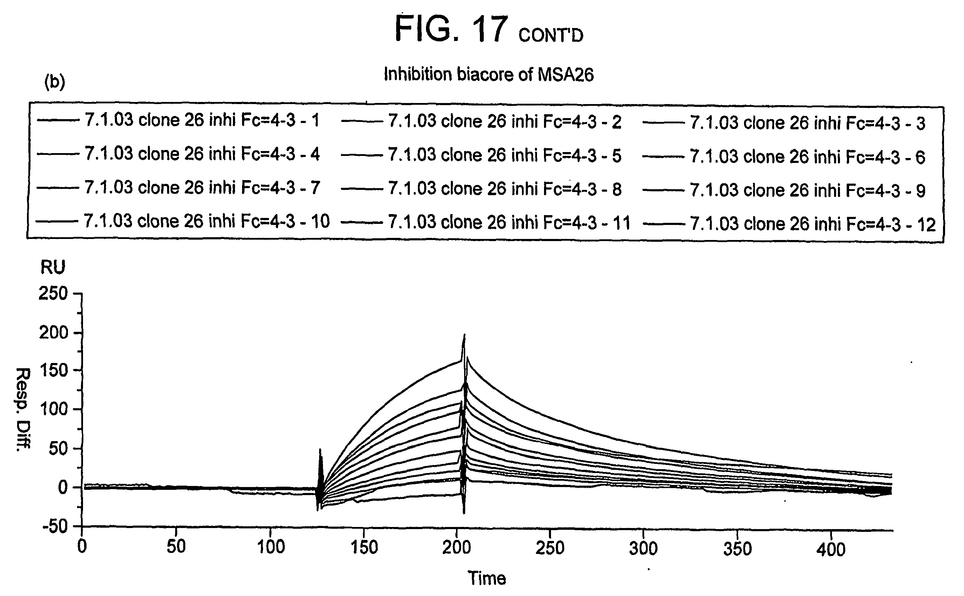

- Figure 17: Inhibition biacore of MSA 16 and 26. Purified dAbs MSA16 and MSA26 were analysed by inhibition biacore to determine K_{d}. Briefly, the dAbs were tested to determine the concentration of dAb required to achieve 200RUs of response on a biacore CM5 chip coated with a high density of MSA. Once the required concentrations of dAb had been determined, MSA antigen at a range of concentrations around the expected K_{d} was premixed with the dAb and incubated overnight. Binding to the MSA coated biacore chip of dAb in each of the premixes was then measured at a high flow-rate of 30 µl/minute.

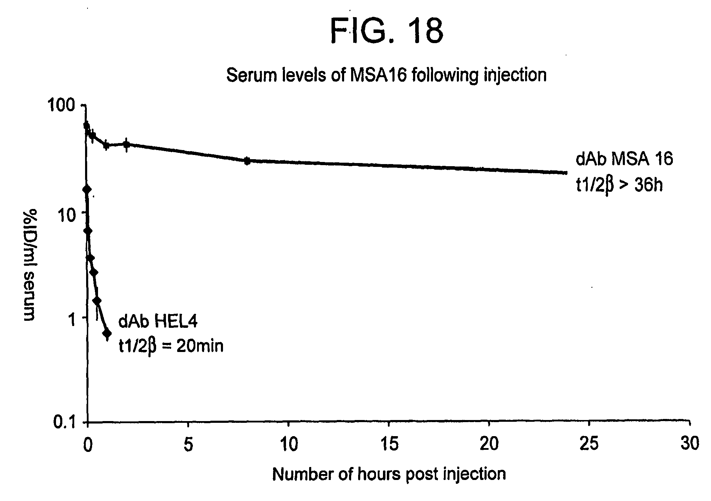

- Figure 18: Serum levels of MSA16 following injection. Serum half life of the dAb MSA16 was determined in mouse. MSA16 was dosed as single i.v. injections at approx 1.5mg/kg into CD1 mice. Modelling with a 2 compartment model showed MSA16 had a t1/2α of 0.98hr, a t1/2β of 36.5hr and an AUC of 913hr.mg/ml. MSA16 had a considerably lengthened half life compared with HEL4 (an anti-hen egg white lysozyme dAb) which had a t1/2α of 0.06hr and a t1/2β of 0.34hr.

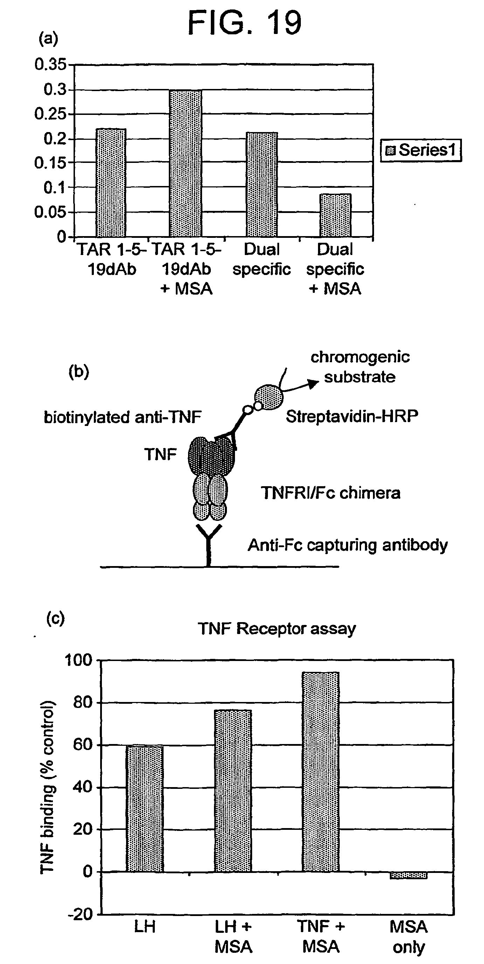

- Figure 19: ELISA (a) and TNF receptor assay (c) showing inhibition of TNF binding with a Fab-like fragment comprising MSA26Ck and TAR1-5-19CH. Addition of MSA with the Fab-like fragment reduces the level of inhibition. An ELISA plate coated with 1µg/ml TNFα was probed with dual specific V_{*K*} C_{H} and V_{*K*} C_{*K*} Fab like fragment and also with a control TNFα binding dAb at a concentration calculated to give a similar signal on the ELISA. Both the dual specific and control dAb were used to probe the ELISA plate in the presence and in the absence of 2mg/ml MSA. The signal in the dual specific well was reduced by more than 50% but the signal in the dAb well was not reduced at all (see figure 19a). The same dual specific protein was also put into the receptor assay with and without MSA and competition by MSA was also shown (see figure 19c). This demonstrates that binding of MSA to the dual specific is competitive with binding to TNFα.

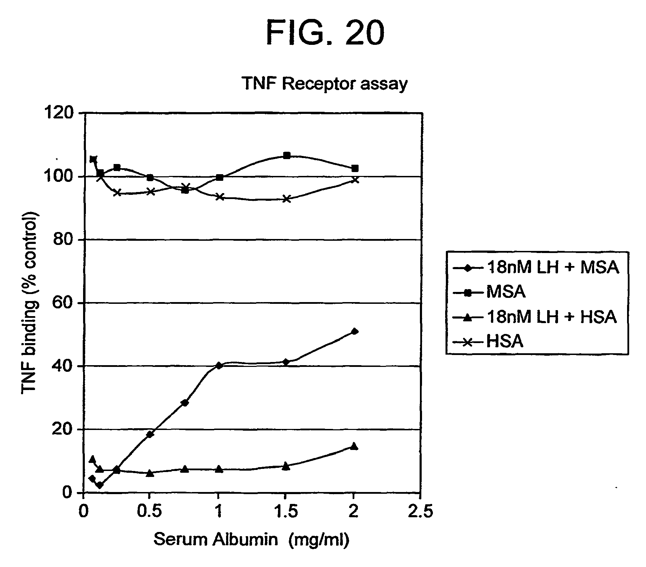

- Figure 20: TNF receptor assay showing inhibiton of TNF binding with a disulphide bonded heterodimer of TAR1-5-19 dAb and MSA16 dAb. Addition of MSA with the dimer reduces the level of inhibiton in a dose dependant manner. The TNF receptor assay (figure 19 (b)) was conducted in the presence of a constant concentration of heterodimer (18nM) and a dilution series of MSA and HSA. The presence of HSA at a range of concentrations (up to 2 mg/ml) did not cause a reduction in the ability of the dimer to inhibit TNFα. However, the addition of MSA caused a dose dependant reduction in the ability of the dimer to inhibit TNFα (figure 19a).This demonstrates that MSA and TNFα compete for binding to the cys bonded TAR1-5-19, MSA16 dimer. MSA and HSA alone did not have an effect on the TNF binding level in the assay.

### Detailed Description of the Invention

### Definitions

Complementary Two immunoglobulin domains are "complementary" where they belong to families of structures which form cognate pairs or groups or are derived from such families and retain this feature. For example, a V_{H} domain and a V_{L} domain of an antibody are complementary; two V_{H} domains are not complementary, and two V_{L} domains are not complementary. Complementary domains may be found in other members of the immunoglobulin superfamily, such as the V_{α} and V_{β} (or γ and δ) domains of the T-cell receptor. In the context of the second configuration of the present invention, non-complementary domains do not bind a target molecule cooperatively, but act independently on different target epitopes which may be on the same or different molecules. Domains which are artificial, such as domains based on protein scaffolds which do not bind epitopes unless engineered to do so, are non-complementary. Likewise, two domains based on (for example) an immunoglobulin domain and a fibronectin domain are not complementary.

**Immunoglobulin** This refers to a family of polypeptides which retain the immunoglobulin fold characteristic of antibody molecules, which contains two β sheets and, usually, a conserved disulphide bond. Members of the immunoglobulin superfamily are involved in many aspects of cellular and non-cellular interactions *in vivo*, including widespread roles in the immune system (for example, antibodies, T-cell receptor molecules and the like), involvement in cell adhesion (for example the ICAM molecules) and intracellular signalling (for example, receptor molecules, such as the PDGF receptor). The present invention is applicable to all immunoglobulin superfamily molecules which possess binding domains. Preferably, the present invention relates to antibodies.

**Combining** Variable domains according to the invention are combined to form a group of domains; for example, complementary domains may be combined, such as V_{L} domains being combined with V_{H} domains. Non-complementary domains may also be combined. Domains may be combined in a number of ways, involving linkage of the domains by covalent or non-covalent means.

**Domain** A domain is a folded protein structure which retains its tertiary structure independently of the rest of the protein. Generally, domains are responsible for discrete functional properties of proteins, and in many cases may be added, removed or transferred to other proteins without loss of function of the remainder of the protein and/or of the domain. By single antibody variable domain is meant a folded polypeptide domain comprising sequences characteristic of antibody variable domains. It therefore includes complete antibody variable domains and modified variable domains, for example in which one or more loops have been replaced by sequences which are not characteristic of antibody variable domains, or antibody variable domains which have been truncated or comprise N- or C-terminal extensions, as well as folded fragments of variable domains which retain at least in part the binding activity and specificity of the full-length domain.

**Repertoire** A collection of diverse variants, for example polypeptide variants which differ in their primary sequence. A library used in the present invention will encompass a repertoire ofpolypeptides comprising at least 1000 members.

**Library** The term library refers to a mixture of heterogeneous polypeptides or nucleic acids. The library is composed of members, each of which have a single polypeptide or nucleic acid sequence. To this extent, *library* is synonymous with *repertoire.* Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. Preferably, each individual organism or cell contains only one or a limited number of library members. Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

A **'closed conformation multi-specific ligand'** describes a multi-specific ligand as herein defined comprising at least two epitope binding domains as herein defined. The term 'closed conformation' (multi-specific ligand) means that the epitope binding domains of the ligand are arranged such that epitope binding by one epitope binding domain competes with epitope binding by another epitope binding domain. That is, cognate epitopes may be bound by each epitope binding domain individually but not simultaneosuly. The closed conformation of the ligand can be achieved using methods herein described.

**Antibody** An antibody (for example IgG, IgM, IgA, IgD or IgE) or fragment (such as a Fab, F(ab')₂, Fv, disulphide linked Fv, scFv, closed conformation multispecific antibody, disulphide-linked scFv, diabody) whether derived from any species naturally producing an antibody, or created by recombinant DNA technology; whether isolated from serum, B-cells, hybridomas, transfectomas, yeast or bacteria).

**Dual-specific ligand** A ligand comprising a first immunoglobulin single variable domain and a second immunoglobulin single variable domain as herein defined, wherein the variable regions are capable of binding to two different antigens or two epitopes on the same antigen which are not normally bound by a monospecific immunoglobulin. For example, the two epitopes may be on the same hapten, but are not the same epitope or sufficiently adjacent to be bound by a monospecific ligand. The dual specific ligands according to the invention are composed of variable domains which have different specificities, and do not contain mutually complementary variable domain pairs which have the same specificity.

**Antigen** A molecule that is bound by a ligand according to the present invention. Typically, antigens are bound by antibody ligands and are capable of raising an antibody response *in vivo*. It may be a polypeptide, protein, nucleic acid or other molecule. Generally, the dual specific ligands according to the invention are selected for target specificity against a particular antigen. In the case of conventional antibodies and fragments thereof, the antibody binding site defined by the variable loops (L1, L2, L3 and H1, H2, H3) is capable of binding to the antigen.

**Epitope** A unit of structure conventionally bound by an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation.

**Generic ligand** A ligand that binds to all members of a repertoire. Generally, not bound through the antigen binding site as defined above. Non-limiting examples include protein A, protein L and protein G.

Selecting Derived by screening, or derived by a Darwinian selection process, in which binding interactions are made between a domain and the antigen or epitope or between an antibody and an antigen or epitope. Thus a first variable domain may be selected for binding to an antigen or epitope in the presence or in the absence of a complementary variable domain.

**Universal framework** A single antibody framework sequence corresponding to the regions of an antibody conserved in sequence as defined by Kabat ("Sequences of Proteins of Immunological Interest", US Department of Health and Human Services) or corresponding to the human germline immunoglobulin repertoire or structure as defined by Chothia and Lesk, (1987) J. Mol. Biol. 196:910-917. The invention provides for the use of a single framework, or a set of such frameworks, which has been found to permit the derivation of virtually any binding specificity though variation in the hypervariable regions alone.

**Half-life** The time taken for the serum concentration of the ligand to reduce by 50%, *in vivo,* for example due to degradation of the ligand and/or clearance or sequestration of the ligand by natural mechanisms. The ligands of the invention are stabilised in *vivo* and their half-life increased by binding to molecules which resist degradation and/or clearance or sequestration. Typically, such molecules are naturally occurring proteins which themselves have a long half-life *in vivo*. The half-life of a ligand is increased if its functional activity persists, *in vivo,* for a longer period than a similar ligand which is not specific for the half-life increasing molecule. Thus, a ligand specific for HSA and a target molecule is compared with the same ligand wherein the specificity for HSA is not present, that it does not bind HSA but binds another molecule. For example, it may bind a second epitope on the target molecule. Typically, the half life is increased by 10%, 20%, 30%, 40%, 50% or more. Increases in the range of 2x, 3x, 4x, 5x, 10x, 20x, 30x, 40x, 50x or more of the half life are possible. Alternatively, or in addition, increases in the range of up to 30x, 40x, 50x, 60x, 70x, 80x, 90x, 100x, 150x of the half life are possible.

**Homogeneous immunoassay** An immunoassay in which analyte is detected without need for a step of separating bound and un-bound reagents.

**Substantially identical (or "substantially homologous")** A first amino acid or nucleotide sequence that contains a sufficient number of identical or equivalent (*e.g.,* with a similar side chain, *e.g.,* conserved amino acid substitutions) amino acid residues or nucleotides to a second amino acid or nucleotide sequence such that the first and second amino acid or nucleotide sequences have similar activities. In the case of antibodies, the second antibody has the same binding specificity and has at least 50% of the affinity of the same.

As used herein, the terms **"low stringency," "medium stringency," "high stringency,"** or **"very high stringency conditions"** describe conditions for nucleic acid hybridization and washing. Guidance for performing hybridization reactions can be found in *Current Protocols in Molecular Biology,* John Wiley & Sons, N.Y. (1989), 6.3.1-6.3.6, which is incorporated herein by reference in its entirety. Aqueous and nonaqueous methods are described in that reference and either can be used. Specific hybridization conditions referred to herein are as follows: (1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); (2) medium stringency hybridization conditions in 6X SSC at about 45°C, followed by one or more washes in 0.2X SSC, 0.1% SDS at 60°C; (3) high stringency hybridization conditions in 6X SSC at about 45°C, followed by one or more washes in 0.2X SSC, 0.1% SDS at 65°C; and preferably (4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

### Detailed Description of the Invention

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods (see generally, Sambrook *et al*., Molecular Cloning: A Laboratory Manual, 2d ed. (1989) Cold Spring Harbor Laboratory Press, Cold Spring Harbor, N.Y. and Ausubel *et al*., Short Protocols in Molecular Biology (1999) 4^{th} Ed, John Wiley & Sons, Inc. and chemical methods.

### Preparation of immunoglobulin based multi-specific ligands

Dual specific ligands according to the invention, whether open or closed in conformation according to the desired configuration of the invention, may be prepared according to previously established techniques, used in the field of antibody engineering, for the preparation of scFv, "phage" antibodies and other engineered antibody molecules. Techniques for the preparation of antibodies, and in particular bispecific antibodies, are for example described in the following reviews and the references cited therein: Winter & Milstein, (1991) Nature 349:293-299; Plueckthun (1992) Immunological Reviews 130:151-188; Wright *et al*., (1992) Crti. Rev. Immunol.12:125-168; Holliger, P. & Winter, G. (1993) Curr. Op. Biotechn. 4, 446-449; Carter, *et al*. (1995) J. Hematother. 4, 463-470; Chester, K.A. & Hawkins, R.E. (1995) Trends Biotechn. 13, 294-300; Hoogenboom, H.R. (1997) Nature Biotechnol. 15, 125-126; Fearon, D. (1997) Nature Biotechnol. 15, 618-619; Plückthun, A. & Pack, P. (1997) Immunotechnology 3, 83-105; Carter, P. & Merchant, A.M. (1997) Curr. Opin. Biotechnol. 8, 449-454; Holliger, P. & Winter, G. (1997) Cancer Immunol. Immunother. 45,128-130.

The invention provides for the selection of variable domains against two different antigens or epitopes, and subsequent combination of the variable domains.

The techniques employed for selection of the variable domains employ libraries and selection procedures which are known in the art. Natural libraries (Marks *et al.* (1991) *J. Mol. Biol.,* **222**: 581; Vaughan *et al*. (1996) *Nature Biotech.,* **14**: 309) which use rearranged V genes harvested from human B cells are well known to those skilled in the art. Synthetic libraries (Hoogenboom & Winter (1992) *J*. *Mol. Biol.,* **227**: 381; Barbas *et al*. (1992) *Proc. Natl. Acad. Sci. USA,* 89: 4457; Nissim *et al*. (1994) *EMBO J*., 13: 692; Griffiths *et al*. (1994) *EMBO J*., 13: 3245; De Kruif *et al*. (1995) *J. Mol. Biol*., **248**: 97) are prepared by cloning immunoglobulin V genes, usually using PCR. Errors in the PCR process can lead to a high degree of randomisation. V_{H} and/or V_{L} libraries may be selected against target antigens or epitopes separately, in which case single domain binding is directly selected for, or together.

A preferred method for making a dual specific ligand according to the present invention comprises using a selection system in which a repertoire of variable domains is selected for binding to a first antigen or epitope and a repertoire of variable domains is selected for binding to a second antigen or epitope. The selected variable first and second variable domains are then combined and the dual-specific ligand selected for binding to both first and second antigen or epitope. Closed conformation ligands are selected for binding both first and second antigen or epitope in isolation but not simultaneously.

### A. Library vector systems

A variety of selection systems are known in the art which are suitable for use in the present invention. Examples of such systems are described below.

Bacteriophage lambda expression systems may be screened directly as bacteriophage plaques or as colonies of lysogens, both as previously described (Huse *et al*. (1989) *Science,* **246:** 1275; Caton and Koprowski (1990) *Proc. Natl. Acad. Sci. U.S.A*., **87;** Mullinax *et al.* (1990) *Proc. Natl. Acad. Sci. U.S.A*., **87:** 8095; Persson *et al*. (1991) *Proc. Natl. Acad Sci. U.S.A*., **88:** 2432) and are of use in the invention. Whilst such expression systems can be used to screen up to 10⁶ different members of a library, they are not really suited to screening of larger numbers (greater than 10⁶ members).

Of particular use in the construction of libraries are selection display systems, which enable a nucleic acid to be linked to the polypeptide it expresses. As used herein, a selection display system is a system that permits the selection, by suitable display means, of the individual members of the library by binding the generic and/or target ligands.

Selection protocols for isolating desired members of large libraries are known in the art, as typified by phage display techniques. Such systems, in which diverse peptide sequences are displayed on the surface of filamentous bacteriophage (Scott and Smith (1990) *Science,* **249**: 386), have proven useful for creating libraries of antibody fragments (and the nucleotide sequences that encoding them) for the *in vitro* selection and amplification of specific antibody fragments that bind a target antigen (McCafferty *et al.,* WO 92/01047). The nucleotide sequences encoding the V_{H} and V_{L} regions are linked to gene fragments which encode leader signals that direct them to the periplasmic space of *E. coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encode the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty *et al*. (1990) *Nature,* **348:** 552; Kang *et al*. (1991) *Proc. Natl. Acad. Sci. U.S.A*., **88:** 4363; Clackson *et al.* (1991) *Nature,* **352:** 624; Lowman *et al.* (1991) *Biochemistry,* **30:** 10832; Burton *et al.* (1991) *Proc. Natl. Acad. Sci U.S.A*., **88:** 10134; Hoogenboom *et al.* (1991) Nucleic Acids Res., **19:** 4133; Chang *et al.* (1991) *J. Immunol*., **147:** 3610; Breitling *et al*. (1991) *Gene,* **104:** 147; Marks *et al*. (1991) supra; Barbas *et al.* (1992) supra; Hawkins and Winter (1992) *J. Immunol.,* **22**: 867; Marks *et al.,* 1992, *J. Biol. Chem.,* **267:** 16007; Lemer *et al.* (1992) *Science,* **258:** 1313.

One particularly advantageous approach has been the use of scFv phage-libraries (Huston *et al*., 1988, Proc. Natl. Acad. Sci U.S.A., 85: 5879-5883; Chaudhary *et al.* (1990) Proc. Natl. Acad. Sci U.S.A., 87: 1066-1070; McCafferty *et al*. (1990) supra; Clackson *et al.* (1991) *Nature,* **352:** 624; Marks *et al*. (1991) *J. Mol. Biol*., **222:** 581; Chiswell *et al.* (1992) Trends Biotech., 10: 80; Marks *et al.* (1992) *J. Biol. Chem*., **267**). Various embodiments of scFv libraries displayed on bacteriophage coat proteins have been described. Refinements of phage display approaches are also known, for example as described in WO96/06213 and WO92/01047 (Medical Research Council *et al*.) and WO97/08320 (Morphosys).

Other systems for generating libraries of polypeptides involve the use of cell-free enzymatic machinery for the *in vitro* synthesis of the library members. In one method, RNA molecules are selected by alternate rounds of selection against a target ligand and PCR amplification (Tuerk and Gold (1990) *Science,* **249**: 505; Ellington and Szostak (1990) *Nature,* **346**: 818). A similar technique may be used to identify DNA sequences which bind a predetermined human transcription factor (Thiesen and Bach (1990) *Nucleic Acids Res.,* **18**: 3203; Beaudry and Joyce (1992) *Science,* **257**: 635; WO92/05258 and WO92/14843). In a similar way, *in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO92/02536. Alternative display systems which are not phage-based, such as those disclosed in WO95/22625 and WO95/11922 (Affymax) use the polysomes to display polypeptides for selection.

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671, WO00/40712 and Tawfik & Griffiths (1998) *Nature Biotechnol* 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

### B. Library Construction.

Libraries intended for selection, may be constructed using techniques known in the art, for example as set forth above, or may be purchased from commercial sources. Libraries which are useful in the present invention are described, for example, in WO99/20749. Once a vector system is chosen and one or more nucleic acid sequences encoding polypeptides of interest are cloned into the library vector, one may generate diversity within the cloned molecules by undertaking mutagenesis prior to expression; alternatively, the encoded proteins may be expressed and selected, as described above, before mutagenesis and additional rounds of selection are performed. Mutagenesis of nucleic acid sequences encoding structurally optimised polypeptides is carried out by standard molecular methods. Of particular use is the polymerase chain reaction, or PCR, (Mullis and Faloona (1987) *Methods Enzymol*., **155:** 335, herein incorporated by reference). PCR, which uses multiple cycles of DNA replication catalysed by a thermostable, DNA-dependent DNA polymerase to amplify the target sequence of interest, is well known in the art. The construction of various antibody libraries has been discussed in Winter *et al.* (1994) Ann. Rev. Immunology 12, 433-55, and references cited therein.

PCR is performed using template DNA (at least lfg; more usefully, 1-1000 ng) and at least 25 pmol of oligonucleotide primers; it may be advantageous to use a larger amount of primer when the primer pool is heavily heterogeneous, as each sequence is represented by only a small fraction of the molecules of the pool, and amounts become limiting in the later amplification cycles. A typical reaction mixture includes: 2µl of DNA, 25 pmol of oligonucleotide primer, 2.5 µl of 10X PCR buffer 1 (Perkin-Elmer, Foster City, CA), 0.4 µl of 1.25 µM dNTP, 0.15 µl (or 2.5 units) of Taq DNA polymerase (Perkin Elmer, Foster City, CA) and deionized water to a total volume of 25 µl. Mineral oil is overlaid and the PCR is performed using a programmable thermal cycler. The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenised, mismatch is required, at least in the first round of synthesis. The ability to optimise the stringency of primer annealing conditions is well within the knowledge of one of moderate skill in the art. An annealing temperature of between 30 °C and 72 °C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above; 1-2 minutes), and extension (72°C for 1-5 minutes, depending on the length of the amplified product). Final extension is generally for 4 minutes at 72°C, and may be followed by an indefinite (0-24 hour) step at 4°C.

### C. Combining single variable domains

Domains useful in the invention, once selected, may be combined by a variety of methods known in the art, including covalent and non-covalent methods.

Preferred methods include the use of polypeptide linkers, as described, for example, in connection with scFv molecules (Bird *et al*., (1988) Science 242:423-426). Discussion of suitable linkers is provided in *Bird et al.* Science 242, 423-426; *Hudson et al*, Journal Immunol Methods 231 (1999) 177-189; *Hudson et al*, Proc Nat Acad Sci USA 85, 5879-5883. Linkers are preferably flexible, allowing the two single domains to interact. One linker example is a (Gly₄ Ser)ₙ linker, where n=1 to 8, eg, 2, 3, 4, 5 or 7. The linkers used in diabodies, which are less flexible, may also be employed (Holliger *et al*., (1993) PNAS (USA) 90:6444-6448).

In one embodiment, the linker employed is not an immunoglobulin hinge region.

Variable domains may be combined using methods other than linkers. For example, the use of disulphide bridges, provided through naturally-occurring or engineered cysteine residues, may be exploited to stabilise V_{H}-V_{H},V_{L}-V_{L} or V_{H}-V_{L} dimers (Reiter *et al*., (1994) Protein Eng. 7:697-704) or by remodelling the interface between the variable domains to improve the "fit" and thus the stability of interaction (Ridgeway *et al*., (1996) Protein Eng. 7:617-621; Zhu *et al*., (1997) Protein Science 6:781-788).

Other techniques for joining or stabilising variable domains of immunoglobulins, and in particular antibody V_{H} domains, may be employed as appropriate.

In accordance with the present invention, dual specific ligands can be in "closed" conformations in solution. A "closed" configuration is that in which the two domains (for example V_{H} and V_{L}) are present in associated form, such as that of an associated V_{H}-V_{L} pair which forms an antibody binding site. For example, scFv may be in a closed conformation, depending on the arrangement of the linker used to link the V_{H} and V_{L} domains. If this is sufficiently flexible to allow the domains to associate, or rigidly holds them in the associated position, it is likely that the domains will adopt a closed conformation.

Similarly, V_{H} domain pairs and V_{L} domain pairs may exist in a closed conformation. Generally, this will be a function of close association of the domains, such as by a rigid linker, in the ligand molecule. Ligands in a closed conformation will be unable to bind both the molecule which increases the half-life of the ligand and a second target molecule. Thus, the ligand will typically only bind the second target molecule on dissociation from the molecule which increases the half-life of the ligand.

Moreover, the construction of V_{H}/V_{H}, V_{L}/V_{L} or V_{H}/V_{L} dimers without linkers provides for competition between the domains.

Ligands according to the invention may moreover be in an open conformation. In such a conformation, the ligands will be able to simultaneously bind both the molecule which increases the half-life of the ligand and the second target molecule. Typically, variable domains in an open configuration are (in the case of V_{H}-V_{L} pairs) held far enough apart for the domains not to interact and form an antibody binding site and not to compete for binding to their respective epitopes. In the case of V_{H}/V_{H} or V_{L}/V_{L} dimers, the domains are not forced together by rigid linkers. Naturally, such domain pairings will not compete for antigen binding or form an antibody binding site.

Fab fragments and whole antibodies will exist primarily in the closed conformation, although it will be appreciated that open and closed dual specific ligands are likely to exist in a variety of equilibria under different circumstances. Binding of the ligand to a target is likely to shift the balance of the equilibrium towards the open configuration. Thus, certain ligands according to the invention can exist in two conformations in solution, one of which (the open form) can bind two antigens or epitopes independently, whilst the alternative conformation (the closed form) can only bind one antigen or epitope; antigens or epitopes thus compete for binding to the ligand in this conformation.

Although the open form of the dual specific ligand may thus exist in equilibrium with the closed form in solution, it is envisaged that the equilibrium will favour the closed form; moreover, the open form can be sequestered by target binding into a closed conformation. Preferably, therefore, certain dual specific ligands of the invention are present in an equilibrium between two (open and closed) conformations.

Dual specific ligands according to the invention may be modified in order to favour an open or closed conformation. For example, stabilisation of V_{H}-V_{L} interactions with disulphide bonds stabilises the closed conformation. Moreover, linkers used to join the domains, including V_{H} domain and V_{L} domain pairs, may be constructed such that the open from is favoured; for example, the linkers may sterically hinder the association of the domains, such as by incorporation of large amino acid residues in opportune locations, or the designing of a suitable rigid structure which will keep the domains physically spaced apart.

### D. Characterisation of the dual-specific ligand.

The binding of the dual-specific ligand to its specific antigens or epitopes can be tested by methods which will be familiar to those skilled in the art and include ELISA. In a preferred embodiment of the invention binding is tested using monoclonal phage BLISA.

Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter *et al.* (1994) Ann. Rev. Immunology 12, 433-55 and references cited therein.

The diversity of the selected phage monoclonal antibodies may also be assessed by gel electrophoresis of PCR products (Marks *et al.* 1991, *supra*; Nissim *et al.* 1994 *supra*), probing (Tomlinson *et al*., 1992) J. Mol. Biol. 227, 776) or by sequencing of the vector DNA.

### E. Structure of 'Dual-specific ligands'.

As described above, an antibody is herein defined as an antibody (for example IgG, IgM, IgA, IgA, IgE) or fragment (Fab, Fv, disulphide linked Fv, scFv, diabody) which comprises at least one heavy and a light chain variable domain, at least two heavy chain variable domains or at least two light chain variable domains. It may be at least partly derived from any species naturally producing an antibody, or created by recombinant DNA technology; whether isolated from serum, B-cells, hybridomas, transfectomas, yeast or bacteria).

In a preferred embodiment of the invention the dual-specific ligand comprises at least one single heavy chain variable domain of an antibody and one single light chain variable domain of an antibody, or two single heavy or light chain variable domains. For example, the ligand may comprise a V_{H}/V_{L} pair, a pair of V_{H} domains or a pair of V_{L} domains.

The first and the second variable domains of such a ligand may be on the same polypeptide chain. Alternatively they may be on separate polypeptide chains. In the case that they are on the same polypeptide chain they may be linked by a linker, which is preferentially a peptide sequence, as described above.

The first and second variable domains may be covalently or non-covalently associated. In the case that they are covalently associated, the covalent bonds may be disulphide bonds.

In the case that the variable domains are selected from V-gene repertoires selected for instance using phage display technology as herein described, then these variable domains comprise a universal framework region, such that is they may be recognised by a specific generic ligand as herein defined. The use of universal frameworks, generic ligands and the like is described in WO99/20749.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary pair. DNA shuffling is known in the art and taught, for example, by Stemmer, 1994, Nature 370: 389-391 and U.S. Patent No. 6,297,053, both of which are incorporated herein by reference. Other methods of mutagenesis are well known to those of skill in the art.

In a preferred embodiment of the invention the 'dual-specific ligand' is a single chain Fv fragment. In an alternative embodiment of the invention, the 'dual-specific ligand' consists of a Fab format.

In a further aspect, the present invention provides nucleic acid encoding at least a 'dual-specific ligand' as herein defined.

One skilled in the art will appreciate that, depending on the aspect of the invention, both antigens or epitopes may bind simultaneously to the same antibody molecule. Alternatively, they may compete for binding to the same antibody molecule. For example, where both epitopes are bound simultaneously, both variable domains of a dual specific ligand are able to independently bind their target epitopes. Where the domains compete, the one variable domain is capable of binding its target, but not at the same time as the other variable domain binds its cognate target; or the first variable domain is capable of binding its target, but not at the same time as the second variable domain binds its cognate target.

The variable regions may be derived from antibodies directed against target antigens or epitopes. Alternatively they may be derived from a repertoire of single antibody domains such as those expressed on the surface of filamentous bacteriophage. Selection may be performed as described below.

In general, the nucleic acid molecules and vector constructs required for the performance of the present invention may be constructed and manipulated as set forth in standard laboratory manuals, such as Sambrook *et al*. (1989) *Molecular Cloning: A Laboratory Manual,* Cold Spring Harbor, USA.

The manipulation of nucleic acids useful in the present invention is typically carried out in recombinant vectors.

Thus in a further aspect, the present invention provides a vector comprising nucleic acid encoding at least a 'dual-specific ligand' as herein defined.

As used herein, vector refers to a discrete element that is used to introduce heterologous DNA into cells for the expression and/or replication thereof. Methods by which to select or construct and, subsequently, use such vectors are well known to one of ordinary skill in the art. Numerous vectors are publicly available, including bacterial plasmids, bacteriophage, artificial chromosomes and episomal vectors. Such vectors may be used for simple cloning and mutagenesis; alternatively gene expression vector is employed. A vector of use according to the invention may be selected to accommodate a polypeptide coding sequence of a desired size, typically from 0.25 kilobase (kb) to 40 kb or more in length A suitable host cell is transformed with the vector after *in vitro* cloning manipulations. Each vector contains various functional components, which generally include a cloning (or "polylinker") site, an origin of replication and at least one selectable marker gene. If given vector is an expression vector, it additionally possesses one or more of the following: enhancer element, promoter, transcription termination and signal sequences, each positioned in the vicinity of the cloning site, such that they are operatively linked to the gene encoding a ligand according to the invention.

Both cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses.

The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are used in mammalian cells able to replicate high levels of DNA, such as COS cells.

Advantageously, a cloning or expression vector may contain a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. arnpicillin, neomycin, methotrexate or tetracycline, complement auxotrophic deficiencies, or supply critical nutrients not available in the growth media.

Since the replication of vectors encoding a ligand according to the present invention is most conveniently performed in *E. coli,* an *E. coli*-selectable marker, for example, the β-lactamase gene that confers resistance to the antibiotic ampicillin, is of use. These can be obtained from *E. coli* plasmids, such as pBR322 or a pUC plasmid such as pUC18 or pUC19.

Expression vectors usually contain a promoter that is recognised by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Delgarno sequence operably linked to the coding sequence.

The preferred vectors are expression vectors that enables the expression of a nucleotide sequence corresponding to a polypeptide library member. Thus, selection with the first and/or second antigen or epitope can be performed by separate propagation and expression of a single clone expressing the polypeptide library member or by use of any selection display system. As described above, the preferred selection display system is bacteriophage display. Thus, phage or phagemid vectors may be used, eg pIT1 or pIT2. Leader sequences useful in the invention include pelB, stII, ompA, phoA, bla and peIA. One example are phagemid vectors which have an *E. coli.* origin of replication (for double stranded replication) and also a phage origin of replication (for production of single-stranded DNA). The manipulation and expression of such vectors is well known in the art (Hoogenboom and Winter (1992) supra; Nssim *et al.* (1994) supra). Briefly, the vector contains a β-lactamase gene to confer selectivity on the phagemid and a lac promoter upstream of a expression cassette that consists (N to C terminal) of a pelB leader sequence (which directs the expressed polypeptide to the periplasmic space), a multiple cloning site (for cloning the nucleotide version of the library member), optionally, one or more peptide tag (for detection), optionally, one or more TAG stop codon and the phage protein pIII. Thus, using various suppressor and non-suppressor strains of *E. coli* and with the addition of glucose, iso-propyl thio-β-D-galactoside (IPTG) or a helper phage, such as VCS M13, the vector is able to replicate as a plasmid with no expression, produce large quantities of the polypeptide library member only or produce phage, some of which contain at least one copy of the polypeptide-pIII fusion on their surface.

Construction of vectors encoding ligands according to the invention employs conventional ligation techniques. Isolated vectors or DNA fragments are cleaved, tailored, and religated in the form desired to generate the required vector. If desired, analysis to confirm that the correct sequences are present in the constructed vector can be performed in a known fashion. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein, *in situ* hybridisation, immunocytochemistry or sequence analysis of nucleic acid or protein molecules. Those skilled in the art will readily envisage how these methods may be modified, if desired.

### Structure of closed conformation multispecific ligands

According to one aspect of the present invention, the two or more non-complementary epitope binding domains are linked so that they are in a closed conformation as herein defined. Advantageously, they may be further attached to a skeleton which may, as a alternative, or on addition to a linker described herein, facilitate the formation and/or maintenance of the closed conformation of the epitope binding sites with respect to one another.

### (I) Skeletons

Skeletons may be based on immunoglobulin molecules or may be non-immunoglobulin in origin as set forth above. Preferred immunoglobulin skeletons as herein defined includes any one or more of those selected from the following: an immunoglobulin molecule comprising at least (i) the CL (kappa or lambda subclass) domain of an antibody; or (ii) the CH1 domain of an antibody heavy chain; an immunoglobulin molecule comprising the CH1 and CH2 domains of an antibody heavy chain; an immunoglobulin molecule comprising the CH1, CH2 and CH3 domains of an antibody heavy chain; or any of the subset (ii) in conjunction with the CL (kappa or lambda subclass) domain of an antibody. A hinge region domain may also be included.. Such combinations of domains may, for example, mimic natural antibodies, such as IgG or IgM, or fragments thereof, such as Fv, scFv, Fab or F(ab')₂ molecules. Those skilled in the art will be aware that this list is not intended to be exhaustive.

### (II) Protein scaffolds

Each epitope binding domain comprises a protein scaffold and one or more CDRs which are involved in the specific interaction of the domain with one or more epitopes. Advantageously, an epitope binding domain according to the present invention comprises three CDRs. Suitable protein scaffolds include any of those selected from the group consisting of the following: those based on immunoglobulin domains, those based on fibronectin, those based on affibodies, those based on CTLA4, those based on chaperones such as GroEL, those based on lipocallin and those based on the bacterial Fc receptors SpA and SpD. Those skilled in the art will appreciate that this list is not intended to be exhaustive.

### F: Scaffolds for use in Constructing Dual Specific Ligands

### i. Selection of the main-chain conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) *J. Mol. Biol*., **196:** 901; Chothia *et al*. (1989) *Nature*, **342:** 877). Analysis of loop lengths and key residues has therefore enabled prediction of the main-chain conformations of H1, H2, L1, L2 and L3 found in the majority of human antibodies (Chothia *et al.* (1992) *J. Mol. Biol.,* **227:** 799; Tomlinson *et al.* (1995) *EMBO J*., 14: 4628; Williams *et al.* (1996) *J. Mol. Biol*., **264:** 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin *et al*. (1996) *J. Mol. Biol*., **263:** 800; Shirai *et al*. (1996) *FEBS Letters,* **399:** 1).

The dual specific ligands of the present invention are advantageously assembled from libraries of domains, such as libraries of V_{H} domains and/or libraries of V_{L} domains. Moreover, the dual specific ligands of the invention may themselves be provided in the form of libraries. In one aspect of the present invention, libraries of dual specific ligands and/or domains are designed in which certain loop lengths and key residues have been chosen to ensure that the main-chain conformation of the members is known. Advantageously, these are real conformations of immunoglobulin superfamily molecules found in nature, to minimise the chances that they are non-functional, as discussed above. Germline V gene segments serve as one suitable basic framework for constructing antibody or T-cell receptor libraries; other sequences are also of use. Variations may occur at a low frequency, such that a small number of functional members may possess an altered main-chain conformation, which does not affect its function.

Canonical structure theory is also of use to assess the number of different main-chain conformations encoded by ligands, to predict the main-chain conformation based on ligand sequences and to chose residues for diversification which do not affect the canonical structure. It is known that, in the human Vₖ domain, the L1 loop can adopt one of four canonical structures, the L2 loop has a single canonical structure and that 90% of human V_{κ} domains adopt one of four or five canonical structures for the L3 loop (Tomlinson *et al.* (1995) supra); thus, in the V_{κ} domain alone, different canonical structures can combine to create a range of different main-chain conformations. Given that the V_{λ} domain encodes a different range of canonical structures for the L1, L2 and L3 loops and that V_{κ} and V_{λ} domains can pair with any V_{H} domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in a preferred aspect, the dual-specific ligands of the invention possess a single known main-chain conformation.

The single main-chain conformation that is chosen is preferably commonplace among molecules of the immunoglobulin superfamily type in question. A conformation is commonplace when a significant number of naturally occurring molecules are observed to adopt it. Accordingly, in a preferred aspect of the invention, the natural occurrence of the different main-chain conformations for each binding loop of an immunoglobulin domain are considered separately and then a naturally occurring variable domain is chosen which possesses the desired combination of main-chain conformations for the different loops. If none is available, the nearest equivalent may be chosen. It is preferable that the desired combination of main-chain conformations for the different loops is created by selecting germline gene segments which encode the desired main-chain conformations. It is more preferable, that the selected germline gene segments are frequently expressed in nature, and most preferable that they are the most frequently expressed of all natural germline gene segments.

In designing dual specific ligands or libraries thereof the incidence of the different main-chain conformations for each of the six antigen binding loops may be considered separately. For H1, H2, L1, L2 and L3, a given conformation that is adopted by between 20% and 100% of the antigen binding loops of naturally occurring molecules is chosen. Typically, its observed incidence is above 35% (i.e. between 35% and 100%) and, ideally, above 50% or even above 65%. Since the vast majority of H3 loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1 - CS 1 (79% of the expressed repertoire), H2 - CS 3 (46%), L1 - CS 2 of V_{κ} (39%), L2 - CS 1 (100%), L3 - CS 1 of V_{κ} (36%) (calculation assumes a κ:λ ratio of 70:30, Hood *et al.* (1967) *Cold Spring Harbor Symp. Quant. Biol*., **48**: 133). For H3 loops that have canonical structures, a CDR3 length (Kabat *et al.* (1991) *Sequences of proteins of immunological interest,* U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modelling (2cgr and 1tet). The most frequently expressed germline gene segments that this combination of canonical structures are the V_{H} segment 3-23 (DP-47), the J_{H} segment JH4b, the V_{κ} segment 02/012 (DPK9) and the J_{κ} segment J_{κ}l. V_{H} segments DP45 and DP38 are also suitable. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is used as the basis for choosing the single main-chain conformation. In the case of antibodies, for example, the natural occurrence of canonical structure combinations for any two, three, four, five or for all six of the antigen binding loops can be determined. Here, it is preferable that the chosen conformation is commonplace in naturally occurring antibodies and most preferable that it observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

### ii. Diversification of the canonical sequence

Having selected several known main-chain conformations or, preferably a single known main-chain conformation, dual specific ligands according to the invention or libraries for use in the invention can be constructed by varying the binding site of the molecule in order to generate a repertoire with structural and/or functional diversity. This means that variants are generated such that they possess sufficient diversity in their structure and/or in their function so that they are capable of providing a range of activities.

The desired diversity is typically generated by varying the selected molecule at one or more positions. The positions to be changed can be chosen at random or are preferably selected. The variation can then be achieved either by randomisation, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error-prone PCR (Hawkins *et al*. (1992) *J. Mol. BioL,* 226: 889), chemical mutagenesis (Deng *et al.* (1994) *J. Biol. Chem*., **269**: 9533) or bacterial mutator strains (Low *et al.* (1996) *J. Mol. Biol*., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas *et al*. (1992) *Proc. Natl. Acad. Sci. USA,* **89**: 4457). Random or semi-random H3 and L3 regions have been appended to germline V gene segments to produce large libraries with unmutated framework regions (Hoogenboom & Winter (1992) *J. Mol. Biol.,* **227**: 381; Barbas *et al*. (1992) *Proc. Natl. Acad Sci. USA,* **89**: 4457; Nissim *et al.* (1994) *EMBO J*., **13**: 692; Griffiths *et al.* (1994) *EMBO J.,* **13**: 3245; De Kruif *et al.* (1995) *J. Mol. Biol*., **248**: 97). Such diversification has been extended to include some or all of the other antigen binding loops (Crameri *et al*. (1996) *Nature Med*., **2**: 100; Riechmann *et al*. (1995) *Bio*/*Technology*, **13**: 475; Morphosys, WO97/08320, supra).

Since loop randomisation has the potential to create approximately more than 10¹⁵ structures for H3 alone and a similarly large number of variants for the other five loops, it is not feasible using current transformation technology or even by using cell free systems to produce a library representing all possible combinations. For example, in one of the largest libraries constructed to date, 6 x 10¹⁰ different antibodies, which is only a fraction of the potential diversity for a library of this design, were generated (Griffiths *et al*. (1994) supra).

In a preferred embodiment, only those residues which are directly involved in creating or modifying the desired function of the molecule are diversified. For many molecules, the function will be to bind a target and therefore diversity should be concentrated in the target binding site, while avoiding changing residues which are crucial to the overall packing of the molecule or to maintaining the chosen main-chain conformation.

### Diversification of the canonical sequence as it applies to antibody domains

In the case of antibody dual-specific ligands, the binding site for the target is most often the antigen binding site. Thus, in a highly preferred aspect, the invention provides libraries of or for the assembly of antibody dual-specific ligands in which only those residues in the antigen binding site are varied. These residues are extremely diverse in the human antibody repertoire and are known to make contacts in high-resolution antibody/antigen complexes. For example, in L2 it is known that positions 50 and 53 are diverse in naturally occurring antibodies and are observed to make contact with the antigen. In contrast, the conventional approach would have been to diversify all the residues in the corresponding Complementarity Determining Region (CDR1) as defined by Kabat *et al.* (1991, supra), some seven residues compared to the two diversified in the library for use according to the invention. This represents a significant improvement in terms of the functional diversity required to create a range of antigen binding specificities.

In nature, antibody diversity is the result of two processes: somatic recombination of germline V, D and J gene segments to create a naive primary repertoire (so called germline and junctional diversity) and somatic hypermutation of the resulting rearranged V genes. Analysis of human antibody sequences has shown that diversity in the primary repertoire is focused at the centre of the antigen binding site whereas somatic hypermutation spreads diversity to regions at the periphery of the antigen binding site that are highly conserved in the primary repertoire (see Tomlinson *et al.* (1996) *J. Mol. Biol.,* **256**: 813). This complementarity has probably evolved as an efficient strategy for searching sequence space and, although apparently unique to antibodies, it can easily be applied to other polypeptide repertoires. The residues which are varied are a subset of those that form the binding site for the target. Different (including overlapping) subsets of residues in the target binding site are diversified at different stages during selection, if desired.

In the case of an antibody repertoire, an initial 'naive' repertoire is created where some, but not all, of the residues in the antigen binding site are diversified. As used herein in this context, the term "naive" refers to antibody molecules that have no pre-determined target. These molecules resemble those which are encoded by the immunoglobulin genes of an individual who has not undergone immune diversification, as is the case with fetal and newborn individuals, whose immune systems have not yet been challenged by a wide variety of antigenic stimuli. This repertoire is then selected against a range of antigens or epitopes. If required, further diversity can then be introduced outside the region diversified in the initial repertoire. This matured repertoire can be selected for modified function, specificity or affinity.

The invention provides two different naive repertoires of binding domains for the construction of dual specific ligands, or a naïve library of dual specific ligands, in which some or all of the residues in the antigen binding site are varied. The "primary" library mimics the natural primary repertoire, with diversity restricted to residues at the centre of the antigen binding site that are diverse in the germline V gene segments (germline diversity) or diversified during the recombination process (junctional diversity). Those residues which are diversified include, but are not limited to, H50, H52, H52a, H53, H55, H56, H58, H95, H96, H97, H98, L50, L53, L91, L92, L93, L94 and L96. In the "somatic" library, diversity is restricted to residues that are diversified during the recombination process (junctional diversity) or are highly somatically mutated). Those residues which are diversified include, but are not limited to: H31, H33, H35, H95, H96, H97, H98, L30, L31, L32, L34 and L96. All the residues listed above as suitable for diversification in these libraries are known to make contacts in one or more antibody-antigen complexes. Since in both libraries, not all of the residues in the antigen binding site are varied, additional diversity is incorporated during selection by varying the remaining residues, if it is desired to do so. It shall be apparent to one skilled in the art that any subset of any of these residues (or additional residues which comprise the antigen binding site) can be used for the initial and/or subsequent diversification of the antigen binding site.

In the construction of libraries for use in the invention, diversification of chosen positions is typically achieved at the nucleic acid level, by altering the coding sequence which specifies the sequence of the polypeptide such that a number of possible amino acids (all 20 or a subset thereof) can be incorporated at that position. Using the IUPAC nomenclature, the most versatile codon is NNK, which encodes all amino acids as well as the TAG stop codon. The NNK codon is preferably used in order to introduce the required diversity. Other codons which achieve the same ends are also of use, including the NNN codon, which leads to the production of the additional stop codons TGA and TAA.

A feature of side-chain diversity in the antigen binding site of human antibodies is a pronounced bias which favours certain amino acid residues. If the amino acid composition of the ten most diverse positions in each of the V_{H}, V_{κ} and V_{λ} regions are summed, more than 76% of the side-chain diversity comes from only seven different residues, these being, serine (24%), tyrosine (14%), asparagine (11%), glycine (9%), alanine (7%), aspartate (6%) and threonine (6%). This bias towards hydrophilic residues and small residues which can provide main-chain flexibility probably reflects the evolution of surfaces which are predisposed to binding a wide range of antigens or epitopes and may help to explain the required promiscuity of antibodies in the primary repertoire.

Since it is preferable to mimic this distribution of amino acids, the distribution of amino acids at the positions to be varied preferably mimics that seen in the antigen binding site of antibodies. Such bias in the substitution of amino acids that permits selection of certain polypeptides (not just antibody polypeptides) against a range of target antigens is easily applied to any polypeptide repertoire. There are various methods for biasing the amino acid distribution at the position to be varied (including the use of tri-nucleotide mutagenesis, see WO97/08320), of which the preferred method, due to ease of synthesis, is the use of conventional degenerate codons. By comparing the amino acid profile encoded by all combinations of degenerate codons (with single, double, triple and quadruple degeneracy in equal ratios at each position) with the natural amino acid use it is possible to calculate the most representative codon. The codons (AGT)(AGC)T, (AGT)(AGC)C and (AGT)(AGC)(CT) - that is, DVT, DVC and DVY, respectively using IUPAC nomenclature - are those closest to the desired amino acid profile: they encode 22% serine and 11% tyrosine, asparagine, glycine, alanine, aspartate, threonine and cysteine. Preferably, therefore, libraries are constructed using either the DVT, DVC or DVY codon at each of the diversified positions.

### G: Antigens capable of increasing ligand half-life

The dual specific ligands according to the invention are capable of binding to one or more molecules which can increase the half-life of the ligand *in vivo*. Typically, such molecules are polypeptides which occur naturally *in vivo* and which resist degradation or removal by endogenous mechanisms which remove unwanted material from the organism. For example, the molecule which increases the half-life of the organism may be selected from the following:

Proteins from the extracellular matrix; for example collagen, laminins, integrins and fibronectin. Collagens are the major proteins of the extracellular matrix. About 15 types of collagen molecules are currently known, found in different parts of the body, eg type I collagen (accounting for 90% of body collagen) found in bone, skin, tendon, ligaments, cornea, internal organs or type II collagen found in cartilage, invertebral disc, notochord, vitreous humour of the eye.

Proteins found in blood, including:
Plasma proteins such as fibrin, α-2 macroglobulin, serum albumin, fibrinogen A, fibrinogen B, serum amyloid protein A, heptaglobin, profilin, ubiquitin, uteroglobulin and β-2-microglobulin;
Enzymes and inhibitors such as plasminogen, lysozyme, cystatin C, alpha-1-antitrypsin and pancreatic trypsin inhibitor. Plasminogen is the inactive precursor of the trypsin-like serine protease plasmin. It is normally found circulating through the blood stream. When plasminogen becomes activated and is converted to plasmin, it unfolds a potent enzymatic domain that dissolves the fibrinogen fibers that entgangle the blood cells in a blood clot. This is called fibrinolysis.

Immune system proteins, such as IgE, IgG, IgM.

Transport proteins such as retinol binding protein, α-1 microglobulin.

Defensins such as beta-defensin 1, Neutrophil defensins 1,2 and 3.

Proteins found at the blood brain barrier or in neural tissues, such as melanocortin receptor, myelin, ascorbate transporter.

Transferrin receptor specific ligand-neuropharmaceutical agent fusion proteins (see US5977307);
brain capillary endothelial cell receptor, transferrin, transferrin receptor, insulin, insulin-like growth factor 1 (IGF 1) receptor, insulin-like growth factor 2 (IGF 2) receptor, insulin receptor.

Proteins localised to the kidney, such as polycystin, type IV collagen, organic anion transporter K1, Heymann's antigen.

Proteins localised to the liver, for example alcohol dehydrogenase, G250.

Blood coagulation factor X
α1 antitrypsin
HNF 1α

Proteins localised to the lung, such as secretory component (binds IgA).

Proteins localised to the Heart, for example HSP 27. This is associated with dilated cardiomyopathy.

Proteins localised to the skin, for example keratin.

Bone specific proteins, such as bone morphogenic proteins (BMPs), which are a subset of the transforming growth factor β superfamily that demonstrate osteogenic activity. Examples include BMP-2, -4, -5, -6, -7 (also referred to as osteogenic protein (OP-I) and -8 (OP-2).

Tumour specific proteins, including human trophoblast antigen, herceptin receptor, oestrogen receptor, cathepsins eg cathepsin B (found in liver and spleen).

Disease-specific proteins, such as antigens expressed only on activated T-cells: including LAG-3 (lymphocyte activation gene), osteoprotegerin ligand (OPGL) see *Nature* **402**, 304-309; 1999, OX40 (a member of the TNF receptor family, expressed on activated T cells and the only costimulatory T cell molecule known to be specifically up-regulated in human T cell leukaemia virus type-I (HTLV-I)-producing cells.) See *J Immunol*. *2000 Jul 1;165(1):263-70*; Metalloproteases (associated with arthritis/cancers), including CG6512 Drosophila, human paraplegin, human FtsH, human AFG3L2, murine ftsH; angiogenic growth factors, including acidic fibroblast growth factor (FGF-1), basic fibroblast growth factor (FGF-2), Vascular endothelial growth factor / vascular permeability factor (VEGF/VPF), transforming growth factor-a (TGF a), tumor necrosis factor-alpha (TNF-α), angiogenin, interleukin-3 (IL-3), interleukin-8 (IL-8), platelet-derived endothelial growth factor (PD-ECGF), placental growth factor (PIGF), midkine platelet-derived growth factor-BB (PDGF), fractalkine.

### Stress proteins (heat shock proteins)

HSPs are normally found intracellularly. When they are found extracellularly, it is an indicator that a cell has died and spilled out its contents. This unprogrammed cell death (necrosis) only occurs when as a result of trauma, disease or injury and therefore *in vivo*, extracellular HSPs trigger a response from the immune system that will fight infection and disease. A dual specific which binds to extracellular HSP can be localised to a disease site:

### Proteins involved in Fc transport

### Brambell receptor (also known as FcRB)

This Fc receptor has two functions, both of which are potentially useful for delivery
The functions are
(1) The transport of IgG from mother to child across the placenta
(2) the protection of IgG from degradation thereby prolonging its serum half life of IgG. It is thought that the receptor recycles IgG from endosome.

See Holliger *et al,* Nat Biotechnol 1997 Ju1;15(7):632-6.

Ligands according to the invention may designed to be specific for the above targets ligands according to the invention can be specific for targets selected from the foregoing which are tissue-specific, thereby enabling tissue-specific targeting of the dual specific ligand, Moreover, where the ligand or dAb monomer targets kidney or liver, this may redirect the ligand or dAb monomer to an alternative clearance pathway in *vivo* (for example, the ligand may be directed away from liver clearance to kidney clearance).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the antibodies or binding proteins thereof in protecting against or treating the disease are available. Methods for the testing of systemic lupus erythematosus (SLE) in susceptible mice are known in the art (Knight *et al*. (1978) *J. Exp. Med*., **147**: 1653; Reinersten *et al*. (1978) *New Eng. J. Med*., **299**: 515). Myasthenia Gravis (MG) is tested in SJL/J female mice by inducing the disease with soluble AchR protein from another species (Lindstrom *et al*. (1988) *Adv. Immunol*., **42**: 233). Arthritis is induced in a susceptible strain of mice by injection of Type II collagen (Stuart *et al*. (1984) *Ann. Rev. Immunol*., **42**: 233). A model by which adjuvant arthritis is induced in susceptible rats by injection of mycobacterial heat shock protein has been described (Van Eden *et al*. (1988) *Nature*, **331**: 171). Thymiditis is induced in mice by administration of thyroglobulin as described (Maron *et al.* (1980) *J. Exp. Med.,* **152**: 1115). Insulin dependent diabetes mellitus (IDDM) occurs naturally or can be induced in certain strains of mice such as those described by Kanasawa *et al.* (1984) *Diabetologia*, **27:** 113. EAE in mouse and rat serves as a model for MS in human. In this model, the demyelinating disease is induced by administration of myelin basic protein (see Paterson (1986) *Textbook of Immunopathology*, Mischer *et al.,* eds., Grune and Stratton, New York, pp. 179-213; McFarlin *et al*. (1973) *Science,* 179: 478: and Satoh *et al.* (1987) *J. Immunol*., **138:** 179).

Generally, the present ligands will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) *Remington's Pharmaceutical Sciences,* 16th Edition).

The ligands of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the ligands of the present invention, or even combinations of lignds according to the present invention having different specificities, such as ligands selected using different target antigens or epitopes, whether or not they are pooled prior to administration.

The route of administration of pharmaceutical compositions according to the invention may be any of those commonly known to those of ordinary skill in the art. For therapy, including without limitation immunotherapy, the selected ligands thereof of the invention can be administered to any patient in accordance with standard techniques. The administration can be by any appropriate mode, including parenterally, intravenously, intramuscularly, intraperitoneally, transdermally, *via* the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

The ligands of this invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present ligands or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of ligand, e.g. antibody, receptor (e.g. a T-cell receptor) or binding protein thereof per kilogram of body weight, with doses of 0.05 to 2.0 mg/kgtdose being more commonly used. For prophylactic applications, compositions containing the present ligands or cocktails thereof may also be administered in similar or slightly lower dosages.

Treatment performed using the compositions described herein is considered "effective" if one or more symptoms is reduced (e.g., by at least 10% or at least one point on a clinical assessment scale), relative to such symptoms present before treatment, or relative to such symptoms in an individual (human or model animal) not treated with such composition. Symptoms will obviously vary depending upon the disease or disorder targeted, but can be measured by an ordinarily skilled clinician or technician. Such symptoms can be measured, for example, by monitoring the level of one or more biochemical indicators of the disease or disorder (e.g., levels of an enzyme or metabolite correlated with the disease, affected cell numbers, etc.), by monitoring physical manifestations (e.g., inflammation, tumor size, etc.), or by an accepted clinical assessment scale, for example, the Expanded Disability Status Scale (for multiple sclerosis), the Irvine Inflammatory Bowel Disease Questionnaire (32 point assessment evaluates quality of life with respect to bowel function, systemic symptoms, social function and emotional status - score ranges from 32 to 224, with higher scores indicating a better quality of life), the Quality of Life Rheumatoid Arthritis Scale, or other accepted clinical assessment scale as known in the field. A sustained (e.g., one day or more, preferably longer) reduction in disease or disorder symptoms by at least 10% or by one or more points on a given clinical scale is indicative of "effective" treatment. Similarly, prophylaxis performed using a composition as described herein is "effective" if the onset or severity of one or more symptoms is delayed, reduced or abolished relative to such symptoms in a similar individual (human or animal model) not treated with the composition.

A composition containing a ligand or cocktail thereof according to the present invention may be utilised in prophylactic and therapeutic settings to aid in the alteration, inactivation, killing or removal of a select target cell population in a mammal. In addition, the selected repertoires of polypeptides described herein may be used extracorporeally or *in vitro* selectively to kill, deplete or otherwise effectively remove a target cell population from a heterogeneous collection of cells. Blood from a mammal may be combined extracorporeally with the ligands, e.g. antibodies, cell-surface receptors or binding proteins thereof whereby the undesired cells are killed or otherwise removed from the blood for return to the mammal in accordance with standard techniques.

### I: Use of half-life enhanced dual-specific ligands according to the invention

Dual-specific ligands according to the method of the present invention may be employed in *in vivo* therapeutic and prophylactic applications, *in vivo* diagnostic applications and the like.

Therapeutic and prophylactic uses of dual-specific ligands prepared according to the invention involve the administration of ligands according to the invention to a recipient mammal, such as a human. Dual specific antibodies according to the invention comprise at least one specificity for a half-life enhancing molecule; one or more further specificities may be directed against target molecules. For example, a dual-specific IgG may be specific for four epitopes, one of which is on a half-life enhancing molecule. Dual-specificity can allow antibodies to bind to multimeric antigen with great avidity. Dual-specific antibodies can allow the cross-linking of two antigens, for example in recruiting cytotoxic T-cells to mediate the killing of tumour cell lines.

Substantially pure ligands or binding proteins thereof, such as dAb monomers, of at least 90 to 95% homogeneity are preferred for administration to a mammal, and 98 to 99% or more homogeneity is most preferred for pharmaceutical uses, especially when the mammal is a human. Once purified, partially or to homogeneity as desired, the ligands may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY).

The ligands of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the dual specific ligands in protecting against or treating the disease are available. Methods for the testing of systemic lupus erythematosus (SLE) in susceptible mice are known in the art (Knight *et al.* (1978) *J. Exp. Med.,* **147**: 1653; Reinersten *et al*. (1978) *New Eng. J. Med.,* **299**: 515). Myasthenia Gravis (MG) is tested in SJL/J female mice by inducing the disease with soluble AchR protein from another species (Lindstrom *et al*. (1988) *Adv. Immunol*., **42**: 233). Arthritis is induced in a susceptible strain of mice by injection of Type II collagen (Stuart *et al.* (1984) *Ann. Rev. Immunol*., **42**: 233). A model by which adjuvant arthritis is induced in susceptible rats by injection of mycobacterial heat shock protein has been described (Van Eden *et al*. (1988) *Nature,* **331**: 171). Thyroiditis is induced in mice by administration of thyroglobulin as described (Maron *et al.* (1980) *J. Exp. Med.,* **152**: 1115). Insulin dependent diabetes mellitus (IDDM) occurs naturally or can be induced in certain strains of mice such as those described by Kanasawa *et al.* (1984) *Diabetologia*, **27**: 113. EAE in mouse and rat serves as a model for MS in human. In this model, the demyelinating disease is induced by administration of myelin basic protein (see Paterson (1986) *Textbook of Immunopathology*, Mischer *et al*., eds., Grune and Stratton, New York, pp. 179-213; McFarlin *et al.* (1973) *Science,* **179:** 478: and Satoh *et al*. (1987) *J. Immunol*., **138**: 179).

Dual specific ligands according to the invention and dAb monomers able to bind to extracellular targets involved in endocytosis (e.g. Clathrin) enable dual specific ligands to be endocytosed, enabling another specificity able to bind to an intracellular target to be delivered to an intracellular environment. This strategy requires a dual specific ligand with physical properties that enable it to remain functional inside the cell. Alternatively, if the final destination intracellular compartment is oxidising, a well folding ligand may not need to be disulphide free.

Generally, the present dual specific ligands will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) *Remington's Pharmaceutical Sciences,* 16th Edition).

The ligands of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the ligands of the present invention.

The route of administration of pharmaceutical compositions according to the invention may be any of those commonly known to those of ordinary skill in the art. For therapy, including without limitation immunotherapy, the ligands of the invention can be administered to any patient in accordance with standard techniques. The administration can be by any appropriate mode, including parenterally, intravenously, intramuscularly, intraperitoneally, transdermally, via the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

The ligands of the invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present ligands or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of ligand *per* kilogram of body weight, with doses of 0.05 to 2.0 mg/kg/dose being more commonly used. For prophylactic applications, compositions containing the present ligands or cocktails thereof may also be administered in similar or slightly lower dosages.

A composition containing a ligand according to the present invention may be utilised in prophylactic and therapeutic settings to aid in the alteration, inactivation, killing or removal of a select target cell population in a mammal.

In addition, the selected repertoires of polypeptides described herein may be used extracorporeally or *in vitro* selectively to kill, deplete or otherwise effectively remove a target cell population from a heterogeneous collection of cells. Blood from a mammal may be combined extracorporeally with the ligands, e.g. antibodies, cell- surface receptors or binding proteins thereof whereby the undesired cells are killed or otherwise removed from the blood for return to the mammal in accordance with standard techniques.

The invention is further described, for the purposes of illustration only, in the following examples. As used herein, for the purposes of dAb nomenclature, human TNFα is referred to as TAR1 and human TNFα receptor 1 (p55 receptor) is referred to as TAR2.

### Example 1. Selection of a dual specific scFv antibody (K8) directed against human serum albumin (HSA) and β-galactosidase (β -gal)

This example explains a method for making a dual specific antibody directed against β-gal and HSA in which a repertoire of V_{κ} variable domains linked to a germline (dummy) V_{H} domain is selected for binding to β-gal and a repertoire of V_{H} variable domains linked to a germline (dummy) V_{κ} domain is selected for binding to HSA. The selected variable V_{H} HSA and V_{κ} β-gal domains are then combined and the antibodies selected for binding to β-gal and HSA. HSA is a half-life increasing protein found in human blood.

Four human phage antibody libraries were used in this experiment.

| | | |
|---|---|---|
| Library 1 | Germline V_{κ}/DVT V_{H} | 8.46 x 107 |
| Library 2 | Germline V_{κ}/NNK V_{H} | 9.64 x 10⁷ |
| Library 3 | Germline V_{H}/DVT V_{κ} | 1.47 x 108 |
| Library 4 | Germline V_{H}/NNK V_{κ} | 1.45 x 10⁸ |

All libraries are based on a single human framework for V_{H} (V3-23/DP47 and J_{H}4b) and V_{κ} (O12/O2/DPK9 and J_{κ}1) with side chain diversity incorporated in complementarity determining regions (CDR2 and CDR3).

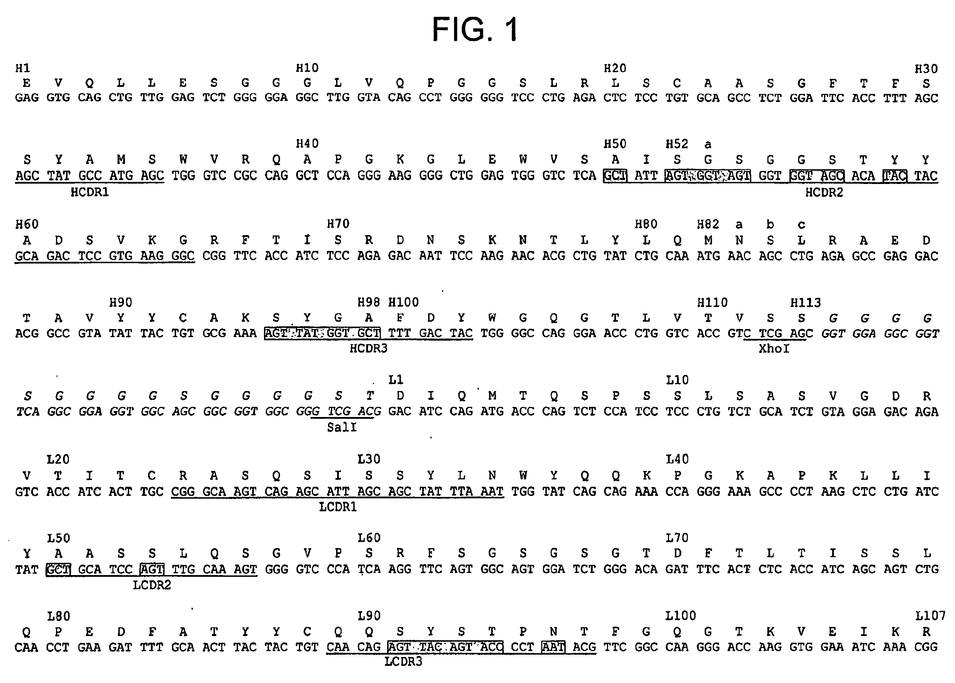

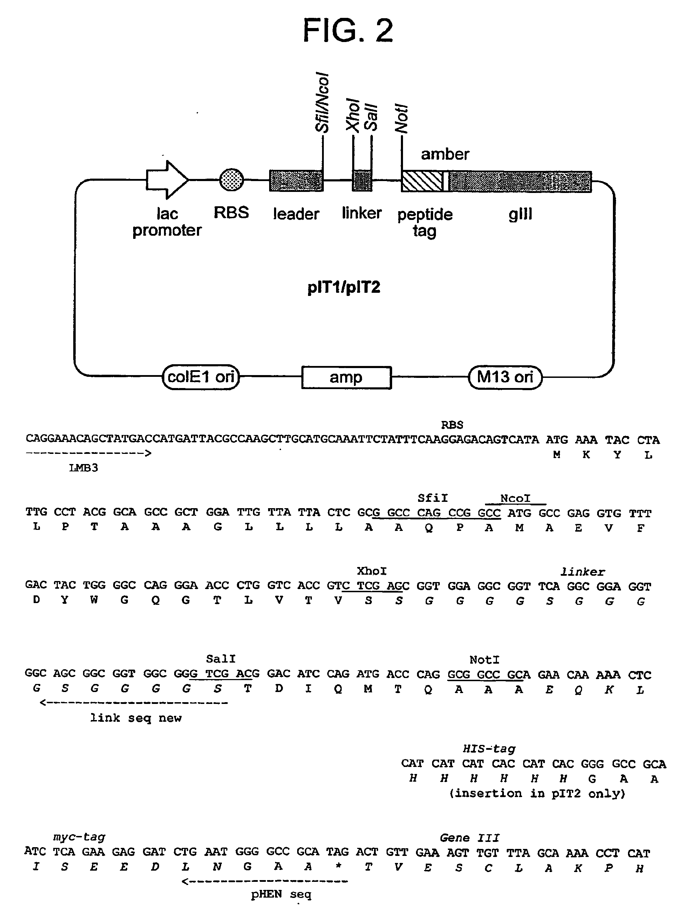

Library 1 and Library 2 contain a dummy V_{κ} sequence, whereas the sequence of V_{H} is diversified at positions H50, H52, H52a, H53, H55, H56, H58, H95, H96, H97 and H98 (DVT or NNK encoded, respectively) (Figure 1). Library 3 and Library 4 contain a dummy V_{H} sequence, whereas the sequence of V_{κ} is diversified at positions L50, L53, L91, L92, L93, L94 and L96 (DVT or NNK encoded, respectively) (Figure 1). The libraries are in phagemid pIT2/ScFv format (Figure 2) and have been preselected for binding to generic ligands, Protein A and Protein L, so that the majority of clones in the unselected libraries are functional. The sizes of the libraries shown above correspond to the sizes after preselection. Library 1 and Library 2 were mixed prior to selections on antigen to yield a single V_{H}/dummy V_{κ} library and Library 3 and Library 4 were mixed to form a single V_{κ}/dummy V_{H} library.

Three rounds of selections were performed on β-gal using V_{κ}/dummy V_{H} library and three rounds of selections were performed on HSA using V_{H}/dummy V_{κ} library. In the case of β-gal the phage titres went up from 1.1 x 10⁶ in the first round to 2.0 x 10⁸ in the third round. In the case of HSA the phage titres went up from 2 x 10⁴ in the first round to 1.4 x 10⁹ in the third round. The selections were performed as described by Griffith *et al*., (1993), except that KM13 helper phage (which contains a pIII protein with a protease cleavage site between the D2 and D3 domains) was used and phage were eluted with 1 mg/ml trypsin in PBS. The addition of trypsin cleaves the pIII proteins derived from the helper phage (but not those from the phagemid) and elutes bound scFv-phage fusions by cleavage in the c-myc tag (Figure 2), thereby providing a further enrichment for phages expressing functional scFvs and a corresponding reduction in background (Kristensen & Winter, Folding & Design 3: 321-328, Jul 9, 1998). Selections were performed using immunotubes coated with either HSA or β-gal at 100µg/ml concentration.

To check for binding, 24 colonies from the third round of each selection were screened by monoclonal phage ELISA. Phage particles were produced as described by Harrison *et al*., Methods Enzymol. 1996;267:83-109. 96-well ELISA plates were coated with 100µl of HSA or β-gal at 10µg/ml concentration in PBS overnight at 4°C. A standard ELISA protocol was followed (Hoogenboom *et al*., 1991) using detection of bound phage with anti-M13-HRP conjugate. A selection of clones gave ELISA signals of greater than 1.0 with 50µl supernatant.

Next, DNA preps were made from V_{H}/dummy V_{κ} library selected on HSA and from V_{κ}/dummy V_{H} library selected on β-gal using the QIAprep Spin Miniprep kit (Qiagen). To access most of the diversity, DNA preps were made from each of the three rounds of selections and then pulled together for each of the antigens. DNA preps were then digested with Sal*I*/Not*I* overnight at 37°C. Following gel purification of the fragments, V_{κ} chains from the V_{κ}/dummy V_{H} library selected on β-gal were ligated in place of a dummy V_{κ} chain of the V_{H}/dummy V_{κ} library selected on HSA creating a library of 3.3 x 10⁹ clones.

This library was then either selected on HSA (first round) and β-gal (second round), HSA/β-gal selection, or on β-gal (first round) and HSA (second round), β-gal/HSA selection. Selections were performed as described above. In each case after the second round 48 clones were tested for binding to HSA and β-gal by the monoclonal phage ELISA (as described above) and by ELISA of the soluble scFv fragments. Soluble antibody fragments were produced as described by Harrison *et al*., (1996), and standard ELISA protocol was followed Hoogenboom *et al.* (1991) Nucleic Acids Res., 19: 4133, except that 2% Tween/PBS was used as a blocking buffer and bound scFvs were detected with Protein L-HRP. Three clones (E4, E5 and E8) from the HSA/β-gal selection and two clones (K8 and K10) from the β-gal/HSA selection were able to bind both antigens. scFvs from these clones were PCR amplified and sequenced as described by Ignatovich *et al.,* (1999) J Mol Biol 1999 Nov 26;294(2):457-65, using the primers LMB3 and pHENseq. Sequence analysis revealed that all clones were identical. Therefore, only one clone encoding a dual specific antibody (K8) was chosen for further work (Figure 3).

### Example 2. Characterisation of the binding properties of the K8 antibody.

Firstly, the binding properties of the K8 antibody were characterised by the monoclonal phage ELISA. A 96-well plate was coated with 100µl of HSA and β-gal alongside with alkaline phosphatase (APS), bovine serum albumin (BSA), peanut agglutinin, lysozyme and cytochrome c (to check for cross-reactivity) at 10µg/ml concentration in PBS overnight at 4°C. The phagemid from K8 clone was rescued with KM13 as described by Harrison *et al*., (1996) and the supernatant (50µl) containing phage assayed directly. A standard ELISA protocol was followed (Hoogenboom *et al*., 1991) using detection of bound phage with anti-M13-HRP conjugate. The dual specific K8 antibody was found to bind to HSA and β-gal when displayed on the surface of the phage with absorbance signals greater than 1.0 (Figure 4). Strong binding to BSA was also observed (Figure 4). Since HSA and BSA are 76% homologous on the amino acid level, it is not surprising that K8 antibody recognised both of these structurally related proteins. No cross-reactivity with other proteins was detected (Figure 4).

Secondly, the binding properties of the K8 antibody were tested in a soluble scFv ELISA. Production of the soluble scFv fragment was induced by IPTG as described by Harrison *et al*., (1996). To determine the expression levels of K8 scFv, the soluble antibody fragments were purified from the supernatant of 50ml inductions using Protein A-Sepharose columns as described by Harlow and Lane, Antibodies: a Laboratory Manual, (1988) Cold Spring Harbor. OD₂₈₀ was then measured and the protein concentration calculated as described by Sambrook *et al.,* (1989). K8 scFv was produced in supernatant at 19mg/l.

A soluble scFv ELISA was then performed using known concentrations of the K8 antibody fragment. A 96-well plate was coated with 100µl of HSA, BSA and β-gal at 10µg/ml and 100µl of Protein A at 1µg/ml concentration. 50µl of the serial dilutions of the K8 scFv was applied and the bound antibody fragments were detected with Protein L-HRP. ELISA results confirmed the dual specific nature of the K8 antibody (Figure 5).

To confirm that binding to β-gal is determined by the V_{K} domain and binding to HSA/BSA by the V_{H} domain of the K8 scFv antibody, the V_{κ} domain was cut out from K8 scFv DNA by Sal*I*/Not*I* digestion and ligated into a Sal*I*/Not*I* digested pIT2 vector containing dummy V_{H} chain (Figures 1 and 2). Binding characteristics of the resulting clone K8V_{κ}/dummy V_{H} were analysed by soluble scFv ELISA. Production of the soluble scFv fragments was induced by IPTG as described by Harrison *et al.,* (1996) and the supernatant (50µ) containing scFvs assayed directly. Soluble scFv ELISA was performed as described in Example 1 and the bound scFvs were detected with Protein L-HRP. The ELISA results revealed that this clone was still able to bind β-gal, whereas binding to HSA was abolished (Figure 6).

### Example 3. Selection of single V_{H} domain antibodies antigens A and B and single V_{κ} domain antibodies directed against antigens C and D.

This example describes a method for making single V_{H} domain antibodies directed against antigens A and B and single V_{κ} domain antibodies directed against antigens C and D by selecting repertoires of virgin single antibody variable domains for binding to these antigens in the absence of the complementary variable domains.

Selections and characterisation of the binding clones is performed as described previously (see Example 5, PCT/GB 02/003014). Four clones are chosen for further work:
VH1- Anti A V_{H}
VH2 - Anti B V_{H}
VK1 - Anti C V_{κ}
VK2 - Anti D V_{κ}

The procedures described above in Examples 1-3 may be used, in a similar manner as that described, to produce dimer molecules comprising combinations of V_{H} domains (i.e., V_{H-}V_{H} ligands) and cominations of V_{L} domains (V_{L}-V_{L} ligands).

### Example 4. Creation and characterisation of the dual specific ScFv antibodies (VH1/VH2 directed against antigens A and B and VK1/VK2 directed against antigens C and D).

This example demonstrates that dual specific ScFv antibodies (VH1/VH2 directed against antigens A and B and VK1/VK2 directed against antigens C and D) could be created by combining V_{κ} and V_{H} single domains selected against respective antigens in a ScFv vector.

To create dual specific antibody VH1/VH2, VH1 single domain is excised from variable domain vector 1 (Figure 7) by *Nco*I/*Xho*I digestion and ligated into *Nco*I/*Xho*I digested variable domain vector 2 (Figure 7) to create VH1/ variable domain vector 2. VH2 single domain is PCR amplified from variable domain vector 1 using primers to introduce *Sal*I restriction site to the 5' end and *Not*I restriction site to the 3' end. The PCR product is then digested with *Sal*I/*Not*I and ligated into *Sal*I/*Not*I digested VH1/ variable domain vector 2 to create VH1/VH2/ variable domain vector 2.

VK1/VK2/ variable domain vector 2 is created in a similar way. The dual specific nature of the produced VH1/VH2 ScFv and VK1/VK2 ScFv is tested in a soluble ScFv ELISA as described previously (see Example 6, PCT/GB 02/003014). Competition ELISA is performed as described previously (see Example 8, PCT/GB 02/003014).

Possible outcomes:
- VH1/VH2 ScFv is able to bind antigens A and B simultaneously
- VK1/VK2 ScFv is able to bind antigens C and D simultaneously
- VH1/VH2 ScFv binding is competitive (when bound to antigen A, VH1/VH2 ScFv cannot bind to antigen B)
- VK1/VK2 ScFv binding is competitive (when bound to antigen C, VK1/VK2 ScFv cannot bind to antigen D)

### Example 5. Construction of dual specific VH1/VH2 Fab and VK1/VK2 Fab and analysis of their binding properties.

To create VH1/VH2 Fab, VH1 single domain is ligated into *Nco*I/*Xho*I digested CH vector (Figure 8) to create VH1/CH and VH2 single domain is ligated into *Sal*I/*Not*I digested CK vector (Figure 9) to create VH2/CK. Plasmid DNA from VH1/CH and VH2/CK is used to co-transform competent *E. coli* cells as descnbed previously (see Example 8, PCT/GB02/003014).

The clone containing VH1/CH and VH2/CK plasmids is then induced by IPTG to produce soluble VH1/VH2 Fab as described previously (see Example 8, PCT/GB 02/003014).

VK1/VK2 Fab is produced in a similar way.

Binding properties of the produced Fabs are tested by competition ELISA as described previously (see Example 8, PCT/GB 02/003014).

Possible outcomes:
- VH1/VH2 Fab is able to bind antigens A and B simultaneously
- VK1/VK2 Fab is able to bind antigens C and D simultaneously
- VH1/VH2 Fab binding is competitive (when bound to antigen A, VH1/VH2 Fab cannot bind to antigen B)
- VK1/VK2 Fab binding is competitive (when bound to antigen C, VK1/VK2 Fab cannot bind to antigen D)

### Example 6

### Chelating dAb Dimers

### Summary

VH and VK homo-dimers are created in a dAb-linker-dAb format using flexible polypeptide linkers. Vectors were created in the dAb linker-dAb format containing glycine-serine linkers of different lengths 3U:(Gly₄Ser)₃, 5U:(Gly₄Ser)₅, 7U:(Gly₄Ser)₇. Dimer libraries were created using guiding dAbs upstream of the linker: TAR1-5 (VK), TAR1-27(VK), TAR2-5(VH) or TAR2-6(VK) and a library of corresponding second dAbs after the linker. Using this method, novel dimeric dAbs were selected. The effect of dimerisation on antigen binding was determined by ELISA and BIAcore studies and in cell neutralisation and receptor binding assays. Dimerisation of both TARI-5 and TAR1-27 resulted in significant improvement in binding affinity and neutralisation levels.

### 1.0 Methods

### 1.1 Library generation

### 1.1.1 Vectors

pEDA3U, pEDA5U and pEDA7U vectors were designed to introduce different linker lengths compatible with the dAb-linker-dAb format. For pEDA3U, sense and anti-sense 73-base pair oligo linkers were annealed using a slow annealing program (95°C-5mins, 80°C-10mins, 70°C-15mins, 56°C-15mins, 42°C until use) in buffer containing 0.1MNaCl, 10mM Tris-HCl pH7.4 and cloned using the *Xhol* and *Notl* restriction sites. The linkers encompassed 3 (Gly₄Ser) units and a stuffer region housed between *Sall* and *Notl* cloning sites (scheme 1). In order to reduce the possibility of monomeric dAbs being selected for by phage display, the stuffer region was designed to include 3 stop codons, a *Sacl* restriction site and a frame shift mutation to put the region out of frame when no second dAb was present. For pEDA5U and 7U due to the length of the linkers required, overlapping oligo-linkers were designed for each vector, annealed and elongated using Klenow. The fragment was then purified and digested using the appropriate enzymes before cloning using the *Xho1* and *NotI* restriction sites.

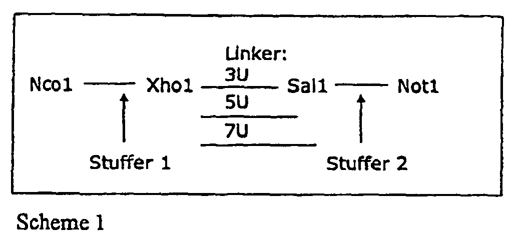

### 1.1.2 Library preparation

The N-terminal V gene corresponding to the guiding dAb was cloned upstream of the linker using *Nco1* and *Xho1* restriction sites. VH genes have existing compatible sites, however cloning VK genes required the introduction of suitable restriction sites. This was achieved by using modifying PCR primers (VK-DLIBF: 5' cggccatggcgtcaacggacat; VKXhoIR: 5' atgtgcgctcgagcgtttgattt 3') in 30 cycles of PCR amplification using a 2:1 mixture of SuperTaq (HTBiotechnology Ltd)and *pfu* turbo (Stratagene). This maintained the *Ncol* site at the 5' end while destroying the adjacent *Sall* site and introduced the *Xhol* site at the 3' end. 5 guiding dAbs were cloned into each of the 3 dimer vectors: TAR1-5 (VK), TAR1-27(VK), TAR2-5(VH), TAR2-6(VK) and TAR2-7(VK). All constructs were verified by sequence analysis.

Having cloned the guiding dAbs upstream of the linker in each of the vectors (pEDA3U, 5U and 7U): TAR1-5 (VK), TAR1-27(VK), TAR2-5(VH) or TAR2-6(VK) a library of corresponding second dAbs were cloned after the linker. To achieve this, the complimentary dAb libraries were PCR amplified from phage recovered from round 1 selections of either a VK library against Human TNFα (at approximately 1 x 10⁶ diversity after round 1) when TAR1-5 or TAR1-27 are the guiding dAbs, or a VH or VK library against human p55 TNF receptor (both at approximately 1 x 10⁵ diversity after round 1) when TAR2-5 or TAR2-6 respectively are the guiding dAbs. For VK libraries PCR amplification was conducted using primers in 30 cycles of PCR amplification using a 2:1 mixture of SuperTaq and *pfu* turbo. VH libraries were PCR amplified using primers in order to introduce a *Sall* restriction site at the 5' end of the gene. The dAb library PCRs were digested with the appropriate restriction enzymes, ligated into the corresponding vectors down stream of the linker, using *Sal1*/*Not1* restriction sites and electroporated into freshly prepared competent TG1 cells.

The titres achieved for each library are as follows:
TARl-5: pEDA3U = 4x10⁸, pEDA5U = 8x10⁷, pEDA7U = 1x10⁸
TARI-27: pEDA3U = 6.2x10⁸, pEDA5U = 1x10⁸, pBDA7U = 1x10⁹
TAR2h-5: pEDA3U = 4x10⁷, pEDA5U = 2 x 10⁸, pEDA7U = 8x10⁷
TAR2h-6: pEDA3U = 7.4x 10⁸, pEDA5U = 1.2 x 10⁸, pEDA7U = 2.2x 10⁸

### 1.2 Selections

### 1.2.1 TNFα

Selections were conducted using human TNFα passively coated on immunotubes. Briefly, Immunotubes are coated overnight with 1-4mls of the required antigen. The immunotubes were then washed 3 times with PBS and blocked with 2%milk powder in PBS for 1-2hrs and washed a further 3 times with PBS. The phage solution is diluted in 2%milk powder in PBS and incubated at room temperature for 2hrs. The tubes are then washed with PBS and the phage eluted with 1mg/ml trypsin-PBS. Three selection strategies were investigated for the TARI-5 dimer libraries. The first round selections were carried out in immunotubes using human TNFα coated at 1µg/ml or 20µg/ml with 20 washes in PBS 0.1%Tween. TG1 cells are infected with the eluted phage and the titres are determined (eg, Marks et al J Mol Biol. 1991 Dec 5;222(3):581-97, Richmann et al Biochemistry. 1993 Aug 31;32(34):8848-55).

The titres recovered were:
pEDA3U = 2.8x10⁷ (1µg/ml TNF) 1.5x10⁸ (20µg/mlTNF),
pEDA5U = 1.8x10⁷ (1µg/ml TNF), 1.6x10⁸ (20µg/ml TNF)
pEDA7U =8x10⁶ (1µg/ml TNF), 7x10⁷ (20µg/ml TNF).

The second round selections were carried out using 3 different methods.
1. In immunotubes, 20 washes with overnight incubation followed by a further 10 washes.
2. In immunotubes, 20 washes followed by 1hr incubation at RT in wash buffer with (1µg/ml TNFα) and 10 further washes.
3. Selection on streptavidin beads using 33 pmoles biotinylated human TNFα (Henderikx *et al*., 2002, *Selection of antibodies against biotinylated antigens*. Antibody Phage Display : Methods and protocols, Ed. O'Brien and Atkin, Humana Press). Single clones from round 2 selections were picked into 96 well plates and crude supernatant preps were made in 2ml 96 well plate format.

| | Round 1 Human TNFαimmuno tube coating concentration | Round 2 selection method 1 | Round 2 selection method 2 | Round 2 selection method 3 |
|---|---|---|---|---|
| pEDA3U | 1µg/ml | 1 x 10⁹ | 1.8 x 10⁹ | 2.4 x 10¹⁰ |
| pEDA3U | 20µg/ml | 6 x 10⁹ | 1.8 x 10¹⁰ | 8.5 x 10¹⁰ |
| pEDA5U | 1µg/ml | 9 x 10⁸ | 1.4 x 10⁹ | 2.8 x 10¹⁰ |
| pEDA5U | 20µg/ml | 9.5 x 10⁹ | 8.5 x 10⁹ | 2.8 x 10¹⁰ |
| pEDA7U | 1µg/ml | 7.8 x 10⁸ | 1.6 x 10⁸ | 4 x 10¹⁰ |
| pEDA7U | 20µg/ml | 1 x 10¹⁰ | 8 x 10⁹ | 1.5 x 10¹⁰ |

For TAR1-27, selections were carried out as described previously with the following modifications. The first round selections were carried out in immunotubes using human TNFα coated at 1µg/ml or 20µg/ml with 20 washes in PBS 0.1%Tween. The second round selections were carried out in immunotubes using 20 washes with overnight incubation followed by a further 20 washes. Single clones from round 2 selections were picked into 96 well plates and crude supernatant preps were made in 2ml 96 well plate format.

TAR1-27 titres are as follows:

| | Human TNFaimmunotube coating conc | Round 1 | Round 2 |
|---|---|---|---|
| pEDA3U | 1µg/ml | 4 x 10⁹ | 6 x 10⁹ |
| pEDA3U | 20µg/ml | 5 x 10⁹ | 4.4 x 10¹⁰ |
| pEDA5U | 1µg/ml | 1.5 x 10⁹ | 1.9 x 10¹⁰ |
| pEDA5U | 20µg/ml | 3.4 x 10⁹ | 3.5 x 10¹⁰ |
| pEDA7U | 1µg/ml | 2.6 x 10⁹ | 5 x 10⁹ |
| pEDA7U | 20µg/ml | 7 x 10⁹ | 1.4 x 10¹⁰ |

### 1.2.2 TNF RECEPTOR 1 (p55 RECEPTOR; TAR2)

Selections were conducted as described previously for the TAR2h-5 libraries only. 3 rounds of selections were carried out in immunotubes using either 1µg/ml human p55 TNF receptor or 10µg/ml human p55 TNF receptor with 20 washes in PBS 0.1%Tween with overnight incubation followed by a further 20 washes. Single clones from round 2 and 3 selections were picked into 96 well plates and crude supernatant preps were made in 2ml 96 well plate format.

TAR2h-5 titres are as follows:

| | Round 1 human p55 TNF receptor immunotube coating concentration | Round 1 | Round 2 | Round 3 |
|---|---|---|---|---|
| pEDA3U | 1µg/ml | 2.4 x 10⁶ | 1.2 x 10⁷ | 1.9 x 10⁹ |
| pEDA3U | 10µg/ml | 3.1 x 10⁷ | 7 x 10⁷ | 1 x 10⁹ |
| pEDA5U | 1µg/ml | 2.5 x 10⁶ | 1.1 x 10⁷ | 5.7 x 10⁸ |
| pEDA5U | 10µg/ml | 3.7 x 10⁷ | 2.3 x 10⁸ | 2.9 x 10⁹ |
| pEDA7U | 1µg/ml | 1.3 x 10⁶ | 1.3 x 10⁷ | 1.4 x 10⁹ |
| pEDA7U | 10µg/ml | 1.6 x 10⁷ | 1.9 x 10⁷ | 3 x 10¹⁰ |

### 1.3 Screening

Single clones from round 2 or 3 selections were picked from each of the 3U, 5U and 7U libraries from the different selections methods, where appropriate. Clones were grown in 2xTY with 100µg/ml ampicillin and 1% glucose overnight at 37°C. A 1/100 dilution of this culture was inoculated into 2mls of 2xTY with 100µg/ml ampicillin and 0.1% glucose in 2ml, 96 well plate format and grown at 37°C shaking until OD600 was approximately 0.9. The culture was then induced with 1mM IPTG overnight at 30°C. The supernatants were clarified by centrifugation at 4000rpm for 15 mins in a sorval plate centrifuge. The supernatant preps the used for initial screening.

### 1.3.1 ELISA

Binding activity of dimeric recombinant proteins was compared to monomer by Protein A/L ELISA or by antigen ELISA. Briefly, a 96 well plate is coated with antigen or Protein A/L overnight at 4°C. The plate washed with 0.05% Tween-PBS, blocked for 2hrs with 2% Tween-PBS. The sample is added to the plate incubated for 1 hr at room temperature. The plate is washed and incubated with the secondary reagent for 1hr at room temperature. The plate is washed and developed with TMB substrate. Protein A/L-HRP or India-HRP was used as a secondary reagent. For antigen ELISAs, the antigen concentrations used were 1µg/ml in PBS for Human TNFα and human THF receptor 1. Due to the presence of the guiding dAb in most cases dimers gave a positive ELISA signal therefore off rate determination was examined by BIAcore.

### 1.3.2 BIAcore

BIAcore analysys was conducted for TAR1-5 and TAR2h-5 clones. For screening, Human TNFαwas coupled to a CM5 chip at high density (approximately 10000 RUs). 50 µl of Human TNFα(50 µg/ml) was coupled to the chip at 5µl/min in acetate buffer - pH5.5. Regeneration of the chip following analysis using the standard methods is not possible due to the instability of Human TNFα, therefore after each sample was analysed, the chip was washed for 10mins with buffer.
For TAR1-5, clones supernatants from the round 2 selection were screened by BIAcore.
48 clones were screened from each of the 3U, 5U and 7U libraries obtained using the following selection methods:
R1: 1µg/ml human TNFα immunotube, R2 1µg/ml human TNFα immunotube, overnight wash.
R1: 20µg/ml human TNFα immunotube, R2 20µg/ml human TNFα immunotube, overnight wash.
R1: 1µg/ml human TNFα immunotube, R2 33 pmoles biotinylated human TNFα on beads.
R1: 20µg/ml human TNFα immunotube, R2 33 pmoles biotinylated human TNFα beads.

For screening, human p55 TNF receptor was coupled to a CM5 chip at high density (approximately 4000 RUs). 100 µl of human p55 TNF receptor (10 µg/ml) was coupled to the chip at 5µl/min in acetate buffer - pH5.5. Standard regeneration conditions were examined (glycine pH2 or pH3) but in each case antigen was removed from the surface of the chip therefore as with TNFα, therefore after each sample was analysed, the chip was washed for 10mins with buffer.
For TAR2-5, clones supernatants from the round 2 selection were screened.
48 clones were screened from each of the 3U, 5U and 7U libraries, using the following selection methods:
R1: 1µg/ml human p55 TNF receptor immunotube, R2 1µg/ml human p55 TNF receptor immunotube, overnight wash.
R1: 10µg/ml human p55 TNF receptor immunotube, R2 10µg/ml human p55 TNF receptor immunotube, overnight wash.

### 1.3.3 Receptor and Cell Assays

The ability of the dimers to neutralise in the receptor assay was conducted as follows:

### Receptor binding

Anti-TNF dAbs were tested for the ability to inhibit the binding of TNF to recombinant TNF receptor 1 (p55). Briefly, Maxisorp plates were incubated overnight with 30mg/ml anti-human Fc mouse monoclonal antibody (Zymed, San Francisco, USA). The wells were washed with phosphate buffered saline (PBS) containing 0.05% Tween-20 and then blocked with 1% BSA in PBS before being incubated with 100ng/ml TNF receptor 1 Fc fusion protein (R&D Systems, Minneapolis, USA). Anti-TNF dAb was mixed with TNF which was added to the washed wells at a final concentration of 10ng/ml. TNF binding was detected with 0.2mg/ml biotinylated anti-TNF antibody (HyCult biotechnology, Uben, Netherlands) followed by 1 in 500 dilution of horse radish peroxidase labelled streptavidin (Amersham Biosciences, UK) and then incubation with TMB substrate (KPL, Gaithersburg, USA). The reaction was stopped by the addition of HCl and the absorbance was read at 450nm. Anti-TNF dAb activity lead to a decrease in TNF binding and therefore a decrease in absorbance compared with the TNF only control.

### L929 Cytotoxicity Assay

Anti-TNF dAbs were also tested for the ability to neutralise the cytotoxic activity of TNF on mouse L929 fibroblasts (Evans, T. (2000) Molecular Biotechnology 15, 243-248). Briefly, L929 cells plated in microtitre plates were incubated overnight with anti-TNF dAb, 100pg/ml TNF and 1mg/ml actinomycin D (Sigma, Poole, UK). Cell viability was measured by reading absorbance at 490nm following an incubation with [3-(4,5-dimethylthiazol-2-yl)-5-(3-carbboxymethoxyphenyl)-2-(4-sulfophenyl)-2H-tetrazolium (Promega, Madison, USA). Anti-TNF dAb activity lead to a decrease in TNF cytotoxicity and therefore an increase in absorbance compared with the TNF only control.

In the initial screen, supernatants prepared for BIAcore analysis, described above, were also used in the receptor assay. Further analysis of selected dimers was also conducted in the receptor and cell assays using purified proteins.

### HeLa IL-8 assay

Anti-TNFR1 or anti-TNF alpha dAbs were tested for the ability to neutralise the induction of IL-8 secretion by TNF in HeLa cells (method adapted from that of Akeson, L. et al (1996) Journal of Biological Chemistry 271, 30517-30523, describing the induction of IL-8 by IL-1 in HUVEC; here we look at induction by human TNF alpha and we use HeLa cells instead of the HUVEC cell line). Briefly, HeLa cells plated in microtitre plates were incubated overnight with dAb and 300pg/ml TNF. Post incubation the supernatant was aspirated off the cells and IL-8 concentration measured via a sandwich ELISA (R&D Systems). Anti-TNFR1 dAb activity lead to a decrease in IL-8 secretion into the supernatant compared with the TNF only control.

The L929 assay is used throughout the following experiments; however, the use of the HeLa IL-8 assay is preferred to measure anti-TNF receptor 1 (p55) ligands; the presence of mouse p55 in the L929 assay poses certain limitations in its use.

### 1.4 Sequence analysis

Dimers that proved to have interesting properties in the BIAcore and the receptor assay screens were sequenced. Sequences are detailed in the sequence listing.

### 1.5 Formatting

### 1.5.1 TAR1-5-19 dimers

The TAR1-5 dimers that were shown to have good neutralisation properties were re-formatted and analysed in the cell and receptor assays. The TAR1-5 guiding dab was substituted with the affinity matured clone TAR1-5-19. To achieve this TAR1-5 was cloned out of the individual dimer pair and substituted with TAR1-5-19 that had been amplified by PCR. In addition, TAR1-5-19 homodimers were also constructed in the 3U, 5U and 7U vectors. The N terminal copy of the gene was amplified by PCR and cloned as described above and the C-terminal gene fragment was cloned using existing *Sal1* and *Not1* restriction sites.

### 1.5.2 Mutagenesis

The amber stop codon present in dAb2, one of the C-terminal dAbs in the TAR1-5 dimer pairs was mutated to a glutamine by site-directed mutagenesis.

### 1.5.3 Fabs

The dimers containing TAR1-5 or TAR1-5-19 were re-formatted into Fab expression vectors. dAbs were cloned into expression vectors containing either the CK or CH genes using *Sfl1* and *Not1* restriction sites and verified by sequence analysis. The CK vector is derived from a pUC based ampicillin resistant vector and the CH vector is derived from a pACYC chloramphenicol resistant vector. For Fab expression the dAb-CH and dAb-CK constructs were co-transformed into HB2151 cells and grown in 2xTY containing 0.1% glucose, 100µg/ml ampicillin and 10µg/ml chloramphenicol.

### 1.5.3 Hinge dimerisation

Dimerisation of dAbs via cystine bond formation was examined. A short sequence of amino acids EPKSGDKTHTCPPCP a modified form of the human IgGC1 hinge was engineered at the C terminal region on the dAb. An oligo linker encoding for this sequence was synthesised and annealed, as described previously. The linker was cloned into the pEDA vector containing TAR1-5-19 using *Xho1* and *Not1* restriction sites. Dimerisation occurs *in situ* in the periplasm.

### 1.6 Expression and purification

### 1.6.1 Expression

Supernatants were prepared in the 2ml, 96-well plate format for the initial screening as described previously. Following the initial screening process selected dimers were analysed further. Dimer constructs were expressed in TOP10F' or HB2151 cells as supernatants. Briefly, an individual colony from a freshly streaked plate was grown overnight at 37°C in 2xTY with 100µg/ml ampicillin and 1% glucose. A 1/100 dilution of this culture was inoculated into 2xTY with 100µg/ml ampicillin and 0.1% glucose and grown at 37°C shaking until OD600 was approximately 0.9. The culture was then induced with 1mM IPTG overnight at 30°C. The cells were removed by centrifugation and the supernatant purified with protein A or L agarose.
Fab and cysteine hinge dimers were expressed as periplasmic proteins in HB2152 cells. A 1/100 dilution of an overnight culture was inoculated into 2xTY with 0.1% glucose and the appropriate antibiotics and grown at 30°C shaking until OD600 was approximately 0.9. The culture was then induced with 1mM IPTG for 3-4 hours at 25°C. The cells were harvested by centrifugation and the pellet resuspended in periplasmic preparation buffer (30mM Tris-HCl pH8.0, 1mM EDTA, 20% sucrose). Following centrifugation the supernatant was retained and the pellet resuspended in 5mM MgSO₄. The supernatant was harvested again by centrifugation, pooled and purified.

### 1.6.2 Protein A/L purification

Optimisation of the purification of dimer proteins from Protein L agarose (Affitech, Norway) or Protein A agarose (Sigma, UK) was examined. Protein was eluted by batch or by column elution using a peristaltic pump. Three buffers were examined 0.1M Phosphate-citrate buffer pH2.6, 0.2M Glycine pH2.5 and 0.1M Glycine pH2.5. The optimal condition was determined to be under peristaltic pump conditions using 0.1M Glycine pH2.5 over 10 column volumes. Purification from protein A was conducted peristaltic pump conditions using 0.1M Glycine pH2.5.

### 1.6.3 FPLC purification

Further purification was carried out by FPLC analysis on the AKTA Explorer 100 system (Amersham Biosciences Ltd). TAR1-5 and TAR1-5-19 dimers were fractionated by cation exchange chromatography (1ml Resource S - Amersham Biosciences Ltd) eluted with a 0-1M NaCl gradient in 50mM acetate buffer pH4. Hinge dimers were purified by ion exchange (1ml Resource Q Amersham Biosciences Ltd) eluted with a 0-1M NaCl gradient in 25mMTris HCl pH 8.0. Fabs were purified by size exclusion chromatography using a superose 12 (Amersham Biosciences Ltd) column run at a flow rate of 0.5ml/min in PBS with 0.05% tween. Following purification samples were concentrated using vivaspin 5K cut off concentrators (Vivascience Ltd).

### 2.0 Results

### 2.1 TAR1-5 dimers

6 x 96 clones were picked from the round 2 selection encompassing all the libraries and selection conditions. Supematant preps were made and assayed by antigen and Protein L ELISA, BIAcore and in the receptor assays. In ELISAs, positive binding clones were identified from each selection method and were distributed between 3U, 5U and 7U libraries. However, as the guiding dAb is always present it was not possible to discriminate between high and low affinity binders by this method therefore BIAcore analysis was conducted.

BIAcore analysis was conducted using the 2ml supernatants. BIAcore analysis revealed that the dimer Koff rates were vastly improved compared to monomeric TAR1-5. Monomer Koff rate was in the range of 10⁻¹M compared with dimer Koff rates which were in the range of 10⁻³ - 10⁻⁴M. 16 clones that appeared to have very slow off rates were selected, these came from the 3U, 5U and 7U libraries and were sequenced. In addition the supernatants were analysed for the ability to neutralise human TNFα in the receptor assay.

6 lead clones (dl-d6 below) that neutralised in these assays and have been sequenced. The results shows that out of the 6 clones obtained there are only 3 different second dAbs (dAb1, dAb2 and dAb3) however where the second dAb is found more than once they are linked with different length linkers.
**TAR1-5d1:** 3U linker 2^{nd} dAb=dAb1 - 1µg/ml Ag immunotube overnight wash
**TAR1-5d2:** 3U linker 2^{nd} dAb=dAb2 - 1µg/ml Ag immunotube overnight wash
**TAR1-5d3:** 5U linker 2^{nd} dAb=dAb2 - 1µg/ml Ag immunotube overnight wash
**TAR1-5d4:** 5U linker 2^{nd} dAb=dAb3 - 20µg/ml Ag immunotube overnight wash
**TAR1-5d5:** 5U linker 2^{nd} dAb=dAb1 - 20µg/ml Ag immunotube overnight wash
**TAR1-5d6:** 7U linker 2^{nd} dAb=dAb1 - R1:1µg/ml Ag immunotube overnight wash, R2:beads

The 6 lead clones were examined further. Protein was produced from the periplasm and supernatant, purified with protein L agarose and examined in the cell and receptor assays. The levels of neutralisation were variable (Table 1). The optimal conditions for protein preparation were determined. Protein produced from HB2151 cells as supernatants gave the highest yield (approximately 10mgs/L of culture). The supernatants were incubated with protein L agarose for 2hrs at room temperature or overnight at 4°C. The beads were washed with PBS/NaCl and packed onto an FPLC column using a peristaltic pump. The beads were washed with 10 column volumes of PBS/NaCl and eluted with 0.1M glycine pH2.5. In general, dimeric protein is eluted after the monomer.

TAR1-5d1-6 dimers were purified by FPLC. Three species were obtained, by FPLC purification and were identified by SDS PAGE. One species corresponds to monomer and the other two species corresponds to dimers of different sizes. The larger of the two species is possibly due to the presence of C terminal tags. These proteins were examined in the receptor assay. The data presented in table 1 represents the optimum results obtained from the two dimeric species (Figure 11)

The three second dAbs from the dimer pairs (ie, dAb1, dAb2 and dAb3) were cloned as monomers and examined by ELISA and in the cell and receptor assay. All three dAbs bind specifically to TNF by antigen ELISA and do not cross react with plastic or BSA. As monomers, none of the dAbs neutralise in the cell or receptor assays.

### 2.1.2 TAR1-5-19 dimers

TAR1-5-19 was substituted for TAR1-5 in the 6 lead clones. Analysis of all TAR1-5-19 dimers in the cell and receptor assays was conducted using total protein (protein L purified only) unless otherwise stated (Table 2). TAR1-5-19d4 and TAR1-5-19d3 have the best ND₅₀ (~5nM) in the cell assay, this is consistent with the receptor assay results and is an improvement over TAR1-5-19 monomer (ND₅₀~30nM). Although purified TAR1-5 dimers give variable results in the receptor and cell assays TAR1-5-19 dimers were more consistent. Variability was shown when using different elution buffers during the protein purification. Elution using 0.1M Phosphate-citrate buffer pH2.6 or 0.2M Glycine pH2.5 although removing all protein from the protein L agarose in most cases rendered it less functional.

TAR1-5-19d4 was expressed in the fermenter and purified on cation exchange FPLC to yield a completely pure dimer. As with TAR1-5d4 three species were obtained, by FPLC purification corresponding to monomer and two dimer species. This dimer was amino acid sequenced. TAR1-5-19 monomer and TAR1-5-19d4 were then examined in the receptor assay and the resulting IC50 for monomer was 30nM and for dimer was 8nM. The results of the receptor assay comparing TAR1-5-19 monomer, TAR1-5-19d4 and TAR1-5d4 is shown in figure 10.

TAR1-5-19 homodimers were made in the 3U, 5U and 7U vectors, expressed and purified on Protein L. The proteins were examined in the cell and receptor assays and the resulting IC₅₀s (for receptor assay) and ND₅₀s (for cell assay) were determined (table 3, figure 12).

### 2.2 Fabs

TAR1-5 and TAR1-5-19 dimers were also cloned into Fab format, expressed and purified on protein L agarose. Fabs were assessed in the receptor assays (Table 4). The results showed that for both TAR1-5-19 and TAR1-5 dimers the neutralisation levels were similar to the original Gly₄Ser linker dimers from which they were derived. A TAR1-5-19 Fab where TAR1-5-19 was displayed on both CH and CK was expressed, protein L purified and assessed in the receptor assay. The resulting IC50 was approximately 1nM.

### 2.3 TAR1-27 dimers

3 x 96 clones were picked from the round 2 selection encompassing all the libraries and selection conditions. 2ml supernatant preps were made for analysis in ELISA and bioassays. Antigen ELISA gave 71 positive clones. The receptor assay of crude supernatants yielded 42 clones with inhibitory properties (TNF binding 0-60%). In the majority of cases inhibitory properties correlated with a strong ELISA signal. 42 clones were sequenced, 39 of these have unique second dAb sequences. The 12 dimers that gave the best inhibitory properties were analysed further.

The 12 neutralising clones were expressed as 200ml supernatant preps and purified on protein L. These were assessed by protein L and antigen ELISA, BIAcore and in the receptor assay. Strong positive ELISA signals were obtained in all cases. BIAcore analysis revealed all clones to have fast on and off rates. The off rates were improved compared to monomeric TAR1-27, however the off rate of TAR1-27 dimers was faster (Koff is approximately in the range of 10⁻¹ and 10⁻²M) than the TAR1-5 dimers examined previously (Koff is approximately in the range of 10⁻³ - 10⁻⁴M). The stability of the purified dimers was questioned and therefore in order to improve stability, the addition on 5%glycerol, 0.5% Triton X100 or 0.5% NP40 (Sigma) was included in the purification of 2 TAR1-27 dimers (d2 and d16). Addition of NP40 or Triton X100^{™} improved the yield of purified product approximately 2 fold. Both dimers were assessed in the receptor assay. TAR1-27d2 gave IC50 of ~30nM under all purification conditions. TAR1-27d16 showed no neutralisation effect when purified without the use of stabilising agents but gave an IC50 of ~50nM when purified under stabilising conditions. No further analysis was conducted.

### 2.4 TAR2-5 dimers

3 x 96 clones were picked from the second round selections encompassing all the libraries and selection conditions. 2ml supernatant preps were made for analysis. Protein A and antigen ELISAs were conducted for each plate. 30 interesting clones were identified as having good off-rates by BIAcore (Koff ranges between 10⁻² - 10⁻³M). The clones were sequenced and 13 unique dimers were identified by sequence analysis.

**Table 1: TAR1-5 dimers**

| **Dimer** | **Cell type** | **Purification** | **Protein Fraction** | **Elution conditions** | **Receptor/ Cell assay** |
|---|---|---|---|---|---|
| TAR1-5d1 | HB2151 | Protein L + FPLC | small dimeric species | 0.1M glycine pH2.5 | RA~30nM |
| TAR1-5d2 | HB2151 | Protein L + FPLC | small dimeric species | 0.1M glycine pH2.5 | RA~50nM |
| TAR1-5d3 | HB2151 | Protein L + FPLC | large dimeric species | 0.1M glycine pH2.5 | RA-300 nM |
| TAR1-5d4 | HB2151 | Protein L + FPLC | small dimeric species | 0.1M glycine pH2.5 | RA-3n M |
| TARl-5d5 | HB2151 | Protein L + FPLC | large dimeric species | 0.1M glycine pH2.5 | RA~200 nM |
| TAR1-5d6 | HB2151 | Protein L +FPLC | Large dimeric species | 0.1M glycine pH2.5 | RA~100 nM |

| | | | | | |
|---|---|---|---|---|---|
| *note dimer 2 and dimer 3 have the same second dAb (called dAb2), however have different linker lengths (d2 = (Gly₄Ser)₃, d3 = (Gly₄Ser)₃). dAb1 is the partner dAb to dimers 1, 5 and 6. dAb3 is the partner dAb to dimer4. None of the partner dAbs neutralise alone. FPLC purification is by cation exchange unless otherwise stated. The optimal dimeric species for each dimer obtained by FPLC was determined in these assays. | | | | | |

**Table 2: TAR1-5-19 dimers**

| Dimer | Cell type | Purification | Protein Fraction | Elution conditions | Recept or/ Cell assay |
|---|---|---|---|---|---|
| TAR1-5-19 d1 | TOP10F' | Protein L | Total protein | 0.1M glycine pH 2.0 | RA~15 nM |
| TAR1-5-19 d2 (no stop codon) | TOP10F' | Protein L | Total protein | 0.1M glycine pH 2.0 + 0.05%NP40 | RA~2n M |
| TAR1-5-19d3 (no stop codon) | TOP10F' | Protein L | Total protein | 0.1M glycine pH 2.5 + 0.05%NP4O | RA~8n M |
| TAR1-5-19d4 | TOP10F' | Protein L + FPLC | FPLC purified fraction | 0.1M glycine pH2.0 | RA~2-5nM |
| | | | | | CA~12 nM |
| TAR1-5-19d5 | TOPIOF' | Protein L | Total protein | 0.1M glycine pH2.0 + NP40 | RA~8n M |
| | | | | | CA~10 nM |
| TAR1-5-19 d6 | TOP10F' | Protein L | Total protein | 0.1M glycine pH 2.0 | RA~10 nM |

**Table 3: TARI-5-19 homodimers**

| Dimer | Cell type | Purification | Protein Fraction | Elution conditions | Recept or/Cell assay |
|---|---|---|---|---|---|
| TAR1-5-19 3U homodimer | HB2151 | Protein L | Total protein | 0.1M glycine pH2.5 | RA-20 nM CA-30 nM |
| TAR1-5-19 5U homodimer | HB2151 | Protein L | Total protein | 0.1M glycine pH2.5 | RA-2n M CA-3n M |
| TAR1-5-19 7U homodimer | HB2151 | Protein L | Total protein | 0.1M glycine pH2.5 | RA~10 nM CA-15 nM |
| TAR1-5-19 cys hinge | HB2151 | Protein L+FPLC | FPLC purified dimer fraction | 0.1M glycine pH2.5 | RA~2nM |
| TAR1-5-19CH/ TAR1-5-19 CK | HB2151 | Protein | Total protein | 0.1M glycine pH2.5 | RA~1nM |

**Table 4: TAR1-5/TAR1-5-19 Fabs**

| **Dimer** | **Cell type** | **Purification** | **Protein Fraction** | **Elution conditions** | **Rece ptor/ Cell assay** |
|---|---|---|---|---|---|
| TAR1-5CH/ dAb1 CK | HB2151 | Protein L | Total protein | 0.1M citrate pH2.6 | RA~90 nM |
| TAR1-5CH/ dAb2 CK | HB2151 | Protein L | Total protein | 0.1M glycine pH2.5 | RA~30 nM |
| | | | | | CA~60 nM |
| dAb3CH/ TAR1-5CK | HB2151 | Protein L | Total protein | 0,1M citrate pH2.6 | RA~10 0nM |
| TAR1-5-19CH/ dAb1 CK | HB2151 | Protein L | Total protein | 0.1M glycine pH2.0 | RA~6n M |
| dAb1 CH/ TAR1-5-19CK | HB2151 | ProteinL | 0.1M glycine pH2,0 | Myc/flag | RA~6n M |
| TAR1-5-19CH/ dAb2 CK | HB2151 | Protein L | Total protein | 0.1M glycine pH2.0 | RA~8n M |
| | | | | | CA~12 nM |
| TAR1-5-19CH/ dAb3CK | HB2151 | Protein L | Total protein | 0.1M glycine pH2.0 | RA~3n M |

### Example 7

### dAb dimerisation by terminal cysteine linkage

### Summary

For dAb dimerisation, a free cysteine has been engineered at the C-terminus of the protein. When expressed the protein forms a dimer which can be purified by a two step purification method.

### PCR construction of TAR1-5-19CYS dimer

See example 8 describing the dAb trimer. The trimer protocol gives rise to a mixture of monomer, dimer and trimer.

### Expression and purification of TAR1-5-19CYS dimer

The dimer was purified from the supernatant of the culture by capture on Protein L agarose as outlined in the example 8.

### Separation of TAR1-5-19CYS monomer from the TAR1-5-19CYS dimer

Prior to cation exchange separation, the mixed monomer/dimer sample was buffer exchanged into 50 mM sodium acetate buffer pH 4.0 using a PD-10 column (Amersham Pharmacia), following the manufacturer's guidelines. The sample was then applied to a ImL Resource S cation exchange column (Amersham Pharmacia), which had been pre-equilibrated with 50 mM sodium acetate pH 4.0. The monomer and dimer were separated using the following salt gradient in 50 mM sodium acetate pH 4.0:
150 to 200 mM sodium chloride over 15 column volumes
200 to 450 mM sodium chloride over 10 column volumes
450 to 1000 mM sodium chloride over 15 column volumes

Fractions containing dimer only were identified using SDS-PAGE and then pooled and the pH increased to 8 by the addition of 1/5 volume of 1M Tris pH 8.0.

### In vitro functional binding assay: TNF receptor assay and cell assay

The affinity of the dimer for human TNFα was determined using the TNF receptor and cell assay. IC50 in the receptor assay was approximately 0.3-0.8 nM; ND50 in the cell assay was approximately 3-8 nM.

### Other possible TAR1-5-19CYS dimer formats

### PEG dimers and custom synthetic maleimide dimers

Nektar (Shearwater) offer a range of bi-maleimide PEGs [mPEG2-(MAL)2 or mPEG-(MAL)2] which would allow the monomer to be formatted as a dimer, with a small linker separating the dAbs and both being linked to a PEG ranging in size from 5 to 40 kDa. It has been shown that the 5kDa mPEG-(MAL)2 (ie, [TAR1-5-19]-Cys-maleimide-PEG x 2, wherein the maleimides are linked together in the dimer) has an affinity in the TNF receptor assay of ∼ 1-3 nM. Also the dimer can also be produced using TMEA (Tris[2-maleimidoethyl]amine) (Pierce Biotechnology) or other bi-functional linkers.

It is also possible to produce the disulphide dimer using a chemical coupling procedure using 2,2'-dithiodipyridine (Sigma Aldrich) and the reduced monomer.

### Addition of a polypeptide linker or hinge to the C-terminus of the dAb.

A small linker, either (Gly₄Ser)ₙ where n=1 to 10, eg, 1, 2, 3, 4, 5, 6 or 7, an immunoglobulin (eg, IgG hinge region or random peptide sequence (eg, selected from a library of random peptide sequences) can be engineered between the dAb and the terminal cysteine residue. This can then be used to make dimers as outlined above.

### Example 8

### dAb trimerisation

### Summary

For dAb trimerisation, a free cysteine is required at the C-terminus of the protein. The cysteine residue, once reduced to give the free thiol, can then be used to specifically couple the protein to a trimeric maleimide molecule, for example TMEA (Tris[2-maleimidoethyl]amine).

### PCR construction of TAR1-5-19CYS

The following oligonucleotides were used to specifically PCR TAR1-5-19 with a *Sal*I and *Bam*HI sites for cloning and also to introduce a C-terminal cysteine residue: (* start of TAR1-5-19CYS sequence)
Forward primer
5'-TGGAGCGCGTCGACGGACATCCAGATGACCCAGTCTCCA-3'
Reverse primer
5'-TTAGCAGCCGGATCCTTATTAGCACCGTTTGATTTCCAC-3'

The PCR reaction (50µL volume) was set up as follows: 200µM dNTPs, 0.4µM of each primer, 5 µL of 10x *Pfu*Turbo buffer (Stratagene), 100 ng of template plasmid (encoding TAR1-5-19), 1µL of *Pfu*Turbo enzyme (Stratagene) and the volume adjusted to 50µL using sterile water. The following PCR conditions were used: initial denaturing step 94 °C for 2 mins, then 25 cycles of 94 °C for 30 secs, 64 °C for 30 sec and 72 °C for 30 sec. A final extension step was also included of 72 °C for 5 mins. The PCR product was purified and digested with *Sal*I and *Bam*HI and ligated into the vector which had also been cut with the same restriction enzymes. Correct clones were verified by DNA sequencing.

### Expression and purification of TAR1-5-19CYS

TAR1-5-19CYS vector was transformed into BL21 (DE3) pLysS chemically competent cells (Novagen) following the manufacturer's protocol. Cells carrying the dAb plasmid were selected for using 100µg/mL carbenicillin and 37 µg/mL chloramphenicol. Cultures were set up in 2L baffled flasks containing 500 mL of terrific broth (Sigma-Aldrich), 100µg/mL carbenicillin and 37 µg/mL chloramphenicol. The cultures were grown at 30 °C at 200rpm to an O.D.600 of 1-1.5 and then induced with 1mM IPTG (isopropyl-beta-D-thiogalactopyranoside, from Melford Laboratories). The expression of the dAb was allowed to continue for 12-16 hrs at 30 °C. It was found that most of the dAb was present in the culture media. Therefore, the cells were separated from the media by centrifugation. (8,000xg for 30 mins), and the supernatant used to purify the dAb. Per litre of supernatant, 30 mL of Protein L agarose (Affitech) was added and the dAb allowed to batch bind with stirring for 2 hours. The resin was then allowed to settle under gravity for a further hour before the supernatant was siphoned off The agarose was then packed into a XK 50 column (Amersham Phamacia) and was washed with 10 column volumes of PBS. The bound dAb was eluted with 100 mM glycine pH 2.0 and protein containing fractions were then neutralized by the addition of 1/5 volume of 1 M Tris pH 8.0. Per litre of culture supernatant 20 mg of pure protein was isolated, which contained a 50:50 ratio of monomer to dimer.

### Trimerisation of TAR1-5-19CYS

2.5 ml of 100 µM TAR1-5-19CYS was reduce with 5 mM dithiothreitol and left at room temperature for 20 minutes. The sample was then buffer exchanged using a PD-10 column (Amersham Pharmacia). The column had been pre-equilibrated with 5 mM EDTA, 50 mM sodium phosphate pH 6.5, and the sample applied and eluted following the manufactures guidelines. The sample was placed on ice until required. TMEA (Tris[2-maleimidoethyl]amine) was purchased from Pierce Biotechnology. A 20 mM stock solution of TMEA was made in 100% DMSO (dimethyl sulphoxide). It was found that a concentration of TNMA greater than 3:1 (molar ratio of dAb:TMEA) caused the rapid precipitation and cross-linking of the protein. Also the rate of precipitation and cross-linking was greater as the pH increased. Therefore using 100 µM reduced TAR1-5-19CYS, 25 µM TMEA was added to trimerise the protein and the reaction allowed to proceed at room temperature for two hours. It was found that the addition of additives such as glycerol or ethylene glycol to 20% (v/v), significantly reduced the precipitation of the trimer as the coupling reaction proceeded. After coupling, SDS-PAGE analysis showed the presence of monomer, dimer and trimer in solution.

### Purification of the trimeric TAR1-5-19CYS

40 µL of 40% glacial acetic acid was added per mL of the TMEA-TAR1-5-19cys reaction to reduce the pH to ~4. The sample was then applied to a 1mL Resource S cation exchange column (Amersham Pharmacia), which had been pre-equilibrated with 50 mM sodium acetate pH 4.0. The dimer and trimer were partially separated using a salt gradient of 340 to 450 mM Sodium chloride, 50 mM sodium acetate pH 4.0 over 30 column volumes. Fractions containing trimer only were identified using SDS-PAGE and then pooled and the pH increased to 8 by the addition of 1/5 volume of 1M Tris pH 8.0. To prevent precipitation of the trimer during concentration steps (using 5K cut off Viva spin concentrators; Vivascience), 10% glycerol was added to the sample.

### In vitro functional binding assay: TNF receptor assay and cell assay

The affinity of the trimer for human TNFα was determined using the TNF receptor and cell assay. IC50 in the receptor assay was 0.3nM; ND50 in the cell assay was in the range of 3 to 10nM (eg, 3nM).

### Other possible TAR1-5-19CYS trimer formats

TAR1-5-19CYS may also be formatted into a trimer using the following reagents:

### PEG trimers and custom synthetic maleimide trimers

Nektar (Shearwater) offer a range of multi arm PEGs, which can be chemically modified at the terminal end of the PEG. Therefore using a PEG trimer with a maleimide functional group at the end of each arm would allow the trimerisation of the dAb in a manner similar to that outlined above using TMEA. The PEG may also have the advantage in increasing the solubility of the trimer thus preventing the problem of aggregation. Thus, one could produce a dAb trimer in which each dAb has a C-terminal cysteine that is linked to a maleimide functional group, the maleimide functional groups being linked to a PEG trimer.

### Addition of a polypeptide linker or hinge to the C-terminus of the dAb

A small linker, either (Gly₄Ser)ₙ where n= 1 to 10, eg, 1, 2, 3, 4, 5, 6 or 7, an immunoglobulin (eg, IgG hinge region or random peptide sequence (eg, selected from a library of random peptide sequences) could be engineered between the dAb and the terminal cysteine residue. When used to make multimers (eg, dimers or trimers), this again would introduce a greater degree of flexibility and distance between the individual monomers, which may improve the binding characteristics to the target, eg a multisubunit target such as human TNFα.

### Example 9.

### Selection of a collection of single domain antibodies (dAbs) directed against human serum albumin (HSA) and mouse serum albumin (MSA).

This example explains a method for making a single domain antibody (dAb) directed against serum albumin. Selection of dAbs against both mouse serum albumin (MSA) and human serum albumin (HSA) is described. Three human phage display antibody libraries were used in this experiment, each based on a single human framework for V_{H} (see Figure 13: sequence of dummy V_{H} based on V3-23/DP47 and JH4b) or V_{κ} (see Figure 15: sequence of dummy V_{κ} based on o12/o2/DPK9 and Jkl) with side chain diversity encoded by NNK codons incorporated in complementarity determining regions (CDRl, CDR2 and CDR3).
Library 1 (V_{H}):
Diversity at positions: H30, H31, H33, H35, H50, H52, H52a, H53, H55, H56, H58, H95, H97, H98.
Library size: 6.2 x 10⁹
Library 2 (V_{H}):
Diversity at positions: H30, H31, H33, H35, H50, H52, H52a, H53, H55, H56, H58, H95, H97, H98, H99, H100, H100a, H100b.
Library size: 4.3 x 10⁹
Library 3 (Vκ):
Diversity at positions: L30, L31, L32, L34, L50, L53, L91, L92, L93, L94, L96
Library size: 2 x 10⁹

The V_{H} and V_{κ} libraries have been preselected for binding to generic ligands protein A and protein L respectively so that the majority of clones in the unselected libraries are functional. The sizes of the libraries shown above correspond to the sizes after preselection.

Two rounds of selection were performed on serum albumin using each of the libraries separately. For each selection, antigen was coated on immunotube (nunc) in 4ml of PBS at a concentration of 100µg/ml. In the first round of selection, each of the three libraries was panned separately against HSA (Sigma) and MSA (Sigma). In the second round of selection, phage from each of the six first round selections was panned against (i) the same antigen again (eg 1^{st} round MSA, 2^{nd} round MSA) and (ii) against the reciprocal antigen (eg 1^{st} round MSA, 2^{nd} round HSA) resulting in a total of twelve 2^{nd} round selections. In each case, after the second round of selection 48 clones were tested for binding to HSA and MSA. Soluble dAb fragments were produced as described for scFv fragments by Harrison *et al*, Methods Enzymol. 1996;267:83-109 and standard ELISA protocol was followed (Hoogenboom *et al*. (1991) Nucleic Acids Res., 19: 4133) except that 2% tween PBS was used as a blocking buffer and bound dAbs were detected with either protein L-HRP (Sigma) (for the Vκs) and protein A -HRP (Amersham Phannacia Biotech) (for the V_{H}s).

dAbs that gave a signal above background indicating binding to MSA, HSA or both were tested in ELISA insoluble form for binding to plastic alone but all were specific for serum albumin. Clones were then sequenced (see table below) revealing that 21 unique dAb sequences had been identified. The minimum similarity (at the amino acid level) between the V_{*K*} dAb clones selected was 86.25% ((69/80)x100; the result when all the diversified residues are different, eg clones 24 and 34). The minimum similarity between the V_{H} dAb clones selected was 94 % ((127/136)x100).

Next, the serum albumin binding dAbs were tested for their ability to capture biotinylated antigen from solution. ELISA protocol (as above) was followed except that ELISA plate was coated with 1µg/ml protein L (for the Vκ clones) and 1µg/ml protein A (for the V_{H} clones). Soluble dAb was captured from solution as in the protocol and detection was with biotinylated MSA or HSA and streptavidin HRP. The biotinylated MSA and HSA had been prepared according to the manufacturer's instructions, with the aim of achieving an average of 2 biotins per serum albumin molecule. Twenty four clones were identified that captured biotinylated MSA from solution in the ELISA. Two of these (clones 2 and 38 below) also captured biotinylated HSA. Next, the dAbs were tested for their ability to bind MSA coated on a CM5 biacore chip. Eight clones were found that bound MSA on the biacore.

| dAb (all capture biotinylated MSA) | H or κ CDR1 | | CDR2 | CDR3 | Binds MSA In biacore? | Captures biotinylated HSA? |
|---|---|---|---|---|---|---|
| V_{κ} library 3 template (dummy) | κ | XXXLX | XASXLQS | QOXXXXPXT | | |
| 2,4,7,41, | κ | SSYLN | RASPLQS | QQTYSVPPT | | 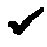 all 4 bind |
| 38,54 | κ | SSYLN | RASPLQS | QQTYRIPPT | | 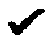 both bind |
| 46,47,52,56 | κ | PKSLK | NASYLQS | QQVVYWPVT | | |
| 13,15 | κ | YYHLK | KASTLQS | QQVRKVPRT | | |
| 30, 35 | κ | RRYLK | QASVLQS | QOGLYPPIT | | |
| 19, | κ | YNWLK | RASSLQS | QQNVVIPRT | | |
| 22, | κ | LWHLR | HASLLQS | QQSAVYPKT | | |
| 23, | κ | FRYLA | HASHLQS | QQRLLYPKT | | |
| 24, | κ | FYHLA | PASKLQS | QQRARWPRT | | |
| 31, | κ | IWHLN | RASRLQS | QQVARVPRT | | |
| 33, | κ | YRYLR | KASSLQS | QQYVGYPRT | | |
| 34, | κ | LKYLK | NASRLQS | QQTTYYPIT | | |
| 53, | κ | LRYLR | KASWLQS | QQVLYYPQT | | |
| 11, | κ | LRSLR | AASRLQS | QQVVYWPAT | 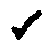 | |
| 12, | κ | FRHLK | AASRLQS | QQVALYPKT | 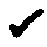 | |
| 17, | κ | RKYLR | TASSLQS | QQNLPWPRT | 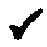 | |
| 18, | κ | RRYLN | AASSLQS | QQMLFYPKT | 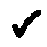 | |
| 16, 21 | κ | IKHLK | GASRLQS | QQGARWPQT | 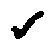 | |
| 25, 26 | κ | YYHLK | KASTLQS | QQVRKVPRT | 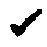 | |
| 27, | κ | YKHLK | NRSHLQS | QQVGRYPRT | 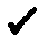 | |
| 55, | κ | FKSLK | NASYLQS | QQVVYWPVT | 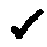 | |
| V_{H} library 1 | | | | | | |
| (and 2) | | | | | | |
| template | | | | | | |
| (dummy) | H | XXYXXX | XIXXXGXXTXYADSVKG | XXXX(XXXX) FDY | | |
| 8, 10 | H | WVYQMD | SISAFGAKTLYADSVKG | LSGKFDY | | |
| 36, | H | WSYQMT | SISSFGSSTLYADSVKG | GRDHNYSLFDY | | |

In all cases the frameworks were identical to the frameworks in the corresponding dummy sequence, with diversity in the CDRs as indicated in the table above.

Of the eight clones that bound MSA on the biacore, two clones that are highly expressed in *E. coli* (clones MSA16 and MSA26) were chosen for further study (see example 10). Full nucleotide and amino acid sequences for MSA16 and 26 are given in figure 16.

### Example 10.

### Determination of affinity and serum half-life in mouse of MSA binding dAbs MSA16 and MSA26.

dAbs MSA16 and MSA26 were expressed in the periplasm of *E. coli* and purified using batch absorbtion to protein L-agarose affinity resin (Affitech, Norway) followed by elution with glycine at pH 2.2. The purified dAbs were then analysed by inhibition biacore to determine K_{d}. Briefly, purified MSA16 and MSA26 were tested to determine the concentration of dAb required to achieve 200RUs of response on a biacore CM5 chip coated with a high density of MSA. Once the required concentrations of dAb had been determined, MSA antigen at a range of concentrations around the expected K_{d} was premixed with the dAb and incubated overnight. Binding to the MSA coated biacore chip of dAb in each of the premixes was then measured at a high flow-rate of 30 µl/minute. The resulting curves were used to create Klotz plots, which gave an estimated K_{d} of 200nM for MSA16 and 70nM for MSA 26 (Figure 17 A & B).

Next, clones MSA16 and MSA26 were cloned into an expression vector with the HA tag (nucleic acid sequence: TATCCTTATGATGTTCCTGATTATGCA and amino acid sequence: YPYDVPDYA) and 2-10 mg quantities were expressed in *E. coli* and purified from the supernatant with protein L-agarose affinity resin (Affitech, Norway) and eluted with glycine at pH2.2. Serum half life of the dAbs was determined in mouse. MSA26 and MSA16 were dosed as single i.v. injections at approx 1.5mg/kg into CD1 mice. Analysis of serum levels was by goat anti-HA (Abcam, UK) capture and protein L-HRP (invitrogen) detection ELISA which was blocked with 4% Marvel. Washing was with 0.05% tween PBS. Standard curves of known concentrations of dAb were set up in the presence of 1xmouse serum to ensure comparability with the test samples. Modelling with a 2 compartment model showed MSA-26 had a t1/2α of 0.16hr, a t1/2β of 14.5hr and an area under the curve (AUC) of 465hr.mg/ml (data not shown) and MSA-16 had a t1/2α of 0.98hr, a t1/2β of 36.5hr and an AUC of 913hr.mg/ml (figure 18). Both anti-MSA clones had considerably lengthened half life compared with HEL4 (an anti-hen egg white lysozyme dAb) which had a t1/2α of 0.06hr, and a t1/2β of 0.34hr.

### Example 11.

### Creation of V_{H}-V_{H} and V_{K}- V_{K} dual specific Fab like fragments

This example describes a method for maldag V_{H}- V_{H} and V_{*K*}-V_{*K*} dual specifics as Fab like fragments. Before constructing each of the Fab like fragments described, dAbs that bind to targets of choice were first selected from dAb libraries similar to those described in example 9. A V_{H} dAb, HEL4, that binds to hen egg lysozyme (Sigma) was isolated and a second V_{H} dAb (TAR2h-5) that binds to TNFα receptor (R and D systems) was also isolated. The sequences of these are given in the sequence listing. A V_{κ} dAb that binds TNFα (TAR1-5-19) was isolated by selection and affinity maturation and the sequence is also set forth in the sequence listing. A second V_{κ} dAb (MSA 26) described in example 9 whose sequence is in figure 17B was also used in these experiments.

DNA from expression vectors containing the four dAbs described above was digested with enzymes SalI and NotI to excise the DNA coding for the dAb. A band of the expected size (300-400bp) was purified by running the digest on an agarose gel and excising the band, followed by gel purification using the Qiagen gel purification kit (Qiagen, UK). The DNA coding for the dAbs was then inserted into either the C_{H} or C_{*K*} vectors (Figs 8 and 9) as indicated in the table below.

| dAb | Target antigen | dAb V_{H} or dAb Vκ | Inserted into vector | tag ( C terminal) | Antibiotic resisitance |
|---|---|---|---|---|---|
| HEL4 | Hen egg lysozyme | V_{H} | C_{H} | myc | Chloramphenicol |
| TAR2-5 | TNF receptor | V_{H} | Cκ | flag | Ampicillin |
| TAR1-5-19 | TNFα | Vκ | C_{H} | myc | Chloramphenicol |
| MSA 26 | Mouse serum albumin | Vκ | Cκ | flag | Ampicillin |

The V_{H} C_{H} and V_{H} Cκ constructs were cotransformed into HB2151 cells. Separately, the V_{*K*} C_{H} and V_{*K*} C_{*K*} constructs were cotransformed into HB2151 cells. Cultures of each of the cotransformed cell lines were grown overnight (in 2xTy containing 5% glucose, 10µg/ml chloramphenicol and 100µg/ml ampicillin to maintain antibiotic selection for both C_{H} and C_{*K*} plasmids). The overnight cultures were used to inoculate fresh media (2xTy, 10µg/ml chloramphenicol and 100µg/ml ampicillin) and grown to OD 0.7-0.9 before induction by the addition of IPTG to express their C_{H} and C_{*K*} constructs. Expressed Fab like fragment was then purified from the periplasm by protein A purification (for the contransformed V_{H} C_{H} and V_{H} Cκ) and MSA affinity resin purification (for the contransformed V_{*K*} C_{H} and V_{*K*} C_{*K*}).

### V_{H}-V_{H} dual specific

Expression of the V_{H} C_{H} and V_{H} C_{*K*} dual specific was tested by running the protein on a gel. The gel was blotted and a band the expected size for the Fab fragment could be detected on the Western blot via both the myc tag and the flag tag, indicating that both the V_{H} C_{H} and V_{H} Cκ parts of the Fab like fragment were present. Next, in order to determine whether the two halves of the dual specific were present in the same Fab-like fragment, an ELISA plate was coated overnight at 4°C with 100 µl per well of hen egg lysozyme (HEL) at 3 mg/ml in sodium bicarbonate buffer. The plate was then blocked (as described in example 1) with 2% tween PBS followed by incubation with the V_{H} C_{H} /V_{H} Cκ dual specific Fab like fragment. Detection of binding of the dual specific to the HEL was via the non cognate chain using 9e10 (a monoclonal antibody that binds the myc tag, Roche) and anti mouse IgG-HRP (Amersham Pharmacia Biotech). The signal for the V_{H} C_{H} /V_{H} Cκ dual specific Fab like fragment was 0.154 compared to a background signal of 0.069 for the V_{H} Cκ chain expressed alone. This demonstrates that the Fab like fragment has binding specificity for target antigen.

### V_{κ}-V_{κ} dual specific

After purifying the contransformed Vκ C_{H} and Vκ Cκ dual specific Fab like fragment on an MSA affinity resin, the resulting protein was used to probe an ELISA plate coated with 1µg/ml TNFα and an ELISA plate coated with 10µg/ml MSA. As predicted, there was signal above background when detected with protein L-HRP on bot ELISA plates (data not shown). This indicated that the fraction of protein able to bind to MSA (and therefore purified on the MSA affinity column) was also able to bind TNFα in a subsequent ELISA, confirming the dual specificity of the antibody fragment. This fraction of protein was then used for two subsequent experiments. Firstly, an ELISA plate coated with 1µg/ml TNFα was probed with dual specific Vκ C_{H} and Vκ Cκ Fab like fragment and also with a control TNFα binding dAb at a concentration calculated to give a similar signal on the ELISA. Both the dual specific and control dAb were used to probe the ELISA plate in the presence and in the absence of 2mg/ml MSA. The signal in the dual specific well was reduced by more than 50% but the signal in the dAb well was not reduced at all (see figure 19a). The same protein was also put into the receptor assay with and without MSA and competition by MSA was also shown (see figure 19c). This demonstrates that binding of MSA to the dual specific is competitive with binding to TNFα.

### Example 12.

### Creation of a Vκ- Vκ dual specific cys bonded dual specific with specificity for mouse serum albumin and TNFα

This example describes a method for making a dual specific antibody fragment specific for both mouse serum albumin and TNFα by chemical coupling via a disulphide bond. Both MSA16 (from example 1) and TAR1-5-19 dAbs were recloned into a pET based vector with a C terminal cysteine and no tags. The two dAbs were expressed at 4-10 mg levels and purified from the supernatant using protein L-agarose affinity resin (Affitiech, Norway). The cysteine tagged dAbs were then reduced with dithiothreitol. The TAR1-5-19 dAb was then coupled with dithiodipyridine to block reformation of disulphide bonds resulting in the formation of PEP 1-5-19 homodimers. The two different dAbs were then mixed at pH 6.5 to promote disulphide bond formation and the generation of TAR1-5-19, MSA16 cys bonded heterodimers. This method for producing conjugates of two unlike proteins was originally described by King *et al*. (King TP, Li Y Kochoumian L Biochemistry. 1978 vol17:1499-506 Preparation of protein conjugates via intermolecular disulfide bond formation.) Heterodimers were separated from monomeric species by cation exchange. Separation was confirmed by the presence of a band of the expected size on a SDS gel. The resulting heterodimeric species was tested in the TNF receptor assay and found to have an IC50 for neutralising TNF of approximately 18 nM. Next, the receptor assay was repeated with a constant concentration of heterodimer (18nM) and a dilution series of MSA and HSA. The presence of HSA at a range of concentrations (up to 2 mg/ml) did not cause a reduction in the ability of the dimer to inhibit TNFα. However, the addition of MSA caused a dose dependant reduction in the ability of the dimer to inhibit TNFα (figure 20).This demonstrates that MSA and TNFα compete for binding to the cys bonded TAR1-5-19, MSA16 dimer.

### Data Summary

A summary of data obtained in the experiments set forth in the foregoing examples is set forth in Annex 4.

All publications mentioned in the present specification, and references cited in said publications, are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

### Annex 1; polypeptides which enhance half-life in vivo.

Alpha-1 Glycoprotein (Orosomucoid) (AAG)
Alpha-1 Antichyromotrypsin (ACT)
Alpha-1 Antitrypsin (AAT)
Alpha-1 Microglobulin (Protein HC) (AIM)
Alpha-2 Macroglobulin (A2M)
Antithrombin III (AT III)
Apolipoprotein A-1 (Apo A-1)
Apoliprotein B (Apo B)
Beta-2-microglobulin (B2M)
Ceruloplasmin (Cp)
Complement Component (C3)
Complement Component (C4)
C1 Esterase Inhibitor (C1 INH)
C-Reactive Protein (CRP)
Cystatin C (Cys C)
Ferritin (FER)
Fibrinogen (FIB)
Fibronectin (FN)
Haptoglobin (Hp)
Hemopexin (HPX)
Immunoglobulin A (IgA)
Immunoglobulin D (IgD)
Immunoglobulin E (IgE)
Immunoglobulin G (IgG)
Immunoglobulin M (IgM)
Immunoglobulin Light Chains (kapa/lambda)
Lipoprotein(a) [Lp(a)]
Mannose-bindign protein (MBP)
Myoglobin (Myo)
Plasminogen (PSM)
Prealbiunin (Transthyretin) (PAL)
Retinol-binding protein (RBP)
Rheomatoid Factor (RF)
Serum Amyloid A (SAA)
Soluble Tranferrin Receptor (sTfR)
Transferrin (Tf)

### Annex 2

| | |
|---|---|
| **Pairing** | **Therapeutic relevant references.** |
| TNF ALPHA/TGF-β | • TGF-b and TNF when injected into the ankle joint of collagen induced arthritis model significantly enhanced joint inflammation. In non-collagen challenged mice there was no effect. |
| TNF ALPHA/IL-1 | • TNF and IL-1 synergize in the pathology of uveitis. |
| | • TNF and IL-1 synergize in the pathology of malaria (hypoglycaemia, NO). |
| | • TNF and IL-1 synergize in the induction of polymorphonuclear (PMN) cells migration in inflammation. |
| | • IL-1 and TNF synergize to induce PMN infiltration into the peritoneum. |
| | • IL-1 and TNF synergize to induce the secretion of IL-1 by endothelial cells. Important in inflammation. |
| | • IL-1 or TNF alone induced some cellular infiltration into knee synovium. IL-1 induced PMNs, TNF - monocytes. Together they induced a more severe infiltration due to increased PMNs. |
| | • Circulating myocardial depressant substance (present in sepsis) is low levels of IL-1 and TNFacting synergistically. |
| TNF ALPHA/IL-2 | • Most relating to synergisitic activation of killer T-cells. |
| TNF ALPHA/IL-3 | • Synergy of interleukin 3 and tumor necrosis factor alpha in stimulating clonal growth of acute myelogenous leukemia blasts is the result of induction of secondary hematopoietic cytokines by tumor necrosis factor alpha. |
| | • Cancer Res. 1992 Apr 15;**52**(8):2197-201. |
| TNF ALPHA/IL-4 | • IL-4 and TNF synergize to induce VCAM expression on endothelial cells. Implied to have a role in asthma. Same for synovium - implicated in RA. |
| | • TNF and IL-4 synergize to induce IL-6 expression in keratinocytes. |
| | • Sustained elevated levels of VCAM-1 in cultured fibroblast-like synoviocytes can be achieved by TNF-alpha in combination with either IL-4 or IL-13 through increased mRNA stability. *Am J Pathol.* 1999 Apr;**154**(4):1149-58 |
| TNF ALPHA/IL-5 | • Relationship between the tumor necrosis factor system and the serum interleukin-4, interleukin-5, interleukin-8, eosinophil cationic protein, and immunoglobulin E levels in the bronchial hyperreactivity of adults and their children. *Allergy Asthma Proc*. 2003 Mar-Apr;**24**(2):111-8. |
| TNF ALPHA/IL-6 | • TNF and IL-6 are potent growth factors for OH-2, a novel human myeloma cell line. *Eur J Haematol*. 1994 Jul;**53**(1):31-7. |
| TNF ALPHA/IL-8 | • TNF and IL-8 synergized with PMNs to activate platelets. Implicated in Acute Respiratory Distress Syndrome. |
| | • See IL-5/TNF (asthma). Synergism between interleukin-8 and tumor necrosis factor-alpha for neutrophil-mediated platelet activation. *Eur Cytokine Netw*. 1994 Sep-Oct;5(5):455-60. (adult respiratory distress syndrome (ARDS)) |
| TNF ALPHA/IL-9 | |
| TNF ALPHA/IL-10 | • IL-10 induces and synergizes with TNF in the induction of HIV expression in chronically infected T-cells. |
| TNF ALPHA/IL-11 | • Cytokines synergistically induce osteoclast differentiation: support by immortalized or normal calvarial cells. *Am J Physiol Cell Physiol*. 2002 Sep;**283**(3):C679-87. (Bone loss) |
| TNF ALPHA/IL-12 | |
| TNF ALPHA/IL-13 | • Sustained elevated levels of VCAM-1 in cultured fibroblast-like synoviocytes can be achieved by TNF-alpha in combination with either IL-4 or IL-13 through increased mRNA stability. Am J Pathol. 1999 Apr;**154**(4):1149-58. |
| | • Interleukin-13 and tumour necrosis factor-alpha synergistically induce eotaxin production in human nasal fibroblasts. Clin Exp Allergy. 2000 Mar;**30**(3):348-55. |
| | • Interleukin-13 and tumour necrosis factor-alpha synergistically induce eotaxin production in human nasal fibroblasts. *Clin Exp Allergy.* 2000 Mar;**30**(3):348-55 (allergic inflammation) |
| | • Implications of serum TNF-beta and IL-13 in the treatment response of childhood nephrotic syndrome. *Cytokine*. 2003 Feb 7;**21**(3):155-9. |
| TNF ALPHA/IL-14 | • Effects of inhaled tumour necrosis factor alpha in subjects with mild asthma. *Thorax.* 2002 Sep;**57**(9):774-8. |
| TNF ALPHA/IL-15 | • Effects of inhaled tumour necrosis factor alpha in subjects with mild asthma. *Thorax.* 2002 Sep;**57**(9):774-8. |
| TNF ALPHA/H, 16 | • Tumor necrosis fictor-alpha-induced synthesis of interleuldn-16 in airway epithelial cells: priming for serotonin stimulation. *Am J Respir Cell Mol Biol.* 2003 Mar,**28**(3):354-62. (airway inflammation) |
| | • Correlation of circulating interleukin 16 with proinflammaxory cytokines in patients with rheumatoid arthritis. *Rheumatology* (Oxford). 2001 Apr,**40**(4):474-5. No abstract available. |
| | • Interleukin 16 is up-regulated in Crohn's disease and participates in TNBS colitis in mice. *Gastroenterology.* 2000 Oct;**119**(4):972-82. |
| TNF ALPHAIIL-17 | • Inhibition of interleukin-17 prevents the development of arthritis in vaccinated mice challenged with Borrelia burgdorferi. *Infect Immun.* 2003 Jun;**71**(6):3437-42. |
| | • Interleukin 17 synergises with tumour necrosis factor alpha to induce cartilage destruction *in vitro. Ann Rheum Dis.* 2002 Oct;**61**(10):870-6. |
| | • A role of GM-CSF in the accumulation of neutrophils in the airways caused byIL-17 andTNF alpha. *Eur RespirJ.* 2003 Mar,**21**(3):387-93. (Airway inflammation) |
| | • Abstract Interleukin-1, tumor necrosis factor alpha, and interleukin-17 synergistically up-regulate nitric oxide and prostaglandin E2 production in explants of human osteoarthritic knee menisci. *Arthritis Rheum*. 2001 Sep;**44**(9):2078-83. |
| TNF ALPHAIIL-18 | • Association of interleukin-18 expression with enhanced levels of both inberleukin-lbeta and tumor necrosis factor alpha in knee synovial tissue of patients with rheumatoid arthritis. *Arthritis Rheum.* 2003 Feb;**48**(2):339-47. |
| | • Abstract Elevated levels of interleukin-18 and tumor necrosis factor-alpha in serum of patients with type 2 diabetes mellitus: relationship with diabetic nephropathy. *Metabolism.* 2003 May;**52**(5):605-8. |
| TNF ALPHA/IL-19 | • Abstract IL-19 induces production of IL-6 and TNF-alpha and results in cell apoptosis through TNF-alpha. *J Immunol*. 2002 Oct 15;**169**(8):4288-97. |
| TNF ALPHA/IL-20 | • Abstract Cytokines: IL-20 - a new effector in skin inflammation. *Curr Biol.* 2001 Jul 10;**11**(13):R531-4 |
| TNF ALPHA/Complem ent | • Inflammation and coagulation: implications for the septic patient. *Clin Infect Dis.* 2003 May 15;**36**(10):1259-65. Epub 2003 May 08. Review. |
| TNF ALPHA/IFN-γ | • MHC induction in the brain. |
| | • Synergize in anti-viral response/IFN-β induction. |
| | • Neutrophil activation/ respiratory burst. |
| | • Endothelial cell activation |
| | • Toxicities noted when patients treated with TNF/IFN-γ as anti-viral therapy |
| | • Fractalkine expression by human asttocytes. |
| | • Many papers on inflammatory responses - i.e. LPS, also macrophage activation. |
| | • Anti-TNF and anti-IFN-γ synergize to protect mice from lethal endotoxemia. |

| | | |
|---|---|---|
| TGF-β/IL-1 | • Prostaglndin synthesis by osteoblasts | |
| | • IL-6 production by intestinal epithelial cells (inflammation model) | |
| | • Stimulates IL-11 and IL-6 in lung fibroblasts (inflammation model) | |
| | • IL-6 and IL-8 production in the retina | |
| TGF-β/IL-6 | | • Chondrocarcoma proliferation |
| IL-1/IL-2 | | • B-cell activation |
| | | • LAK cell activation |
| | | • T-cell activation |
| | | • IL-1 synergy with IL-2 in the generation of lymphokine activated killer cells is mediated by TNF-alpha and beta (lymphotoxin). *Cytokine*. 1992 Nov;4(6):479-87. |
| IL-1/IL-3 | | |
| IL-1/IL-4 | | • B-cell activation |
| | | • IL-4 induces IL-1 expression in endothelial cell activation. |
| IL-1/IL-5 | | |
| IL-1/IL-6 | | • B cell activation |
| | | • T cell activation (can replace accessory cells) |
| | | • IL-1 induces IL-6 expression |
| | | • C3 and serum amyloid expression (acute phase response) |
| | | • HIV expression |
| | | • Cartilage collagen breakdown. |
| IL-1/IL-7 | | • IL-7 is requisite for IL-1-induced thymocyte proliferation. Involvement of IL-7 in the synergistic effects of granulocyte-macrophage colony-stimulating factor or tumor necrosis factor with IL-1. *J Immunol*. 1992 Jan 1;**148**(1):99-105. |
| IL-1/IL-8 | | |
| IL-1/IL-10 | | |
| IL-1/IL-11 | | • Cytokines synergistically induce osteoclast differentiation: support by immortalized or normal calvarial cells. *Am J Physiol Cell Physiol*. 2002 Sep;**283**(3):C679-87. (Bone loss) |
| IL-1/IL-16 | | • Correlation of circulating interleukin 16 with proinflammatory cytokines in patients with rheumatoid arthritis. *Rheumatology* (Oxford). 2001 Apr;**40**(4):474-5. No abstract available. |
| IL-1/IL-17 | | • Inhibition of interleukin-17 prevents the development of arthritis in vaccinated mice challenged with Borrelia burgdorferi. *Infect Immun*. 2003 Jun;**71**(6):3437-42. |
| | | • Contribution of interleukin 17 to human cartilage degradation and synovial inflammation in osteoarthritis. *Osteoarthritis Cartilage*. 2002 Oct;**10**(10):799-807. |
| | | • Abstract Interleukin-1, tumor necrosis factor alpha, and interleukin-17 synergistically up-regulate nitric oxide and prostaglandin E2 production in explants of human osteoarthritic knee menisci. *Arthritis Rheum*. 2001 Sep;**44**(9):2078-83. |
| IL-1/IL-18 | | • Association of interleukin-18 expression with enhanced levels of both interleukin-lbeta and tumor necrosis factor alpha in knee synovial tissue of patients with rheumatoid arthritis. *Arthritis Rheum*. 2003 Feb;**48**(2):339-47. |
| IL-1/IFN-g | | |
| IL-2/IL-3 | | • T-cell proliferation |
| | | • B cell proliferation |
| IL-2/IL-4 | | • B-cell proliferation |
| | | • T-cell proliferation |
| | | • (selectively inducing activation of CD8 and NK lymphocytes)IL-2R beta agonist P1-30 acts in synergy with IL-2, IL-4, IL-9, and IL-15: biological and molecular effects. *J Immunol*. 2000 Oct 15;**165**(8): 4312-8. |
| IL-2/IL-5 | | • B-cell proliferation/ Ig secretion |
| | | • IL-5 induces IL-2 receptors on B-cells |
| IL-2/IL-6 | | • Development of cytotoxic T-cells |
| IL-2/IL-7 | | |
| IL-2/IL-9 | | • See IL-2/IL-4 (NK-cells) |
| IL-2/IL-10 | | • B-cell activation |
| IL-2/IL-12 | | • IL-12 synergizes with IL-2 to induce lymphokine-activated cytotoxicity and perforin and granzyme gene expression in fresh human NK cells. *Cell Immunol*. 1995 Oct 1;**165**(1):33-43. (T-cell activation) |
| IL-2/IL-15 | | • See IL-2/IL-4 (NK cells) |
| | | • (T cell activation and proliferation) IL-15 and IL-2: a matter of life and death for T cells *in vivo. Nat Med.* 2001 Jan;7(1):114-8. |
| IL-2/IL-16 | | • Synergistic activation of CD4+ T cells by IL-16 and IL-2. *J Immunol*. 1998 Mar 1;**160**(5):2115-20. |
| IL-2/IL-17 | | • Evidence for the early involvement of interleukin 17 in human and experimental renal allograft rejection. *J Pathol.* 2002 Jul;**197**(3):322-32. |
| IL-2/IL-18 | | • Interleukin 18 (IL-18) in synergy with IL-2 induces lethal lung injury in mice: a potential role for cytokines, chemokines, and natural killer cells in the pathogenesis of interstitial pneumonia. *Blood* 2002 Feb 15;**99**(4):1289-98. |
| IL-2/TGF-β | | • Control of CD4 effector fate: transforming growth factor beta 1 and interleukin 2 synergize to prevent apoptosis and promote effector expansion. *J Exp Med*. 1995 Sep 1;**182**(3):699-709. |
| IL-2/IFN-γ | | • Ig secretion by B-cells |
| | | • IL-2 induces IFN-γ expression by T-cells |
| IL-2/IFN-α/β | | • None |
| IL-3/IL-4 | | • Synergize in mast cell growth |
| | | • Synergistic effects of IL-4 and either GM-CSF or IL-3 on the induction of CD23 expression by human monocytes: regulatory effects of IFN-alpha and IFN-gamma. *Cytokine*. 1994 Jul;**6**(4):407-13. |
| IL-3/IL-5 | | |
| IL-3/IL-6 | | |
| IL-3/IFN-γ | | • IL-4 and IFN-gamma synergistically increase total polymeric IgA receptor levels in human intestinal epithelial cells. Role of protein tyrosine kinases. *J Immunol*. 1996 Jun 15;**156**(12):4807-14. |
| IL-3/GM-CSF | | • Differential regulation of human eosinophil IL-3, IL-5, and GM-CSF receptor alpha-chain expression by cytokines: IL-3, IL-5, and GM-CSF down-regulate IL-5 receptor alpha expression with loss of IL-5 responsiveness, but up-regulate IL-3 receptor alpha expression. *J Immunol*. 2003 Jun 1;**170**(11):5359-66. (allergic inflammation) |
| IL-4/IL-2 | | • IL-4 synergistically enhances both IL-2- and IL-12-induced IFN-{gamma} expression in murine NK cells. *Blood.* 2003 Mar 13 [Epub ahead of print] |
| IL-4/IL-5 | | • Enhanced mast cell histamine etc. secretion in response to IgE |
| | | • A Th2-like cytokine response is involved in bullous pemphigoid, the role of IL-4 and IL-5 in the pathogenesis of the disease. *Int J Immunopathol Pharmacol*. 1999 May-Aug;**12**(2):55-61. |
| IL-4/IL-6 | | |
| IL-4/IL-10 | | |
| IL-4/IL-11 | | • Synergistic interactions between interleukin-11 and interleukin-4 in support of proliferation of primitive hematopoietic progenitors of mice. *Blood* |
| | | 1991 Sep 15;78(6):1448-51. |
| IL-4/IL-12 | | • Synergistic effects of IL-4 and IL-18 on IL-12-dependent IFN-gamma production by dendritic cells. *J Immunol.* 2000 Jan 1;164(1):64-71. (increase Th1/Th2 differentiation) |
| | | • IL-4 synergistically enhances both TL-2- and IL-12-induced IFN-{gamma} expression in murine NK cells. *Blood.* 2003 Mar 13 [Epub ahead of print] |
| IL-4/IL-13 | | • Abstract Interleuldn-4 and interleukin-13 signaling connections maps. *Science.* 2003 Jun 6;**300**(5625):1527-8. (allergy, asthma) |
| | | • Inhibition of the IL-4/IL-13 receptor system prevents allergic sensitization without affecting established allergy in a mouse model for allergic asthma. *J Allergy Clin Immunol*. 2003 Jun;**111**(6):1361-1369. |
| IL-4/IL-16 | | • (asthma) Interleukin (IL)-4/IL-9 and exogenous IL-16 induce IL-16 production by BEAS-2B cells, a bronchial epithelial cell line. *Cell Immunol*. 2001 Feb 1;**207**(2):75-80 |
| IL-4/IL-17 | | • Interleukin (IL)-4 and IL-17 synergistically stimulate IL-6 secretion in human colonic myofibroblasts. *Int J Mol Med.* 2002 Nov;**10**(5):631-4. (Gut inflammation) |
| IL-4/IL-24 | | • IL-24 is expressed by rat and human macrophages. *Immunobiology*. 2002 Jul;**205**(3):321-34. |
| IL-4/IL-25 | | • Abstract New IL-17 family members promote Th1 or Th2 responses in the lung: in vivo function of the novel cytokine IL-25. *J Immunol*. 2002 Jul 1;**169**(1):443-53. (allergic inflammation) |
| | | • Abstract Mast cells produce interleukin-25 upon Fcepsilon RI-mediated activation. *Blood*. 2003 May 1;101(9):3594-6. Epub 2003 Jan 02. (allergic inflammation) |
| IL-4/IFN-γ | | • Abstract Interleukin 4 induces interleukin 6 production by endothelial cells: synergy with interferon-gamma. *Eur J Immunol*. 1991 Jan;**21**(1):97-101. |
| IL-4/SCF | | • Regulation of human intestinal mast cells by stem cell factor and IL-4. *Immunol Rev.* 2001 Feb;**179**:57-60. Review. |
| IL-5/IL-3 | | • Differential regulation of human eosinophil IL-3. IL-5, and GM-CSF receptor alpha-chain expression by cytokines: IL-3, IL-5. and GM-CSF down-regulate IL-5 receptor alpha expression with loss of IL-5 responsiveness, but up-regulate IL-3 receptor alpha expression. *J Immunol*. 2003 Jun 1;**170**(11):5359-66. (Allergic inflammation see abstract) |
| IL-5/IL-6 | | |
| IL-5/IL-13 | | • Inhibition of allergic airways inflammation and airway hyperresponsiveness in mice by dexamethasone: role of eosinophils, IL-5, eotaxin, andIL-13. *J Allergy Clin Immunol.* 2003 May;**111**(5):1049-61. |
| IL-5/IL-17 | | • Interleukin-17 orchestrates the granulocyte influx into airways after allergen inhalation in a mouse model of allergic asthma. *Am J Respir Cell Mol Biol*. 2003 Jan;**28**(1):42-50. |
| IL-5/IL-25 | | • Abstract New IL-17 family members promote Th1 or Th2 responses in the lung: in vivo function of the novel cytokine IL-25. *J Immunol.* 2002 Jul 1;**169**(1):443-53. (allergic inflammation) |
| | | • Abstract Mast cells produce interleukin-25 upon Fcepsilon RI-mediated activation. *Blood*. 2003 May 1;101(9):3594-6. Epub 2003 Jan 02. (allergic inflammation) |
| IL-5/EFN-γ | | |
| IL-5/GM-CSF | | • Differential regulation of human eosinophil IL-3, IL-5, and GM-CSF receptor alpha-chain expression by cytokines: IL-3, IL-5, and GM-CSF down-regulate IL-5 receptor alpha expression with loss of IL-5 responsiveness, but up-regulate IL-3 receptor alpha expression. *J Immunol*. 2003 Jun 1;**170**(11):5359-66. (Allergic inflammation) |
| IL-6/IL-10 | | |
| IL-6/IL-11 | | |
| IL-6/IL-16 | | • Interleukin-16 stimulates the expression and production of pro- |
| | | inflammatory cytokines by human monocytes. *Immunology.* 2000 May;**100**(1):63-9. |
| IL-6/IL-17 | | • Stimulation of airway mucin gene expression by interleukin (IL)-17 through IL-6 paracrine/autocrine loop. *J Biol Chem*. 2003 May 9;**278**(19):17036-43. Epub 2003 Mar 06. (airway inflammation, asthma) |
| IL-6/IL-19 | | • Abstract IL-19 induces production of IL-6 and TNF-alpha and results in cell apoptosis through TNF-alpha. *J Immunol*. 2002 Oct 15;**169**(8):4288-97. |
| IL-6/IFN-g | | |
| IL-7/IL-2 | | • Interleukin 7 worsens graft-versus-host disease. *Blood*. 2002 Oct 1;**100**(7):2642-9. |
| IL-7/IL-12 | | • Synergistic effects of IL-7 and IL-12 on human T cell activation. *J Immunol.* 1995 May 15;**154**(10):5093-102. |
| IL-7/IL-15 | | • Interleukin-7 and interleukin-15 regulate the expression of the bcl-2 and c-myb genes in cutaneous T-cell lymphoma cells. *Blood* 2001 Nov 1;**98**(9):2778-83. (growth factor) |
| IL-8/IL-11 | | • Abnormal production of interleukin (IL)-11 and IL-8 in polycythaemia vera. *Cytokine.* 2002 Nov 21;**20**(4):178-83. |
| IL,8/IL-17 | | • The Role of IL-17 in Joint Destruction. *Drug News Perspect.* 2002 Jan;**15**(1):17-23. (arthritis) |
| | | • Abstract Interleukin-17 stimulates the expression of interleukin-8. growth-related oncogene-alpha, and granulocyte-colony-stimulating factor by human airway epithelial cells. *Am J Respir Cell Mol Biol.* 2002 Jun;**26**(6):748-53. (airway inflammation) |
| IL-8/GSF | | • Interleukin-8: an autocrine/paracrine growth factor for human hematopoietic progenitors acting in synergy with colony stimulating factor-1 to promote monocyte-macrophage growth and differentiation. *Exp Hematol*. 1999 Jan;**27**(1):28-36. |
| IL-8/VGEF | | • Intracavitary VEGF, bFGF, IL-8, IL-12 levels in primary and recurrent malignant glioma. *J Neurooncol*. 2003 May;**62**(3):297-303. |
| IL-9/IL-4 | | • Anti-interleukin-9 antibody treatment inhibits airway inflammation and hyperreactivity in mouse asthma model. *Am J Respir Crit Care Med*. 2002 Aug 1;**166**(3):409-16. |
| IL-9/IL-5 | | • pulmonary overexpression of IL-9 induces Th2 cytokine expression, leading to immune pathology. *J Clin Invest*. 2002 Jan;**109**(1):29-39. |
| | | • Th2 cytokines and asthma. Interleulan-9 as a therapeutic target for asthma. *Respir Res.* 2001;**2**(2):80-4. Epub 2001 Feb 15. Review. |
| | | • Abstract Interleukin-9 enhances interleukin-5 receptor expression, differentiation, and survival of human eosinophils. Blood 2000 Sep 15;**96**(6):2163-71 (asthma) |
| IL-9/IL-13 | | • Anti-interleukin-9 antibody treatment inhibits airway inflammation and hyperreactivity in mouse asthma model. *Am J Respir Crit Care Med*. 2002 Aug 1;**166**(3):409-16. |
| | | • Direct effects of interleukin-13 on epithelial cells cause airway hyperreactivity and mucus overproduction in asthma. *Nat Med*. 2002 Aug;**8**(8):885-9. |
| IL-9/IL-16 | | • See IL-4/IL-16 |
| IL-10/IL-2 | | • The interplay of interleukin-10 (IL-10) and interleukin-2 (IL-2) in humoral immune responses: IL-10 synergizes with IL-2 to enhance responses of human B lymphocytes in a mechanism which is different from upregulation of CD25 expression. *Cell Immunol.* 1994 Sep;**157**(2):478-88. |
| IL-10/IL-12 | | |
| IL-10/TGF-β | | • IL-10 and TGF-beta cooperate in the regulatory T cell response to mucosal allergens in normal immunity and specific immunotherapy. *Eur J Immunol.* 2003 May;**33**(5):1205-14. |
| IL-10/IFN-γ | | |
| IL-11/IL-6 | | • Interleukin-6 and interleukin-11 support human osteoclast formation by a RANKL-independent mechanism. *Bone*. 2003 Jan;**32**(1):1-7. (bone resorption in inflammation) |
| IL-11/IL-17 | | • Polarized in vivo expression of IL-11 and IL-17 between acute and chronic skin lesions. *J Allergy Clin Immunol*. 2003 Apr;**111**(4):875-81. (allergic dermatitis) |
| | | • IL-17 promotes bone erosion in murine collagen-induced arthritis through loss of the receptor activator of NF-kappa B ligand/osteoprotegerin balance. *J Immunol*. 2003 Mar 1;**170**(5):2655-62. |
| IL-11/TGF-β | | • Polarized in vivo expression of IL-11 and IL-17 between acute and chronic skin lesions. *J Allergy Clin Immunol*. 2003 Apr;**111**(4):875-81. (allergic dermatitis) |
| IL-12/IL-13 | | • Relationship of Interleukin-12 and Interleukin-13 imbalance with class-specific rheumatoid factors and anticardiolipin antibodies in systemic lupus erythematosus. *Clin Rheumatol*. 2003 May;**22**(2):107-11. |
| IL-12/IL-17 | | • Upregulation of interleukin-12 and -17 in active inflammatory bowel disease. *Scand J Gastroenterol.* 2003 Feb;**38**(2):180-5. |
| IL-12/IL-18 | | • Synergistic proliferation and activation of natural killer cells by interleukin 12 and interleukin 18. *Cytokine*. 1999 Nov;**11**(11):822-30. |
| | | • Inflammatory Liver Steatosis Caused by IL-12 and IL-18. *J Interferon Cytokine Res*. 2003 Mar;**23**(3):155-62. |
| IL-12/IL-23 | | • nterleukin-23 rather than interleukin-12 is the critical cytokine for autoimmune inflammation of the brain. *Nature*. 2003 Feb 13;**421**(6924):744-8. |
| | | • Abstract A unique role for IL-23 in promoting cellular immunity. *J Leukoc Biol*. 2003 Jan;**73**(1):49-56. Review. |
| IL-12/IL-27 | | • Abstract IL-27, a heterodimeric cytokine composed of EBI3 and p28 protein, induces proliferation of naive CD4(+) T cells. *Immunity.* 2002 Jun;**16**(6):779-90. |
| IL-12/IFN-γ | | • IL-12 induces IFN-γ expression by B and T-cells as part of immune stimulation. |
| IL-13/IL-5 | | • See IL-5/IL-13 |
| IL-13/IL-25 | | • Abstract New IL-17 family members promote Th1 or Th2 responses in the lung: in vivo function of the novel cytokine IL-25. *J Immunol.* 2002 Jul 1;**169**(1):443-53. (allergic inflammation) |
| | | • Abstract Mast cells produce interleukin-25 upon Fcepsilon RI-mediated activation. *Blood.* 2003 May 1;**101**(9):3594-6. Epub 2003 Jan 02. (allergic inflammation) |
| IL-15/IL-13 | | • Differential expression of interleukins (IL)-13 and IL-15 in ectopic and entopic endometrium of women with endometriosis and normal fertile women. *Am J Reprod Immunol*. 2003 Feb;**49**(2):75-83. |
| IL-15/IL-16 | | • IL-15 and IL-16 overexpression in cutaneous T-cell lymphomas: stage-dependent increase in mycosis fungoides progression. *Exp Dermatol*. 2000 Aug;**9**(4):248-51. |
| IL-15/IL-17 | | • Abstract IL-17, produced by lymphocytes and neutrophils, is necessary for lipopolysaccharide-induced airway neutrophilia: IL-15 as a possible trigger. *J Immunol*. 2003 Feb 15;**170**(4):2106-12. (airway inflammation) |
| IL-15/IL-21 | | • IL-21 in Synergy with IL-15 or IL-18 Enhances IFN-gamma Production in Human NK and T Cells. *J Immunol*. 2003 Jun 1;**170**(11):5464-9. |
| IL-17/IL-23 | | • Interleukin-23 promotes a distinct CD4 T cell activation state characterized by the production of interleukin-17. *J Biol Chem*. 2003 Jan 17,**278**(3):1910-4. Epub 2002 Nov 03 |
| IL-17/TGF-β | | • Polarized in vivo expression of IL-11 and IL-17 between acute and chronic skin lesions. *J Allergy Clin Immunol*. 2003 Apr;**111**(4):875-81. (allergic dermatitis) |
| IL-18/IL-12 | | • Synergistic proliferation and activation of natural killer cells by interleukin |
| | | 12 and interleukin 18. *Cytokine*. 1999 Nov;**11**(11):822-30. |
| | | • Abstract Inhibition of in vitro immunoglobulin production by IL-12 in murine chronic graft-vs.-host disease: synergism with IL-18. *Eur J Immunol*. 1998 Jun;**28**(6):2017-24. |
| IL-18/IL-21 | | • IL-21 in Synergy with IL-15 or IL-18 Enhances IFN-gamma Production in Human NK and T Cells. *J Immunol*. 2003 Jun 1;**170**(11):5464-9. |
| IL-18/TGF-β | | • Interleukin 18 and transforming growth factor beta1 in the serum of patients with Graves' ophthalinopathy treated with corticosteroids. *Int Immunopharmacol*. 2003 Apr,**3**(4):549-52. |
| IL-18/IFN-γ | | |
| Anti-TNF ALPHA/anti-CD4 | | • Synergistic therapeutic effect in DBA/I arthritic mice. |

### Annex 3: Oncology combinations

| **Target** | **Disease** | **Pair with** |
|---|---|---|
| CD89* | Use as cytotoxic cell recruiter | all |
| | | |
| CD19 | B cell lymphomas | HLA-DR |
| | | CD5 |
| HLA-DR | B cell lymphomas | CD89 |
| | | CD19 |
| | | CD5 |
| CD38 | Multiple myeloma | CD138 |
| | | CD56 |
| | | HLA-DR |
| CD138 | Multiple myeloma | CD38 |
| | | CD56 |
| | | HLA-DR |
| CD138 | Lung cancer | CD56 |
| | | CEA |
| CD33 | Acute myelod lymphoma | CD34 |
| | | HLA-DR |
| CD56 | Lung cancer | CD138 |
| | | CEA |
| CEA | Pan carcinoma | MET receptor |
| VEGF | Pan carcinoma | MET receptor |
| VEGF | Pan carcinoma | MET receptor |
| receptor | | |
| IL-13 | Asthma/pulmonary | IL-4 |
| | inflammation | IL-5 |
| | | Eotaxin(s) |
| | | MDC |
| | | TARC |
| | | TNFα |
| | | IL-9 |
| | | EGFR |
| | | CD40L |
| | | IL-25 |
| | | MCP-1 |
| | | TGFβ |
| IL-4 | Asthma | IL-13 |
| | | IL-5 |
| | | Eotaxin(s) |
| | | MDC |
| | | TARC |
| | | TNFα |
| | | IL-9 |
| | | EGFR |
| | | CD40L |
| | | IL-25 |
| | | MCP-1 |
| | | TGFβ |
| Eotaxin | Asthma | IL-5 |
| | | Eotaxin-2 |
| | | Eotaxin-3 |
| EGFR | cancer | HER2/neu |
| | | HER3 |
| | | HER4 |
| HER2 | cancer | HER3 |
| | | HER4 |
| TNFR1 | RA/Crohn's disease | IL-1R |
| | | IL-6R |
| | | IL-18R |
| TNFα | RA/Crohn's disease | IL-1α/β |
| | | IL-6 |
| | | IL-18 |
| | | ICAM-1 |
| | | IL-15 |
| | | IL-17 |
| IL-1R | RA/Crohn's disease | IL-6R |
| | | IL-18R |
| IL-18R | RA/Crohn's disease | IL-6R |

### Annex 4

**Data Summary**

| **TARGET** | **dAb** | **Equilibrium dissocation constant (Kd = Koff/Kon)** | **Koff** | **IC50 for ligand assay** | **ND50 for cell based neutralisn assay** |
|---|---|---|---|---|---|
| TAR1 | TAR1 monomers | 300nM to 5pM (ie, 3 x 10⁻⁷ to 5 x 10⁻¹²), preferably 50nM to 20pM | 5 x 10⁻¹ to 1 x 10⁻⁷ | 500nM to 100pM | 500nM to 50pM |
| | TAR1 dimers | As TAR1 monomer | As TAR1 monomer | As TAR1 monomer | As TAR1 monomer |
| | TAR1 trimers | As TAR1 monomer | As TAR1 monomer | As TAR1 monomer | As TAR1 monomer |
| | TAR1-S | | | | |
| | TAR1-27 | | | | |
| | TAR1-5-19 monomer | 30nM | | | |
| | TAR1-5-19 homodimer | | | With (Gly₄Ser)₃ linker = 20nm | =30nM =3nM =15nM |
| | | | | With (Gly₄Ser)₅ linker = 2nm | |
| | | | | With (Gly₄Ser)₇ linker = 10nm | |
| | | | | In Fab format =1nM | |
| | | | | | |
| | TAR1-5-19 heterodimers | | | With (Gly₄Ser)ₙ linker | = 12nM =10nM = 12nM |
| | | | | TAR1-5-19 d2 = 2nM | |
| | | | | TAR1-5-19 d3 = 8nM | |
| | | | | TAR1-5-19 d4 = 2-5nM | |
| | | | | TAR1-5-19 d5 = 8nM | |
| | | | | In Fab format | |
| | | | | TAR1-5-19CH d1CK = 6nM | |
| | | | | TAR1-5-19CK d1CH = 6nM | |
| | | | | TAR1-5-19CH d2CK = 8nM | |
| | | | | TAR1-5-19CH d3CK = 3nM | |
| | | | | | |
| | TAR1-5 | | | With (Gly₄Ser)ₙ linker | |
| | heterodimers | | | TAR1-5d1 =30nM | |
| | | | | TAR1-5d2 = 50nM | |
| | | | | TAR1-5d3 = 300nM | |
| | | | | TAR1-5d4 = 3nM | |
| | | | | TAR1-5d5 = 200nM | |
| | | | | TAR1-5d6 = 100nM | |
| | | | | In Fab format | |
| | | | | TAR1-5CH d2CK=30nM | = 60nM |
| | | | | TAR1-5CK d3CH =100nM | |
| | TAR1-5-19 homotrimer | | | 0.3nM | 3.10nM (eg, 3nM) |
| | | | | | |
| TAR2 | TAR2 monomers | As TAR1 monomer | As TAR1 monomer | 500nM to 100pM | 500nM to 50pM |
| | TAR2-10 | | | | |
| | TAR2-5 | | | | |
| Serum Albumin | Anti-SA monomers | 1nM to 500µM, preferably 100nM to 10 µM | | 1nM to 500µM, preferably 100nM to 10 µM | |
| | | In Dual Specific format, target affinity is 1 to 100,000 x affinity of SA dAb affinity, eg 100pM (target) and 10 µM SA affinity. | | In Dual Specific format, target affinity is 1 to 100,000 x affinity of SA dAb affinity, eg 100pM (target) and 10 µM SA affinity. | |
| | MSA-16 | 200nM | | | |
| | MSA-26 | 70nM | | | |

### SEQUENCE LISTING

<110> Domantis Limited
   Winter, Greg
   Tomlinson, Ian
   Ignatovich, Olga
   Holt, Lucy
   De Angelis, Elena
<120> Ligand
<130> P014435WO ATM
<140> PCT/GB 03/02804
   <141> 2003-06-30
<150> PCT/GB2002/003014
   <151> 2002-06-28
<150> GB 0230202.4
   <151> 2002-12-27
<160> 240
<170> PatentIn version 3.1
<210> 1
   <211> 360
   <212> DNA
   <213> Artificial sequence
<220>
   <223> HEL4
<220>
   <221> CDS
   <222> (1)..(360)
   <223>
<400> 1
   gag gtg cag ctg ttg gag tct ggg gga ggc ttg gta cag cct ggg ggg       48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttt agg att agc gat gag        96
   Ser Leu Arg Leu Ser Cys Ala Ala Ser Gly Phe Arg Ile Ser Asp Glu
               20                  25                  30
   gat atg ggc tgg gtc cgc cag gct cca ggg aag ggt cta gag tgg gta       144
   Asp Met Gly Trp Val Arg Gln Ala Pro Gly Lys Gly Leu Glu Trp Val
           35                  40                  45
   tca agc att tat ggc cct agc ggt agc aca tac tac gca gac tcc gtg       192
   Ser Ser Ile Tyr Gly Pro Ser Gly Ser Thr Tyr Tyr Ala Asp Ser Val
       50                  55                  60
   aag ggc cgg ttc acc atc tcc cgt gac aat tcc aag aac acg ctg tat       240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tat tgc       288
   Leu Gln Met Asn Ser Leu Arg Ala Glu Asp Thr Ala Val Tyr Tyr Cys
                   85                  90                  95
   gcg agt gct ttg gag ccg ctt tcg gag ccc ctg ggc ttt tgg ggt cag       336
   Ala Ser Ala Leu Glu Pro Leu Ser Glu Pro Leu Gly Phe Trp Gly Gln
               100                 105                 110
   gga acc ctg gtc acc gtc tcg agc                                       360
   Gly Thr Leu Val Thr Val Ser Ser
           115                 120
<210> 2
   <211> 120
   <212> PRT
   <213> Artificial sequence
<220>
   <223> HEL4
<400> 2
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Arg Ile Ser Asp Glu
               20                  25                  30
   Asp Met Gly Trp Val Arg Gln Ala Pro Gly Lys Gly Leu Glu Trp Val
           35                  40                  45
   Ser Ser Ile Tyr Gly Pro Ser Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Ala Leu Glu Pro Leu Ser Glu Pro Leu Gly Phe Trp Gly Gln
               100                 105                 110
   Gly Thr Leu Val Thr Val Ser Ser
           115                 120
<210> 3
   <211> 348
   <212> DNA
   <213> Artificial sequence
<220>
   <223> TAR2-5
<220>
   <221> CDS
   <222> (1)..(348)
   <223>
<400> 3
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt gat ctt tat      96
   Ser Leu Arg Leu Ser Cys Ala Ala Ser Gly Phe Thr Phe Asp Leu Tyr
               20                  25                  30
   aat atg ttt tgg gtc cgc cag gct cca ggg aag ggt cta gag tgg gtc       144
   Asn Met Phe Trp Val Arg Gln Ala Pro Gly Lys Gly Leu Glu Trp Val
           35                  40                  45
   tca ttt att agt cag act ggt agg ctt aca tgg tac gca gac tcc gtg       192
   Ser Phe Ile Ser Gln Thr Gly Arg Leu Thr Trp Tyr Ala Asp Ser Val
       50                  55                  60
   aag ggc cgg ttc acc atc tcc cgc gac aat tcc aag aac acg ctg tat       240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt       288
   Leu Gln Met Asn Ser Leu Arg Ala Glu Asp Thr Ala Val Tyr Tyr Cys
                   85                  90                  95
   gcg aaa acg ctg gag gat ttt gac tac tgg ggc cag gga acc ctg gtc       336
   Ala Lys Thr Leu Glu Asp Phe Asp Tyr Trp Gly Gln Gly Thr Leu Val
               100                 105                 110
   acc gtc tcg agc                                                       348
   Thr Val Ser Ser
           115
<210> 4
   <211> 116
   <212> PRT
   <213> Artificial sequence
<220>
   <223> TAR2-5
<400> 4
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Asp Leu Tyr
               20                  25                  30
   Asn Met Phe Trp Val Arg Gln Ala Pro Gly Lys Gly Leu Glu Trp Val
           35                  40                  45
   Ser Phe Ile Ser Gln Thr Gly Arg Leu Thr Trp Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Thr Leu Glu Asp Phe Asp Tyr Trp Gly Gln Gly Thr Leu Val
   100 105 110
   Thr Val Ser Ser
   115
<210> 5
   <211> 324
   <212> DNA
   <213> Artificial sequence
<220>
   <223> TAR1-5-19
<220>
   <221> CDS
   <222> (1)..(324)
   <223>
<400> 5
   gac atc cag atg acc cag tct cca tcc tcc ctg tct gca tct gta gga 48
   Asp Ile Gln Met Thr Gln Ser Pro Ser Ser Leu Ser Ala Ser Val Gly
   1 5 10 15
   gac cgt gtc acc atc act tgc cgg gca agt cag agc gtt aag gag ttt 96
   Asp Arg Val Thr Ile Thr Cys Arg Ala Ser Gln Ser Val Lys Glu Phe
   20 25 30
   tta tgg tgg tac cag cag aaa cca ggg aaa gcc cct aag ctc ctg atc 144
   Leu Trp Trp Tyr Gln Gln Lys Pro Gly Lys Ala Pro Lys Leu Leu Ile
   35 40 45
   tat atg gca tcc aat ttg caa agt ggg gtc cca tca cgt ttc agt ggc       192
   Tyr Met Ala Ser Asn Leu Gln Ser Gly Val Pro Ser Arg Phe Ser Gly
       50                  55                  60
   agt gga tct ggg aca gat ttc act ctc acc atc agc agt ctg caa cct       240
   Ser Gly Ser Gly Thr Asp Phe Thr Leu Thr Ile Ser Ser Leu Gln Pro
   65                  70                  75                  80
   gaa gat ttt gct acg tac tac tgt caa cag aag ttt aag ctg cct cgt       288
   Glu Asp Phe Ala Thr Tyr Tyr Cys Gln Gln Lys Phe Lys Leu Pro Arg
                   85                  90                  95
   acg ttc ggc caa ggg acc aag gtg gaa atc aaa cgg                       324
   Thr Phe Gly Gln Gly Thr Lys Val Glu Ile Lys Arg
               100                 105
<210> 6
   <211> 108
   <212> PRT
   <213> Artificial sequence
<220>
   <223> TAR1-5-19
<400> 6
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Val Lys Glu Phe
               20                  25                  30
   Leu Trp Trp Tyr Gln Gln Lys Pro Gly Lys Ala Pro Lys Leu Leu Ile
           35                  40                  45
   Tyr Met Ala Ser Asn Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Lys Phe Lys Leu Pro Arg
   85 90 95
   Thr Phe Gly Gln Gly Thr Lys Val Glu Ile Lys Arg
   100 105
<210> 7
   <211> 362
   <212> DNA
   <213> Artificial sequence
<220>
   <223> TAR2-10
<400> 7
   gaggtgcagc tgttggagtc tgggggaggc ttggtacagc ctggggggtc cctgcgtctc 60
   tcctgtgcag cctccggatt cacctttgag tggtattgga tgggttgggt ccgccaggct 120
   ccagggaagg gtctagagtg ggtctcagct attagtggta gtggtggtag cacatactac 180
   gcagactccg tgaagggccg gttcaccatc tcccgcgaca attccaagaa cacgctgtat 240
   ctgcaaatga acagcctgcg tgccgaggac accgcggtat attactgtgc gaaagttaag 300
   ttgggggggg ggcctaattt tgactactgg ggccagggaa ccctggtcac cgtctcgagc 360
   gc 362
<210> 8
   <211> 360
   <212> DNA
   <213> Artificial sequence
<220>
   <223> TAR2-10
<220>
   <221> CDS
   <222> (1)..(360)
   <223>
<400> 8
   gag gtg cag ctg ttg gag tct ggg gga ggc ttg gta cag cct ggg ggg        48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt gag tgg tat        96
   Ser Leu Arg Leu Ser Cys Ala Ala Ser Gly Phe Thr Phe Glu Trp Tyr
               20                  25                  30
   tgg atg ggt tgg gtc cgc cag gct cca ggg aag ggt cta gag tgg gtc       144
   Trp Met Gly Trp Val Arg Gln Ala Pro Gly Lys Gly Leu Glu Trp Val
           35                  40                  45
   tca gct att agt ggt agt ggt ggt agc aca tac tac gca gac tcc gtg       192
   Ser Ala Ile Ser Gly Ser Gly Gly Ser Thr Tyr Tyr Ala Asp Ser Val
       50                  55                  60
   aag ggc cgg ttc acc atc tcc cgc gac aat tcc aag aac acg ctg tat       240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt       288
   Leu Gln Met Asn Ser Leu Arg Ala Glu Asp Thr Ala Val Tyr Tyr Cys
                   85                  90                  95
   gcg aaa gtt aag ttg ggg ggg ggg cct aat ttt gac tac tgg ggc cag       336
   Ala Lys Val Lys Leu Gly Gly Gly Pro Asn Phe Asp Tyr Trp Gly Gln
               100                 105                 110
   gga acc ctg gtc acc gtc tcg agc                                       360
   Gly Thr Leu Val Thr Val Ser Ser
           115                 120
<210> 9
   <211> 120
   <212> PRT
   <213> Artificial sequence
<220>
   <223> TAR2-10
<400> 9
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Glu Trp Tyr
   20 25 30
   Trp Met Gly Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Val Lys Leu Gly Gly Gly Pro Asn Phe Asp Tyr Trp Gly Gln
   100 105 110
   Gly Thr Leu Val Thr Val Ser Ser
   115 120
<210> 10
   <211> 324
   <212> DNA
   <213> Artificial sequence
<220>
   <223> TAR1-5-19
<220>
   <221> CDS
   <222> (1)..(324)
   <223>
<400> 10
   gac atc cag atg acc cag tct cca tcc tct ctg tct gca tct gta gga 48
   Asp Ile Gln Met Thr Gln Ser Pro Ser Ser Leu Ser Ala Ser Val Gly
   1 5 10 15
   gac cgt gtc acc atc act tgc cgg gca agt cag agc att gat agt tat       96
   Asp Arg Val Thr Ile Thr Cys Arg Ala Ser Gln Ser Ile Asp Ser Tyr
               20                  25                  30
   tta cat tgg tac cag cag aaa cca ggg aaa gcc cct aag ctc ctg atc      144
   Leu His Trp Tyr Gln Gln Lys Pro Gly Lys Ala Pro Lys Leu Leu Ile
           35                  40                  45
   tat agt gca tcc gag ttg caa agt ggg gtc cca tca cgt ttc agt ggc      192
   Tyr Ser Ala Ser Glu Leu Gln Ser Gly Val Pro Ser Arg Phe Ser Gly
       50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
   65                  70                  75                  80
   gaa gat ttt gct acg tac tac tgt caa cag gtt gtg tgg cgt cct ttt      288
   Glu Asp Phe Ala Thr Tyr Tyr Cys Gln Gln Val Val Trp Arg Pro Phe
                   85                  90                  95
   acg ttc ggc caa ggg acc aag gtg gaa atc aaa cgc                      324
   Thr Phe Gly Gln Gly Thr Lys Val Glu Ile Lys Arg
               100                 105
<210> 11
   <211> 108
   <212> PRT
   <213> Artificial sequence
<220>
   <223> TAR1-5-19
<400> 11
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Asp Ser Tyr
               20                  25                  30
   Leu His Trp Tyr Gln Gln Lys Pro Gly Lys Ala Pro Lys Leu Leu Ile
           35                  40                  45
   Tyr Ser Ala Ser Glu Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Val Val Trp Arg Pro Phe
   85 90 95
   Thr Phe Gly Gln Gly Thr Lys Val Glu Ile Lys Arg
   100 105
<210> 12
   <211> 324
   <212> DNA
   <213> Artificial sequence
<220>
   <223> TAR1-5-19
<400> 12
   gcgtttgatt tccaccttgg tcccttggcc gaacgtaaaa ggacgccaca caacctgttg 60
   acagtagtac gtagcaaaat cttcaggttg cagactgctg atggtgagag tgaaatctgt 120
   cccagatcca ctgccactga aacgtgatgg gaccccactt tgcaactcgg atgcactata 180
   gatcaggagc ttaggggctt tccctggttt ctgctggtac caatgtaaat aactatcaat 240
   gctctgactt gcccggcaag tgatggtgac acggtctcct acagatgcag acagagagga 300
   tggagactgg gtcatctgga tgtc 324
<210> 13
   <211> 324
   <212> DNA
   <213> Artificial sequence
<220>
   <223> TAR1-5
<220>
   <221> CDS
   <222> (1)..(324)
   <223>
<400> 13
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att ttt atg aat        96
   Asp Arg Val Thr Ile Thr Cys Arg Ala Ser Gln Ser Ile Phe Met Asn
               20                  25                  30
   tta ttg tgg tac cag cag aaa cca ggg aaa gcc cct aag ctc ctg atc       144
   Leu Leu Trp Tyr Gln Gln Lys Pro Gly Lys Ala Pro Lys Leu Leu Ile
           35                  40                  45
   tat aat gca tcc gtg ttg caa agt ggg gtc cca tca cgt ttc agt ggc       192
   Tyr Asn Ala Ser Val Leu Gln Ser Gly Val Pro Ser Arg Phe Ser Gly
       50                  55                  60
   agt gga tct ggg aca gat ttc act ctc acc atc agc agt ctg caa cct       240
   Ser Gly Ser Gly Thr Asp Phe Thr Leu Thr Ile Ser Ser Leu Gln Pro
   65                  70                  75                  80
   gaa gat ttt gct acg tac tac tgt caa cag gtt gtg tgg cgt cct ttt       288
   Glu Asp Phe Ala Thr Tyr Tyr Cys Gln Gln Val Val Trp Arg Pro Phe
                   85                  90                  95
   acg ttc ggc caa ggg acc aag gtg gaa atc aaa cgg                       324
   Thr Phe Gly Gln Gly Thr Lys Val Glu Ile Lys Arg
               100                 105
<210> 14
   <211> 108
   <212> PRT
   <213> Artificial sequence
<220>
   <223> TAR1-5
<400> 14
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Phe Met Asn
               20                  25                  30
   Leu Leu Trp Tyr Gln Gln Lys Pro Gly Lys Ala Pro Lys Leu Leu Ile
           35                  40                  45
   Tyr Asn Ala Ser Val Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Val Val Trp Arg Pro Phe
                   85                  90                  95
   Thr Phe Gly Gln Gly Thr Lys Val Glu Ile Lys Arg
               100                 105
<210> 15
   <211> 324
   <212> DNA
   <213> Artificial sequence
<220>
   <223> TAR1-5
<400> 15
   ccgtttgatt tccaccttgg tcccttggcc gaacgtaaaa ggacgccaca caacctgttg 60
   acagtagtac gtagcaaaat cttcaggttg cagactgctg atggtgagag tgaaatctgt 120
   cccagatcca ctgccactga aacgtgatgg gaccccactt tgcaacacgg atgcattata 180
   gatcaggagc ttaggggctt tccctggttt ctgctggtac cacaataaat tcataaaaat 240
   gctctgactt gcccggcaag tgatggtgac acggtctcct acagatgcag acagggagga 300
   tggagactgg gtcatctgga tgtc 324
<210> 16
   <211> 324
   <212> DNA
   <213> Artificial sequence
<220>
   <223> Monomer - dAb1: Partner dAb in TAR1-5d1 (3Ulinker), d5 (5U linker ), d6 (7U linker)
<220>
   <221> CDS
   <222> (1)..(324)
   <223>
<400> 16
   gac atc cag atg acc cag tct cca tcc tcc ctg tct gca tct gta gga       48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att tat gat gcg       96
   Asp Arg Val Thr Ile Thr Cys Arg Ala Ser Gln Ser Ile Tyr Asp Ala
               20                  25                  30
   tta gag tgg tac cag cag aaa cca ggg aaa gcc cct aag ctc ctg atc      144
   Leu Glu Trp Tyr Gln Gln Lys Pro Gly Lys Ala Pro Lys Leu Leu Ile
           35                  40                  45
   tat act gca tcc cgg ttg caa agt ggg gtc cca tca cgt ttc agt ggc      192
   Tyr Thr Ala Ser Arg Leu Gln Ser Gly Val Pro Ser Arg Phe Ser Gly
       50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
   65                  70                  75                  80
   gaa gat ttt gct acg tac tac tgt caa cag gtt atg cag cgt cct gtt      288
   Glu Asp Phe Ala Thr Tyr Tyr Cys Gln Gln Val Met Gln Arg Pro Val
                   85                  90                  95
   acg ttc ggc caa ggg acc aag gtg gaa atc aaa cgg                      324
   Thr Phe Gly Gln Gly Thr Lys Val Glu Ile Lys Arg
               100                 105
<210> 17
   <211> 108
   <212> PRT
   <213> Artificial sequence
<220>
   <223> Monomer - dAb1: Partner dAb in TAR1-5d1 (3Ulinker), d5 (5U linker ), d6 (7U linker)
<400> 17
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Tyr Asp Ala
   20 25 30
   Leu Glu Trp Tyr Gln Gln Lys Pro Gly Lys Ala Pro Lys Leu Leu Ile
   35 40 45
   Tyr Thr Ala Ser Arg Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Val Met Gln Arg Pro Val
   85 90 95
   Thr Phe Gly Gln Gly Thr Lys Val Glu Ile Lys Arg
   100 105
<210> 18
   <211> 324
   <212> DNA
   <213> Artificial sequence
<220>
   <223> Monomer - dAb1: Partner dAb in TAR1-5d1 (3Ulinker), d5 (5U linker ), d6 (7U linker)
<400> 18
   ccgtttgatt tccaccttgg tcccttggcc gaacgtaaca ggacgctgca taacctgttg 60
   acagtagtac gtagcaaaat cttcaggttg cagactgctg atggtgagag tgaaatctgt 120
   cccagatcca ctgccactga aacgtgatgg gaccccactt tgcaaccggg atgcagtata 180
   gatcaggagc ttaggggctt tccctggttt ctgctggtac cactctaacg catcataaat 240
   gctctgactt gcccggcaag tgatggtgac acggtctcct acagatgcag acagggagga 300
   tggagactgg gtcatctgga tgtc 324
<210> 19
   <211> 324
   <212> DNA
   <213> Artificial sequence
<220>
   <223> Monomer - dAb2: Partner dAb in TAR1-5d2 (3U linker), d3 (5U linke r)
<400> 19
   gacatccaga tgacccagtc tccatcctcc ctgtctgcat ctgtaggaga ccgtgtcacc      60
   atcacttgcc gggcaagtca gagcatttat gatgctttac agtggtacca gcagaaacca     120
   gggaaagccc ctaagctcct gatctatact gcatcccggt tgcaaagtgg ggtcccatca     180
   cgtttcagtg gcagtggatc tgggacagat ttcactctca ccatcagcag tctgcaacct     240
   gaagattttg ctacgtacca ctgtcaacag gttatgcagc gtcctgttac gttcggccaa     300
   gggaccaagg tggaaatcaa acgg                                            324
<210> 20
   <211> 33
   <212> PRT
   <213> Artificial sequence
<220>
   <223> Monomer - dAb2: Partner dAb in TAR1-5d2 (3U linker), d3 (5U linke r)
<400> 20
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Tyr Asp Ala
               20                  25                  30
   Leu
<210> 21
   <211> 74
   <212> PRT
   <213> Artificial sequence
<220>
   <223> Monomer - dAb2: Partner dAb in TAR1-5d2 (3U linker), d3 (5U linke r)
<400> 21
   Trp Tyr Gln Gln Lys Pro Gly Lys Ala Pro Lys Leu Leu Ile Tyr Thr
   1               5                   10                  15
   Ala Ser Arg Leu Gln Ser Gly Val Pro Ser Arg Phe Ser Gly Ser Gly
               20                  25                  30
   Ser Gly Thr Asp Phe Thr Leu Thr Ile Ser Ser Leu Gln Pro Glu Asp
           35                  40                  45
   Phe Ala Thr Tyr His Cys Gln Gln Val Met Gln Arg Pro Val Thr Phe
       50                  55                  60
   Gly Gln Gly Thr Lys Val Glu Ile Lys Arg
   65                  70
<210> 22
   <211> 324
   <212> DNA
   <213> Artificial sequence
<220>
   <223> Monomer - dAb2: Partner dAb in TAR1-5d2 (3U linker), d3 (5U linke r)
<400> 22
   ccgtttgatt tccaccttgg tcccttggcc gaacgtaaca ggacgctgca taacctgttg 60
   acagtggtac gtagcaaaat cttcaggttg cagactgctg atggtgagag tgaaatctgt 120
   cccagatcca ctgccactga aacgtgatgg gaccccactt tgcaaccggg atgcagtata 180
   gatcaggagc ttaggggctt tccctggttt ctgctggtac cactgtaaag catcataaat 240
   gctctgactt gcccggcaag tgatggtgac acggtctcct acagatgcag acagggagga 300
   tggagactgg gtcatctgga tgtc 324
<210> 23
   <211> 324
   <212> DNA
   <213> Artificial sequence
<220>
   <223> Monomer - dAb3: Partner dAb in TAR1-5d4 (5U linker)
<220>
   <221> CDS
   <222> (1)..(324)
   <223>
<400> 23
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac cgt gtc acc atc act tgc cgg gca agt cag agc gtt aag gag ttt        96
   Asp Arg Val Thr Ile Thr Cys Arg Ala Ser Gln Ser Val Lys Glu Phe
               20                  25                  30
   tta tgg tgg tac cag cag aaa cca ggg aaa gcc cct aag ctc ctg atc       144
   Leu Trp Trp Tyr Gln Gln Lys Pro Gly Lys Ala Pro Lys Leu Leu Ile
           35                  40                  45
   tat atg gca tcc aat ttg caa agt ggg gtc cca tca cgt ttc agt ggc       192
   Tyr Met Ala Ser Asn Leu Gln Ser Gly Val Pro Ser Arg Phe Ser Gly
       50                  55                  60
   agt gga tct ggg aca gat ttc act ctc acc atc agc agt ctg caa cct       240
   Ser Gly Ser Gly Thr Asp Phe Thr Leu Thr Ile Ser Ser Leu Gln Pro
   65                  70                  75                  80
   gaa gat ttt gct acg tac tac tgt caa cag aag ttt aag ctg cct cgt       288
   Glu Asp Phe Ala Thr Tyr Tyr Cys Gln Gln Lys Phe Lys Leu Pro Arg
                   85                  90                  95
   acg ttc ggc caa ggg acc aag gtg gaa atc aaa cgg                       324
   Thr Phe Gly Gln Gly Thr Lys Val Glu Ile Lys Arg
               100                 105
<210> 24
   <211> 108
   <212> PRT
   <213> Artificial sequence
<220>
   <223> Monomer - dAb3: Partner dAb in TAR1-5d4 (5U linker)
<400> 24
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Val Lys Glu Phe
   20 25 30
   Leu Trp Trp Tyr Gln Gln Lys Pro Gly Lys Ala Pro Lys Leu Leu Ile
   35 40 45
   Tyr Met Ala Ser Asn Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Lys Phe Lys Leu Pro Arg
   85 90 95
   Thr Phe Gly Gln Gly Thr Lys Val Glu Ile Lys Arg
   100 105
<210> 25
   <211> 324
   <212> DNA
   <213> Artificial sequence
<220>
   <223> Monomer - dAb3: Partner dAb in TAR1-5d4 (5U linker)
<400> 25
   ccgtttgatt tccaccttgg tcccttggcc gaacgtacga ggcagcttaa acttctgttg 60
   acagtagtac gtagcaaaat cttcaggttg cagactgctg atggtgagag tgaaatctgt 120
   cccagatcca ctgccactga aacgtgatgg gaccccactt tgcaaattgg atgccatata 180
   gatcaggagc ttaggggctt tccctggttt ctgctggtac caccataaaa actccttaac 240
   gctctgactt gcccggcaag tgatggtgac acggtctcct acagatgcag acagggagga 300
   tggagactgg gtcatctgga tgtc 324
<210> 26
   <211> 324
   <212> DNA
   <213> Artificial sequence
<220>
   <223> TAR1-27
<220>
   <221> CDS
   <222> (1)..(324)
   <223>
<400> 26
   gac atc cag atg acc cag tct cca tcc tcc ctg tct gca tct gta gga      48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att tgg acg aag      96
   Asp Arg Val Thr Ile Thr Cys Arg Ala Ser Gln Ser Ile Trp Thr Lys
               20                  25                  30
   tta cat tgg tac cag cag aaa cca ggg aaa gcc cct aag ctc ctg atc      144
   Leu His Trp Tyr Gln Gln Lys Pro Gly Lys Ala Pro Lys Leu Leu Ile
           35                  40                  45
   tat atg gca tcc agt ttg caa agt ggg gtc cca tca cgt ttc agt ggc      192
   Tyr Met Ala Ser Ser Leu Gln Ser Gly Val Pro Ser Arg Phe Ser Gly
       50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
   65                  70                  75                  80
   gaa gat ttt gct acg tac tac tgt caa cag tgg ttt agt aat cct agt      288
   Glu Asp Phe Ala Thr Tyr Tyr Cys Gln Gln Trp Phe Ser Asn Pro Ser
                   85                  90                  95
   acg ttc ggc caa ggg acc aag gtg gaa atc aaa cgc                      324
   Thr Phe Gly Gln Gly Thr Lys Val Glu Ile Lys Arg
               100                 105
<210> 27
   <211> 108
   <212> PRT
   <213> Artificial sequence
<220>
   <223> TAR1-27
<400> 27
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Trp Thr Lys
               20                  25                  30
   Leu His Trp Tyr Gln Gln Lys Pro Gly Lys Ala Pro Lys Leu Leu Ile
           35                  40                  45
   Tyr Met Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Trp Phe Ser Asn Pro Ser
                   85                  90                  95
   Thr Phe Gly Gln Gly Thr Lys Val Glu Ile Lys Arg
               100                 105
<210> 28
   <211> 324
   <212> DNA
   <213> Artificial sequence
<220>
   <223> TAR1-27
<400> 28
   gcgtttgatt tccaccttgg tcccttggcc gaacgtacta ggattactaa accactgttg 60
   acagtagtac gtagcaaaat cttcaggttg cagactgctg atggtgagag tgaaatctgt 120
   cccagatcca ctgccactga aacgtgatgg gaccccactt tgcaaactgg atgccatata 180
   gatcaggagc ttaggggctt tccctggttt ctgctggtac caatgtaact tcgtccaaat 240
   gctctgactt gcccggcaag tgatggtgac acggtctcct acagatgcag acagggagga 300
   tggagactgg gtcatctgga tgtc 324
<210> 29
   <211> 324
   <212> DNA
   <213> Artificial sequence
<220>
   <223> Partner dAb monomer in TAR1-27d1 (3U linker)
<220>
   <221> CDS
   <222> (1)..(324)
   <223>
<400> 29
   gac atc cag atg acc cag tct cca tcc tcc ctg tct gca tct gta gga      48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att tag ccg att      96
   Asp Arg Val Thr Ile Thr Cys Arg Ala Ser Gln Ser Ile     Pro Ile
               20                  25                      30
   tta tgt tgg tac cag cag aaa cca ggg aaa gcc cct aag ctc ctg atc      144
   Leu Cys Trp Tyr Gln Gln Lys Pro Gly Lys Ala Pro Lys Leu Leu Ile
               35                  40                  45
   tat gct gca tcc agt ttg caa agt ggg gtc cca tca cgt ttc agt ggc      192
   Tyr Ala Ala Ser Ser Leu Gln Ser Gly Val Pro Ser Arg Phe Ser Gly
           50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
       65                  70                  75
   gaa gat ttt gct acg tac tac tgt caa cag att cag cat att cct gtg      288
   Glu Asp Phe Ala Thr Tyr Tyr Cys Gln Gln Ile Gln His Ile Pro Val
   80                  85                  90                  95
   acg ttc ggc caa ggg acc aag gtg gaa atc aaa cgg                      324
   Thr Phe Gly Gln Gly Thr Lys Val Glu Ile Lys Arg
                   100                 105
<210> 30
   <211> 29
   <212> PRT
   <213> Artificial sequence
<220>
   <223> Partner dAb monomer in TAR1-27d1 (3U linker)
<400> 30
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile
   20 25
<210> 31
   <211> 78
   <212> PRT
   <213> Artificial sequence
<220>
   <223> Partner dAb monomer in TAR1-27d1 (3U linker)
<400> 31
   Pro Ile Leu Cys Trp Tyr Gln Gln Lys Pro Gly Lys Ala Pro Lys Leu
   1 5 10 15
   Leu Ile Tyr Ala Ala Ser Ser Leu Gln Ser Gly Val Pro Ser Arg Phe
   20 25 30
   Ser Gly Ser Gly Ser Gly Thr Asp Phe Thr Leu Thr Ile Ser Ser Leu
   35 40 45
   Gln Pro Glu Asp Phe Ala Thr Tyr Tyr Cys Gln Gln Ile Gln His Ile
   50 55 60
   Pro Val Thr Phe Gly Gln Gly Thr Lys Val Glu Ile Lys Arg
   65 70 75
<210> 32
   <211> 324
   <212> DNA
   <213> Artificial sequence
<220>
   <223> Partner dAb monomer in TAR1-27d1 (3U linker)
<400> 32
   ccgtttgatt tccaccttgg tcccttggcc gaacgtcaca ggaatatgct gaatctgttg     60
   acagtagtac gtagcaaaat cttcaggttg cagactgctg atggtgagag tgaaatctgt    120
   cccagatcca ctgccactga aacgtgatgg gaccccactt tgcaaactgg atgcagcata    180
   gatcaggagc ttaggggctt tccctggttt ctgctggtac caacataaaa tcggctaaat    240
   gctctgactt gcccggcaag tgatggtgac acggtctcct acagatgcag acagggagga    300
   tggagactgg gtcatctgga tgtc                                           324
<210> 33
   <211> 324
   <212> DNA
   <213> Artificial sequence
<220>
   <223> Partner dAb monomer in TAR1-27d2 (3U linker)
<220>
   <221> CDS
   <222> (1)..(324)
   <223>
<400> 33
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att ggg tag gat        96
   Asp Arg Val Thr Ile Thr Cys Arg Ala Ser Gln Ser Ile Gly     Asp
               20                  25                  30
   tta cat tgg tac cag cag aaa cca ggg aaa gcc cct aag ctc ctg atc        144
   Leu His Trp Tyr Gln Gln Lys Pro Gly Lys Ala Pro Lys Leu Leu Ile
               35                  40                  45
   tat acg gca tcc ctt ttg caa agt ggg gtc cca tca cgt ttc agt ggc        192
   Tyr Thr Ala Ser Leu Leu Gln Ser Gly Val Pro Ser Arg Phe Ser Gly
           50                  55                  60
   agt gga tct ggg aca gat ttc act ctc acc atc agc agt ctg caa cct        240
   Ser Gly Ser Gly Thr Asp Phe Thr Leu Thr Ile Ser Ser Leu Gln Pro
       65                  70                  75
   gaa gat ttt gct acg tac tac tgt caa cag cag agt gct ttt cct aat        288
   Glu Asp Phe Ala Thr Tyr Tyr Cys Gln Gln Gln Ser Ala Phe Pro Asn
   80                  85                  90                  95
   acg ctc ggc caa ggg acc aag gtg gaa atc aaa cgg                        324
   Thr Leu Gly Gln Gly Thr Lys Val Glu Ile Lys Arg
                   100                 105
<210> 34
   <211> 30
   <212> PRT
   <213> Artificial sequence
<220>
   <223> Partner dAb monomer in TAR1-27d2 (3U linker)
<400> 34
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Gly
               20                  25                  30
<210> 35
   <211> 77
   <212> PRT
   <213> Artificial sequence
<220>
   <223> Partner dAb monomer in TAR1-27d2 (3U linker)
<400> 35
   Asp Leu His Trp Tyr Gln Gln Lys Pro Gly Lys Ala Pro Lys Leu Leu
   1 5 10 15
   Ile Tyr Thr Ala Ser Leu Leu Gln Ser Gly Val Pro Ser Arg Phe Ser
   20 25 30
   Gly Ser Gly Ser Gly Thr Asp Phe Thr Leu Thr Ile Ser Ser Leu Gln
   35 40 45
   Pro Glu Asp Phe Ala Thr Tyr Tyr Cys Gln Gln Gln Ser Ala Phe Pro
   50 55 60
   Asn Thr Leu Gly Gln Gly Thr Lys Val Glu Ile Lys Arg
   65 70 75
<210> 36
   <211> 324
   <212> DNA
   <213> Artificial sequence
<220>
   <223> Partner dAb monomer in TAR1-27d2 (3U linker)
<400> 36
   ccgtttgatt tccaccttgg tcccttggcc gagcgtatta ggaaaagcac tctgctgttg 60
   acagtagtac gtagcaaaat cttcaggttg cagactgctg atggtgagag tgaaatctgt 120
   cccagatcca ctgccactga aacgtgatgg gaccccactt tgcaaaaggg atgccgtata 180
   gatcaggagc ttaggggctt tccctggttt ctgctggtac caatgtaaat cctacccaat 240
   gctctgactt gcccggcaag tgatggtgac acggtctcct acagatgcag acagggagga 300
   tggagactgg gtcatctgga tgtc 324
<210> 37
   <211> 324
   <212> DNA
   <213> Artificial sequence
<220>
   <223> Partner dAb monomer in TAR1-27d7 (3Ulinker)
<220>
   <221> CDS
   <222> (1)..(324)
   <223>
<400> 37
   gac atc cag atg acc cag tct cca tcc tcc ctg tct gca tcc gta gga       48
   Asp Ile Gln Met Thr Gln Ser Pro Ser Ser Leu Ser Ala Ser Val Gly
   1               5                   10                  15
   gac cgt gtc acc atc act tgc cgg gca agt cag agc ata acg aag aat       96
   Asp Arg Val Thr Ile Thr Cys Arg Ala Ser Gln Ser Ile Thr Lys Asn
               20                  25                  30
   tta ctt tgg tac cag cag aaa cca ggg aaa gcc cct aag ctc ctg atc      144
   Leu Leu Trp Tyr Gln Gln Lys Pro Gly Lys Ala Pro Lys Leu Leu Ile
           35                  40                  45
   tat tag gca tcc tct ttg caa agt ggg gtc cca tca cgt ttc agt ggc      192
   Tyr     Ala Ser Ser Leu Gln Ser Gly Val Pro Ser Arg Phe Ser Gly
           50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
       65                  70                  75
   gaa gat ttt gct acg tac tac tgt caa cag ctt cgt cat aag cct ccg      288
   Glu Asp Phe Ala Thr Tyr Tyr Cys Gln Gln Leu Arg His Lys Pro Pro
   80                  85                  90                  95
   acg ttc ggc caa ggg acc aag gtg gaa atc aaa cgg                      324
   Thr Phe Gly Gln Gly Thr Lys Val Glu Ile Lys Arg
                   100                 105
<210> 38
   <211> 49
   <212> PRT
   <213> Artificial sequence
<220>
   <223> Partner dAb monomer in TAR1-27d7 (3Ulinker)
<400> 38
<210> 39
   <211> 58
   <212> PRT
   <213> Artificial sequence
<220>
   <223> Partner dAb monomer in TAR1-27d7 (3Ulinker)
<400> 39
   Ala Ser Ser Leu Gln Ser Gly Val Pro Ser Arg Phe Ser Gly Ser Gly
   1               5                   10                  15
   Ser Gly Thr Asp Phe Thr Leu Thr Ile Ser Ser Leu Gln Pro Glu Asp
               20                  25                  30
   Phe Ala Thr Tyr Tyr Cys Gln Gln Leu Arg His Lys Pro Pro Thr Phe
           35                  40                  45
   Gly Gln Gly Thr Lys Val Glu Ile Lys Arg
       50                  55
<210> 40
   <211> 324
   <212> DNA
   <213> Artificial sequence
<220>
   <223> Partner dAb monomer in TAR1-27d7 (3Ulinker)
<400> 40
   ccgtttgatt tccaccttgg tcccttggcc gaacgtcgga ggcttatgac gaagctgttg      60
   acagtagtac gtagcaaaat cttcaggttg cagactgctg atggtgagag tgaaatctgt     120
   cccagatcca ctgccactga aacgtgatgg gaccccactt tgcaaagagg atgcctaata     180
   gatcaggagc ttaggggctt tccctggttt ctgctggtac caaagtaaat tcttcgttat     240
   gctctgactt gcccggcaag tgatggtgac acggtctcct acggatgcag acagggagga     300
   tggagactgg gtcatctgga tgtc                                            324
<210> 41
   <211> 324
   <212> DNA
   <213> Artificial sequence
<220>
   <223> Partner dAb monomer in TAR1-27d8 (3Ulinker)
<220>
   <221> CDS
   <222> (1)..(324)
   <223>
<400> 41
   gac atc cag atg acc cag tct cca tcc tcc ctg tct gca tct gta gga       48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att tag aag tct       96
   Asp Arg Val Thr Ile Thr Cys Arg Ala Ser Gln Ser Ile     Lys Ser
               20                  25                  30
   tta agg tgg tac cag cag aaa cca ggg aaa gcc cct aag ctc ctg atc      144
   Leu Arg Trp Tyr Gln Gln Lys Pro Gly Lys Ala Pro Lys Leu Leu Ile
               35                  40                  45
   tat cat gca tcc gat ttg caa agt ggg gtc cca tca cgt ttc agt ggc      192
   Tyr His Ala Ser Asp Leu Gln Ser Gly Val Pro Ser Arg Phe Ser Gly
           50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
       65                  70                  75
   gaa gat ttt gct acg tac tac tgt caa cag atg gtt aat agt cct gtt      288
   Glu Asp Phe Ala Thr Tyr Tyr Cys Gln Gln Met Val Asn Ser Pro Val
   80                  85                  90                  95
   acg ttc ggc caa ggg acc aag gtg gaa atc aaa cgg                      324
   Thr Phe Gly Gln Gly Thr Lys Val Glu Ile Lys Arg
                   100                 105
<210> 42
   <211> 29
   <212> PRT
   <213> Artificial sequence
<220>
   <223> Partner dAb monomer in TAR1-27d8 (3Ulinker)
<400> 42
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile
               20                  25
<210> 43
   <211> 78
   <212> PRT
   <213> Artificial sequence
<220>
   <223> Partner dAb monomer in TAR1-27d8 (3Ulinker)
<210> 44
   <211> 324
   <212> DNA
   <213> Artificial sequence
<220>
   <223> Partner dAb monomer in TAR1-27d8 (3Ulinker)
<400> 44
   ccgtttgatt tccaccttgg tcccttggcc gaacgtaaca ggactattaa ccatctgttg 60
   acagtagtac gtagcaaaat cttcaggttg cagactgctg atggtgagag tgaaatctgt 120
   cccagatcca ctgccactga aacgtgatgg gaccccactt tgcaaatcgg atgcatgata 180
   gatcaggagc ttaggggctt tccctggttt ctgctggtac caccttaaag acttctaaat 240
   gctctgactt gcccggcaag tgatggtgac acggtctcct acagatgcag acagggagga 300
   tggagactgg gtcatctgga tgtc 324
<210> 45
   <211> 324
   <212> DNA
   <213> Artificial sequence
<220>
   <223> Partner dAb monomer in TAR1-27d12 (3Ulinker)
<220>
   <221> CDS
   <222> (1)..(324)
   <223>
<400> 45
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att tag acg gcg        96
   Asp Arg Val Thr Ile Thr Cys Arg Ala Ser Gln Ser Ile     Thr Ala
               20                  25                      30
   tta cat tgg tac cag cag aaa cca ggg aaa gcc cct aag ctc ctg atc        144
   Leu His Trp Tyr Gln Gln Lys Pro Gly Lys Ala Pro Lys Leu Leu Ile
               35                  40                  45
   tat tct gca tcc agt ttg caa agt ggg gtc cca tca cgt ttc agt ggc        192
   Tyr Ser Ala Ser Ser Leu Gln Ser Gly Val Pro Ser Arg Phe Ser Gly
           50                  55                  60
   agt gga tct ggg aca gat ttc act ctc acc atc agc agt ctg caa cct        240
   Ser Gly Ser Gly Thr Asp Phe Thr Leu Thr Ile Ser Ser Leu Gln Pro
       65                  70                  75
   gaa gat ttt gct acg tac tac tgt caa cag tcg agt ttt ttg cct ttt        288
   Glu Asp Phe Ala Thr Tyr Tyr Cys Gln Gln Ser Ser Phe Leu Pro Phe
   80                  85                  90                  95
   acg ttc ggc caa ggg acc aag gtg gaa atc aaa cgg                        324
   Thr Phe Gly Gln Gly Thr Lys Val Glu Ile Lys Arg
                   100                 105
<210> 46
   <211> 29
   <212> PRT
   <213> Artificial sequence
<220>
   <223> Partner dAb monomer in TAR1-27d12 (3Ulinker)
<400> 46
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile
               20                  25
<210> 47
   <211> 78
   <212> PRT
   <213> Artificial sequence
<220>
   <223> Partner dAb monomer in TAR1-27d12 (3Ulinker)
<400> 47
   Thr Ala Leu His Trp Tyr Gln Gln Lys Pro Gly Lys Ala Pro Lys Leu
   1               5                   10                  15
   Leu Ile Tyr Ser Ala Ser Ser Leu Gln Ser Gly Val Pro Ser Arg Phe
               20                  25                  30
   Ser Gly Ser Gly Ser Gly Thr Asp Phe Thr Leu Thr Ile Ser Ser Leu
           35                  40                  45
   Gln Pro Glu Asp Phe Ala Thr Tyr Tyr Cys Gln Gln Ser Ser Phe Leu
       50                  55                  60
   Pro Phe Thr Phe Gly Gln Gly Thr Lys Val Glu Ile Lys Arg
   65                  70                  75
<210> 48
   <211> 324
   <212> DNA
   <213> Artificial sequence
<220>
   <223> Partner dAb monomer in TAR1-27d12 (3Ulinker)
<400> 48
   ccgtttgatt tccaccttgg tcccttggcc gaacgtaaaa ggcaaaaaac tcgactgttg     60
   acagtagtac gtagcaaaat cttcaggttg cagactgctg atggtgagag tgaaatctgt    120
   cccagatcca ctgccactga aacgtgatgg gaccccactt tgcaaactgg atgcagaata    180
   gatcaggagc ttaggggctt tccctggttt ctgctggtac caatgtaacg ccgtctaaat    240
   gctctgactt gcccggcaag tgatggtgac acggtctcct acagatgcag acagggagga    300
   tggagactgg gtcatctgga tgtc                                           324
<210> 49
   <211> 324
   <212> DNA
   <213> Artificial sequence
<220>
   <223> Partner dAb monomer in TARl-27dl6 (3U linker)
<220>
   <221> CDS
   <222> (1)..(324)
   <223>
<400> 49
   gac atc cag atg acc cag tct cca tcc tcc ctg tct gca tct gta gga      48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att ggg ccg aat      96
   Asp Arg Val Thr Ile Thr Cys Arg Ala Ser Gln Ser Ile Gly Pro Asn
               20                  25                  30
   tta gag tgg tac cag cag aaa cca ggg aaa gcc cct aag ctc ctg atc     144
   Leu Glu Trp Tyr Gln Gln Lys Pro Gly Lys Ala Pro Lys Leu Leu Ile
           35                  40                  45
   tat gct gca tcc agt ttg caa agt ggg gtc cca tca cgt ttc agt ggc     192
   Tyr Ala Ala Ser Ser Leu Gln Ser Gly Val Pro Ser Arg Phe Ser Gly
       50                  55                  60
   agt gga tct ggg aca gat ttc act ctc acc atc agc agt ctg caa cct     240
   Ser Gly Ser Gly Thr Asp Phe Thr Leu Thr Ile Ser Ser Leu Gln Pro
   65                  70                  75                  80
   gaa gat ttt gct acg tac tac tgt caa cag cag atg ggg cgt cct cgg     288
   Glu Asp Phe Ala Thr Tyr Tyr Cys Gln Gln Gln Met Gly Arg Pro Arg
                   85                  90                  95
   acg ttc ggc caa ggg acc aag gtg gaa atc aaa cgg                     324
   Thr Phe Gly Gln Gly Thr Lys Val Glu Ile Lys Arg
               100                 105
<210> 50
   <211> 108
   <212> PRT
   <213> Artificial sequence
<220>
   <223> Partner dAb monomer in TAR1-27d16 (3U linker)
<400> 50
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Gly Pro Asn
   20 25 30
   Leu Glu Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Gln Met Gly Arg Pro Arg
   85 90 95
   Thr Phe Gly Gln Gly Thr Lys Val Glu Ile Lys Arg
   100 105
<210> 51
   <211> 324
   <212> DNA
   <213> Artificial sequence
<220>
   <223> Partner dAb monomer in TAR1-27d16 (3U linker)
<400> 51
   ccgtttgatt tccaccttgg tcccttggcc gaacgtccga ggacgcccca tctgctgttg 60
   acagtagtac gtagcaaaat cttcaggttg cagactgctg atggtgagag tgaaatctgt 120
   cccagatcca ctgccactga aacgtgatgg gaccccactt tgcaaactgg atgcagcata 180
   gatcaggagc ttaggggctt tccctggttt ctgctggtac cactctaaat tcggcccaat 240
   gctctgactt gcccggcaag tgatggtgac acggtctcct acagatgcag acagggagga 300
   tggagactgg gtcatctgga tgtc 324
<210> 52
   <211> 324
   <212> DNA
   <213> Artificial sequence
<220>
   <223> Partner dAb monomer in TARI-27d23 (5U linker)
<220>
   <221> CDS
   <222> (1)..(324)
   <223>
<400> 52
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att aag cat tag        96
   Asp Arg Val Thr Ile Thr Cys Arg Ala Ser Gln Ser Ile Lys His
               20                  25                  30
   tta gct tgg tac cag cag aaa cca ggg aaa gcc cct aag ctc ctg atc       144
   Leu Ala Trp Tyr Gln Gln Lys Pro Gly Lys Ala Pro Lys Leu Leu Ile
               35                  40                  45
   tat aag gca tcc gtg ttg caa agt ggg gtc cca tca cgt ttc agt ggc       192
   Tyr Lys Ala Ser Val Leu Gln Ser Gly Val Pro Ser Arg Phe Ser Gly
           50                  55                  60
   agt gga tct ggg aca gat ttc act ctc acc atc agc agt ctg caa cct       240
   Ser Gly Ser Gly Thr Asp Phe Thr Leu Thr Ile Ser Ser Leu Gln Pro
       65                  70                  75
   gaa gat ttt gct acg tac tac tgt caa cag ctt agg cgt cgt cct act       288
   Glu Asp Phe Ala Thr Tyr Tyr Cys Gln Gln Leu Arg Arg Arg Pro Thr
   80                  85                  90                  95
   acg ttc ggc caa ggg acc aag gtg gaa atc aaa cgg                       324
   Thr Phe Gly Gln Gly Thr Lys Val Glu Ile Lys Arg
                   100                 105
<210> 53
   <211> 31
   <212> PRT
   <213> Artificial sequence
<220>
   <223> Partner dAb monomer in TAR1-27d23 (5U linker)
<400> 53
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Lys His
               20                  25                  30
<210> 54
   <211> 76
   <212> PRT
   <213> Artificial sequence
<220>
   <223> Partner dAb monomer in TAR1-27d23 (5U linker)
<400> 54
   Leu Ala Trp Tyr Gln Gln Lys Pro Gly Lys Ala Pro Lys Leu Leu Ile
   1               5                   10                  15
   Tyr Lys Ala Ser Val Leu Gln Ser Gly Val Pro Ser Arg Phe Ser Gly
               20                  25                  30
   Ser Gly Ser Gly Thr Asp Phe Thr Leu Thr Ile Ser Ser Leu Gln Pro
           35                  40                  45
   Glu Asp Phe Ala Thr Tyr Tyr Cys Gln Gln Leu Arg Arg Arg Pro Thr
       50                  55                  60
   Thr Phe Gly Gln Gly Thr Lys Val Glu Ile Lys Arg
   65                  70                  75
<210> 55
   <211> 324
   <212> DNA
   <213> Artificial sequence
<220>
   <223> Partner dAb monomer in TAR1-27d23 (5U linker)
<400> 55
   ccgtttgatt tccaccttgg tcccttggcc gaacgtagta ggacgacgcc taagctgttg 60
   acagtagtac gtagcaaaat cttcaggttg cagactgctg atggtgagag tgaaatctgt 120
   cccagatcca ctgccactga aacgtgatgg gaccccactt tgcaacacgg atgccttata 180
   gatcaggagc ttaggggctt tccctggttt ctgctggtac caagctaact aatgcttaat 240
   gctctgactt gcccggcaag tgatggtgac acggtctcct acagatgcag acagggagga 300
   tggagactgg gtcatctgga tgtc 324
<210> 56
   <211> 324
   <212> DNA
   <213> Artificial sequence
<220>
   <223> Partner dAb monomer in TAR1-27d30 (7U linker)
<220>
   <221> CDS
   <222> (1)..(324)
   <223>
<400> 56
   gac atc cag atg acc cag tct cca tcc tcc ctg tct gca tct gta gga       48
   Asp Ile Gln Met Thr Gln Ser Pro Ser Ser Leu Ser Ala Ser Val Gly
   1               5                   10                  15
   gac cgt gtc acc atc act tgc cgg gca agt cag agc gtt aag gct tag       96
   Asp Arg Val Thr Ile Thr Cys Arg Ala Ser Gln Ser Val Lys Ala
               20                  25                  30
   tta act tgg tac cag cag aaa cca ggg aaa gcc cct aag ctc ctg atc      144
   Leu Thr Trp Tyr Gln Gln Lys Pro Gly Lys Ala Pro Lys Leu Leu Ile
               35                  40                  45
   tat aag gca tcc act ttg caa agt ggg gtc cca tca cgt ttc agt ggc      192
   Tyr Lys Ala Ser Thr Leu Gln Ser Gly Val Pro Ser Arg Phe Ser Gly
           50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
       65                  70                  75
   gaa gat ttt gct acg tac tac tgt caa cag cat agt tct agg cct tat      288
   Glu Asp Phe Ala Thr Tyr Tyr Cys Gln Gln His Ser Ser Arg Pro Tyr
   80                  85                  90                  95
   acg ttc ggc caa ggg acc aag gtg gaa atc aaa cgg                      324
   Thr Phe Gly Gln Gly Thr Lys Val Glu Ile Lys Arg
                   100                 105
<210> 57
   <211> 31
   <212> PRT
   <213> Artificial sequence
<220>
   <223> Partner dAb monomer in TAR1-27d30 (7U linker)
<400> 57
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Val Lys Ala
               20                  25                  30
<210> 58
   <211> 76
   <212> PRT
   <213> Artificial sequence
<220>
   <223> Partner dAb monomer in TAR1-27d30 (7U linker)
<400> 58
   Leu Thr Trp Tyr Gln Gln Lys Pro Gly Lys Ala Pro Lys Leu Leu Ile
   1               5                   10                  15
   Tyr Lys Ala Ser Thr Leu Gln Ser Gly Val Pro Ser Arg Phe Ser Gly
               20                  25                  30
   Ser Gly Ser Gly Thr Asp Phe Thr Leu Thr Ile Ser Ser Leu Gln Pro
           35                  40                  45
   Glu Asp Phe Ala Thr Tyr Tyr Cys Gln Gln His Ser Ser Arg Pro Tyr
       50                  55                  60
   Thr Phe Gly Gln Gly Thr Lys Val Glu Ile Lys Arg
   65                  70                  75
<210> 59
   <211> 324
   <212> DNA
   <213> Artificial sequence
<220>
   <223> Partner dAb monomer in TAR1-27d30 (7U linker)
<400> 59
   ccgtttgatt tccaccttgg tcccttggcc gaacgtataa ggcctagaac tatgctgttg      60
   acagtagtac gtagcaaaat cttcaggttg cagactgctg atggtgagag tgaaatctgt     120
   cccagatcca ctgccactga aacgtgatgg gaccccactt tgcaaagtgg atgccttata     180
   gatcaggagc ttaggggctt tccctggttt ctgctggtac caagttaact aagccttaac     240
   gctctgactt gcccggcaag tgatggtgac acggtctcct acagatgcag acagggagga     300
   tggagactgg gtcatctgga tgtc                                            324
<210> 60
   <211> 324
   <212> DNA
   <213> Artificial sequence
<220>
   <223> Partner dAb monomer in TAR1-27d31 (7U linker)
<220>
   <221> CDS
   <222> (1)..(324)
   <223>
<400> 60
   gac atc cag atg acc cag tct cca tcc tcc ctg tct gca tct gta gga 48
   Asp Ile Gln Met Thr Gln Ser Pro Ser Ser Leu Ser Ala Ser Val Gly
   1 5 10 15
   gac cgt gtc acc atc act tgc cgg gca agt cag agc att gag aat cgg 96
   Asp Arg Val Thr Ile Thr Cys Arg Ala Ser Gln Ser Ile Glu Asn Arg
   20 25 30
   tta ggt tgg tac cag cag aaa cca ggg aaa gcc cct aag ctc ctg atc 144
   Leu Gly Trp Tyr Gln Gln Lys Pro Gly Lys Ala Pro Lys Leu Leu Ile
   35 40 45
   tat tag gcg tcc ttg ttg caa agt ggg gtc cca tca cgt ttc agt ggc 192
   Tyr Ala Ser Leu Leu Gln Ser Gly Val Pro Ser Arg Phe Ser Gly
   50 55 60
   agt gga tct ggg aca gat ttc act ctc acc atc agc agt ctg caa cct 240
   Ser Gly Ser Gly Thr Asp Phe Thr Leu Thr Ile Ser Ser Leu Gln Pro
   65 70 75
   gaa gat ttt gct acg tac tac tgt caa cag gat tcg tat ttt cct cgt 288
   Glu Asp Phe Ala Thr Tyr Tyr Cys Gln Gln Asp Ser Tyr Phe Pro Arg
   80 85 90 95
   acg ttc ggc caa ggg acc aag gtg gaa atc aaa cgg 324
   Thr Phe Gly Gln Gly Thr Lys Val Glu Ile Lys Arg
   100 105
<210> 61
   <211> 49
   <212> PRT
   <213> Artificial sequence
<220>
   <223> Partner dAb monomer in TAR1-27d31 (7U linker)
<400> 61
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Glu Asn Arg
   20 25 30
   Leu Gly Trp Tyr Gln Gln Lys Pro Gly Lys Ala Pro Lys Leu Leu Ile
   35 40 45
   Tyr
<210> 62
   <211> 58
   <212> PRT
   <213> Artificial sequence
<220>
   <223> Partner dAb monomer in TAR1-27d31 (7U linker)
<400> 62
   Ala Ser Leu Leu Gln Ser Gly Val Pro Ser Arg Phe Ser Gly Ser Gly
   1 5 10 15
   Ser Gly Thr Asp Phe Thr Leu Thr Ile Ser Ser Leu Gln Pro Glu Asp
   20 25 30
   Phe Ala Thr Tyr Tyr Cys Gln Gln Asp Ser Tyr Phe Pro Arg Thr Phe
   35 40 45
   Gly Gln Gly Thr Lys Val Glu Ile Lys Arg
   50 55
<210> 63
   <211> 324
   <212> DNA
   <213> Artificial sequence
<220>
   <223> Partner dAb monomer in TAR1-27d31 (7U linker)
<400> 63
   ccgtttgatt tccaccttgg tcccttggcc gaacgtacga ggaaaatacg aatcctgttg 60
   acagtagtac gtagcaaaat cttcaggttg cagactgctg atggtgagag tgaaatctgt 120
   cccagatcca ctgccactga aacgtgatgg gaccccactt tgcaacaagg acgcctaata 180
   gatcaggagc ttaggggctt tccctggttt ctgctggtac caacctaacc gattctcaat 240
   gctctgactt gcccggcaag tgatggtgac acggtctcct acagatgcag acagggagga 300
   tggagactgg gtcatctgga tgtc 324
<210> 64
   <211> 324
   <212> DNA
   <213> Artificial sequence
<220>
   <223> Partner dAb monomer in TAR1-27d36 (7U linker)
<220>
   <221> CDS
   <222> (1)..(324)
   <223>
<400> 64
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att atg gat aag        96
   Asp Arg Val Thr Ile Thr Cys Arg Ala Ser Gln Ser Ile Met Asp Lys
               20                  25                  30
   tta aag tgg tac cag cag aaa cca ggg aaa gcc cct aag ctc ctg atc        144
   Leu Lys Trp Tyr Gln Gln Lys Pro Gly Lys Ala Pro Lys Leu Leu Ile
           35                  40                  45
   tat tag gca tcc att ttg caa agt ggg gtc cca tca cgt ttc agt ggc        192
   Tyr     Ala Ser Ile Leu Gln Ser Gly Val Pro Ser Arg Phe Ser Gly
           50                  55                  60
   agt gga tct ggg aca gat ttc act ctc acc atc agc agt ctg caa cct        240
   Ser Gly Ser Gly Thr Asp Phe Thr Leu Thr Ile Ser Ser Leu Gln Pro
       65                  70                  75
   gaa gat ttt gct acg tac tac tgt caa cag gat agt ggg ggt cct aat        288
   Glu Asp Phe Ala Thr Tyr Tyr Cys Gln Gln Asp Ser Gly Gly Pro Asn
   80                  85                  90                  95
   acg ttc ggc caa ggg acc aag gtg gaa atc aaa cgg                        324
   Thr Phe Gly Gln Gly Thr Lys Val Glu Ile Lys Arg
                   100                 105
<210> 65
   <211> 49
   <212> PRT
   <213> Artificial sequence
<220>
   <223> Partner dAb monomer in TAR1-27d36 (7U linker)
<400> 65
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Met Asp Lys
   20 25 30
   Leu Lys Trp Tyr Gln Gln Lys Pro Gly Lys Ala Pro Lys Leu Leu Ile
   35 40 45
   Tyr
<210> 66
   <211> 58
   <212> PRT
   <213> Artificial sequence
<220>
   <223> Partner dAb monomer in TAR1-27d36 (7U linker)
<400> 66
   Ala Ser Ile Leu Gln Ser Gly Val Pro Ser Arg Phe Ser Gly Ser Gly
   1 5 10 15
   Ser Gly Thr Asp Phe Thr Leu Thr Ile Ser Ser Leu Gln Pro Glu Asp
   20 25 30
   Phe Ala Thr Tyr Tyr Cys Gln Gln Asp Ser Gly Gly Pro Asn Thr Phe
   35 40 45
   Gly Gln Gly Thr Lys Val Glu Ile Lys Arg
   50 55
<210> 67
   <211> 324
   <212> DNA
   <213> Artificial sequence
<220>
   <223> Partner dAb monomer in TAR1-27d36 (7U linker)
<400> 67
   ccgtttgatt tccaccttgg tcccttggcc gaacgtatta ggacccccac tatcctgttg      60
   acagtagtac gtagcaaaat cttcaggttg cagactgctg atggtgagag tgaaatctgt     120
   cccagatcca ctgccactga aacgtgatgg gaccccactt tgcaaaatgg atgcctaata     180
   gatcaggagc ttaggggctt tccctggttt ctgctggtac cactttaact tatccataat     240
   gctctgactt gcccggcaag tgatggtgac acggtctcct acagatgcag acagggagga     300
   tggagactgg gtcatctgga tgtc                                            324
<210> 68
   <211> 324
   <212> DNA
   <213> Artificial sequence
<220>
   <223> Partner dAb monomer in TAR1-27d37 (7U linker)
<220>
   <221> CDS
   <222> (1)..(324)
   <223>
<400> 68
   gac atc cag atg acc cag tct cca tcc tcc ctg tct gca tct gta gga      48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att ggg agg aat 96
   Asp Arg Val Thr Ile Thr Cys Arg Ala Ser Gln Ser Ile Gly Arg Asn
   20 25 30
   tta gag tgg tac cag cag aaa cca ggg aaa gcc cct aag ctc ctg atc 144
   Leu Glu Trp Tyr Gln Gln Lys Pro Gly Lys Ala Pro Lys Leu Leu Ile
   35 40 45
   tat gat gca tcc cat ttg caa agt ggg gtc cca tca cgt ttc agt ggc 192
   Tyr Asp Ala Ser His Leu Gln Ser Gly Val Pro Ser Arg Phe Ser Gly
   50 55 60
   agt gga tct ggg aca gat ttc act ctc acc atc agc agt ctg caa cct 240
   Ser Gly Ser Gly Thr Asp Phe Thr Leu Thr Ile Ser Ser Leu Gln Pro
   65 70 75 80
   gaa gat ttt gct acg tac tac tgt caa cag tcg cgt tgg ctt cct cgt 288
   Glu Asp Phe Ala Thr Tyr Tyr Cys Gln Gln Ser Arg Trp Leu Pro Arg
   85 90 95
   acg ttc ggc caa ggg acc aag gtg gaa atc aaa cgg 324
   Thr Phe Gly Gln Gly Thr Lys Val Glu Ile Lys Arg
   100 105
<210> 69
   <211> 108
   <212> PRT
   <213> Artificial sequence
<220>
   <223> Partner dAb monomer in TAR1-27d37 (7U linker)
<400> 69
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Gly Arg Asn
   20 25 30
   Leu Glu Trp Tyr Gln Gln Lys Pro Gly Lys Ala Pro Lys Leu Leu Ile
   35 40 45
   Tyr Asp Ala Ser His Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Arg Trp Leu Pro Arg
   85 90 95
   Thr Phe Gly Gln Gly Thr Lys Val Glu Ile Lys Arg
   100 105
<210> 70
   <211> 324
   <212> DNA
   <213> Artificial sequence
<220>
   <223> Partner dAb monomer in TAR1-27d37 (7U linker)
<400> 70
   ccgtttgatt tccaccttgg tcccttggcc gaacgtacga ggaagccaac gcgactgttg 60
   acagtagtac gtagcaaaat cttcaggttg cagactgctg atggtgagag tgaaatctgt 120
   cccagatcca ctgccactga aacgtgatgg gaccccactt tgcaaatggg atgcatcata 180
   gatcaggagc ttaggggctt tccctggttt ctgctggtac cactctaaat tcctcccaat 240
   gctctgactt gcccggcaag tgatggtgac acggtctcct acagatgcag acagggagga 300
   tggagactgg gtcatctgga tgtc 324
<210> 71
   <211> 324
   <212> DNA
   <213> Artificial sequence
<220>
   <223> Partner dAb monomer in TAR1-27d39 (7U linker)
<220>
   <221> CDS
   <222> (1)..(324)
   <223>
<400> 71
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att agg aag atg        96
   Asp Arg Val Thr Ile Thr Cys Arg Ala Ser Gln Ser Ile Arg Lys Met
               20                  25                  30
   tta gtt tgg tac cag cag aaa cca ggg aaa gcc cct aag ctc ctg atc       144
   Leu Val Trp Tyr Gln Gln Lys Pro Gly Lys Ala Pro Lys Leu Leu Ile
           35                  40                  45
   tat cgg gca tcc tat ttg caa agt ggg gtc cca tca cgt ttc agt ggc       192
   Tyr Arg Ala Ser Tyr Leu Gln Ser Gly Val Pro Ser Arg Phe Ser Gly
       50                  55                  60
   agt gga tct ggg aca gat ttc act ctc acc atc agc agt ctg caa cct       240
   Ser Gly Ser Gly Thr Asp Phe Thr Leu Thr Ile Ser Ser Leu Gln Pro
   65                  70                  75                  80
   gaa gat ttt gct acg tac tac tgt caa cag gct ttt cgg cgg cct agg       288
   Glu Asp Phe Ala Thr Tyr Tyr Cys Gln Gln Ala Phe Arg Arg Pro Arg
                   85                  90                  95
   acg ttc ggc caa ggg acc aag gtg gaa atc aaa cgg                       324
   Thr Phe Gly Gln Gly Thr Lys Val Glu Ile Lys Arg
               100                 105
<210> 72
   <211> 108
   <212> PRT
   <213> Artificial sequence
<220>
   <223> Partner dAb monomer in TAR1-27d39 (7U linker)
<400> 72
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Arg Lys Met
   20 25 30
   Leu Val Trp Tyr Gln Gln Lys Pro Gly Lys Ala Pro Lys Leu Leu Ile
   35 40 45
   Tyr Arg Ala Ser Tyr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ala Phe Arg Arg Pro Arg
   85 90 95
   Thr Phe Gly Gln Gly Thr Lys Val Glu Ile Lys Arg
   100 105
<210> 73
   <211> 324
   <212> DNA
   <213> Artificial sequence
<220>
   <223> Partner dAb monomer in TAR1-27d39 (7U linker)
<210> 74
   <211> 345
   <212> DNA
   <213> Artificial sequence
<220>
   <223> TAR2h-5
<220>
   <221> CDS
   <222> (1)..(345)
   <223>
<400> 74
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt gat ctt tat      96
   Ser Leu Arg Leu Ser Cys Ala Ala Ser Gly Phe Thr Phe Asp Leu Tyr
               20                  25                  30
   aat atg ttt tgg gtc cgc cag gct cca ggg aag ggt cta gag tgg gtc      144
   Asn Met Phe Trp Val Arg Gln Ala Pro Gly Lys Gly Leu Glu Trp Val
           35                  40                  45
   tca ttt att agt cag act ggt agg ctt aca tgg tac gca gac tcc gtg      192
   Ser Phe Ile Ser Gln Thr Gly Arg Leu Thr Trp Tyr Ala Asp Ser Val
       50                  55                  60
   aag ggc cgg ttc acc atc tcc cgc gac aat tcc aag aac acg ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
                   85                  90                  95
   gcg aaa acg ctg gag gat ttt gac tac tgg ggc cag gga acc ctg gtc      336
   Ala Lys Thr Leu Glu Asp Phe Asp Tyr Trp Gly Gln Gly Thr Leu Val
               100                 105                 110
   acc gtc tcg                                                          345
   Thr Val Ser
           115
<210> 75
   <211> 115
   <212> PRT
   <213> Artificial sequence
<220>
   <223> TAR2h-5
<400> 75
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Asp Leu Tyr
               20                  25                  30
   Asn Met Phe Trp Val Arg Gln Ala Pro Gly Lys Gly Leu Glu Trp Val
           35                  40                  45
   Ser Phe Ile Ser Gln Thr Gly Arg Leu Thr Trp Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Thr Leu Glu Asp Phe Asp Tyr Trp Gly Gln Gly Thr Leu Val
               100                 105                 110
   Thr Val Ser
           115
<210> 76
   <211> 345
   <212> DNA
   <213> Artificial sequence
<220>
   <223> TAR2h-5
<400> 76
   cgagacggtg accagggttc cctggcccca gtagtcaaaa tcctccagcg ttttcgcaca 60
   gtaatatacc gcggtgtcct cggcacgcag gctgttcatt tgcagataca gcgtgttctt 120
   ggaattgtcg cgggagatgg tgaaccggcc cttcacggag tctgcgtacc atgtaagcct 180
   accagtctga ctaataaatg agacccactc tagacccttc cctggagcct ggcggaccca 240
   aaacatatta taaagatcaa aggtgaatcc ggaggctgca caggagagac gcagggaccc 300
   cccaggctgt accaagcctc ccccagactc caacagctgc acctc 345
<210> 77
   <211> 357
   <212> DNA
   <213> Artificial sequence
<220>
   <223> Partner dAb monomer in TAR2h-5d1 (3U linker)
<220>
   <221> CDS
   <222> (1)..(357)
   <223>
<400> 77
   gag gtg cag ctg ttg gag tct ggg gga ggc ttg gta cag cct ggg ggg          48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt ccg gtt tat          96
   Ser Leu Arg Leu Ser Cys Ala Ala Ser Gly Phe Thr Phe Pro Val Tyr
               20                  25                  30
   atg atg ggt tgg gtc cgc cag gct cca ggg aag ggt cta gag tgg gtc         144
   Met Met Gly Trp Val Arg Gln Ala Pro Gly Lys Gly Leu Glu Trp Val
           35                  40                  45
   tca tcg att gat gct ctt ggt ggg cgg aca ggt tac gca gac tcc gtg         192
   Ser Ser Ile Asp Ala Leu Gly Gly Arg Thr Gly Tyr Ala Asp Ser Val
       50                  55                  60
   aag ggc cgg ttc acc atc tcc cgc gac aat tcc aag aac acg ctg tat         240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt         288
   Leu Gln Met Asn Ser Leu Arg Ala Glu Asp Thr Ala Val Tyr Tyr Cys
                   85                  90                  95
   gcg aaa act atg tcg aat aag acg cat acg ttt gac tac tgg ggc cag         336
   Ala Lys Thr Met Ser Asn Lys Thr His Thr Phe Asp Tyr Trp Gly Gln
               100                 105                 110
   gga acc ctg gtc acc gtc tcg                                             357
   Gly Thr Leu Val Thr Val Ser
           115
<210> 78
   <211> 119
   <212> PRT
   <213> Artificial sequence
<220>
   <223> Partner dAb monomer in TAR2h-5d1 (3U linker)
<400> 78
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Pro Val Tyr
   20 25 30
   Met Met Gly Trp Val Arg Gln Ala Pro Gly Lys Gly Leu Glu Trp Val
   35 40 45
   Ser Ser Ile Asp Ala Leu Gly Gly Arg Thr Gly Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Thr Met Ser Asn Lys Thr His Thr Phe Asp Tyr Trp Gly Gln
   100 105 110
   Gly Thr Leu Val Thr Val Ser
   115
<210> 79
   <211> 357
   <212> DNA
   <213> Artificial sequence
<220>
   <223> Partner dAb monomer in TAR2h-5d1 (3U linker)
<400> 79
   cgagacggtg accagggttc cctggcccca gtagtcaaac gtatgcgtct tattcgacat 60
   agttttcgca cagtaatata ccgcggtgtc ctcggcacgc aggctgttca tttgcagata 120
   cagcgtgttc ttggaattgt cgcgggagat ggtgaaccgg cccttcacgg agtctgcgta 180
   acctgtccgc ccaccaagag catcaatcga tgagacccac tctagaccct tccctggagc 240
   ctggcggacc caacccatca tataaaccgg aaaggtgaat ccggaggctg cacaggagag 300
   acgcagggac cccccaggct gtaccaagcc tcccccagac tccaacagct gcacctc 357
<210> 80
   <211> 345
   <212> DNA
   <213> Artificial sequence
<220>
   <223> Partner dAb monomer in TAR2h-5d2 (3U linker)
<220>
   <221> CDS
   <222> (1)..(345)
   <223>
<400> 80
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt gtg gct tat      96
   Ser Leu Arg Leu Ser Cys Ala Ala Ser Gly Phe Thr Phe Val Ala Tyr
               20                  25                  30
   aat atg act tgg gtc cgc cag gct cca ggg aag ggt cta gag tgg gtc      144
   Asn Met Thr Trp Val Arg Gln Ala Pro Gly Lys Gly Leu Glu Trp Val
           35                  40                  45
   tca agt att aat act ttt ggt aat tag aca agg tac gca gac tcc gtg      192
   Ser Ser Ile Asn Thr Phe Gly Asn     Thr Arg Tyr Ala Asp Ser Val
       50                  55                  60
   aag ggc cgg ttc acc atc tcc cgc gac aat tcc aag aac acg ctg tat      240
   Lys Gly Arg Phe Thr Ile Ser Arg Asp Asn Ser Lys Asn Thr Leu Tyr
       65                  70                  75
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
   80                  85                  90                  95
   gcg aaa ggt agt agg cct ttt gac tac tgg ggc cag gga acc ctg gtc      336
   Ala Lys Gly Ser Arg Pro Phe Asp Tyr Trp Gly Gln Gly Thr Leu Val
                   100                 105                 110
   acc gtc tcg                                                          345
   Thr Val Ser
<210> 81
   <211> 56
   <212> PRT
   <213> Artificial sequence
<220>
   <223> Partner dAb monomer in TAR2h-5d2 (3U linker)
<400> 81
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Val Ala Tyr
               20                  25                  30
   Asn Met Thr Trp Val Arg Gln Ala Pro Gly Lys Gly Leu Glu Trp Val
           35                  40                  45
   Ser Ser Ile Asn Thr Phe Gly Asn
       50                  55
<210> 82
   <211> 58
   <212> PRT
   <213> Artificial sequence
<220>
   <223> Partner dAb monomer in TAR2h-5d2 (3U linker)
<400> 82
   Thr Arg Tyr Ala Asp Ser Val Lys Gly Arg Phe Thr Ile Ser Arg Asp
   1               5                   10                  15
   Asn Ser Lys Asn Thr Leu Tyr Leu Gln Met Asn Ser Leu Arg Ala Glu
               20                  25                  30
   Asp Thr Ala Val Tyr Tyr Cys Ala Lys Gly Ser Arg Pro Phe Asp Tyr
           35                  40                  45
   Trp Gly Gln Gly Thr Leu Val Thr Val Ser
       50                  55
<210> 83
   <211> 345
   <212> DNA
   <213> Artificial sequence
<220>
   <223> Partner dAb monomer in TAR2h-5d2 (3U linker)
<400> 83
   cgagacggtg accagggttc cctggcccca gtagtcaaaa ggcctactac ctttcgcaca      60
   gtaatatacc gcggtgtcct cggcacgcag gctgttcatt tgcagataca gcgtgttctt     120
   ggaattgtcg cgggagatgg tgaaccggcc cttcacggag tctgcgtacc ttgtctaatt     180
   accaaaagta ttaatacttg agacccactc tagacccttc cctggagcct ggcggaccca     240
   agtcatatta taagccacaa aggtgaatcc ggaggctgca caggagagac gcagggaccc     300
   cccaggctgt accaagcctc ccccagactc caacagctgc acctc                     345
<210> 84
   <211> 357
   <212> DNA
   <213> Artificial sequence
<220>
   <223> Partner dAb monomer in TAR2h-5d3 (3U linker)
<220>
   <221> CDS
   <222> (1)..(357)
   <223>
<400> 84
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt tag ggg tat      96
   Ser Leu Arg Leu Ser Cys Ala Ala Ser Gly Phe Thr Phe     Gly Tyr
               20                  25                      30
   cgt atg ggt tgg gtc cgc cag gct cca ggg aag ggt cta gag tgg gtc      144
   Arg Met Gly Trp Val Arg Gln Ala Pro Gly Lys Gly Leu Glu Trp Val
               35                  40                  45
   tca tgg att acg cgt act ggt ggg acg aca cag tac gca gac tcc gtg      192
   Ser Trp Ile Thr Arg Thr Gly Gly Thr Thr Gln Tyr Ala Asp Ser Val
           50                  55                  60
   aag ggc cgg ttc acc atc tcc cgc gac aat tcc aag aac acg ctg tat      240
   Lys Gly Arg Phe Thr Ile Ser Arg Asp Asn Ser Lys Asn Thr Leu Tyr
       65                  70                  75
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
   80                  85                  90                  95
   gcg aaa ccg gcg aag ctt gtt ggg gtt ggg ttt gac tac tgg ggc cag      336
   Ala Lys Pro Ala Lys Leu Val Gly Val Gly Phe Asp Tyr Trp Gly Gln
                   100                 105                 110
   gga acc ctg gtc acc gtc tcg                                          357
   Gly Thr Leu Val Thr Val Ser
               115
<210> 85
   <211> 29
   <212> PRT
   <213> Artificial sequence
<220>
   <223> Partner dAb monomer in TAR2h-5d3 (3U linker)
<400> 85
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe
               20                  25
<210> 86
   <211> 89
   <212> PRT
   <213> Artificial sequence
<220>
   <223> Partner dAb monomer in TAR2h-5d3 (3U linker)
<400> 86
   Gly Tyr Arg Met Gly Trp Val Arg Gln Ala Pro Gly Lys Gly Leu Glu
   1 5 10 15
   Trp Val Ser Trp Ile Thr Arg Thr Gly Gly Thr Thr Gln Tyr Ala Asp
   20 25 30
   Ser Val Lys Gly Arg Phe Thr Ile Ser Arg Asp Asn Ser Lys Asn Thr
   35 40 45
   Leu Tyr Leu Gln Met Asn Ser Leu Arg Ala Glu Asp Thr Ala Val Tyr
   50 55 60
   Tyr Cys Ala Lys Pro Ala Lys Leu Val Gly Val Gly Phe Asp Tyr Trp
   65 70 75 80
   Gly Gln Gly Thr Leu Val Thr Val Ser
   85
<210> 87
   <211> 357
   <212> DNA
   <213> Artificial sequence
<220>
   <223> Partner dAb monomer in TAR2h-5d3 (3U linker)
<400> 87
   cgagacggtg accagggttc cctggcccca gtagtcaaac ccaaccccaa caagcttcgc     60
   cggtttcgca cagtaatata ccgcggtgtc ctcggcacgc aggctgttca tttgcagata    120
   cagcgtgttc ttggaattgt cgcgggagat ggtgaaccgg cccttcacgg agtctgcgta    180
   ctgtgtcgtc ccaccagtac gcgtaatcca tgagacccac tctagaccct tccctggagc    240
   ctggcggacc caacccatac gataccccta aaaggtgaat ccggaggctg cacaggagag    300
   acgcagggac cccccaggct gtaccaagcc tcccccagac tccaacagct gcacctc        357
<210> 88
   <211> 357
   <212> DNA
   <213> Artificial sequence
<220>
   <223> Partner dAb monomer in TAR2h-5d4 (3U linker)
<220>
   <221> CDS
   <222> (1)..(357)
   <223>
<400> 88
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt cgg aag tat      96
   Ser Leu Arg Leu Ser Cys Ala Ala Ser Gly Phe Thr Phe Arg Lys Tyr
               20                  25                  30
   tag atg ggg tgg gtc cgc cag gct cca ggg aag ggt cta gag tgg gtc     144
       Met Gly Trp Val Arg Gln Ala Pro Gly Lys Gly Leu Glu Trp Val
               35                  40                  45
   tca cag att ggt gcg aag ggt cag tct aca gat tac gca gac tcc gtg     192
   Ser Gln Ile Gly Ala Lys Gly Gln Ser Thr Asp Tyr Ala Asp Ser Val
           50                  55                  60
   aag ggc cgg ttc acc atc tcc cgc gac aat tcc aag aac acg ctg tat     240
   Lys Gly Arg Phe Thr Ile Ser Arg Asp Asn Ser Lys Asn Thr Leu Tyr
       65                  70                  75
   ctg caa atg aac agc ctg cgt gcc gag gac acc gcg gta tat tac tgt     288
   Leu Gln Met Asn Ser Leu Arg Ala Glu Asp Thr Ala Val Tyr Tyr Cys
   80                  85                  90                  95
   gcg aaa aag aag agg ggg gag aat tat ttt ttt gac tac tgg ggc cag     336
   Ala Lys Lys Lys Arg Gly Glu Asn Tyr Phe Phe Asp Tyr Trp Gly Gln
                   100                 105                 110
   gga acc ctg gtc acc gtc tcg                                          357
   Gly Thr Leu Val Thr Val Ser
               115
<210> 89
   <211> 32
   <212> PRT
   <213> Artificial sequence
<220>
   <223> Partner dAb monomer in TAR2h-5d4 (3U linker)
<400> 89
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Arg Lys Tyr
               20                  25                  30
<210> 90
   <211> 86
   <212> PRT
   <213> Artificial sequence
<220>
   <223> Partner dAb monomer in TAR2h-5d4 (3U linker)
<400> 90
   Met Gly Trp Val Arg Gln Ala Pro Gly Lys Gly Leu Glu Trp Val Ser
   1               5                   10                  15
   Gln Ile Gly Ala Lys Gly Gln Ser Thr Asp Tyr Ala Asp Ser Val Lys
               20                  25                  30
   Gly Arg Phe Thr Ile Ser Arg Asp Asn Ser Lys Asn Thr Leu Tyr Leu
           35                  40                  45
   Gln Met Asn Ser Leu Arg Ala Glu Asp Thr Ala Val Tyr Tyr Cys Ala
       50                  55                  60
   Lys Lys Lys Arg Gly Glu Asn Tyr Phe Phe Asp Tyr Trp Gly Gln Gly
   65                  70                  75                  80
   Thr Leu Val Thr Val Ser
                   85
<210> 91
   <211> 357
   <212> DNA
   <213> Artificial sequence
<220>
   <223> Partner dAb monomer in TAR2h-5d4 (3U linker)
<400> 91
   cgagacggtg accagggttc cctggcccca gtagtcaaaa aaataattct cccccctctt     60
   ctttttcgca cagtaatata ccgcggtgtc ctcggcacgc aggctgttca tttgcagata    120
   cagcgtgttc ttggaattgt cgcgggagat ggtgaaccgg cccttcacgg agtctgcgta 180
   atctgtagac tgacccttcg caccaatctg tgagacccac tctagaccct tccctggagc 240
   ctggcggacc caccccatct aatacttccg aaaggtgaat ccggaggctg cacaggagag 300
   acgcagggac cccccaggct gtaccaagcc tcccccagac tccaacagct gcacctc 357
<210> 92
   <211> 357
   <212> DNA
   <213> Artificial sequence
<220>
   <223> Partner dAb monomer in TAR2h-5d5 (3U linker)
<220>
   <221> CDS
   <222> (1)..(357)
   <223>
<400> 92
   gag gtg cag ctg ttg gag tct ggg gga ggc ttg gta cag cct ggg ggg       48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt cgg cgg tat       96
   Ser Leu Arg Leu Ser Cys Ala Ala Ser Gly Phe Thr Phe Arg Arg Tyr
               20                  25                  30
   agt atg tcg tgg gtc cgc cag gct cca ggg aag ggt cta gag tgg gtc       144
   Ser Met Ser Trp Val Arg Gln Ala Pro Gly Lys Gly Leu Glu Trp Val
           35                  40                  45
   tca gat att tct cgt tct ggt cgg tat aca cat tac gca gac tcc gtg       192
   Ser Asp Ile Ser Arg Ser Gly Arg Tyr Thr His Tyr Ala Asp Ser Val
       50                  55                  60
   aag ggc cgg ttc acc atc tcc cgc gac aat tcc aag aac acg ctg tat       240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
                   85                  90                  95
   gcg aaa cgt att gat tct tct cag aat ggg ttt gac tac tgg ggc cag      336
   Ala Lys Arg Ile Asp Ser Ser Gln Asn Gly Phe Asp Tyr Trp Gly Gln
               100                 105                 110
   gga acc ctg gtc acc gtc tcg                                          357
   Gly Thr Leu Val Thr Val Ser
           115
<210> 93
   <211> 119
   <212> PRT
   <213> Artificial sequence
<220>
   <223> Partner dAb monomer in TAR2h-5d5 (3U linker)
<400> 93
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Arg Arg Tyr
               20                  25                  30
   Ser Met Ser Trp Val Arg Gln Ala Pro Gly Lys Gly Leu Glu Trp Val
           35                  40                  45
   Ser Asp Ile Ser Arg Ser Gly Arg Tyr Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Arg Ile Asp Ser Ser Gln Asn Gly Phe Asp Tyr Trp Gly Gln
   100 105 110
   Gly Thr Leu Val Thr Val Ser
   115
<210> 94
   <211> 357
   <212> DNA
   <213> Artificial sequence
<220>
   <223> Partner dAb monomer in TAR2h-5d5 (3U linker)
<400> 94
   cgagacggtg accagggttc cctggcccca gtagtcaaac ccattctgag aagaatcaat 60
   acgtttcgca cagtaatata ccgcggtgtc ctcggcacgc aggctgttca tttgcagata 120
   cagcgtgttc ttggaattgt cgcgggagat ggtgaaccgg cccttcacgg agtctgcgta 180
   atgtgtatac cgaccagaac gagaaatatc tgagacccac tctagaccct tccctggagc 240
   ctggcggacc cacgacatac tataccgccg aaaggtgaat ccggaggctg cacaggagag 300
   acgcagggac cccccaggct gtaccaagcc tcccccagac tccaacagct gcacctc 357
<210> 95
   <211> 345
   <212> DNA
   <213> Artificial sequence
<220>
   <223> Partner dAb monomer in TAR2h-5d6 (3U linker)
<220>
   <221> CDS
   <222> (1)..(345)
   <223>
<400> 95
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt tag ggg tat      96
   Ser Leu Arg Leu Ser Cys Ala Ala Ser Gly Phe Thr Phe     Gly Tyr
               20                  25                      30
   aag atg ttt tgg gtc cgc cag gct cca ggg aag ggt cta gag tgg gtc      144
   Lys Met Phe Trp Val Arg Gln Ala Pro Gly Lys Gly Leu Glu Trp Val
               35                  40                  45
   tca gct att agt ggt agt ggt ggt agc aca tac tac gca gac tcc gtg      192
   Ser Ala Ile Ser Gly Ser Gly Gly Ser Thr Tyr Tyr Ala Asp Ser Val
           50                  55                  60
   aag ggc cgg ttc acc atc tcc cgc gac aat tcc aag aac acg ctg tat      240
   Lys Gly Arg Phe Thr Ile Ser Arg Asp Asn Ser Lys Asn Thr Leu Tyr
       65                  70                  75
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
   80                  85                  90                  95
   gcg aaa cag aag gag aat ttt gac tac tgg ggc cag gga acc ctg gtc      336
   Ala Lys Gln Lys Glu Asn Phe Asp Tyr Trp Gly Gln Gly Thr Leu Val
               100                 105                 110
   acc gtc tcg                                                          345
   Thr Val Ser
<210> 96
   <211> 29
   <212> PRT
   <213> Artificial sequence
<220>
   <223> Partner dAb monomer in TAR2h-5d6 (3U linker)
<400> 96
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe
               20                  25
<210> 97
   <211> 85
   <212> PRT
   <213> Artificial sequence
<220>
   <223> Partner dAb monomer in TAR2h-5d6 (3U linker)
<400> 97
   Gly Tyr Lys Met Phe Trp Val Arg Gln Ala Pro Gly Lys Gly Leu Glu
   1               5                   10                  15
   Trp Val Ser Ala Ile Ser Gly Ser Gly Gly Ser Thr Tyr Tyr Ala Asp
               20                  25                  30
   Ser Val Lys Gly Arg Phe Thr Ile Ser Arg Asp Asn Ser Lys Asn Thr
           35                  40                  45
   Leu Tyr Leu Gln Met Asn Ser Leu Arg Ala Glu Asp Thr Ala Val Tyr
       50                  55                  60
   Tyr Cys Ala Lys Gln Lys Glu Asn Phe Asp Tyr Trp Gly Gln Gly Thr
   65                  70                  75                  80
   Leu Val Thr Val Ser
                   85
<210> 98
   <211> 345
   <212> DNA
   <213> Artificial sequence
<220>
   <223> Partner dAb monomer in TAR2h-5d6 (3U linker)
<400> 98
   cgagacggtg accagggttc cctggcccca gtagtcaaaa ttctccttct gtttcgcaca      60
   gtaatatacc gcggtgtcct cggcacgcag gctgttcatt tgcagataca gcgtgttctt     120
   ggaattgtcg cgggagatgg tgaaccggcc cttcacggag tctgcgtagt atgtgctacc     180
   accactacca ctaatagctg agacccactc tagacccttc cctggagcct ggcggaccca     240
   aaacatctta tacccctaaa aggtgaatcc ggaggctgca caggagagac gcagggaccc     300
   cccaggctgt accaagcctc ccccagactc caacagctgc acctc                     345
<210> 99
   <211> 357
   <212> DNA
   <213> Artificial sequence
<220>
   <223> Partner dAb monomer in TAR2h-5d7 (3U linker)
<220>
   <221> CDS
   <222> (1)..(357)
   <223>
<400> 99
   gag gtg cag ctg ttg gag tct ggg gga ggc ttg gta cag cct ggg ggg       48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt ggg gat tat       96
   Ser Leu Arg Leu Ser Cys Ala Ala Ser Gly Phe Thr Phe Gly Asp Tyr
               20                  25                  30
   gct atg tgg tgg gtc cgc cag gct cca ggg aag ggt cta gag tgg gtc      144
   Ala Met Trp Trp Val Arg Gln Ala Pro Gly Lys Gly Leu Glu Trp Val
           35                  40                  45
   tca gtg att agt tcg aat ggt ggg agt aca ttt tac gca gac tcc gtg      192
   Ser Val Ile Ser Ser Asn Gly Gly Ser Thr Phe Tyr Ala Asp Ser Val
       50                  55                  60
   aag ggc cgg ttc acc atc tcc cgc gac aat tcc aag aac acg ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
                   85                  90                  95
   gcg aaa cgt gtt cgt aag agg act cct gag ttt gac tac tgg ggc cag      336
   Ala Lys Arg Val Arg Lys Arg Thr Pro Glu Phe Asp Tyr Trp Gly Gln
               100                 105                 110
   gga acc ctg gtc acc gtc tcg                                          357
   Gly Thr Leu Val Thr Val Ser
           115
<210> 100
   <211> 119
   <212> PRT
   <213> Artificial sequence
<220>
   <223> Partner dAb monomer in TAR2h-5d7 (3U linker)
<400> 100
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Gly Asp Tyr
               20                  25                  30
   Ala Met Trp Trp Val Arg Gln Ala Pro Gly Lys Gly Leu Glu Trp Val
           35                  40                  45
   Ser Val Ile Ser Ser Asn Gly Gly Ser Thr Phe Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
   85 90 95
   Ala Lys Arg Val Arg Lys Arg Thr Pro Glu Phe Asp Tyr Trp Gly Gln
   100 105 110
   Gly Thr Leu Val Thr Val Ser
   115
<210> 101
   <211> 357
   <212> DNA
   <213> Artificial sequence
<220>
   <223> Partner dAb monomer in TAR2h-5d7 (3U linker)
<400> 101
   cgagacggtg accagggttc cctggcccca gtagtcaaac tcaggagtcc tcttacgaac 60
   acgtttcgca cagtaatata ccgcggtgtc ctcggcacgc aggctgttca tttgcagata 120
   cagcgtgttc ttggaattgt cgcgggagat ggtgaaccgg cccttcacgg agtctgcgta 180
   aaatgtactc ccaccattcg aactaatcac tgagacccac tctagaccct tccctggagc 240
   ctggcggacc caccacatag cataatcccc aaaggtgaat ccggaggctg cacaggagag 300
   acgcagggac cccccaggct gtaccaagcc tcccccagac tccaacagct gcacctc 357
<210> 102
   <211> 357
   <212> DNA
   <213> Artificial sequence
<220>
   <223> Partner dAb monomer in TAR2h-5d8 (3U linker)
<220>
   <221> CDS
   <222> (1)..(357)
   <223>
<400> 102
   gag gtg cag ctg ttg gag tct ggg gga ggc ttg gta cag cct ggg ggg        48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt agg agg tat        96
   Ser Leu Arg Leu Ser Cys Ala Ala Ser Gly Phe Thr Phe Arg Arg Tyr
               20                  25                  30
   aag atg ggt tgg gtc cgc cag gct cca ggg aag ggt cta gag tgg gtc       144
   Lys Met Gly Trp Val Arg Gln Ala Pro Gly Lys Gly Leu Glu Trp Val
           35                  40                  45
   tca gcg att ggg agg aat ggt acg aag aca aat tac gca gac tcc gtg       192
   Ser Ala Ile Gly Arg Asn Gly Thr Lys Thr Asn Tyr Ala Asp Ser Val
       50                  55                  60
   aag ggc cgg ttc acc atc tcc cgc gac aat tcc aag aac acg ctg tat       240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt       288
   Leu Gln Met Asn Ser Leu Arg Ala Glu Asp Thr Ala Val Tyr Tyr Cys
                   85                  90                  95
   gcg aaa att tat acg ggg aag cct gct gcg ttt gac tac tgg ggc cag       336
   Ala Lys Ile Tyr Thr Gly Lys Pro Ala Ala Phe Asp Tyr Trp Gly Gln
               100                 105                 110
   gga acc ctg gtc acc gtc tcg                                           357
   Gly Thr Leu Val Thr Val Ser
           115
<210> 103
   <211> 119
   <212> PRT
   <213> Artificial sequence
<220>
   <223> Partner dAb monomer in TAR2h-5d8 (3U linker)
<400> 103
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Arg Arg Tyr
   20 25 30
   Lys Met Gly Trp Val Arg Gln Ala Pro Gly Lys Gly Leu Glu Trp Val
   35 40 45
   Ser Ala Ile Gly Arg Asn Gly Thr Lys Thr Asn Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ile Tyr Thr Gly Lys Pro Ala Ala Phe Asp Tyr Trp Gly Gln
   100 105 110
   Gly Thr Leu Val Thr Val Ser
   115
<210> 104
   <211> 357
   <212> DNA
   <213> Artificial sequence
<220>
   <223> Partner dAb monomer in TAR2h-5d8 (3U linker)
<400> 104
   cgagacggtg accagggttc cctggcccca gtagtcaaac gcagcaggct tccccgtata 60
   aattttcgca cagtaatata ccgcggtgtc ctcggcacgc aggctgttca tttgcagata 120
   cagcgtgttc ttggaattgt cgcgggagat ggtgaaccgg cccttcacgg agtctgcgta 180
   atttgtcttc gtaccattcc tcccaatcgc tgagacccac tctagaccct tccctggagc 240
   ctggcggacc caacccatct tatacctcct aaaggtgaat ccggaggctg cacaggagag 300
   acgcagggac cccccaggct gtaccaagcc tcccccagac tccaacagct gcacctc 357
<210> 105
   <211> 357
   <212> DNA
   <213> Artificial sequence
<220>
   <223> Partner dAb monomer in TAR2h-5d9 (3U linker)
<220>
   <221> CDS
   <222> (1)..(357)
   <223>
<400> 105
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt aag aag tat      96
   Ser Leu Arg Leu Ser Cys Ala Ala Ser Gly Phe Thr Phe Lys Lys Tyr
               20                  25                  30
   tag atg tct tgg gtc cgc cag gct cca ggg aag ggt cta gag tgg gtc     144
       Met Ser Trp Val Arg Gln Ala Pro Gly Lys Gly Leu Glu Trp Val
               35                  40                  45
   tca gct att agt ggt agt ggt ggt agc aca tac tac gca gac tcc gtg     192
   Ser Ala Ile Ser Gly Ser Gly Gly Ser Thr Tyr Tyr Ala Asp Ser Val
           50                  55                  60
   aag ggc cgg ttc acc atc tcc cgc gac aat tcc aag aac acg ctg tat     240
   Lys Gly Arg Phe Thr Ile Ser Arg Asp Asn Ser Lys Asn Thr Leu Tyr
       65                  70                  75
   ctg caa atg aac agc ctg cgt gcc gag gac acc gcg gta tat tac tgt     288
   Leu Gln Met Asn Ser Leu Arg Ala Glu Asp Thr Ala Val Tyr Tyr Cys
   80                  85                  90                  95
   gcg aaa atg ctg agg act aag aat aag gtg ttt gac tac tgg ggc cag      336
   Ala Lys Met Leu Arg Thr Lys Asn Lys Val Phe Asp Tyr Trp Gly Gln
                   100                 105                 110
   gga acc ctg gtc acc gtc tcg                                          357
   Gly Thr Leu Val Thr Val Ser
               115
<210> 106
   <211> 32
   <212> PRT
   <213> Artificial sequence
<220>
   <223> Partner dAb monomer in TAR2h-5d9 (3U linker)
<400> 106
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Lys Lys Tyr
               20                  25                  30
<210> 107
   <211> 86
   <212> PRT
   <213> Artificial sequence
<220>
   <223> Partner dAb monomer in TAR2h-5d9 (3U linker)
<400> 107
   Met Ser Trp Val Arg Gln Ala Pro Gly Lys Gly Leu Glu Trp Val Ser
   1               5                   10                  15
   Ala Ile Ser Gly Ser Gly Gly Ser Thr Tyr Tyr Ala Asp Ser Val Lys
               20                  25                  30
   Gly Arg Phe Thr Ile Ser Arg Asp Asn Ser Lys Asn Thr Leu Tyr Leu
           35                  40                  45
   Gln Met Asn Ser Leu Arg Ala Glu Asp Thr Ala Val Tyr Tyr Cys Ala
       50                  55                  60
   Lys Met Leu Arg Thr Lys Asn Lys Val Phe Asp Tyr Trp Gly Gln Gly
   65                  70                  75                  80
   Thr Leu Val Thr Val Ser
                   85
<210> 108
   <211> 357
   <212> DNA
   <213> Artificial sequence
<220>
   <223> Partner dAb monomer in TAR2h-5d9 (3U linker)
<400> 108
   cgagacggtg accagggttc cctggcccca gtagtcaaac accttattct tagtcctcag      60
   cattttcgca cagtaatata ccgcggtgtc ctcggcacgc aggctgttca tttgcagata     120
   cagcgtgttc ttggaattgt cgcgggagat ggtgaaccgg cccttcacgg agtctgcgta     180
   gtatgtgcta ccaccactac cactaatagc tgagacccac tctagaccct tccctggagc     240
   ctggcggacc caagacatct aatacttctt aaaggtgaat ccggaggctg cacaggagag     300
   acgcagggac cccccaggct gtaccaagcc tcccccagac tccaacagct gcacctc        357
<210> 109
   <211> 357
   <212> DNA
   <213> Artificial sequence
<220>
   <223> Partner dAb monomer in TAR2h-5d10 (5U linker)
<220>
   <221> CDS
   <222> (1)..(357)
   <223>
<400> 109
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt agg agg tat      96
   Ser Leu Arg Leu Ser Cys Ala Ala Ser Gly Phe Thr Phe Arg Arg Tyr
               20                  25                  30
   aag atg ggt tgg gtc cgc cag gct cca ggg aag ggt cta gag tgg gtc      144
   Lys Met Gly Trp Val Arg Gln Ala Pro Gly Lys Gly Leu Glu Trp Val
           35                  40                  45
   tca gcg att ggg agg aat ggt acg aag aca aat tac gca gac tcc gtg      192
   Ser Ala Ile Gly Arg Asn Gly Thr Lys Thr Asn Tyr Ala Asp Ser Val
       50                  55                  60
   aag ggc cgg ttc acc atc tcc cgc gac aat tcc aag aac acg ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
                   85                  90                  95
   gcg aaa att tat acg ggg aag cct gct gcg ttt gac tac tgg ggc cag      336
   Ala Lys Ile Tyr Thr Gly Lys Pro Ala Ala Phe Asp Tyr Trp Gly Gln
               100                 105                 110
   gga acc ctg gtc acc gtc tcg                                          357
   Gly Thr Leu Val Thr Val Ser
           115
<210> 110
   <211> 119
   <212> PRT
   <213> Artificial sequence
<220>
   <223> Partner dAb monomer in TAR2h-5d10 (5U linker)
<400> 110
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Arg Arg Tyr
               20                  25                  30
   Lys Met Gly Trp Val Arg Gln Ala Pro Gly Lys Gly Leu Glu Trp Val
           35                  40                  45
   Ser Ala Ile Gly Arg Asn Gly Thr Lys Thr Asn Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ile Tyr Thr Gly Lys Pro Ala Ala Phe Asp Tyr Trp Gly Gln
               100                 105                 110
   Gly Thr Leu Val Thr Val Ser
           115
<210> 111
   <211> 357
   <212> DNA
   <213> Artificial sequence
<220>
   <223> Partner dAb monomer in TAR2h-5d10 (5U linker)
<400> 111
   cgagacggtg accagggttc cctggcccca gtagtcaaac gcagcaggct tccccgtata      60
   aattttcgca cagtaatata ccgcggtgtc ctcggcacgc aggctgttca tttgcagata     120
   cagcgtgttc ttggaattgt cgcgggagat ggtgaaccgg cccttcacgg agtctgcgta     180
   atttgtcttc gtaccattcc tcccaatcgc tgagacccac tctagaccct tccctggagc     240
   ctggcggacc caacccatct tatacctcct aaaggtgaat ccggaggctg cacaggagag     300
   acgcagggac cccccaggct gtaccaagcc tcccccagac tccaacagct gcacctc        357
<210> 112
   <211> 357
   <212> DNA
   <213> Artificial sequence
<220>
   <223> Partner dAb monomer in TAR2h-5d11 (5U linker)
<220>
   <221> CDS
   <222> (1)..(357)
   <223>
<400> 112
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt tag agt tat      96
   Ser Leu Arg Leu Ser Cys Ala Ala Ser Gly Phe Thr Phe     Ser Tyr
               20                  25                      30
   cgg atg ggt tgg gtc cgc cag gct cca ggg aag ggt cta gag tgg gtc      144
   Arg Met Gly Trp Val Arg Gln Ala Pro Gly Lys Gly Leu Glu Trp Val
               35                  40                  45
   tca agt att tcg tcg agg ggt agg cat aca tct tac gca gac tcc gtg      192
   Ser Ser Ile Ser Ser Arg Gly Arg His Thr Ser Tyr Ala Asp Ser Val
           50                  55                  60
   aag ggc cgg ttc acc atc tcc cgc gac aat tcc aag aac acg ctg tat      240
   Lys Gly Arg Phe Thr Ile Ser Arg Asp Asn Ser Lys Asn Thr Leu Tyr
       65                  70                  75
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
   80                  85                  90                  95
   gcg aaa agg gtt ccg ggt cgg ggg cgt tct ttt gac tac tgg ggc cag      336
   Ala Lys Arg Val Pro Gly Arg Gly Arg Ser Phe Asp Tyr Trp Gly Gln
                   100                 105                 110
   gga acc ctg gtc acc gtc tcg                                          357
   Gly Thr Leu Val Thr Val Ser
               115
<210> 113
   <211> 29
   <212> PRT
   <213> Artificial sequence
<220>
   <223> Partner dAb monomer in TAR2h-5d11 (5U linker)
<400> 113
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe
               20                  25
<210> 114
   <211> 89
   <212> PRT
   <213> Artificial sequence
<220>
   <223> Partner dAb monomer in TAR2h-5d11 (5U linker)
<400> 114
   Ser Tyr Arg Met Gly Trp Val Arg Gln Ala Pro Gly Lys Gly Leu Glu
   1 5 10 15
   Trp Val Ser Ser Ile Ser Ser Arg Gly Arg His Thr Ser Tyr Ala Asp
   20 25 30
   Ser Val Lys Gly Arg Phe Thr Ile Ser Arg Asp Asn Ser Lys Asn Thr
   35 40 45
   Leu Tyr Leu Gln Met Asn Ser Leu Arg Ala Glu Asp Thr Ala Val Tyr
   50 55 60
   Tyr Cys Ala Lys Arg Val Pro Gly Arg Gly Arg Ser Phe Asp Tyr Trp
   65 70 75 80
   Gly Gln Gly Thr Leu Val Thr Val Ser
   85
<210> 115
   <211> 357
   <212> DNA
   <213> Artificial sequence
<220>
   <223> Partner dAb monomer in TAR2h-5d11 (5U linker)
<400> 115
   cgagacggtg accagggttc cctggcccca gtagtcaaaa gaacgccccc gacccggaac 60
   ccttttcgca cagtaatata ccgcggtgtc ctcggcacgc aggctgttca tttgcagata 120
   cagcgtgttc ttggaattgt cgcgggagat ggtgaaccgg cccttcacgg agtctgcgta 180
   agatgtatgc ctacccctcg acgaaatact tgagacccac tctagaccct tccctggagc 240
   ctggcggacc caacccatcc gataactcta aaaggtgaat ccggaggctg cacaggagag 300
   acgcagggac cccccaggct gtaccaagcc tcccccagac tccaacagct gcacctc 357
<210> 116
   <211> 357
   <212> DNA
   <213> Artificial sequence
<220>
   <223> Partner dAb monomer in TAR2h-5d12 (5U linker)
<220>
   <221> CDS
   <222> (1)..(357)
   <223>
<400> 116
   gag gtg cag ctg ttg gag tct ggg gga ggc ttg gta cag cct ggg ggg       48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc ccc ttt cgt cgg tat       96
   Ser Leu Arg Leu Ser Cys Ala Ala Ser Gly Phe Pro Phe Arg Arg Tyr
               20                  25                  30
   cgg atg agg tgg gtc cgc cag gct cca ggg aag ggt cta gag tgg gtc      144
   Arg Met Arg Trp Val Arg Gln Ala Pro Gly Lys Gly Leu Glu Trp Val
           35                  40                  45
   tca ggt att tct ccg ggt ggt aag cat aca acg tac gca gac tcc gtg      192
   Ser Gly Ile Ser Pro Gly Gly Lys His Thr Thr Tyr Ala Asp Ser Val
       50                  55                  60
   aag ggc cgg ttc acc atc tcc cgc gac aat tcc aag aac acg ctg tat      240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt      288
   Leu Gln Met Asn Ser Leu Arg Ala Glu Asp Thr Ala Val Tyr Tyr Cys
                   85                  90                  95
   gcg aaa ggt gag ggg ggg gcg agt tct gcg ttt gac tac tgg ggc cag      336
   Ala Lys Gly Glu Gly Gly Ala Ser Ser Ala Phe Asp Tyr Trp Gly Gln
               100                 105                 110
   gga acc ctg gtc acc gtc tcg                                          357
   Gly Thr Leu Val Thr Val Ser
           115
<210> 117
   <211> 119
   <212> PRT
   <213> Artificial sequence
<220>
   <223> Partner dAb monomer in TAR2h-5d12 (5U linker)
<400> 117
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Pro Phe Arg Arg Tyr
               20                  25                  30
   Arg Met Arg Trp Val Arg Gln Ala Pro Gly Lys Gly Leu Glu Trp Val
           35                  40                  45
   Ser Gly Ile Ser Pro Gly Gly Lys His Thr Thr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Glu Gly Gly Ala Ser Ser Ala Phe Asp Tyr Trp Gly Gln
               100                 105                 110
   Gly Thr Leu Val Thr Val Ser
           115
<210> 118
   <211> 357
   <212> DNA
   <213> Artificial sequence
<220>
   <223> Partner dAb monomer in TAR2h-5d12 (5U linker)
<400> 118
   cgagacggtg accagggttc cctggcccca gtagtcaaac gcagaactcg cccccccctc 60
   acctttcgca cagtaatata ccgcggtgtc ctcggcacgc aggctgttca tttgcagata 120
   cagcgtgttc ttggaattgt cgcgggagat ggtgaaccgg cccttcacgg agtctgcgta 180
   cgttgtatgc ttaccacccg gagaaatacc tgagacccac tctagaccct tccctggagc 240
   ctggcggacc cacctcatcc gataccgacg aaaggggaat ccggaggctg cacaggagag 300
   acgcagggac cccccaggct gtaccaagcc tcccccagac tccaacagct gcacctc 357
<210> 119
   <211> 357
   <212> DNA
   <213> Artificial sequence
<220>
   <223> Partner dAb monomer in TAR2h-5d13 (5U linker)
<220>
   <221> CDS
   <222> (1)..(357)
   <223>
<400> 119
   gag gtg cag ctg ttg gag tct ggg gga ggc ttg gta cag cct ggg ggg        48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt tag cgg tat        96
   Ser Leu Arg Leu Ser Cys Ala Ala Ser Gly Phe Thr Phe     Arg Tyr
               20                  25                      30
   ggg atg gtt tgg gtc cgc cag gct cca ggg aag ggt cta gag tgg gtc       144
   Gly Met Val Trp Val Arg Gln Ala Pro Gly Lys Gly Leu Glu Trp Val
               35                  40                  45
   tca gct att agt ggt agt ggt ggt agc aca tac tac gca gac tcc gtg       192
   Ser Ala Ile Ser Gly Ser Gly Gly Ser Thr Tyr Tyr Ala Asp Ser Val
           50                  55                  60
   aag ggc cgg ttc acc atc tcc cgc gac aat tcc aag aac acg ctg tat       240
   Lys Gly Arg Phe Thr Ile Ser Arg Asp Asn Ser Lys Asn Thr Leu Tyr
       65                  70                  75
   ctg caa atg aac agc ctg cgt gcc gag gac acc gcg gta tat tac tgt       288
   Leu Gln Met Asn Ser Leu Arg Ala Glu Asp Thr Ala Val Tyr Tyr Cys
   80                  85                  90                  95
   gcg aaa cgg cat agt tct gag gct agg cag ttt gac tac tgg ggc cag       336
   Ala Lys Arg His Ser Ser Glu Ala Arg Gln Phe Asp Tyr Trp Gly Gln
                   100                 105                 110
   gga acc ctg gtc acc gtc tcg                                           357
   Gly Thr Leu Val Thr Val Ser
               115
<210> 120
   <211> 29
   <212> PRT
   <213> Artificial sequence
<220>
   <223> Partner dAb monomer in TAR2h-5d13 (5U linker)
<400> 120
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe
               20                  25
<210> 121
   <211> 89
   <212> PRT
   <213> Artificial sequence
<220>
   <223> Partner dAb monomer in TAR2h-5d13 (5U linker)
<400> 121
   Arg Tyr Gly Met Val Trp Val Arg Gln Ala Pro Gly Lys Gly Leu Glu
   1               5                   10                  15
   Trp Val Ser Ala Ile Ser Gly Ser Gly Gly Ser Thr Tyr Tyr Ala Asp
               20                  25                  30
   Ser Val Lys Gly Arg Phe Thr Ile Ser Arg Asp Asn Ser Lys Asn Thr
           35                  40                  45
   Leu Tyr Leu Gln Met Asn Ser Leu Arg Ala Glu Asp Thr Ala Val Tyr
       50                  55                  60
   Tyr Cys Ala Lys Arg His Ser Ser Glu Ala Arg Gln Phe Asp Tyr Trp
   65                  70                  75                  80
   Gly Gln Gly Thr Leu Val Thr Val Ser
                   85
<210> 122
   <211> 357
   <212> DNA
   <213> Artificial sequence
<220>
   <223> Partner dAb monomer in TAR2h-5d13 (5U linker)
<400> 122
   cgagacggtg accagggttc cctggcccca gtagtcaaac tgcctagcct cagaactatg      60
   ccgtttcgca cagtaatata ccgcggtgtc ctcggcacgc aggctgttca tttgcagata     120
   cagcgtgttc ttggaattgt cgcgggagat ggtgaaccgg cccttcacgg agtctgcgta     180
   gtatgtgcta ccaccactac cactaatagc tgagacccac tctagaccct tccctggagc     240
   ctggcggacc caaaccatcc cataccgcta aaaggtgaat ccggaggctg cacaggagag     300
   acgcagggac cccccaggct gtaccaagcc tcccccagac tccaacagct gcacctc        357
<210> 123
   <211> 720
   <212> DNA
   <213> Artificial sequence
<220>
   <223> Dual specific antibody
<220>
   <221> CDS
   <222> (1)..(720)
   <223>
<400> 123
   gag gtg cag ctg ttg gag tct ggg gga ggc ttg gta cag cct ggg ggg      48
   Glu Val Gln Leu Leu Glu Ser Gly Gly Gly Leu Val Gln Pro Gly Gly
   1               5                   10                  15
   tcc ctg aga ctc tcc tgt gca gcc tct gga ttc acc ttt agc agc tat      96
   Ser Leu Arg Leu Ser Cys Ala Ala Ser Gly Phe Thr Phe Ser Ser Tyr
               20                  25                  30
   gcc atg agc tgg gtc cgc cag gct cca ggg aag ggg ctg gag tgg gtc     144
   Ala Met Ser Trp Val Arg Gln Ala Pro Gly Lys Gly Leu Glu Trp Val
           35                  40                  45
   tca gct att agt ggt agt ggt ggt agc aca tac tac gca gac tcc gtg     192
   Ser Ala Ile Ser Gly Ser Gly Gly Ser Thr Tyr Tyr Ala Asp Ser Val
       50                  55                  60
   aag ggc cgg ttc acc atc tcc aga gac aat tcc aag aac acg ctg tat      240
   Lys Gly Arg Phe Thr Ile Ser Arg Asp Asn Ser Lys Asn Thr Leu Tyr
   65                  70                  75                  80
   ctg caa atg aac agc ctg aga gcc gag gac acg gcc gta tat tac tgt      288
   Leu Gln Met Asn Ser Leu Arg Ala Glu Asp Thr Ala Val Tyr Tyr Cys
                   85                  90                  95
   gcg aaa agt tat ggt gct ttt gac tac tgg ggc cag gga acc ctg gtc      336
   Ala Lys Ser Tyr Gly Ala Phe Asp Tyr Trp Gly Gln Gly Thr Leu Val
               100                 105                 110
   acc gtc tcg agc ggt gga ggc ggt tca ggc gga ggt ggc agc ggc ggt      384
   Thr Val Ser Ser Gly Gly Gly Gly Ser Gly Gly Gly Gly Ser Gly Gly
           115                 120                 125
   ggc ggg tcg acg gac atc cag atg acc cag tct cca tcc tcc ctg tct      432
   Gly Gly Ser Thr Asp Ile Gln Met Thr Gln Ser Pro Ser Ser Leu Ser
       130                 135                 140
   gca tct gta gga gac aga gtc acc atc act tgc cgg gca agt cag agc      480
   Ala Ser Val Gly Asp Arg Val Thr Ile Thr Cys Arg Ala Ser Gln Ser
   145                 150                 155                 160
   att agc agc tat tta aat tgg tat cag cag aaa cca ggg aaa gcc cct      528
   Ile Ser Ser Tyr Leu Asn Trp Tyr Gln Gln Lys Pro Gly Lys Ala Pro
                   165                 170                 175
   aag ctc ctg atc tat gct gca tcc agt ttg caa agt ggg gtc cca tca      576
   Lys Leu Leu Ile Tyr Ala Ala Ser Ser Leu Gln Ser Gly Val Pro Ser
               180                 185                 190
   agg ttc agt ggc agt gga tct ggg aca gat ttc act ctc acc atc agc      624
   Arg Phe Ser Gly Ser Gly Ser Gly Thr Asp Phe Thr Leu Thr Ile Ser
           195                 200                 205
   agt ctg caa cct gaa gat ttt gca act tac tac tgt caa cag agt tac      672
   Ser Leu Gln Pro Glu Asp Phe Ala Thr Tyr Tyr Cys Gln Gln Ser Tyr
       210                 215                 220
   agt acc cct aat acg ttc ggc caa ggg acc aag gtg gaa atc aaa cgg      720
   Ser Thr Pro Asn Thr Phe Gly Gln Gly Thr Lys Val Glu Ile Lys Arg
   225                 230                 235                 240
<210> 124
   <211> 240
   <212> PRT
   <213> Artificial sequence
<220>
   <223> Dual specific antibody
<400> 124
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Tyr Gly Ala Phe Asp Tyr Trp Gly Gln Gly Thr Leu Val
   100 105 110
   Thr Val Ser Ser Gly Gly Gly Gly Ser Gly Gly Gly Gly Ser Gly Gly
   115 120 125
   Gly Gly Ser Thr Asp Ile Gln Met Thr Gln Ser Pro Ser Ser Leu Ser
   130 135 140
   Ala Ser Val Gly Asp Arg Val Thr Ile Thr Cys Arg Ala Ser Gln Ser
   145 150 155 160
   Ile Ser Ser Tyr Leu Asn Trp Tyr Gln Gln Lys Pro Gly Lys Ala Pro
   165 170 175
   Lys Leu Leu Ile Tyr Ala Ala Ser Ser Leu Gln Ser Gly Val Pro Ser
   180 185 190
   Arg Phe Ser Gly Ser Gly Ser Gly Thr Asp Phe Thr Leu Thr Ile Ser
   195 200 205
   Ser Leu Gln Pro Glu Asp Phe Ala Thr Tyr Tyr Cys Gln Gln Ser Tyr
   210 215 220
   Ser Thr Pro Asn Thr Phe Gly Gln Gly Thr Lys Val Glu Ile Lys Arg
   225 230 235 240
<210> 125
   <211> 332
   <212> DNA
   <213> Artificial sequence
<220>
   <223> Variable domain vectors 1 and 2
<220>
   <221> CDS
   <222> (69)..(332)
   <223>
<400> 125
   caggaaacag ctatgaccat gattacgcca agcttgcatg caaattctat ttcaaggaga 60
   cagtcata atg aaa tac cta ttg cct acg gca gcc gct gga ttg tta tta 110
   Met Lys Tyr Leu Leu Pro Thr Ala Ala Ala Gly Leu Leu Leu
   1 5 10
   ctc gcg gcc cag ccg gcc atg gcc gag gtg ttt gac tac tgg ggc cag        158
   Leu Ala Ala Gln Pro Ala Met Ala Glu Val Phe Asp Tyr Trp Gly Gln
   15                  20                  25                  30
   gga acc ctg gtc acc gtc tcg agc ggt gga ggc ggt tca ggc gga ggt        206
   Gly Thr Leu Val Thr Val Ser Ser Gly Gly Gly Gly Ser Gly Gly Gly
                   35                  40                  45
   ggc agc ggc ggt ggc ggg tcg acg gac atc cag atg acc cag gcg gcc        254
   Gly Ser Gly Gly Gly Gly Ser Thr Asp Ile Gln Met Thr Gln Ala Ala
               50                  55                  60
   gca gaa caa aaa ctc atc tca gaa gag gat ctg aat ggg gcc gca tag        302
   Ala Glu Gln Lys Leu Ile Ser Glu Glu Asp Leu Asn Gly Ala Ala
           65                  70                  75
   act gtt gaa agt tgt tta gca aaa cct cat                                332
   Thr Val Glu Ser Cys Leu Ala Lys Pro His
           80                  85
<210> 126
   <211> 77
   <212> PRT
   <213> Artificial sequence
<220>
   <223> Variable domain vectors 1 and 2
<400> 126
   Met Lys Tyr Leu Leu Pro Thr Ala Ala Ala Gly Leu Leu Leu Leu Ala
   1               5                   10                  15
   Ala Gln Pro Ala Met Ala Glu Val Phe Asp Tyr Trp Gly Gln Gly Thr
               20                  25                  30
   Leu Val Thr Val Ser Ser Gly Gly Gly Gly Ser Gly Gly Gly Gly Ser
           35                  40                  45
   Gly Gly Gly Gly Ser Thr Asp Ile Gln Met Thr Gln Ala Ala Ala Glu
       50                  55                  60
   Gln Lys Leu Ile Ser Glu Glu Asp Leu Asn Gly Ala Ala
   65                  70                  75
<210> 127
   <211> 10
   <212> PRT
   <213> Artificial sequence
<220>
   <223> Variable domain vectors 1 and 2
<400> 127
   Thr Val Glu Ser Cys Leu Ala Lys Pro His
   1               5                   10
<210> 128
   <211> 27
   <212> DNA
   <213> Artificial sequence
<220>
   <223> Insertion in V domain vector 2 only
<220>
   <221> CDS
   <222> (1)..(27)
   <223>
<400> 128
   cat cat cat cac cat cac ggg gcc gca 27
   His His His His His His Gly Ala Ala
   1               5
<210> 129
   <211> 9
   <212> PRT
   <213> Artificial sequence
<220>
   <223> Insertion in V domain vector 2 only
<400> 129
   His His His His His His Gly Ala Ala
   1               5
<210> 130
   <211> 116
   <212> PRT
   <213> Artificial sequence
<220>
   <223> VH chain K8
<400> 130
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser His Ile Ser Pro Tyr Gly Ala Asn Thr Arg Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Leu Arg Ala Phe Asp Tyr Trp Gly Gln Gly Thr Leu Val
               100                 105                 110
   Thr Val Ser Ser
           115
<210> 131
   <211> 116
   <212> PRT
   <213> Artificial sequence
<220>
   <223> VH chain VH2
<400> 131
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Asp Ile Gly Ala Thr Gly Ser Lys Thr Gly Tyr Ala Asp Pro Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Lys Val Leu Thr Phe Asp Tyr Trp Gly Gln Gly Thr Leu Val
               100                 105                 110
   Thr Val Ser Ser
           115
<210> 132
   <211> 55
   <212> PRT
   <213> Artificial sequence
<220>
   <223> VH chain VH4
<400> 132
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Arg Ile Asn Gly Pro Gly
       50                  55
<210> 133
   <211> 60
   <212> PRT
   <213> Artificial sequence
<220>
   <223> VH chain VH4
<400> 133
   Ala Thr Gly Tyr Ala Asp Ser Val Lys Gly Arg Phe Thr Ile Ser Arg
   1               5                   10                  15
   Asp Asn Ser Lys Asn Thr Leu Tyr Leu Gln Ile Asn Ser Leu Arg Ala
               20                  25                  30
   Glu Asp Thr Ala Val Tyr Tyr Cys Ala Lys His Gly Ala Pro Phe Asp
           35                  40                  45
   Tyr Trp Gly Gln Gly Thr Leu Val Thr Val Ser Ser
       50                  55                  60
<210> 134
   <211> 116
   <212> PRT
   <213> Artificial sequence
<220>
   <223> VH chain VHC11
<400> 134
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Asn Trp Val Arg Gln Ala Pro Gly Lys Gly Leu Glu Trp Val
           35                  40                  45
   Ser Ser Ile Pro Ala Ser Gly Leu His Thr Arg Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Pro Gly Leu Gly Phe Asp Tyr Trp Gly Gln Gly Thr Leu Val
               100                 105                 110
   Thr Val Ser Ser
           115
<210> 135
   <211> 56
   <212> PRT
   <213> Artificial sequence
<220>
   <223> VH chain VHA10sd
<400> 135
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Asp Ile Glu Arg Thr Gly Tyr
   50 55
<210> 136
   <211> 59
   <212> PRT
   <213> Artificial sequence
<220>
   <223> VH chain VHA10sd
<400> 136
   Thr Arg Tyr Ala Asp Ser Val Lys Gly Arg Phe Thr Ile Ser Arg Asp
   1 5 10 15
   Asn Ser Lys Asn Thr Leu Tyr Leu Gln Met Asn Ser Leu Arg Ala Glu
   20 25 30
   Asp Thr Ala Val Tyr Tyr Cys Ala Lys Lys Val Leu Val Phe Asp Tyr
   35 40 45
   Trp Gly Gln Gly Thr Leu Val Thr Val Ser Ser
   50 55
<210> 137
   <211> 116
   <212> PRT
   <213> Artificial sequence
<220>
   <223> VH chain VHA1sd
<400> 137
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Glu Ile Ser Ala Asn Gly Ser Lys Thr Gln Tyr Ala Asp Ser Val
   50 55 60
   Lys Gly Arg Leu Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Lys Val Leu Gln Phe Asp Tyr Trp Gly Gln Gly Thr Leu Val
   100 105 110
   Thr Val Ser Ser
   115
<210> 138
   <211> 55
   <212> PRT
   <213> Artificial sequence
<220>
   <223> VH chain vha5sd
<400> 138
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Thr Ile Pro Ala Asn Gly
       50                  55
<210> 139
   <211> 60
   <212> PRT
   <213> Artificial sequence
<220>
   <223> VH chain VHA5sd
<400> 139
   Val Thr Arg Tyr Ala Asp Ser Val Lys Gly Arg Phe Thr Ile Ser Arg
   1               5                   10                  15
   Asp Asn Ser Lys Asn Thr Leu Tyr Leu Gln Met Asn Ser Leu Arg Ala
               20                  25                  30
   Glu Asp Thr Ala Val Tyr Tyr Cys Ala Lys Ser Leu Leu Gln Phe Asp
           35                  40                  45
   Tyr Trp Gly Gln Gly Thr Leu Val Thr Val Ser Ser
       50                  55                  60
<210> 140
   <211> 116
   <212> PRT
   <213> Artificial sequence
<220>
   <223> VH chain VHC5sd
<400> 140
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Asp Ile Ala Ala Thr Gly Ser Ala Thr Ser Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Lys Ile Leu Lys Phe Asp Tyr Trp Gly Gln Gly Thr Leu Val
   100 105 110
   Thr Val Ser Ser
   115
<210> 141
   <211> 116
   <212> PRT
   <213> Artificial sequence
<220>
   <223> VH chain VHC11sd
<400> 141
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Ser Thr Phe Ser Ser Tyr
               20                  25                  30
   Ala Met Ser Trp Val Arg Gln Ala Pro Gly Lys Gly Leu Glu Trp Val
           35                  40                  45
   Ser Thr Ile Ser Ser Val Gly Gln Ser Thr Arg Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asn Leu Met Ser Phe Asp Tyr Trp Gly Gln Gly Thr Leu Val
               100                 105                 110
   Thr Val Ser Ser
           115
<210> 142
   <211> 108
   <212> PRT
   <213> Artificial sequence
<220>
   <223> Vkappa chain K8
<400> 142
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Arg Ala Ser His Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Pro Trp Arg Ser Pro Gly
   85 90 95
   Thr Phe Gly Gln Gly Thr Lys Val Glu Ile Lys Arg
   100 105
<210> 143
   <211> 108
   <212> PRT
   <213> Artificial sequence
<220>
   <223> Vkappa chain E5sd
<400> 143
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Val Ser Ser Tyr
   20 25 30
   Leu Asn Trp Tyr Gln Gln Lys Pro Gly Lys Ala Pro Lys Leu Leu Ile
   35 40 45
   Tyr Leu Ala Ser Arg Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Asn Trp Trp Leu Pro Pro
   85 90 95
   Thr Phe Gly Gln Gly Thr Lys Val Glu Ile Lys Arg
   100 105
<210> 144
   <211> 49
   <212> PRT
   <213> Artificial sequence
<220>
   <223> Vkappa chain C3
<400> 144
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr
<210> 145
   <211> 58
   <212> PRT
   <213> Artificial sequence
<220>
   <223> Vkappa chain C3
<400> 145
   Ala Ser Leu Leu Gln Ser Gly Val Pro Ser Arg Phe Ser Gly Ser Gly
   1 5 10 15
   Ser Gly Thr Asp Phe Thr Leu Thr Ile Ser Ser Leu Gln Pro Glu Asp
   20 25 30
   Phe Ala Thr Tyr Tyr Cys Gln Gln Arg Val Tyr Asp Pro Leu Thr Phe
   35 40 45
   Gly Gln Gly Thr Lys Val Glu Ile Lys Arg
   50 55
<210> 146
   <211> 348
   <212> DNA
   <213> Artificial sequence
<220>
   <223> Dummy VH sequence for library 1
<220>
   <221> CDS
   <222> (1)..(348)
   <223>
<400> 146
   gag gtg cag ctg ttg gag tct ggg gga ggc ttg gta cag cct ggg ggg        48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt agc agc tat        96
   Ser Leu Arg Leu Ser Cys Ala Ala Ser Gly Phe Thr Phe Ser Ser Tyr
               20                  25                  30
   gcc atg agc tgg gtc cgc cag gct cca ggg aag ggt cta gag tgg gtc       144
   Ala Met Ser Trp Val Arg Gln Ala Pro Gly Lys Gly Leu Glu Trp Val
           35                  40                  45
   tca gct att agt ggt agt ggt ggt agc aca tac tac gca gac tcc gtg       192
   Ser Ala Ile Ser Gly Ser Gly Gly Ser Thr Tyr Tyr Ala Asp Ser Val
       50                  55                  60
   aag ggc cgg ttc acc atc tcc cgt gac aat tcc aag aac acg ctg tat       240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt       288
   Leu Gln Met Asn Ser Leu Arg Ala Glu Asp Thr Ala Val Tyr Tyr Cys
                   85                  90                  95
   gcg aaa agt tat ggt gct ttt gac tac tgg ggc cag gga acc ctg gtc       336
   Ala Lys Ser Tyr Gly Ala Phe Asp Tyr Trp Gly Gln Gly Thr Leu Val
               100                 105                 110
   acc gtc tcg agc                                                       348
   Thr Val Ser Ser
           115
<210> 147
   <211> 116
   <212> PRT
   <213> Artificial sequence
<220>
   <223> Dummy VH sequence for library 1
<400> 147
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Tyr Gly Ala Phe Asp Tyr Trp Gly Gln Gly Thr Leu Val
               100                 105                 110
   Thr Val Ser Ser
           115
<210> 148
   <211> 348
   <212> DNA
   <213> Artificial sequence
<220>
   <223> Dummy VH sequence for library 1
<400> 148
   gctcgagacg gtgaccaggg ttccctggcc ccagtagtca aaagcaccat aacttttcgc 60
   acagtaatat accgcggtgt cctcggcacg caggctgttc atttgcagat acagcgtgtt 120
   cttggaattg tcacgggaga tggtgaaccg gcccttcacg gagtctgcgt agtatgtgct 180
   accaccacta ccactaatag ctgagaccca ctctagaccc ttccctggag cctggcggac 240
   ccagctcatg gcatagctgc taaaggtgaa tccggaggct gcacaggaga gacgcaggga 300
   ccccccaggc tgtaccaagc ctcccccaga ctccaacagc tgcacctc 348
<210> 149
   <211> 360
   <212> DNA
   <213> Artificial sequence
<220>
   <223> Dummy VH sequence for library 2
<220>
   <221> CDS
   <222> (1)..(360)
   <223>
<220>
   <221> misc feature
   <222> (307)..(308)
   <223> n = a or t or c or g
<220>
   <221> misc feature
   <222> (310)..(311)
   <223> n = a or t or c or g
<220>
   <221> misc_feature
   <222> (313)..(314)
   <223> n = a or t or c or g
<220>
   <221> misc_feature
   <222> (316)..(317)
   <223> n = a or t or c or g
<400> 149
   gag gtg cag ctg ttg gag tct ggg gga ggc ttg gta cag cct ggg ggg        48
   Glu Val Gln Leu Leu Glu Ser Gly Gly Gly Leu Val Gln Pro Gly Gly
   1               5                   10                  15
   tcc ctg cgt ctc tcc tgt gca gcc tcc gga ttc acc ttt agc agc tat        96
   Ser Leu Arg Leu Ser Cys Ala Ala Ser Gly Phe Thr Phe Ser Ser Tyr
               20                  25                  30
   gcc atg agc tgg gtc cgc cag gct cca ggg aag ggt cta gag tgg gtc        144
   Ala Met Ser Trp Val Arg Gln Ala Pro Gly Lys Gly Leu Glu Trp Val
           35                  40                  45
   tca gct att agt ggt agt ggt ggt agc aca tac tac gca gac tcc gtg        192
   Ser Ala Ile Ser Gly Ser Gly Gly Ser Thr Tyr Tyr Ala Asp Ser Val
       50                  55                  60
   aag ggc cgg ttc acc atc tcc cgt gac aat tcc aag aac acg ctg tat        240
   Lys Gly Arg Phe Thr Ile Ser Arg Asp Asn Ser Lys Asn Thr Leu Tyr
   65                  70                  75                  80
   ctg caa atg aac agc ctg cgt gcc gag gac acc gcg gta tat tac tgt        288
   Leu Gln Met Asn Ser Leu Arg Ala Glu Asp Thr Ala Val Tyr Tyr Cys
                   85                  90                  95
   gcg aaa agt tat ggt gct nnk nnk nnk nnk ttt gac tac tgg ggc cag        336
   Ala Lys Ser Tyr Gly Ala Xaa Xaa Xaa Xaa Phe Asp Tyr Trp Gly Gln
               100                 105                 110
   gga acc ctg gtc acc gtc tcg agc                                        360
   Gly Thr Leu Val Thr Val Ser Ser
           115                 120
<210> 150
   <211> 120
   <212> PRT
   <213> Artificial sequence
<220>
   <221> misc feature
   <222> (103)..(103)
   <223> The 'Xaa' at location 103 is encoded by nnk, which encodes all amino acids as well as the TAG codon
<220>
   <221> misc_feature
   <222> (104)..(104)
   <223> The 'Xaa' at location 104 is encoded by nnk, which encodes all amino acids as well as the TAG codon
<220>
   <221> misc_feature
   <222> (105)..(105)
   <223> The 'Xaa' at location 105 is encoded by nnk, which encodes all amino acids as well as the TAG codon
<220>
   <221> misc_feature
   <222> (106)..(106)
   <223> The 'Xaa' at location 106 is encoded by nnk, which encodes all amino acids as well as the TAG codon
<220>
   <223> Dummy VH sequence for library 2
<400> 150
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Tyr Gly Ala Xaa Xaa Xaa Xaa Phe Asp Tyr Trp Gly Gln
               100                 105                 110
   Gly Thr Leu Val Thr Val Ser Ser
           115                 120
<210> 151
   <211> 360
   <212> DNA
   <213> Artificial sequence
<220>
   <223> Dummy VH sequence for library 2
<220>
   <221> misc_feature
   <222> (44)..(45)
   <223> n = a or t or c or g
<220>
   <221> misc feature
   <222> (47)..(48)
   <223> n = a or t or c or g
<220>
   <221> misc feature
   <222> (50)..(51)
   <223> n = a or t or c or g
<220>
   <221> misc_feature
   <222> (53)..(54)
   <223> n = a or t or c or g
<400> 151
   gctcgagacg gtgaccaggg ttccctggcc ccagtagtca aaknnknnkn nknnagcacc      60
   ataacttttc gcacagtaat ataccgcggt gtcctcggca cgcaggctgt tcatttgcag     120
   atacagcgtg ttcttggaat tgtcacggga gatggtgaac cggcccttca cggagtctgc     180
   gtagtatgtg ctaccaccac taccactaat agctgagacc cactctagac ccttccctgg     240
   agcctggcgg acccagctca tggcatagct gctaaaggtg aatccggagg ctgcacagga     300
   gagacgcagg gaccccccag gctgtaccaa gcctccccca gactccaaca gctgcacctc     360
<210> 152
   <211> 324
   <212> DNA
   <213> Artificial sequence
<220>
   <223> Dummy Vkappa sequence for library 3
<220>
   <221> CDS
   <222> (1)..(324)
   <223>
<400> 152
   gac atc cag atg acc cag tct cca tcc tcc ctg tct gca tct gta gga      48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att agc agc tat      96
   Asp Arg Val Thr Ile Thr Cys Arg Ala Ser Gln Ser Ile Ser Ser Tyr
               20                  25                  30
   tta aat tgg tac cag cag aaa cca ggg aaa gcc cct aag ctc ctg atc      144
   Leu Asn Trp Tyr Gln Gln Lys Pro Gly Lys Ala Pro Lys Leu Leu Ile
           35                  40                  45
   tat gct gca tcc agt ttg caa agt ggg gtc cca tca cgt ttc agt ggc      192
   Tyr Ala Ala Ser Ser Leu Gln Ser Gly Val Pro Ser Arg Phe Ser Gly
       50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
   65                  70                  75                  80
   gaa gat ttt gct acg tac tac tgt caa cag agt tac agt acc cct aat      288
   Glu Asp Phe Ala Thr Tyr Tyr Cys Gln Gln Ser Tyr Ser Thr Pro Asn
                   85                  90                  95
   acg ttc ggc caa ggg acc aag gtg gaa atc aaa cgg                      324
   Thr Phe Gly Gln Gly Thr Lys Val Glu Ile Lys Arg
               100                 105
<210> 153
   <211> 108
   <212> PRT
   <213> Artificial sequence
<220>
   <223> Dummy Vkappa sequence for library 3
<400> 153
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Tyr Ser Thr Pro Asn
   85 90 95
   Thr Phe Gly Gln Gly Thr Lys Val Glu Ile Lys Arg
   100 105
<210> 154
   <211> 324
   <212> DNA
   <213> Artificial sequence
<220>
   <223> Dummy Vkappa sequence for library 3
<400> 154
   ccgtttgatt tccaccttgg tcccttggcc gaacgtatta ggggtactgt aactctgttg 60
   acagtagtac gtagcaaaat cttcaggttg cagactgctg atggtgagag tgaaatctgt 120
   cccagatcca ctgccactga aacgtgatgg gaccccactt tgcaaactgg atgcagcata 180
   gatcaggagc ttaggggctt tccctggttt ctgctggtac caatttaaat agctgctaat 240
   gctctgactt gcccggcaag tgatggtgac acggtctcct acagatgcag acagggagga 300
   tggagactgg gtcatctgga tgtc 324
<210> 155
   <211> 324
   <212> DNA
   <213> Artificial sequence
<220>
   <223> anti MSA dAb MSA 16
<220>
   <221> CDS
   <222> (1)..(324)
   <223>
<400> 155
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att att aag cat        96
   Asp Arg Val Thr Ile Thr Cys Arg Ala Ser Gln Ser Ile Ile Lys His
               20                  25                  30
   tta aag tgg tac cag cag aaa cca ggg aaa gcc cct aag ctc ctg atc        144
   Leu Lys Trp Tyr Gln Gln Lys Pro Gly Lys Ala Pro Lys Leu Leu Ile
           35                  40                  45
   tat ggt gca tcc cgg ttg caa agt ggg gtc cca tca cgt ttc agt ggc        192
   Tyr Gly Ala Ser Arg Leu Gln Ser Gly Val Pro Ser Arg Phe Ser Gly
       50                  55                  60
   agt gga tct ggg aca gat ttc act ctc acc atc agc agt ctg caa cct        240
   Ser Gly Ser Gly Thr Asp Phe Thr Leu Thr Ile Ser Ser Leu Gln Pro
   65                  70                  75                  80
   gaa gat ttt gct acg tac tac tgt caa cag ggg gct cgg tgg cct cag        288
   Glu Asp Phe Ala Thr Tyr Tyr Cys Gln Gln Gly Ala Arg Trp Pro Gln
                   85                  90                  95
   acg ttc ggc caa ggg acc aag gtg gaa atc aaa cgg                        324
   Thr Phe Gly Gln Gly Thr Lys Val Glu Ile Lys Arg
               100                 105
<210> 156
   <211> 108
   <212> PRT
   <213> Artificial sequence
<220>
   <223> anti MSA dAb MSA 16
<400> 156
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ile Lys His
   20 25 30
   Leu Lys Trp Tyr Gln Gln Lys Pro Gly Lys Ala Pro Lys Leu Leu Ile
   35 40 45
   Tyr Gly Ala Ser Arg Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Gly Ala Arg Trp Pro Gln
   85 90 95
   Thr Phe Gly Gln Gly Thr Lys Val Glu Ile Lys Arg
   100 105
<210> 157
   <211> 324
   <212> DNA
   <213> Artificial sequence
<220>
   <223> anti MSA dAb MSA 26
<220>
   <221> CDS
   <222> (1)..(324)
   <223>
<400> 157
   gac atc cag atg acc cag tct cca tcc tcc ctg tct gca tct gta gga      48
   Asp Ile Gln Met Thr Gln Ser Pro Ser Ser Leu Ser Ala Ser Val Gly
   1               5                   10                  15
   gac cgt gtc acc atc act tgc cgg gca agt cag agc att tat tat cat      96
   Asp Arg Val Thr Ile Thr Cys Arg Ala Ser Gln Ser Ile Tyr Tyr His
               20                  25                  30
   tta aag tgg tac cag cag aaa cca ggg aaa gcc cct aag ctc ctg atc      144
   Leu Lys Trp Tyr Gln Gln Lys Pro Gly Lys Ala Pro Lys Leu Leu Ile
           35                  40                  45
   tat aag gca tcc acg ttg caa agt ggg gtc cca tca cgt ttc agt ggc      192
   Tyr Lys Ala Ser Thr Leu Gln Ser Gly Val Pro Ser Arg Phe Ser Gly
       50                  55                  60
   agt gga tct ggg aca gat ttc act ctc acc atc agc agt ctg caa cct      240
   Ser Gly Ser Gly Thr Asp Phe Thr Leu Thr Ile Ser Ser Leu Gln Pro
   65                  70                  75                  80
   gaa gat ttt gct acg tac tac tgt caa cag gtt cgg aag gtg cct cgg      288
   Glu Asp Phe Ala Thr Tyr Tyr Cys Gln Gln Val Arg Lys Val Pro Arg
                   85                  90                  95
   acg ttc ggc caa ggg acc aag gtg gaa atc aaa cgg                      324
   Thr Phe Gly Gln Gly Thr Lys Val Glu Ile Lys Arg
               100                 105
<210> 158
   <211> 108
   <212> PRT
   <213> Artificial sequence
<220>
   <223> anti MSA dAb MSA 26
<400> 158
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Tyr Tyr His
   20 25 30
   Leu Lys Trp Tyr Gln Gln Lys Pro Gly Lys Ala Pro Lys Leu Leu Ile
   35 40 45
   Tyr Lys Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Val Arg Lys Val Pro Arg
   85 90 95
   Thr Phe Gly Gln Gly Thr Lys Val Glu Ile Lys Arg
   100 105
<210> 159
   <211> 5
   <212> PRT
   <213> Artificial sequence
<220>
   <223> Polypeptide linker
<400> 159
   Gly Gly Gly Gly Ser
   1 5
<210> 160
   <211> 10
   <212> PRT
   <213> Artificial sequence
<220>
   <223> Polypeptide linker
<400> 160
   Gly Gly Gly Gly Ser Gly Gly Gly Gly Ser
   1 5 10
<210> 161
   <211> 15
   <212> PRT
   <213> Artificial sequence
<220>
   <223> Polypeptide linker
<400> 161
   Gly Gly Gly Gly Ser Gly Gly Gly Gly Ser Gly Gly Gly Gly Ser
   1 5 10 15
<210> 162
   <211> 20
   <212> PRT
   <213> Artificial sequence
<220>
   <223> Polypeptide linker
<400> 162
   Gly Gly Gly Gly Ser Gly Gly Gly Gly Ser Gly Gly Gly Gly Ser Gly
   1 5 10 15
   Gly Gly Gly Ser
   20
<210> 163
   <211> 25
   <212> PRT
   <213> Artificial sequence
<220>
   <223> Polypeptide linker
<400> 163
   Gly Gly Gly Gly Ser Gly Gly Gly Gly Ser Gly Gly Gly Gly Ser Gly
   1 5 10 15
   Gly Gly Gly Ser Gly Gly Gly Gly Ser
   20 25
<210> 164
   <211> 30
   <212> PRT
   <213> Artificial sequence
<220>
   <223> Polypeptide linker
<400> 164
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser
               20                  25                  30
<210> 165
   <211> 35
   <212> PRT
   <213> Artificial sequence
<220>
   <223> Polypeptide linker
<400> 165
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser Gly Gly
               20                  25                  30
   Gly Gly Ser
           35
<210> 166
   <211> 40
   <212> PRT
   <213> Artificial sequence
<220>
   <223> Polypeptide linker
<400> 166
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser Gly Gly
               20                  25                  30
   Gly Gly Ser Gly Gly Gly Gly Ser
           35                  40
<210> 167
   <211> 45
   <212> PRT
   <213> Artificial sequence
<220>
   <223> Polypeptide linker
<400> 167
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser Gly Gly
               20                  25                  30
   Gly Gly Ser Gly Gly Gly Gly Ser Gly Gly Gly Gly Ser
           35                  40                  45
<210> 168
   <211> 50
   <212> PRT
   <213> Artificial sequence
<220>
   <223> Polypeptide linker
<400> 168
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser Gly Gly
               20                  25                  30
   Gly Gly Ser Gly Gly Gly Gly Ser Gly Gly Gly Gly Ser Gly Gly Gly
           35                  40                  45
   Gly Ser
       50
<210> 169
   <211> 22
   <212> DNA
   <213> Artificial sequence
<220>
   <223> Primer
<400> 169
   cggccatggc gtcaacggac at 22
<210> 170
   <211> 23
   <212> DNA
   <213> Artificial sequence
<220>
   <223> Primer
<400> 170
   atgtgcgctc gagcgtttga ttt 23
<210> 171
   <211> 15
   <212> PRT
   <213> Artificial sequence
<220>
   <223> Modified form of the human IgGC1 hinge
<400> 171
   Glu Pro Lys Ser Gly Asp Lys Thr His Thr Cys Pro Pro Cys Pro
   1               5                   10                  15
<210> 172
   <211> 357
   <212> DNA
   <213> Artificial sequence
<220>
   <223> TAR1-5-19CYS
<220>
   <221> CDS
   <222> (1)..(357)
   <223>
<400> 172
   tgg agc gcg tcg acg gac atc cag atg acc cag tct cca tcc tct ctg       48
   Trp Ser Ala Ser Thr Asp Ile Gln Met Thr Gln Ser Pro Ser Ser Leu
   1               5                   10                  15
   tct gca tct gta gga gac cgt gtc acc atc act tgc cgg gca agt cag       96
   Ser Ala Ser Val Gly Asp Arg Val Thr Ile Thr Cys Arg Ala Ser Gln
               20                  25                  30
   agc att gat agt tat tta cat tgg tac cag cag aaa cca ggg aaa gcc      144
   Ser Ile Asp Ser Tyr Leu His Trp Tyr Gln Gln Lys Pro Gly Lys Ala
           35                  40                  45
   cct aag ctc ctg atc tat agt gca tcc gag ttg caa agt ggg gtc cca      192
   Pro Lys Leu Leu Ile Tyr Ser Ala Ser Glu Leu Gln Ser Gly Val Pro
       50                  55                  60
   tca cgt ttc agt ggc agt gga tct ggg aca gat ttc act ctc acc atc      240
   Ser Arg Phe Ser Gly Ser Gly Ser Gly Thr Asp Phe Thr Leu Thr Ile
   65                  70                  75                  80
   agc agt ctg caa cct gaa gat ttt gct acg tac tac tgt caa cag gtt      288
   Ser Ser Leu Gln Pro Glu Asp Phe Ala Thr Tyr Tyr Cys Gln Gln Val
                   85                  90                  95
   gtg tgg cgt cct ttt acg ttc ggc caa ggg acc aag gtg gaa atc aaa      336
   Val Trp Arg Pro Phe Thr Phe Gly Gln Gly Thr Lys Val Glu Ile Lys
               100                 105                 110
   cgg tgc taa taa gga tcc ggc                                          357
   Arg Cys         Gly Ser Gly
                   115
<210> 173
   <211> 114
   <212> PRT
   <213> Artificial sequence
<220>
   <223> TAR1-5-19CYS
<400> 173
   Trp Ser Ala Ser Thr Asp Ile Gln Met Thr Gln Ser Pro Ser Ser Leu
   1 5 10 15
   Ser Ala Ser Val Gly Asp Arg Val Thr Ile Thr Cys Arg Ala Ser Gln
   20 25 30
   Ser Ile Asp Ser Tyr Leu His Trp Tyr Gln Gln Lys Pro Gly Lys Ala
   35 40 45
   Pro Lys Leu Leu Ile Tyr Ser Ala Ser Glu Leu Gln Ser Gly Val Pro
   50 55 60
   Ser Arg Phe Ser Gly Ser Gly Ser Gly Thr Asp Phe Thr Leu Thr Ile
   65 70 75 80
   Ser Ser Leu Gln Pro Glu Asp Phe Ala Thr Tyr Tyr Cys Gln Gln Val
   85 90 95
   Val Trp Arg Pro Phe Thr Phe Gly Gln Gly Thr Lys Val Glu Ile Lys
   100 105 110
   Arg Cys
<210> 174
   <211> 357
   <212> DNA
   <213> Artificial sequence
<220>
   <223> TAR1-5-19CYS
<400> 174
   gccggatcct tattagcacc gtttgatttc caccttggtc ccttggccga acgtaaaagg 60
   acgccacaca acctgttgac agtagtacgt agcaaaatct tcaggttgca gactgctgat 120
   ggtgagagtg aaatctgtcc cagatccact gccactgaaa cgtgatggga ccccactttg 180
   caactcggat gcactataga tcaggagctt aggggctttc cctggtttct gctggtacca 240
   atgtaaataa ctatcaatgc tctgacttgc ccggcaagtg atggtgacac ggtctcctac 300
   agatgcagac agagaggatg gagactgggt catctggatg tccgtcgacg cgctcca 357
<210> 175
   <211> 39
   <212> DNA
   <213> Artificial sequence
<220>
   <223> Primer
<400> 175
   tggagcgcgt cgacggacat ccagatgacc cagtctcca 39
<210> 176
   <211> 39
   <212> DNA
   <213> Artificial sequence
<220>
   <223> Primer
<400> 176
   ttagcagccg gatccttatt agcaccgttt gatttccac 39
<210> 177
   <211> 5
   <212> PRT
   <213> Artificial sequence
<220>
   <223> Vkappa CDR1
<400> 177
   Ser Ser Tyr Leu Asn
   1 5
<210> 178
   <211> 5
   <212> PRT
   <213> Artificial sequence
<220>
   <223> Vkappa CDR1
<400> 178
   Phe Lys Ser Leu Lys
   1 5
<210> 179
   <211> 5
   <212> PRT
   <213> Artificial sequence
<220>
   <223> Vkappa CDR1
<400> 179
   Tyr Tyr His Leu Lys
   1 5
<210> 180
   <211> 5
   <212> PRT
   <213> Artificial sequence
<220>
   <223> Vkappa CDR1
<400> 180
   Arg Arg Tyr Leu Lys
   1 5
<210> 181
   <211> 5
   <212> PRT
   <213> Artificial sequence
<220>
   <223> Vkappa CDR1
<400> 181
   Tyr Asn Trp Leu Lys
   1 5
<210> 182
   <211> 5
   <212> PRT
   <213> Artificial sequence
<220>
   <223> Vkappa CDR1
<400> 182
   Leu Trp His Leu Arg
   1 5
<210> 183
   <211> 5
   <212> PRT
   <213> Artificial sequence
<220>
   <223> Vkappa CDR1
<400> 183
   Phe Arg Tyr Leu Ala
   1 5
<210> 184
   <211> 5
   <212> PRT
   <213> Artificial sequence
<220>
   <223> Vkappa CDR1
<400> 184
   Phe Tyr His Leu Ala
   1 5
<210> 185
   <211> 5
   <212> PRT
   <213> Artificial sequence
<220>
   <223> Vkappa CDR1
<400> 185
   Ile Trp His Leu Asn
   1 5
<210> 186
   <211> 5
   <212> PRT
   <213> Artificial sequence
<220>
   <223> Vkappa CDR1
<400> 186
   Tyr Arg Tyr Leu Arg
   1 5
<210> 187
   <211> 5
   <212> PRT
   <213> Artificial sequence
<220>
   <223> Vkappa CDR1
<400> 187
   Leu Lys Tyr Leu Lys
   1 5
<210> 188
   <211> 5
   <212> PRT
   <213> Artificial sequence
<220>
   <223> Vkappa CDR1
<400> 188
   Leu Arg Tyr Leu Arg
   1 5
<210> 189
   <211> 5
   <212> PRT
   <213> Artificial sequence
<220>
   <223> Vkappa CDR1
<400> 189
   Leu Arg Ser Leu Lys
   1 5
<210> 190
   <211> 5
   <212> PRT
   <213> Artificial sequence
<220>
   <223> Vkappa CDR1
<400> 190
   Phe Arg His Leu Lys
   1 5
<210> 191
   <211> 5
   <212> PRT
   <213> Artificial sequence
<220>
   <223> Vkappa CDR1
<400> 191
   Arg Lys Tyr Leu Arg
   1 5
<210> 192
   <211> 5
   <212> PRT
   <213> Artificial sequence
<220>
   <223> Vkappa CDR1
<400> 192
   Arg Arg Tyr Leu Asn
   1 5
<210> 193
   <211> 5
   <212> PRT
   <213> Artificial sequence
<220>
   <223> Vkappa CDR1
<400> 193
   Ile Lys His Leu Lys
   1 5
<210> 194
   <211> 5
   <212> PRT
   <213> Artificial sequence
<220>
   <223> Vkappa CDR1
<400> 194
   Tyr Lys His Leu Lys
   1 5
<210> 195
   <211> 6
   <212> PRT
   <213> Artificial sequence
<220>
   <223> VH CDR1
<400> 195
   Trp Val Tyr Gln Met Asp
   1 5
<210> 196
   <211> 6
   <212> PRT
   <213> Artificial sequence
<220>
   <223> VH CDR1
<400> 196
   Trp Ser Tyr Gln Met Thr
   1 5
<210> 197
   <211> 7
   <212> PRT
   <213> Artificial sequence
<220>
   <223> Vkappa dummy CDR2
<220>
   <221> MISC FEATURE
   <222> (1)..(1)
   <223> Xaa is encoded by nnk, which encodes all amino acids as well as t he TAG codon
<220>
   <221> MISC_FEATURE
   <222> (4)..(4)
   <223> Xaa is encoded by nnk, which encodes all amino acids as well as t he TAG codon
<400> 197
   Xaa Ala Ser Xaa Leu Gln Ser
   1 5
<210> 198
   <211> 7
   <212> PRT
   <213> Artificial sequence
<220>
   <223> Vkappa CDR2
<400> 198
   Arg Ala Ser Pro Leu Gln Ser
   1 5
<210> 199
   <211> 7
   <212> PRT
   <213> Artificial sequence
<220>
   <223> Vkappa CDR2
<400> 199
   Asn Ala Ser Tyr Leu Gln Ser
   1 5
<210> 200
   <211> 7
   <212> PRT
   <213> Artificial sequence
<220>
   <223> Vkappa CDR2
<400> 200
   Lys Ala Ser Thr Leu Gln Ser
   1 5
<210> 201
   <211> 7
   <212> PRT
   <213> Artificial sequence
<220>
   <223> Vkappa CDR2
<400> 201
   Gln Ala Ser Val Leu Gln Ser
   1 5
<210> 202
   <211> 7
   <212> PRT
   <213> Artificial sequence
<220>
   <223> Vkappa CDR2
<400> 202
   Arg Ala Ser Ser Leu Gln Ser
   1 5
<210> 203
   <211> 7
   <212> PRT
   <213> Artificial sequence
<220>
   <223> Vkappa CDR2
<400> 203
   His Ala Ser Leu Leu Gln Ser
   1 5
<210> 204
   <211> 7
   <212> PRT
   <213> Artificial sequence
<220>
   <223> Vkappa CDR2
<400> 204
   His Ala Ser His Leu Gln Ser
   1 5
<210> 205
   <211> 7
   <212> PRT
   <213> Artificial sequence
<220>
   <223> Vkappa CDR2
<400> 205
   Pro Ala Ser Lys Leu Gln Ser
   1 5
<210> 206
   <211> 7
   <212> PRT
   <213> Artificial sequence
<220>
   <223> Vkappa CDR2
<400> 206
   Arg Ala Ser Arg Leu Gln Ser
   1 5
<210> 207
   <211> 7
   <212> PRT
   <213> Artificial sequence
<220>
   <223> Vkappa CDR2
<400> 207
   Lys Ala Ser Ser Leu Gln Ser
   1 5
<210> 208
   <211> 7
   <212> PRT
   <213> Artificial sequence
<220>
   <223> Vkappa CDR2
<400> 208
   Asn Ala Ser His Leu Gln Ser
   1 5
<210> 209
   <211> 7
   <212> PRT
   <213> Artificial sequence
<220>
   <223> Vkappa CDR2
<400> 209
   Lys Ala Ser Trp Leu Gln Ser
   1 5
<210> 210
   <211> 7
   <212> PRT
   <213> Artificial sequence
<220>
   <223> Vkappa CDR2
<400> 210
   Ala Ala Ser Arg Leu Gln Ser
   1 5
<210> 211
   <211> 7
   <212> PRT
   <213> Artificial sequence
<220>
   <223> Vkappa CDR2
<400> 211
   Thr Ala Ser Ser Leu Gln Ser
   1 5
<210> 212
   <211> 7
   <212> PRT
   <213> Artificial sequence
<220>
   <223> Vkappa CDR2
<400> 212
   Ala Ala Ser Ser Leu Gln Ser
   1 5
<210> 213
   <211> 7
   <212> PRT
   <213> Artificial sequence
<220>
   <223> Vkappa CDR2
<400> 213
   Gly Ala Ser Arg Leu Gln Ser
   1 5
<210> 214
   <211> 17
   <212> PRT
   <213> Artificial sequence
<220>
   <223> VH dummy CDR2
<220>
   <221> MISC_FEATURE
   <222> (1)..(1)
   <223> Xaa is encoded by nnk, which encodes all amino acids as well as t he TAG codon
<220>
   <221> MISC_FEATURE
   <222> (3)..(5)
   <223> Xaa is encoded by nnk, which encodes all amino acids as well as t he TAG codon
<220>
   <221> MISC_FEATURE
   <222> (7)..(8)
   <223> Xaa is encoded by nnk, which encodes all amino acids as well as t he TAG codon
<220>
   <221> MISC FEATURE
   <222> (10)..(10)
   <223> Xaa is encoded by nnk, which encodes all amino acids as well as t he TAG codon
<400> 214
   Xaa Ile Xaa Xaa Xaa Gly Xaa Xaa Thr Xaa Tyr Ala Asp Ser Val Lys
   1 5 10 15
   Gly
<210> 215
   <211> 17
   <212> PRT
   <213> Artificial sequence
<220>
   <223> VH CDR2
<400> 215
   Ser Ile Ser Ala Phe Gly Ala Lys Thr Leu Tyr Ala Asp Ser Val Lys
   1 5 10 15
   Gly
<210> 216
   <211> 17
   <212> PRT
   <213> Artificial sequence
<220>
   <223> VH CDR2
<400> 216
   Ser Ile Ser Ser Phe Gly Ser Ser Thr Leu Tyr Ala Asp Ser Val Lys
   1 5 10 15
   Gly
<210> 217
   <211> 9
   <212> PRT
   <213> Artificial sequence
<220>
   <223> Vkappa dummy CDR3
<220>
   <221> MISC FEATURE
   <222> (3)..(6)
   <223> Xaa is encoded by nnk, which encodes all amino acids as well as t he TAG codon
<220>
   <221> MISC_FEATURE
   <222> (8)..(8)
   <223> Xaa is encoded by nnk, which encodes all amino acids as well as t he TAG codon
<400> 217
   Gln Gln Xaa Xaa Xaa Xaa Pro Xaa Thr
   1 5
<210> 218
   <211> 9
   <212> PRT
   <213> Artificial sequence
<220>
   <223> Vkappa CDR3
<400> 218
   Gln Gln Thr Tyr Ser Val Pro Pro Thr
   1 5
<210> 219
   <211> 9
   <212> PRT
   <213> Artificial sequence
<220>
   <223> Vkappa CDR3
<400> 219
   Gln Gln Thr Tyr Arg Ile Pro Pro Thr
   1 5
<210> 220
   <211> 9
   <212> PRT
   <213> Artificial sequence
<220>
   <223> Vkappa CDR3
<400> 220
   Gln Gln Val Val Tyr Trp Pro Val Thr
   1 5
<210> 221
   <211> 9
   <212> PRT
   <213> Artificial sequence
<220>
   <223> Vkappa CDR3
<400> 221
   Gln Gln Val Arg Lys Val Pro Arg Thr
   1 5
<210> 222
   <211> 9
   <212> PRT
   <213> Artificial sequence
<220>
   <223> Vkappa CDR3
<400> 222
   Gln Gln Gly Leu Tyr Pro Pro Ile Thr
   1 5
<210> 223
   <211> 9
   <212> PRT
   <213> Artificial sequence
<220>
   <223> Vkappa CDR3
<400> 223
   Gln Gln Asn Val Val Ile Pro Arg Thr
   1 5
<210> 224
   <211> 9
   <212> PRT
   <213> Artificial sequence
<220>
   <223> Vkappa CDR3
<400> 224
   Gln Gln Ser Ala Val Tyr Pro Lys Thr
   1 5
<210> 225
   <211> 9
   <212> PRT
   <213> Artificial sequence
<220>
   <223> Vkappa CDR3
<400> 225
   Gln Gln Arg Leu Leu Tyr Pro Lys Thr
   1 5
<210> 226
   <211> 9
   <212> PRT
   <213> Artificial sequence
<220>
   <223> Vkappa CDR3
<400> 226
   Gln Gln Arg Ala Arg Trp Pro Arg Thr
   1 5
<210> 227
   <211> 9
   <212> PRT
   <213> Artificial sequence
<220>
   <223> Vkappa CDR3
<400> 227
   Gln Gln Val Ala Arg Val Pro Arg Thr
   1 5
<210> 228
   <211> 9
   <212> PRT
   <213> Artificial sequence
<220>
   <223> Vkappa CDR3
<400> 228
   Gln Gln Tyr Val Gly Tyr Pro Arg Thr
   1 5
<210> 229
   <211> 9
   <212> PRT
   <213> Artificial sequence
<220>
   <223> Vkappa CDR3
<400> 229
   Gln Gln Thr Thr Tyr Tyr Pro Ile Thr
   1 5
<210> 230
   <211> 9
   <212> PRT
   <213> Artificial sequence
<220>
   <223> Vkappa CDR3
<400> 230
   Gln Gln Val Leu Tyr Tyr Pro Gln Thr
   1 5
<210> 231
   <211> 9
   <212> PRT
   <213> Artificial sequence
<220>
   <223> Vkappa CDR3
<400> 231
   Gln Gln Val Val Tyr Trp Pro Ala Thr
   1 5
<210> 232
   <211> 9
   <212> PRT
   <213> Artificial sequence
<220>
   <223> Vkappa CDR3
<400> 232
   Gln Gln Val Ala Leu Tyr Pro Lys Thr
   1 5
<210> 233
   <211> 9
   <212> PRT
   <213> Artificial sequence
<220>
   <223> Vkappa CDR3
<400> 233
   Gln Gln Asn Leu Phe Trp Pro Arg Thr
   1 5
<210> 234
   <211> 9
   <212> PRT
   <213> Artificial sequence
<220>
   <223> Vkappa CDR3
<400> 234
   Gln Gln Met Leu Phe Tyr Pro Lys Thr
   1 5
<210> 235
   <211> 9
   <212> PRT
   <213> Artificial sequence
<220>
   <223> Vkappa CDR3
<400> 235
   Gln Gln Gly Ala Arg Trp Pro Gln Thr
   1 5
<210> 236
   <211> 9
   <212> PRT
   <213> Artificial sequence
<220>
   <223> Vkappa CDR3
<400> 236
   Gln Gln Val Gly Arg Tyr Pro Lys Thr
   1 5
<210> 237
   <211> 7
   <212> PRT
   <213> Artificial sequence
<220>
   <223> VH CDR3
<400> 237
   Leu Ser Gly Lys Phe Asp Tyr
   1 5
<210> 238
   <211> 11
   <212> PRT
   <213> Artificial sequence
<220>
   <223> VH CDR3
<400> 238
   Gly Arg Asp His Asn Tyr Ser Leu Phe Asp Tyr
   1 5 10
<210> 239
   <211> 27
   <212> DNA
   <213> Artificial sequence
<220>
   <223> HA tag
<400> 239
   tatccttatg atgttcctga ttatgca 27
<210> 240
   <211> 9
   <212> PRT
   <213> Artificial sequence
<220>
   <223> HA tag
<400> 240
   Tyr Pro Tyr Asp Val Pro Asp Tyr Ala
   1 5

## Claims

1. A dual-specific ligand comprising a first, heavy chain immunoglobulin single variable domain having a binding specificity to a first epitope or antigen and a second, flight chain single variable domain having a binding activity to a second epitope or antigen, wherein binding to one or both of said antigens or epitopes acts to increase the half-life of the ligand *in vivo* and wherein said first and second domains are not a heavy chain variable domain and light chain variable domain which share the same specificity, provided that said dual specific ligand does not consist of an anti-HSA V_{H} domain and an anti-β galactosidase Vₖ domain.

2. A dual specific ligand according to claim 1 wherein the variable domains are provided as an antibody scFv fragment.

3. A dual-specific ligand according to claim 1 wherein the variable domains are provided as an antibody Fab region.

4. A four chain IgG immunoglobulin comprising a dual specific ligand of any preceding claim.

5. A four chain IgG immunoglobulin according to claim 4, wherein said IgG comprises two dual specific ligands, said dual specific ligands being identical in their variable domains.

6. A four chain IgG immunoglobulin according to claim 4, wherein said IgG comprises two dual specific ligands, said dual specific ligands being different in their variable domains.

7. A ligand comprising a first immunoglobulin single variable domain having a first antigen or epitope binding specificity and a second immunoglobulin single variable domain having a second antigen or epitope binding specificity wherein one or both of said first and second variable domains bind to an antigen or epitope which increases the half-life of the ligand *in vivo,* and either
(i) the first and the second immunoglobulin variable domains are heavy chain variable domains; or
(ii) the first and the second immunoglobulin variable domains are light chain variable domains.
provided that when the first and second immunoglobulin variable domains are Camelid VHH domains, the VHH domain which is specific for an antigen which increases the half-life of the ligand in vivo does not bind hen egg white lysozyme (HEL), porcine pancreatic alpha-amylase; NmC-A, hcg, BSA-linked RR6 azo dye or *S. mutans* HG982 cells.

8. A ligand according to claim 7, wherein the ligand comprises first and second Camelid VHH domains.

9. A ligand according to claim 7, wherein the first and/or second variable domain is human.

10. The dual-specific ligand according to claim 8 or 9, wherein the single variable domains are identical.

11. A ligand according to claim any preceding claim, wherein the first and second domains bind independently, such that the dual specific ligand may simultaneously bind both the first and second epitopes or antigens.

12. A ligand according to claim 11, wherein the dual specific ligand comprises a first form and a second form in equilibrium in solution, wherein both epitopes or antigens bind to the first form independently but compete for binding to the second form.

13. A ligand according to any preceding claim, wherein said first and second epitopes are present on separate antigens.

14. A ligand according to any one of claims 1 to 12, wherein said first and second epitopes are present on the same antigen.

15. A dual-specific ligand according to any preceding claim wherein the variable domains are non-covalently associated.

16. A dual-specific ligand according to any one of claims 1 to 14 wherein the variable regions are covalently associated.

17. A dual-specific ligand to claim 16 wherein the covalent association is mediated by disulphide bonds.

18. A ligand according to any preceding claim, comprising a single variable domain specific for TNFα, which has a dissociation constant (K_{d}) of 1x10⁻⁸ M or less for human TNFα, and a K_{off} rate constant of 1x10⁻³ s⁻¹ or less, as determined by surface plasmon resonance.

19. A ligand according to claim 18 wherein the domain specific for TNFα is a V_{*k*}.

20. A ligand according to any one of claims 1 to 17, comprising a single variable domain specific for TNF receptor 1 (p55), which has a dissociation constant (K_{d}) of 1x10⁻⁸ M or less for human TNF receptor 1, and a K_{off} rate constant of 1x10⁻³ s⁻¹ or less, as determined by surface plasmon resonance.

21. A ligand according to any one of claims 18 to 20, wherein the TNFα- or p55-specific domain neutralises human TNFα or TNF receptor 1 in a standard cell assay with a ND50 of 50nM or less.

22. A ligand according to any one of claims 1 to 17, comprising a single variable domain specific for TNF receptor 1 (p55), wherein the dAb antagonises the activity of the TNF receptor 1 in a standard cell assay with an ND₅₀ of ≤100nM and at a concentration of ≤10µM the dAb agonises the activity of the TNF receptor 1 by ≤5% in the assay.

23. A ligand according to any preceding claim, comprising a single variable domain specific for serum albumin (SA) which has a dissociation constant (K_{d}) of 1nM to 500µM for SA, as determined by surface plasmon resonance.

24. A ligand according to claim 23, where the SA-specific domain binds SA in a standard ligand binding assay with an IC50 of 1nM to 500µM.

25. A ligand according to any one of claims 1 to 17 comprising a single variable domain specific for TNFα, and which comprises the amino acid sequence of TAR1-5-19 idenlified by its sequence on pages 2 and 3 of the sequence listing or a sequence that is at least 80% homologous thereto.

26. A ligand according to any one of claims 1 to 17, comprising a single variable domain specific for TNFα, and which comprises the amino acid sequence of TAR1-5 idenlified by its sequence on pages 2 and 3 of the sequence listingor a sequence that is at least 80% homologous thereto.

27. A ligand according to any one of claims 1 to 17, comprising a single variable domain specific for TNFα, and which comprises the amino acid sequence of TAR1-27 identified by its sequence on page 4 of the seqence listing or a sequence that is at least 80% homologous thereto.

28. A ligand according to any one of claims 1 to 17, comprising a single variable domain specific for TNF receptor 1, and which comprises the amino acid sequence of TAR2-10 identified by its sequence on page 2 of the sequence listing or a sequence that is at least 80% homologous thereto.

29. A ligand according to any one of claims 1 to 17, comprising a single variable domain specific for TNF receptor 1, and which comprises the amino acid sequence of TAR2-10 identified by its sequence on page 2 of the sequence listing or a sequence that is at least 90% homologous thereto.

30. A ligand according to any one of claims 1 to 17, comprising a single variable domain specific for TNF receptor 1, and which comprises the amino acid sequence of TAR2-5 identified by its sequence an page 1 of the sequence listing or a sequence that is at least 80% homologous thereto.

31. A ligand according to any one of claims 1 to 17, comprising a single variable domain specific for TNF receptor 1, and which comprises the amino acid sequence of TAR2-5 identified by its sequence an page 1 of the sequence listing or a sequence that is at least 90% homologous thereto.

32. A ligand according to any one of claims 1 to 17, comprising a single variable domain specific for SA, and which comprises the amino acid sequence of MSA-16 identified by its sequence in figure 16 or a sequence that is at least 80% homologous thereto.

33. A ligand according to any one of claims 1 to 17, comprising a single variable domain specific for SA, and which comprises the amino acid sequence of MSA-26 identified by its sequence in figure 16 or a sequence that is at last 80% homologous thereto.

34. A ligand according to any one of claims 25 to 31, comprising an SA specific single variable domain as defined in claim 23 or 24.

35. A ligand according to any one of claims 18 to 34, wherein the TNFα, TNF receptor 1 or SA is in human form.

36. A dual-specific ligand comprising an anti-human TNF alpha dAb and an anti-SA dAb.

37. The ligand according to claim 36, wherein the dAbs are Camelid V_{HH} domains.

38. The ligand according to claim 37, wherein the anti-TNF alpha dAb is a TNFα-specific domain as defined in any one of claims 18,19, 21 and 25 to 27.

39. The ligand according to claim 37, wherein the anti-SA dAb is an SA-specific domain as defined in any one of claims 23, 24, 32 and 33.

40. A ligand according to any preceding claim, which comprises a universal framework.

41. A ligand according to any preceding claim comprising a V_{H} framework selected from the group consisting of DP47, DP45 and DP38; and/or a V_{L} framework which is DPK9.

42. A ligand according to any preceding claim which comprises a binding site for a generic ligand.

43. The ligand of claim 42, wherein the generic ligand binding site is selected from the group consisting of protein A, protein L and protein G.

44. A ligand according to any preceding claim wherein the ligand comprises a variable domain having one or more framework regions comprising an amino acid sequence that is the same as the amino acid sequence of a corresponding framework region encoded by a human germline antibody gene segment, or the amino acid sequences of one or more of said framework regions collectively comprises up to 5 amino acid differences relative to the amino acid sequence of said corresponding framework region encoded by a human germline antibody gene segment.

45. A ligand according to any one of claims 1 to 43, wherein the ligand comprises a variable domain, wherein the amino acid sequences of FW1, FW2, FW3 and FW4 are the same as the amino acid sequences of corresponding framework regions encoded by a human germline antibody gene segment, or the amino acid sequences of FW1, FW2, FW3 and FW4 collectively contain up to 10 amino acid differences relative to the amino acid sequences of corresponding framework regions encoded by said human germline antibody gene segment.

46. The ligand according to claim 44 or claim 45, which comprises an antibody variable domain comprising FW1, FW2 and FW3 regions, and the amino acid sequences of said FW1, FW2 and FW3 are the same as the amino acid sequences of corresponding framework regions encoded by human germline antibody gene segments.

47. The ligand according to any of claims 44 to 46, wherein said human germline antibody gene segment is selected from the group consisting of DP47, DP45, DP48 and DPK9.

48. A ligand according to claim 1 or 7 wherein the or each heavy chain variable domain is not a Camelid immunoglobulin variable domain.

49. The ligand according to claim 1 or 7 comprising at least one heavy chain variable domain that does not contain one or more amino acids that are specific to Camelid immunoglobulin variable domains as compared to human V_{H} domains.

50. A method for producing a ligand according to any preceding claim, comprising a first immunoglobulin single variable domain having a first binding specificity and a second single immunoglobulin single variable domain having a second binding specificity, one or both of the binding specificities being specific for a protein which increases the half-life of the ligand *in vivo,* the method comprising the steps of:
(a) selecting a first variable domain by its ability to bind to a first epitope,
(b) selecting a second variable region by its ability to bind to a second epitope,
(c) combining the variable regions; and
(d) selecting the ligand by its ability to bind to said first and second epitopes; wherein, when said variable domains are a heavy and a light chain variable domain, the heavy chain variable domain is not a V_{H} domain specific for HSA.

51. A method according to claim 50, wherein said first variable domain is selected for binding to said first epitope in absence of a complementary variable domain.

52. A method according to claim 51, wherein said first variable domain is selected for binding to said first epitope in the presence of a third complementary variable domain in which said third variable domain is different from said second variable domain.

53. Nucleic acid encoding at least a dual-specific ligand according to any one of claims 1 to 49.

54. A nucleic acid according to claim 53, comprising the nucleic acid sequence of TAR1-5-19 identified by its sequence on pages 2 and 3 of the sequence listing or a sequence that is at least 70% homologous thereto.

55. A nucleic acid according to claim 53, comprising the nucleic acid sequence of TAR1-5 identified by its sequence on page 3 of the sequence listing or a sequence that is at least 70% homologous thereto.

56. A nucleic acid according to claim 53, comprising the nucleic acid sequence of TAR1-27 identified by its sequence on page 4 of the sequence listing or a sequence that is at least 70% homologous thereto.

57. A nucleic acid according to claim 53, comprising the nucleic acid sequence of TAR2-10 identified by its sequence on page 2 of the sequence listing or a sequence that is at least 70% homologous thereto.

58. A nucleic acid according to claim 53, comprising the nucleic acid sequence of TAR2-10 identified by its sequence on page 2 of the sequence listing or a sequence that is at least 80% homologous thereto.

59. A nucleic acid according to claim 53, comprising the nucleic acid sequence of TAR2-5 identified by its sequence on page 1 of the sequence listing or a sequence that is at least 70% homologous thereto.

60. A nucleic acid according to claim 53, comprising the nucleic acid sequence of TAR2-5 identified by its sequence on page 1 of the sequence listing or a sequence that is at least 80% homologous thereto.

61. A nucleic acid according to claim 53, comprising the nucleic acid sequence of MSA-16 identified by its sequence in figure 16 or a sequence that is at least 70% homologous thereto.

62. A nucleic acid according to claim 53, comprising the nucleic acid sequence of MSA-26 identified by its sequence in figure 16 or a sequence that is at least 70% homologous thereto.

63. A vector comprising nucleic acid according to any one of claims 53 to 62.

64. A vector according to claim 63, further comprising components necessary for the expression of a dual-specific ligand.

65. A host cell comprising a vector according to claim 64.

66. A ligand according to any one of claims 1 to 49 for use in therapy.

67. A pharmaceutical composition comprising a ligand according to any one of claims 1 to 49, and a pharmaceutically acceptable excipient, carrier or diluent.

68. A dual-specific ligand comprising a first immunoglobulin single variable domain having binding specificity for serum albumin (SA), and a second immunoglobulin single variable domain having binding specificity for an antigen selected from the group consisting of EPO receptor, ApoE, Apo-SAA, BDNF, Cardiotrophin-1, EGF, EGF receptor, ENA-78, Eotaxin, Eotaxin-2, Exodus-2, EpoR, FGF-acidic, FGF-basic, fibroblast growth factor-10, FLT3 ligand, Fractalkine (CX3C), GDNF, G-CSF, GM-CSF, GF-β1, insulin, IFN-γ, IGF-I, IGF-II, IL-1α, IL-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-γ, HCC1, 1-309, HER 1, HER 2, HER 3 and HER 4.

69. A dual-specific ligand comprising a first immunoglobulin single variable domain having binding specificity for serum albumin (SA), and a second immunoglobulin single variable domain having binding specificity for an antigen selected from the group consisting of human or animal proteins, cytokines, cytokine receptors, enzymes, enzyme co-factors and DNA binding proteins.

70. A dual-specific ligand comprising a first immunoglobulin single variable domain having binding specificity for serum albumin (SA), and a second single immunoglobulin variable domain having binding specificity for a receptor for a cytokine listed in claim 68.

71. The ligand of claim 68, 69 or 70, where each of the first and second domains is
(i) a heavy chain variable domain or
(ii) a light chain variable domain.

72. The ligand of any one of claims 68 to 71, wherein the anti-SA domain is as defined in any one of claims 23, 24, 32 and 33.

73. Nucleic acid comprising said nucleic acid sequences as defined in any one of claims 53 to 62.

74. A vector comprising nucleic acid according to claim 73.

75. A host cell comprising a vector according to claim 74.

76. A ligand according to any one of claims 68 to 72 for use in therapy.

77. A pharmaceutical composition comprising a ligand according to any one of claims 68 to 72 and a pharmaceutically acceptable excipient, carrier or diluent.

## Patentansprüche

1. Bispezifischer Ligand, welcher eine erste einzelne variable Domäne einer schweren Kette von Immunglobulin mit einer Bindungsspezifität für ein erstes Epitop oder Antigen und eine zweite einzelne variable Domäne einer leichten Kette mit Bindungsaktivität für ein zweites Epitop oder Antigen umfaßt, wobei die Bindung an eines oder beide der Antigene oder Epitope so wirkt, daß sie die Halbwertszeit des Liganden *in vivo* erhöht, und wobei die erste und die zweite Domäne keine variable Domäne einer schweren Kette und keine variable Domäne einer leichten Kette, die dieselbe Spezifität gemeinsam haben, mit der Maßgabe, daß der bispezifische Ligand nicht aus einer Anti-HSA V_{H}-Domäne und einer Anti-β-Galactosidase-Vₖ-Domäne besteht.

2. Bispezifischer Ligand nach Anspruch 1, wobei die variablen Domänen als ein scFv-Antikörperfragment bereitgestellt werden.

3. Bispezifischer Ligand nach Anspruch 1, wobei die variablen Domänen als ein Fab-Antikörperbereich bereitgestellt werden.

4. Vierketten-IgG-Immunglobulin, welches einen bispezifischen Liganden nach einem der vorangegangenen Ansprüche umfaßt.

5. Vierketten-IgG-Immunglobulin nach Anspruch 4, wobei das IgG zwei bispezifische Liganden umfaßt, wobei die bispezifischen Liganden in ihren variablen Domänen identisch sind.

6. Vierketten-IgG-Immunglobulin nach Anspruch 4, wobei das IgG zwei bispezifische Liganden umfaßt, wobei die bispezifischen Liganden sich in ihren variablen Domänen unterscheiden.

7. Ligand, welcher eine erste einzelne variable Immunglobulindomäne mit einer ersten Antigen- oder Epitop-Bindungsspezifität und eine zweite einzelne variable Immunglobulindomäne mit einer zweiten Antigen- oder Epitop-Bindungsspezifität umfaßt, wobei eine oder beide der ersten und zweiten variablen Domänen an ein Antigen oder Epitop bindet/binden, welches die Halbwertszeit des Liganden *in vivo* erhöht, und entweder
(i) die erste und die zweite variable lmmunglobulindomäne variable Domänen einer schweren Kette sind oder
(ii) die erste und die zweite variable Immunglobulindomäne variable Domänen einer leichten Kette sind,
mit der Maßgabe, daß, wenn die erste und die zweite variable Immunglobulindomäne Camelid-VHH-Domänen sind, die VHH-Domäne, die für ein Antigen spezifisch ist, welches die Halbwertszeit des Liganden *in vivo* erhöht, nicht an Hühnerei-Lysozym (HEL), Alpha-Amylase aus der Bauchspeicheldrüse vom Schwein, NmC-A, hcg, BSA-verknüpften RR6-Azofarbstoff oder *S. mutans* HG982-Zellen bindet.

8. Ligand nach Anspruch 7, wobei der Ligand erste und zweite Camelid-VHH-Domänen umfaßt.

9. Ligand nach Anspruch 7, wobei die erste und/oder die zweite variable Domäne menschlich ist.

10. Bispezifischer Ligand nach Anspruch 8 oder 9, wobei die einzelnen variablen Domänen identisch sind.

11. Ligand nach einem der vorangegangenen Ansprüche, wobei die erste und die zweite Domäne unabhängig voneinander binden, so daß der bispezifische Ligand gleichzeitig sowohl an das erste als auch an das zweite Epitop oder Antigen binden kann.

12. Ligand nach Anspruch 11, wobei der bispezifische Ligand in Lösung eine erste Form und eine zweite Form im Gleichgewicht umfaßt, wobei beide Epitope oder Antigene unabhängig voneinander an die erste Form binden, aber um die Bindung an die zweite Form konkurrieren.

13. Ligand nach einem der vorangegangenen Ansprüche, wobei das erste und das zweite Epitop auf separaten Antigenen vorliegen.

14. Ligand nach einem der Ansprüche 1 bis 12, wobei das erste und das zweite Epitop auf demselben Antigen vorliegen.

15. Bispezifischer Ligand nach einem der vorangegangenen Ansprüche, wobei die variablen Bereiche nicht kovalent verknüpft sind.

16. Bispezifischer Ligand nach einem der Ansprüche 1 bis 14, wobei die variablen Bereiche kovalent verknüpft sind.

17. Bispezifischer Ligand nach Anspruch 16, wobei die kovalente Verknüpfung durch Disulfidbindungen vermittelt wird.

18. Ligand nach einem der vorangegangenen Ansprüche, welcher eine einzelne variable Domäne, die für TNFα spezifisch ist, umfaßt, die für humanen TNFα eine Dissoziationskonstante (K_{d}) von 1x10⁻⁸ M oder weniger und eine K_{off}-Ratenkonstante von 1X10⁻³ s⁻¹ oder weniger, bestimmt durch Oberflächenplasmonresonanz, hat.

19. Ligand nach Anspruch 18, wobei die für TNFα spezifische Domäne eine Vₖ ist.

20. Ligand nach einem der Ansprüche 1 bis 17, welcher eine einzelne variable Domäne, die für TNF-Rezeptor 1 (p55) spezifisch ist, umfaßt, die für humanen TNF-Rezeptor 1 eine Dissoziationskonstante (K_{d}) von 1x10⁻⁸ M oder weniger und eine K_{off}-Ratenkonstante von 1x10⁻³ s⁻¹ oder weniger, bestimmt durch Oberflächenplasmonresonanz, hat.

21. Ligand nach einem der Ansprüche 18 bis 20, wobei die TNFα- oder p55-spezifische Domäne humanen TNFα oder TNF-Rezeptor 1 in einem Standardzelltest mit einem ND50 von 50 nM oder weniger neutralisiert.

22. Ligand nach einem der Ansprüche 1 bis 17, welcher eine einzelne variable Domäne, die für TNF-Rezeptor 1 (p55) spezifisch ist, umfaßt, wobei der dAb der Aktivität des TNF-Rezeptors 1 in einem Standardzelltest mit einem ND₅₀ von ≤ 100 nM entgegenwirkt und bei einer Konzentration von ≤ 10 µM der dAb der Aktivität des TNF-Rezeptors 1 um ≤ 5% in dem Test entgegenwirkt.

23. Ligand nach einem der vorangegangenen Ansprüche, welcher eine einzelne variable Domäne, die für Serumalbumin (SA) spezifisch ist, umfaßt, die für SA eine Dissoziationskonstante (K_{d}) von 1 nM bis 500 µm, bestimmt durch Oberflächenplasmonresonanz, hat.

24. Ligand nach Anspruch 23, wobei die SA-spezifische Domäne in einem Standard-Ligandenbindungstest mit einem IC50 von 1 nM bis 500 µM an SA bindet.

25. Ligand nach einem der Ansprüche 1 bis 17, welcher eine einzelne variable Domäne umfaßt, die für TNFα spezifisch ist und die Aminosäuresequenz von TAR1-5-19, gezeigt durch ihre Sequenz auf Seiten 2 und 3 des Sequenzprotokolls, oder eine Sequenz, die zu wenigstens 80% homolog dazu ist, umfaßt.

26. Ligand nach einem der Ansprüche 1 bis 17, welcher eine einzelne variable Domäne umfaßt, die für TNFα spezifisch ist und die Aminosäuresequenz von TAR1-5, gezeigt durch ihre Sequenz auf den Seiten 2 und 3 des Sequenzprotokolls, oder eine Sequenz, die zu wenigstens 80% homolog dazu ist, umfaßt.

27. Ligand nach einem der Ansprüche 1 bis 17, welcher eine einzelne variable Domäne umfaßt, die für TNFα spezifisch ist und die Aminosäuresequenz von TAR1-27, gezeigt durch ihre Sequenz auf Seite 4 des Sequenzprotokolls, oder eine Sequenz, die zu wenigstens 80% homolog dazu ist, umfaßt.

28. Ligand nach einem der Ansprüche 1 bis 17, welcher eine einzelne variable Domäne umfaßt, die für TNF-Rezeptor 1 spezifisch ist und die Aminosäuresequenz von TAR2-10, gezeigt durch ihre Sequenz auf Seite 2 des Sequenzprotokolls, oder eine Sequenz, die zu wenigstens 80% homolog dazu ist, umfaßt.

29. Ligand nach einem der Ansprüche 1 bis 17, welcher eine einzelne variable Domäne umfaßt, die für TNF-Rezeptor 1 spezifisch ist und die Aminosäuresequenz von TAR2-10, gezeigt durch ihre Sequenz auf Seite 2 des Sequenzprotokolls, oder eine Sequenz, die zu wenigstens 90% homolog dazu ist, umfaßt.

30. Ligand nach einem der Ansprüche 1 bis 17, welcher eine einzelne variable Domäne umfaßt, die für TNF-Rezeptor 1 spezifisch ist und die Aminosäuresequenz von TAR2-5, gezeigt durch ihre Sequenz auf Seite 1 des Sequenzprotokolls, oder eine Sequenz, die zu wenigstens 80% homolog dazu ist, umfaßt.

31. Ligand nach einem der Ansprüche 1 bis 17, welcher eine einzelne variable Domäne umfaßt, die für TNF-Rezeptor 1 spezifisch ist und die Aminosäuresequenz von TAR2-5, gezeigt durch ihre Sequenz auf Seite 1 des Sequenzprotokolls, oder eine Sequenz, die zu wenigstens 90% homolog dazu ist, umfaßt.

32. Ligand nach einem der Ansprüche 1 bis 17, welcher eine einzelne variable Domäne umfaßt, die für SA spezifisch ist und die Aminosäuresequenz von MSA-16, gezeigt durch ihre Sequenz in Figur 16, oder eine Sequenz, die zu wenigstens 80% homolog dazu ist, umfaßt.

33. Ligand nach einem der Ansprüche 1 bis 17, welcher eine einzelne variable Domäne umfaßt, die für SA spezifisch ist und die Aminosäuresequenz von MSA-26, gezeigt durch ihre Sequenz in Figur 16, oder eine Sequenz, die zu wenigstens 80% homolog dazu ist, umfaßt.

34. Ligand nach einem der Ansprüche 25 bis 31, welcher eine einzelne variable SA-spezifische Domäne, wie sie in einem der Ansprüche 23 oder 24 definiert ist, umfaßt.

35. Ligand nach einem der Ansprüche 18 bis 34, wobei der TNFα, der TNF-Rezeptor 1 oder SA in humaner Form vorliegen.

36. Bispezifischer Ligand, welcher einen Anti-Mensch-TNF-alpha-dAb und einen Anti-SA-dAb umfaßt.

37. Ligand nach Anspruch 36, wobei die dAb Camelid-V_{HH}-Domänen sind.

38. Ligand nach Anspruch 37, wobei Anti-TNF-alpha-dAb eine TNFα-spezifische Domäne ist, wie sie in einem der Ansprüche 18, 19, 21 und 25 bis 27 definiert ist.

39. Ligand nach Anspruch 37, wobei Anti-SA-dAb eine SA-spezifische Domäne ist, wie sie in einem der Ansprüche 23, 24, 32 und 33 definiert ist.

40. Ligand nach einem der vorangegangenen Ansprüche, welcher ein universales Gerüst umfaßt.

41. Ligand nach einem der vorangegangenen Ansprüche, welcher ein V_{H}-Gerüst, ausgewählt aus der Gruppe, bestehend aus DP47, DP45 und DP38, und/oder ein V_{L}-Gerüst, welches DPK9 ist, umfaßt.

42. Ligand nach einem der vorangegangenen Ansprüche, welcher eine Bindungsstelle für einen generischen Liganden umfaßt.

43. Ligand nach Anspruch 42, wobei die Bindungsstelle für den generischen Liganden aus der Gruppe ausgewählt ist, bestehend aus Protein A, Protein L und Protein G.

44. Ligand nach einem der vorangegangenen Ansprüche, wobei der Ligand eine variable Domäne mit einem oder mehreren Gerüstbereichen aufweisen, welche eine Aminosäuresequenz umfassen, die dieselbe ist wie die Aminosäuresequenz eines korrespondierenden Gerüstbereichs, ein durch das humane Keimbahn-Antikörper-Gensegment codiert wird, oder die Aminosäuresequenzen eines oder mehrerer der Gerüstbereiche gemeinsam bis zu 5 Aminosäureunterschiede in Bezug auf die Aminosäuresequenz des korrespondierenden Gerüstbereichs, der durch ein humanes Keimbahn-Antikörper-Gensegment codiert wird, aufweisen.

45. Ligand nach einem der Ansprüche 1 bis 43, wobei der Ligand eine variable Domäne umfaßt, wobei die Aminosäuresequenzen von FW1, FW2, FW3 und FW4 dieselben sind wie die Aminosäuresequenzen von korrespondierenden Gerüstbereichen, die durch ein humanes Keimbahn-Antikörper-Gensegment codiert werden, oder die Aminosäuresequenzen von FW1, FW2, FW3 und FW4 gemeinsam bis zu 10 Aminosäureunterschiede in Bezug auf die Aminosäuresequenzen der korrespondierenden Gerüstbereiche, die von dem humanen Keimbahn-Antikörper-Gensegment codiert werden, aufweisen.

46. Ligand nach Anspruch 44 oder Anspruch 45, welcher eine variable Antikörperdomäne umfaßt, die die Bereiche FW1, FW2 und FW3 beinhaltet, und die Aminosäuresequenzen von FW1, FW2 und FW3 dieselben sind wie die Aminosäuresequenzen von korrespondierenden Gerüstbereichen, die durch humane Keimbahn-Antikörper-Gensegmente codiert werden.

47. Ligand nach einem der Ansprüche 44 bis 46, wobei das humane Keimbahn-Antikörper Gensegment aus der Gruppe ausgewählt ist, bestehend aus DP47, DP45, DP48 und DPK9.

48. Ligand nach Anspruch 1 oder 7, wobei die oder jede der variablen Domänen einer schweren Kette keine variable Camelid-Immunglobulindomäne ist.

49. Ligand nach Anspruch 1 oder 7, welcher wenigstens eine variable Domäne einer schweren Kette umfaßt, die nicht eine oder mehrere Aminosäuren, die im Vergleich zu humanen V_{H}-Domänen für variable Camelid-Immunglobulindomänen spezifisch sind, enthält.

50. Verfahren zum Herstellen eines Liganden nach einem der vorangegangenen Ansprüche, welcher eine erste einzelne variable Immunglobulindomäne mit einer ersten Bindungsspezifität und eine zweite einzelne variable Immunglobulindomäne mit einer zweiten Bindungsspezifität umfaßt, wobei eine der oder beide Bindungsspezifitäten für ein Protein, welches die Halbwertszeit des Liganden *in vivo* erhöht, spezifisch ist/sind, wobei das Verfahren die folgenden Stufen umfaßt:
(a) Auswählen einer ersten variablen Domäne anhand ihrer Fähigkeit, an ein erstes Epitop zu binden,
(b) Auswählen eines zweiten variablen Bereichs anhand seiner Fähigkeit, an ein zweites Epitop zu binden,
(c) Kombinieren der variablen Bereiche und
(d) Auswählen des Liganden anhand seiner Fähigkeit, an das erste und das zweite Epitop zu binden, wobei, wenn die variablen Domänen variable Domänen einer schweren und einer leichten Kette sind, die variable Domäne einer schweren Kette keine für HSA spezifische V_{H}-Domäne ist.

51. Verfahren nach Anspruch 50, wobei die erste variable Domäne hinsichtlich Bindung an das erste Epitop in Abwesenheit einer komplementären variablen Domäne ausgewählt wird.

52. Verfahren nach Anspruch 51, wobei die erste variable Domäne hinsichtlich Bindung an das erste Epitop in Gegenwart einer komplementären dritten variablen Domäne ausgewählt wird, wobei die dritte variable Domäne sich von der zweiten variablen Domäne unterscheidet.

53. Nukleinsäure, die wenigstens einen bispezifischen Liganden gemäß einem der Ansprüche 1 bis 49 codiert.

54. Nukleinsäure nach Anspruch 53, welche die Nukleinsäuresequenz von TAR1-5-19, gezeigt durch ihre Sequenz auf den Seiten 2 und 3 des Sequenzprotokolls, oder eine Sequenz, die zu wenigstens 70% homolog dazu ist, umfaßt.

55. Nukleinsäure nach Anspruch 53, welche die Nukleinsäuresequenz von TAR1-5, gezeigt durch ihre Sequenz auf Seite 3 des Sequenzprotokolls, oder eine Sequenz, die zu wenigstens 70% homolog dazu ist, umfaßt.

56. Nukleinsäure nach Anspruch 53, welche die Nukleinsäuresequenz von TAR1-27, gezeigt durch ihre Sequenz auf Seite 4 des Sequenzprotokolls, oder eine Sequenz, die zu wenigstens 70% homolog dazu ist, umfaßt.

57. Nukleinsäure nach Anspruch 53, welche die Nukleinsäuresequenz von TAR2-10, gezeigt durch ihre Sequenz auf Seite 2 des Sequenzprotokolls, oder eine Sequenz, die zu wenigstens 70% homolog dazu ist, umfaßt.

58. Nukleinsäure nach Anspruch 53, welche die Nukleinsäuresequenz von TAR2-10, gezeigt durch ihre Sequenz auf Seite 2 des Sequenzprotokolls, oder eine Sequenz, die zu wenigstens 80% homolog dazu ist, umfaßt.

59. Nukleinsäure nach Anspruch 53, welche die Nukleinsäuresequenz von TAR2-5, gezeigt durch ihre Sequenz auf Seite 1 des Sequenzprotokolls, oder eine Sequenz, die zu wenigstens 70% homolog dazu ist, umfaßt.

60. Nukleinsäure nach Anspruch 53, welche die Nukleinsäuresequenz von TAR2-5, gezeigt durch ihre Sequenz auf Seite 1 des Sequenzprotokolls, oder eine Sequenz, die zu wenigstens 80% homolog dazu ist, umfaßt.

61. Nukleinsäure nach Anspruch 53, welche die Nukleinsäuresequenz von MSA-16, gezeigt durch ihre Sequenz in Figur 16, oder eine Sequenz, die zu wenigstens 70% homolog dazu ist, umfaßt.

62. Nukleinsäure nach Anspruch 53, welche die Nukleinsäuresequenz von MSA-26, gezeigt durch ihre Sequenz in Figur 16, oder eine Sequenz, die zu wenigstens 70% homolog dazu ist, umfaßt.

63. Vektor, welcher die Nukleinsäure nach einem der Ansprüche 53 bis 62 umfaßt.

64. Vektor nach Anspruch 63, welcher weiterhin Komponenten umfaßt, die für die Expression eines bispezifischen Liganden notwendig sind.

65. Wirtszelle, die einen Vektor nach Anspruch 64 umfaßt.

66. Ligand nach einem der Ansprüche 1 bis 49 zur Verwendung in der Therapie.

67. Pharmazeutische Zusammensetzung, welche einen Liganden nach einem der Ansprüche 1 bis 49 und einen pharmazeutisch verträglichen Hilfsstoff, einen Träger oder ein Verdünnungsmittel umfaßt.

68. Bispezifischer Ligand mit einer ersten einzelnen variablen Immunglobulindomäne mit Bindungsspezifität für Serumalbumin (SA) und einer zweiten einzelnen variablen Immunglobulindomäne mit Bindungsspezifität für ein Antigen, ausgewählt aus der Gruppe, bestehend aus EPO-Rezeptor, ApoE, Apo-SAA, BDNF, Cardiotrophin-1, EGF, EGF-Rezeptor, ENA-78, Eotaxin, Eotaxin-2, Exodus-2, EpoR, saurem FGF-, basischem FGF, Fibroblastenwachstumsfaktor-10, FLT3-Ligand, Fractalkin (CX3C), GDNF, G-CSF, GM-CSF, GF-β1, Insulin, IFN-γ, IGF-I, IGF-II, IL-1α, IL-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, Keratinozytenwachstumsfaktor-2 (KGF-2), KGF, Leptin, LIF, Lymphotactin, Müllerian Inhibitory Substance, Monozyten-koloniehemmendem Faktor, Monozyten anziehendem Protein, M-CSF, MDC (67 a.a.), MDC (69 a.a.), MCP-1 (MCAF), MCP-2, MCP-3, MCP-4, MDC (67a.a.), MDC (69 a.a.), MIG, MIP-1α, MIP-1β, MIP-3α, MIP-3β, MIP-4, myeloischem Progenitorinhibitorfaktor-1 (MPIF-1), NAP-2, Neurturin, Nervenwachstumsfaktor, β-NGF, NT-3, NT-4, Oncostatin M, PDGF-AA, PDGF-AB, PDGF-BB, PF-4, RANTES, SDF1α, SDF1β, SCF, SCGF, Stammzellfaktor (SCF), TARC, TGF-α, TGF-β, TGF-β2, TGF-β3, Tumornekrosefaktor (TNF), TNF-α, TNF-β, TNF-Rezeptor I, TNF-Rezeptor II, TNIL-1, TPO, VEGF, VEGF-Rezeptor 1, VEGF-Rezeptor 2, VEGF-Rezeptor 3, GCP-2, GRO/MGSA, GRO-β, GRO-γ, HCC1, 1-309, HER 1, HER 2, HER 3 und HER 4.

69. Bispezifischer Ligand, welcher eine erste einzelne variable Immunglobulindomäne mit Bindungsspezifität für Serumalbumin (SA) und eine zweite einzelne variable Immunglobulindomäne mit Bindungsspezifität für ein Antigen, ausgewählt aus der Gruppe, bestehend aus menschlichen oder tierischen Proteinen, Zytokinen, Zytokinrezeptoren, Enzymen, Enzym-Cofakoren und DNA-Bindungsproteinen, umfaßt.

70. Bispezifischer Ligand, welcher eine erste einzelne variable Immunglobulindomäne mit Bindungsspezifität für Serumalbumin (SA) und eine zweite einzelne variable Immunglobulindomäne mit Bindungsspezifität für einen Rezeptor für ein in Anspruch 68 aufgeführtes Zytokin umfaßt.

71. Ligand nach einem der Ansprüche 68, 69 oder 70, wobei jede der ersten und zweiten Domänen
(i) eine variable Domäne einer schweren Kette oder
(ii) eine variable Domäne einer leichten Kette ist.

72. Ligand nach einem der Ansprüche 68 bis 71, wobei die Anti-SA-Domäne wie in einem der Ansprüche 23, 24, 32 und 33 definiert ist.

73. Nukleinsäure, welche die Nukleinsäuresequenzen, wie sie in einem der Ansprüche 53 bis 62 definiert sind, umfaßt.

74. Vektor, welcher die Nukleinsäure nach Anspruch 73 umfaßt.

75. Wirtszelle, welche einen Vektor nach Anspruch 74 umfaßt.

76. Ligand nach einem der Ansprüche 68 bis 72 zur Verwendung in der Therapie.

77. Pharmazeutische Zusammensetzung, welche einen Liganden nach einem der Ansprüche 68 bis 72 und einen pharmazeutisch verträglichen Hilfsstoff, einen Träger oder ein Verdünnungsmittel umfaßt.

## Revendications

1. Ligand à double spécificité, comprenant un premier domaine variable unique de chaîne lourde d'immunoglobuline ayant une spécificité de liaison à un premier épitope ou antigène et un second domaine variable unique de chaîne légère ayant une activité de liaison à un second épitope ou antigène, dans lequel la liaison de l'un des ou des deux antigènes ou épitopes agit en augmentant la demi-vie du ligand *in vivo*, et dans lequel lesdits premier et second domaines ne sont pas un domaine variable de chaîne lourde et un domaine variable de chaîne légère qui ont en commun la même spécificité, sous réserve que ledit ligand à double spécificité ne consiste pas en un domaine V_{H} anti-HSA et un domaine V_{K} anti-β-galactosidase.

2. Ligand à double spécificité suivant la revendication 1, dans lequel les domaines variables sont fournis sous forme d'un fragment scFv d'anticorps.

3. Ligand à double spécificité suivant la revendication 1, dans lequel les domaines variables sont fournis sous forme d'une région Fab d'anticorps.

4. Immunoglobuline IgG à quatre chaînes comprenant un ligand à double spécificité de l'une quelconque des revendications précédentes.

5. Immunoglobuline IgG à quatre chaînes suivant la revendication 4, ladite IgG comprenant deux ligands à double spécificité, lesdits ligands à double spécificité étant identiques dans leurs domaines variables.

6. Immunoglobuline IgG à quatre chaînes suivant la revendication 4, ladite IgG comprenant deux ligands à double spécificité, lesdits ligands à double spécificité étant différents dans leurs domaines variables.

7. Ligand comprenant un premier domaine variable unique d'immunoglobuline ayant une première spécificité de liaison à un antigène ou épitope et un second domaine variable unique d'immunoglobuline ayant une seconde spécificité de liaison à un antigène ou épitope, dans lequel l'un des ou les deux premier et second domaines variables se lient à un antigène ou épitope qui augmente la demi-vie du ligand *in vivo*, et soit
(i) les premier et second domaines variables d'immunoglobuline sont des domaines variables de chaîne lourde ; soit
(ii) les premier et second domaines variables d'immunoglobuline sont des domaines variables de chaîne légère,
sous réserve que, lorsque les premier et second domaines variables d'immunoglobuline sont des domaines VHH de camélidés, le domaine VHH qui est spécifique d'un antigène qui augmente la demi-vie du ligand *in vivo* ne se lie pas au lysozyme de blanc d'oeuf de poule (HEL), à l'alpha-amylase pancréatique porcine, à NmC-A, à la hcg, au colorant azoïque RR6 lié à la SAB, ou à des cellules de *S. mutans* HG982.

8. Ligand suivant la revendication 7, le ligand comprenant des premier et second domaines VHH de camélidés.

9. Ligand suivant la revendication 7, dans lequel les premier et/ou second domaines variables sont d'origine humaine.

10. Ligand à double spécificité suivant la revendication 8 ou 9, dans lequel les domaines variables uniques sont identiques.

11. Ligand suivant l'une quelconque des revendications précédentes, dans lequel les premier et second domaines se lient indépendamment, de sorte que le ligand à double spécificité peut se lier simultanément aux premier et second épitopes ou antigènes.

12. Ligand suivant la revendication 11, dans lequel le ligand à double spécificité comprend une première forme et une seconde forme à l'équilibre en solution, les deux épitopes ou antigènes se liant à la première forme indépendamment mais entrant en compétition pour la liaison à la seconde forme.

13. Ligand suivant l'une quelconque des revendications précédentes, dans lequel lesdits premier et second épitopes sont présents sur des antigènes distincts.

14. Ligand suivant l'une quelconque des revendications 1 à 12, dans lequel lesdits premier et second épitopes sont présents sur le même antigène.

15. Ligand à double spécificité suivant l'une quelconque des revendications précédentes, dans lequel les domaines variables sont associés de manière non covalente.

16. Ligand à double spécificité suivant l'une quelconque des revendications 1 à 14, dans lequel les régions variables sont associées de manière covalente.

17. Ligand à double spécificité suivant la revendication 16, dans lequel l'association covalente est soumise à une médiation par des liaisons disulfures.

18. Ligand suivant l'une quelconque des revendications précédentes, comprenant un domaine variable unique spécifique du TNFα qui a une constante de dissociation (K_{d}) égale ou inférieure à 1 x 10⁻⁸ M pour le TNFα humain, et une constante de vitesse K_{off} égale ou inférieure à 1 x 10⁻³ s⁻¹, de la manière déterminée par résonance de plasmon de surface.

19. Ligand suivant la revendication 18, dans lequel le domaine spécifique du TNFα est le domaine V_{κ}.

20. Ligand suivant l'une quelconque des revendications 1 à 17, comprenant un domaine variable unique spécifique du récepteur 1 de TNF (p55) qui a une constante de dissociation (K_{d}) égale ou inférieure à 1 x 10⁻⁸ M pour le récepteur 1 du TNF humain, et une constante de vitesse K_{off} égale ou inférieure à 1 x 10⁻³ s⁻¹, telle que déterminée par résonance de plasmon de surface.

21. Ligand suivant l'une quelconque des revendications 18 à 20, dans lequel le domaine spécifique du TNFα ou de p55 neutralise le TNFα humain ou le récepteur 1 du TNF humain dans une analyse cellulaire classique avec une DN₅₀ égale ou inférieure à 50 nM.

22. Ligand suivant l'une quelconque des revendications 1 à 17, comprenant un domaine variable unique spécifique du récepteur 1 du TNF (p55), dans lequel le dAb antagonise l'activité du récepteur 1 du TNF dans une analyse cellulaire classique avec une DN₅₀ ≤ 100 nM et, à une concentration < 10 µM, le dAb agonise l'activité du récepteur 1 du TNF d'une valeur < 5 % dans l'analyse.

23. Ligand suivant l'une quelconque des revendications précédentes, comprenant un domaine variable unique spécifique de la sérumalbumine (SA) qui a une constante de dissociation (K_{d}) de 1 nM à 500 µM pour la SA, comme déterminée par résonance du plasmon de surface.

24. Ligand suivant la revendication 23, dans lequel le domaine spécifique de SA se lie à la SA dans une analyse de liaison de ligand classique avec une CI₅₀ de 1 nM à 500 µM.

25. Ligand suivant l'une quelconque des revendications 1 à 17, comprenant un domaine variable unique spécifique du TNFα, et qui comprend la séquence d'aminoacides de TAR1-5-19 identifiée par sa séquence aux pages 2 et 3 de la liste des séquences, ou une séquence qui est homologue à au moins 80 % avec celle-ci.

26. Ligand suivant l'une quelconque des revendications 1 à 17, comprenant un domaine variable unique spécifique du TNFα, et qui comprend la séquence d'aminoacides de TAR1-5 identifiée par sa séquence aux pages 2 et 3 de la liste des séquences, ou une séquence qui est homologue à au moins 80 % avec celle-ci.

27. Ligand suivant l'une quelconque des revendications 1 à 17, comprenant un domaine variable unique spécifique du TNFα, et qui comprend la séquence d'aminoacides de TAR1-27 identifiée par sa séquence à la page 4 de la liste des séquences, ou une séquence qui est homologue à au moins 80 % avec celle-ci.

28. Ligand suivant l'une quelconque des revendications 1 à 17, comprenant un domaine variable unique spécifique du récepteur 1 du TNF, et qui comprend la séquence d'aminoacides de TAR2-10 identifiée par sa séquence à la page 2 de la liste des séquences, ou une séquence qui est homologue à au moins 80 % avec celle-ci.

29. Ligand suivant l'une quelconque des revendications 1 à 17, comprenant un domaine variable unique spécifique du récepteur 1 du TNF, et qui comprend la séquence d'aminoacides de TAR2-10 identifiée par sa séquence à la page 2 de la liste des séquences, ou une séquence qui est homologue à au moins 90 % avec celle-ci.

30. Ligand suivant l'une quelconque des revendications 1 à 17, comprenant un domaine variable unique spécifique du récepteur 1 du TNF, et qui comprend la séquence d'aminoacides de TAR2-5 identifiée par sa séquence à la page 1 de la liste des séquences, ou une séquence qui est homologue à au moins 80 % avec celle-ci.

31. Ligand suivant l'une quelconque des revendications 1 à 17, comprenant un domaine variable unique spécifique du récepteur 1 du TNF, et qui comprend la séquence d'aminoacides de TAR2-5 identifiée par sa séquence à la page 1 de la liste des séquences, ou une séquence qui est homologue à au moins 90 % avec celle-ci.

32. Ligand suivant l'une quelconque des revendications 1 à 17, comprenant un domaine variable unique spécifique de la SA, et qui comprend la séquence d'aminoacides de MSA-16 identifiée par sa séquence sur la figure 16, ou une séquence qui est homologue à au moins 80 % avec celle-ci.

33. Ligand suivant l'une quelconque des revendications 1 à 17, comprenant un domaine variable unique spécifique de la SA, et qui comprend la séquence d'aminoacides de MSA-26 identifiée par sa séquence sur la figure 16, ou une séquence qui est homologue à au moins 80 % avec celle-ci.

34. Ligand suivant l'une quelconque des revendications 25 à 31, comprenant un domaine variable unique spécifique de la SA tel que défini dans la revendication 23 ou 24.

35. Ligand suivant l'une quelconque des revendications 18 à 34, dans lequel le TNFα, le récepteur 1 du TNF ou la SA est sous forme humaine.

36. Ligand à double spécificité comprenant un dAb anti-TNFα humain ou un dAb anti-SA.

37. Ligand suivant la revendication 36, dans lequel les dAb sont des domaines VHH de camélidés.

38. Ligand suivant la revendication 37, dans lequel le dAb anti-TNFα est un domaine spécifique du TNFα tel que défini dans l'une quelconque des revendications 18, 19, 21 et 25 à 27.

39. Ligand suivant la revendication 37, dans lequel le dAb anti-SA est un domaine spécifique de la SA tel que défini dans l'une quelconque des revendications 23, 24, 32 et 33.

40. Ligand suivant l'une quelconque des revendications précédentes, qui comprend une séquence d'ossature universelle.

41. Ligand suivant l'une quelconque des revendications précédentes, comprenant une séquence d'ossature V_{H} choisie dans le groupe consistant en DP47, DP45 et DP38 ; et/ou une séquence d'ossature V_{L} qui est DPK9.

42. Ligand suivant l'une quelconque des revendications précédentes, qui comprend un site de liaison pour un ligand général.

43. Ligand suivant la revendication 42, dans lequel le site de liaison pour un ligand général est choisi dans le groupe consistant en la protéine A, la protéine L et la protéine G.

44. Ligand suivant l'une quelconque des revendications précédentes, le ligand comprenant un domaine variable ayant une ou plusieurs régions de séquence d'ossature comprenant une séquence d'aminoacides qui est identique à la séquence d'aminoacides d'une région de séquence d'ossature correspondante codée par un segment de gène d'anticorps de lignée germinale humain, ou bien les séquences d'aminoacides d'une ou plusieurs desdites régions de séquence d'ossature comprennent collectivement jusqu'à 5 différences d'aminoacides par rapport à la séquence d'aminoacides de ladite région de séquence d'ossature correspondante codée par un segment de gène d'anticorps de lignée germinale humain.

45. Ligand suivant l'une quelconque des revendications 1 à 43, le ligand comprenant un domaine variable, dans lequel les séquences d'aminoacides de FW1, FW2, FW3 et FW4 sont identiques aux séquences d'aminoacides des régions de séquence d'ossature correspondantes codées par un segment de gène d'anticorps de lignée germinale humain, ou bien les séquences d'aminoacides de FW1, FW2, FW3 et FW4 contiennent collectivement jusqu'à 10 différences d'aminoacides par rapport aux séquences d'aminoacides des régions de séquence d'ossature correspondantes codées par ledit segment de gène d'anticorps de lignée germinale humain.

46. Ligand suivant la revendication 44 ou la revendication 45, qui comprend un domaine variable d'anticorps comprenant les régions FW1, FW2 et FW3, et les séquences d'aminoacides desdites régions FW1, FW2 et FW3 sont identiques aux séquences d'aminoacides des régions de séquence d'ossature correspondantes codées par des segments de gène d'anticorps de lignée germinale humain.

47. Ligand suivant l'une quelconque des revendications 44 à 46, dans lequel ledit segment de gène d'anticorps de lignée germinale humain est choisi dans le groupe consistant en DP47, DP45, DP48 et DPK9.

48. Ligand suivant la revendication 1 ou 7, dans lequel le ou chaque domaine variable de chaîne lourde n'est pas un domaine variable d'immunoglobuline de camélidés.

49. Ligand suivant la revendication 1 ou 7, comprenant au moins un domaine variable de chaîne lourde qui ne contient pas un ou plusieurs aminoacides qui sont spécifiques de domaines variables d'immunoglobulines de camélidés par comparaison avec des domaines V_{H} humains.

50. Procédé pour la production d'un ligand suivant l'une quelconque des revendications précédentes, comprenant un premier domaine variable unique d'immunoglobuline ayant une première spécificité de liaison et un domaine variable unique d'immunoglobuline unique ayant une seconde spécificité de liaison, l'une des ou les deux spécificités de liaison étant spécifiques pour une protéine qui augmente la demi-vie du ligand *in vivo,* procédé comprenant les étapes consistant à :
(a) sélectionner un premier domaine variable par son aptitude à se lier à un premier épitope,
(b) sélectionner un second domaine variable par son aptitude à se lier à un second épitope,
(c) combiner les régions variables ; et
(d) sélectionner le ligand par son aptitude à se lier auxdits premier et second épitopes ; dans lequel lesdits domaines variables sont un domaine variable de chaîne lourde et un domaine variable de chaîne légère, le domaine variable de chaîne lourde n'étant pas un domaine V_{H} spécifique de la SAH.

51. Procédé suivant la revendication 50, dans lequel ledit premier domaine variable est sélectionné pour la liaison audit premier épitope en l'absence d'un domaine variable complémentaire.

52. Procédé suivant la revendication 51, dans lequel ledit premier domaine variable est sélectionné pour la liaison audit premier épitope en présence d'un troisième domaine variable complémentaire, dans lequel ledit troisième domaine variable est différent dudit deuxième domaine variable.

53. Acide nucléique codant pour au moins un ligand à double spécificité suivant l'une quelconque des revendications 1 à 49.

54. Acide nucléique suivant la revendication 53, comprenant la séquence d'acide nucléique de TAR1-5-19, identifiée par sa séquence aux pages 2 et 3 de la liste des séquences, ou une séquence qui est homologue à au moins 70 % avec celle-ci.

55. Acide nucléique suivant la revendication 53, comprenant la séquence d'acide nucléique de TAR1-5, identifiée par sa séquence à la page 3 de la liste des séquences, ou une séquence qui est homologue à au moins 70 % avec celle-ci.

56. Acide nucléique suivant la revendication 53, comprenant la séquence d'acide nucléique de TAR1-27, identifiée par sa séquence à la page 4 de la liste des séquences, ou une séquence qui est homologue à au moins 70 % avec celle-ci.

57. Acide nucléique suivant la revendication 53, comprenant la séquence d'acide nucléique de TAR2-10, identifiée par sa séquence à la page 2 de la liste des séquences, ou une séquence qui est homologue à au moins 70 % avec celle-ci.

58. Acide nucléique suivant la revendication 53, comprenant la séquence d'acide nucléique de TAR2-10, identifiée par sa séquence à la page 2 de la liste des séquences, ou une séquence qui est homologue à au moins 80 % avec celle-ci.

59. Acide nucléique suivant la revendication 53, comprenant la séquence d'acide nucléique de TAR2-5, identifiée par sa séquence à la page 1 de la liste des séquences, ou une séquence qui est homologue à au moins 70 % avec celle-ci.

60. Acide nucléique suivant la revendication 53, comprenant la séquence d'acide nucléique de TAR2-5, identifiée par sa séquence à la page 1 de la liste des séquences, ou une séquence qui est homologue à au moins 80 % avec celle-ci.

61. Acide nucléique suivant la revendication 53, comprenant la séquence d'acide nucléique de MSA-16, identifiée par sa séquence sur la figure 16, ou une séquence qui est homologue à au moins 70 % avec celle-ci.

62. Acide nucléique suivant la revendication 53, comprenant la séquence d'acide nucléique de MSA-26, identifiée par sa séquence sur la figure 16, ou une séquence qui est homologue à au moins 70 % avec celle-ci.

63. Vecteur comprenant l'acide nucléique suivant l'une quelconque des revendications 53 à 62.

64. Vecteur suivant la revendication 63, comprenant en outre les constituants nécessaires pour l'expression d'un ligand à double spécificité.

65. Cellule hôte comprenant un vecteur suivant la revendication 64.

66. Ligand suivant l'une quelconque des revendications 1 à 49, destiné à être utilisé en thérapeutique.

67. Composition pharmaceutique comprenant un ligand suivant l'une quelconque des revendications 1 à 49, et un excipient, support ou diluant pharmaceutiquement acceptable.

68. Ligand à double spécificité comprenant un premier domaine variable unique d'immunoglobuline ayant une spécificité de liaison pour la sérumalbumine (SA), et un second domaine variable unique d'immunoglobuline ayant une spécificité de liaison pour un antigène choisi dans le groupe consistant en le récepteur d'EPO, l'ApoE, l'Apo-SAA, le BDNF, la cardiotrophine-1, l'EGF, le récepteur d'EGF, l'ENA-78, l'Eotaxine, l'Eotaxine-2, l'Exodus-2, l'EpoR, le FGF acide, le FGF basique, le facteur de croissance des fibroblastes 10, le ligand FLT3, la fractalkine (CX3C), le GDNF, le G-CSF, le GM-CSF, le GF-β1, l'insuline, l'IFN-γ, l'IGF-I, l'IGF-II, l'IL-1α, l'IL-1β, l'IL-2, l'IL-3, l'IL-4, l'IL-5, l'IL-6, l'IL-7, l'IL-8 (72 a.a.), l'IL-8 (77 a.a.), l'IL-9, l'IL-10, l'IL-11, l'IL-12, l'IL-13, l'IL-15, l'IL-16, l'IL-17, l'IL-18 (IGIF), l'inhibine α, l'inhibine β, l'IP-10, le facteur de croissance des kératinocytes 2 (KGF-2), le KGF, la leptine, le LIF, la lymphotactine, la substance inhibitrice mullérienne, le facteur inhibiteur des colonies de monocytes, la protéine d'attraction des monocytes, le M-CSF, le MDC (67 a.a.), le MDC (69 a.a.), le MCP-1 (MCAF), le MCP-2, le MCP-3, le MCP-4, le MDC (67 a.a.), le MDC (69 a.a.), le MIG, le MIP-1α, le MIP-1β, le MIP-3α, le MIP-3β, le MIP-4, le facteur inhibiteur des cellules souches myéloïdes 1 (MPIF-1), le NAP-2, la neurturine, le facteur de croissance neurale, le β-NGF, le NT-3, le NT-4, l'oncostatine M, le PDGF-AA, le PDGF-AB, le PDGF-BB, le PF-4, le RANTES, le SDF1α, le SDF1β, le SCF, le SCGF, le facteur des cellules souches (SCF), le TARC, le TGFα, le TGFβ, le TGFβ2, le TGFβ3, le facteur de nécrose de tumeur (TNF), le TNFα, le TNFβ, le récepteur 1 de TNF, le récepteur 2 de TNF, le TNIL-1, le TPO, le VEGF, le récepteur 1 du VEGF, le récepteur 2 du VEGF, le récepteur 3 du VEGF, le GCP-2, le GRO/MGSA, le GRO-β, le GRO-γ, le HCC1, le 1-309, le HER 1, le HER 2, le HER 3 et le HER 4.

69. Ligand à double spécificité comprenant un premier domaine variable unique d'immunoglobuline ayant une spécificité de liaison pour la sérumalbumine (SA), et un second domaine variable unique d'immunoglobuline ayant une spécificité de liaison pour un antigène choisi dans le groupe consistant en des protéines humaines ou animales, des cytokines, des récepteurs de cytokines, des enzymes, des cofacteurs d'enzymes et des protéines se liant à l'ADN.

70. Ligand à double spécificité comprenant un premier domaine variable unique d'immunoglobuline ayant une spécificité de liaison pour la sérumalbumine (SA), et un second domaine variable unique d'immunoglobuline ayant une spécificité de liaison pour un récepteur d'une cytokine indiquée dans la revendication 68.

71. Ligand suivant la revendication 68, 69 ou 70, dans lequel chacun des premier et second domaines est :
(i) un domaine variable de chaîne lourde ou
(ii) un domaine variable de chaîne légère.

72. Ligand suivant l'une des revendications 68 à 71, dans lequel le domaine anti-SA est tel que défini dans l'une quelconque des revendications 23, 24, 32 et 33.

73. Acide nucléique comprenant lesdites séquences d'acide nucléique telles que définies dans l'une quelconque des revendications 53 à 62.

74. Vecteur comprenant un acide nucléique suivant la revendication 73.

75. Cellule hôte comprenant un vecteur suivant la revendication 74.

76. Ligand suivant l'une quelconque des revendications 68 à 72, destiné à être utilisé en thérapeutique.

77. Composition pharmaceutique comprenant un ligand suivant l'une quelconque des revendications 68 à 72 et un excipient, support ou diluant pharmaceutiquement acceptable.
